# EUROPEAN PATENT APPLICATION

(11) **EP 1 433 792 A2**
(43) Date of publication of application: **30.06.2004**
(21) Application number: 04005422.3
(22) Date of filing: 23.05.2001
(51) Int. Cl.: C07K 14/705, C07K 16/28, C12N 15/12, C12N 15/62, C12N 5/10, C12N 1/21, A61K 38/17, A61K 39/395

(54) **Human receptor proteins, related reagents and methods**

(30) Priority: 25.05.2000 US 207558 P
(62) Divisional of application: 01939361.0
(71) Applicant: SCHERING CORPORATION, Kenilworth, New Jersey 07033-0530 (US)
(72) Inventor: Hardiman, Gerard T., San Diego, CA 92128 (US); Rock, Fernando L., La Honda, CA 94020 (US); Bazan, J. Fernando, Palo Alto, CA 94301 (US); Ho, Stephen W.K., Sunnywale, CA 94087 (US); Liu, Yong-Jun, Palo Alto, CA 94306 (US)
(74) Representative: Taylor, Kathryn May

(57) **Abstract**

Nucleic acids encoding mammalian, e.g., human receptors, purified receptor proteins and fragments thereof. Antibodies, both polyclonal and monoclonal, are also provided. Methods of using the compositions for both diagnostic and therapeutic utilities are provided.

## Description

### FIELD OF THE INVENTION

The present invention relates to compositions and methods for affecting mammalian physiology, including morphogenesis or immune system function. In particular, it provides nucleic acids, proteins, and antibodies which regulate development and/or the immune system. Diagnostic and therapeutic uses of these materials are also disclosed.

### BACKGROUND OF THE INVENTION

Recombinant DNA technology refers generally to techniques of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host.

For some time, it has been known that the mammalian immune response is based on a series of complex cellular interactions, called the "immune network". Recent research has provided new insights into the inner workings of this network. While it remains clear that much of the immune response does, in fact, revolve around the network-like interactions of lymphocytes, macrophages, granulocytes, and other cells, immunologists now generally hold the opinion that soluble proteins, known as lymphokines, cytokines, or monokines, play critical roles in controlling these cellular interactions. Thus, there is considerable interest in the isolation, characterization, and mechanisms of action of cell modulatory factors, an understanding of which will lead to significant advancements in the diagnosis and therapy of numerous medical abnormalities, e.g., immune system disorders.

Lymphokines apparently mediate cellular activities in a variety of ways. They have been shown to support the proliferation, growth, and/or differentiation of pluripotential hematopoietic stem cells into vast numbers of progenitors comprising diverse cellular lineages which make up a complex immune system. Proper and balanced interactions between the cellular components are necessary for a healthy immune response. The different cellular lineages often respond in a different manner when lymphokines are administered in conjunction with other agents.

Cell lineages especially important to the immune response include two classes of lymphocytes: B-cells, which can produce and secrete immunoglobulins (proteins with the capability of recognizing and binding to foreign matter to effect its removal), and T-cells of various subsets that secrete lymphokines and induce or suppress the B-cells and various other cells (including other T-cells) making up the immune network. These lymphocytes interact with many other cell types.

Another important cell lineage is the mast cell (which has not been positively identified in all mammalian species), which is a granule-containing connective tissue cell located proximal to capillaries throughout the body. These cells are found in especially high concentrations in the lungs, skin, and gastrointestinal and genitourinary tracts. Mast cells play a central role in allergy-related disorders, particularly anaphylaxis as follows: when selected antigens crosslink one class of immunoglobulins bound to receptors on the mast cell surface, the mast cell degranulates and releases mediators, e.g., histamine, serotonin, heparin, and prostaglandins, which cause allergic reactions, e.g., anaphylaxis.

Research to better understand and treat various immune disorders has been hampered by the general inability to maintain cells of the immune system in vitro. Immunologists have discovered that culturing many of these cells can be accomplished through the use of T-cell and other cell supernatants, which contain various growth factors, including many of the lymphokines.

The interleukin-1 family of proteins includes the IL-1α, the IL-1β, the IL-1RA, and recently the IL-lγ (also designated Interferon-Gamma Inducing Factor, IGIF). This related family of genes have been implicated in a broad range of biological functions. See Dinarello (1994) FASEB J. 8:1314-1325; Dinarello (1991) Blood 77:1627-1652; and Okamura, et al. (1995) Nature 378:88-91.

In addition, various growth and regulatory factors exist which modulate morphogenetic development. This includes, e.g., the Toll ligands, which signal through binding to receptors which share structural, and mechanistic, features characteristic of the IL-1 receptors. See, e.g., Lemaitre, et al. (1996) Cell 86:973-983; and Belvin and Anderson (1996) Ann. Rev. Cell & Devel. Biol. 12: 393-416.

From the foregoing, it is evident that the discovery and development of new soluble proteins and their receptors, including ones similar to lymphokines, should contribute to new therapies for a wide range of degenerative or abnormal conditions which directly or indirectly involve development, differentiation, or function, e.g., of the immune system and/or hematopoietic cells. In particular, the discovery and understanding of novel receptors for lymphokine-like molecules which enhance or potentiate the beneficial activities of other lymphokines would be highly advantageous. The present invention provides new receptors for ligands exhibiting similarity to interleukin-1 like compositions and related compounds, and methods for their use.

### BRIEF DESCRIPTION OF THE DRAWINGS

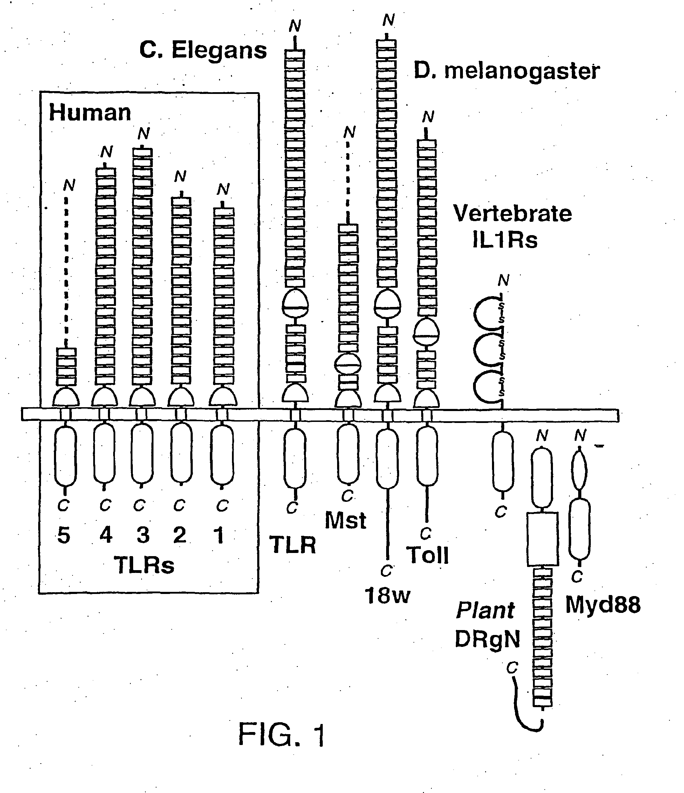

Figure 1 shows a schematic comparison of the protein architectures of Drosophila, Caenorabditis, and human DTLRs, and their relationship to vertebrate IL-1 receptors and plant disease resistance proteins. Three Drosophila (Dm) DTLRs (Toll, 18w, and the Mst ORF fragment) (Morisato and Anderson (1995) Ann. Rev. Genet. 29:371-399; Chiang and Beachy (1994) Mech. Develop. 47:225-239; Mitcham, et al. (1996) J. Biol. Chem. 271:5777-5783; and Eldon, et al. (1994) Develop. 120:885-899) are arrayed beside four complete (DTLRs 1-4) and one partial (DTLR5) human (Hu) receptors. Individual LRRs in the receptor ectodomains that are flagged by PRINTS (Attwood, et al. (1997) Nucleic Acids Res. 25:212-217) are explicitly noted by boxes; 'top' and 'bottom' Cys-rich clusters that flank the C- or N-terminal ends of LRR arrays are respectively drawn by opposed half-circles. The loss of the internal Cys-rich region in DTLRs 1-5 largely accounts for their smaller ectodomains (558, 570, 690, and 652 aa, respectively) when compared to the 784 and 977 aa extensions of Toll and 18w. The incomplete chains of DmMst and HuDTLR5 (about 519 and 153 aa ectodomains, respectively) are represented by dashed lines. The intracellular signaling module common to DTLRs, IL-1-type receptors (IL-1Rs), the intracellular protein Myd88, and the tobacco disease resistance gene N product (DRgN) is indicated below the membrane. See, e.g., Hardiman, et al. (1996) Oncogene 13:2467-2475; and Rock, et al. (1998) Proc. Nat'l Acad. Sci. USA 95:588-Additional domains include the trio of Ig-like modules in IL-1Rs (disulfide-linked loops); the DRgN protein features an NTPase domain (box) and Myd88 has a death domain (black oval).

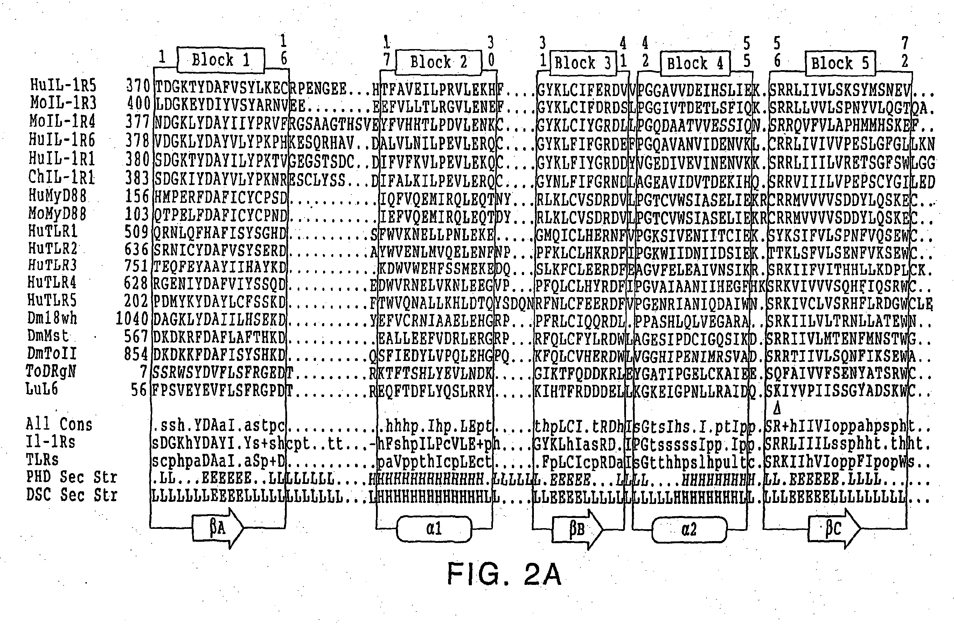

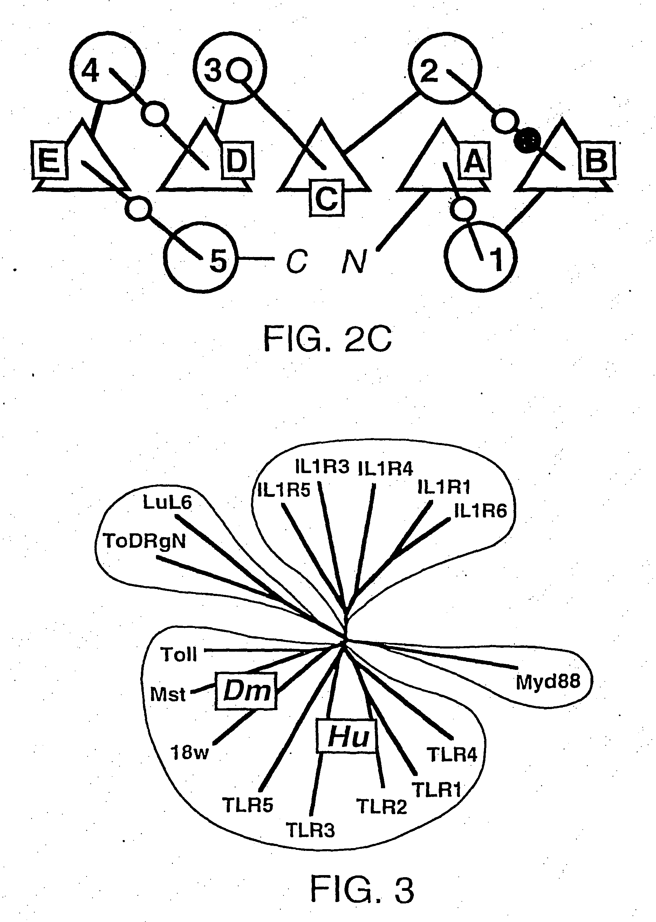

Figures 2A-2C show conserved structural patterns in the signaling domains of Toll- and IL-1-like cytokine receptors, and two divergent modular proteins. Figures 2A-2B show a sequence alignment of the common TH domain. DTLRs are labeled as in Figure 1; the human (Hu) or mouse (Mo) IL-1 family receptors (IL-1R1-6) are sequentially numbered as earlier proposed (Hardiman, et al. (1996) Oncogene 13:2467-2475); Myd88 and the sequences from tobacco (To) and flax, L. usitatissimum (Lu), represent C-and N-terminal domains, respectively, of larger, multidomain molecules. Ungapped blocks of sequence (numbered 1-10) are boxed. Triangles indicate deleterious mutations, while truncations N-terminal of the arrow eliminate bioactivity in human IL-1R1 (Heguy, et al. (1992) J. Biol. Chem. 267:2605-2609). PHD (Rost and Sander (1994) Proteins 19:55-72) and DSC (King and Sternberg (1996) Protein Sci. 5:2298-2310) secondary structure predictions of α-helix (H), β-strand (E), or coil (L) are marked. The amino acid shading scheme depicts chemically similar residues: hydrophobic, acidic, basic, Cys, aromatic, structure-breaking, and tiny. Diagnostic sequence patterns for IL-1Rs, DTLRs,- and full alignment (ALL) were derived by Consensus at a stringency of 75%. Symbols for amino acid subsets are (see internet site for detail): o, alcohol; l, aliphatic; •, any amino acid; a, aromatic; c, charged; h, hydrophobic; -, negative; p, polar; +, positive; s, small; u, tiny; t, turnlike. Figure 2C shows a topology diagram of the proposed TH β/α domain fold. The parallel β-sheet (with β-strands A-E as yellow triangles) is seen at its C-terminal end; α-helices (circles labeled 1-5) link the β-strands; chain connections are to the front (visible) or back (hidden). Conserved, charged residues at the C-end of the β-sheet are noted in gray (Asp) or as a lone black (Arg) residue (see text).
Figure 3 shows evolution of a signaling domain superfamily. The multiple TH module alignment of Figures 2A-2B was used to derive a phylogenetic tree by the Neighbor-Joining method (Thompson, et al. (1994) Nucleic Acids Res. 22:4673-4680). Proteins labeled as in the alignment; the tree was rendered with TreeView.

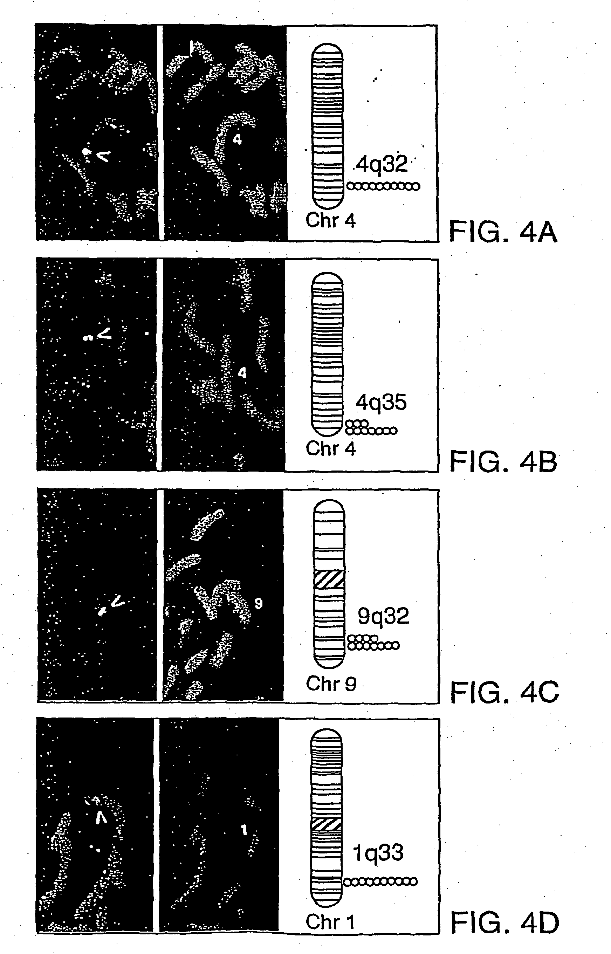

Figures 4A-4D depict FISH chromosomal mapping of human DTLR genes. Denatured chromosomes from synchronous cultures of human lymphocytes were hybridized to biotinylated DTLR cDNA probes for localization. The assignment of the FISH mapping data (left, Figures 4A, DTLR2; 4B, DTLR3; 4C, DTLR4; 4D, DTLR5) with chromosomal bands was achieved by superimposing FISH signals with DAPI banded chromosomes (center panels). Heng and Tsui (1994) Meth. Molec. Biol. 33:109-122. Analyses are summarized in the form of human chromosome ideograms (right panels).

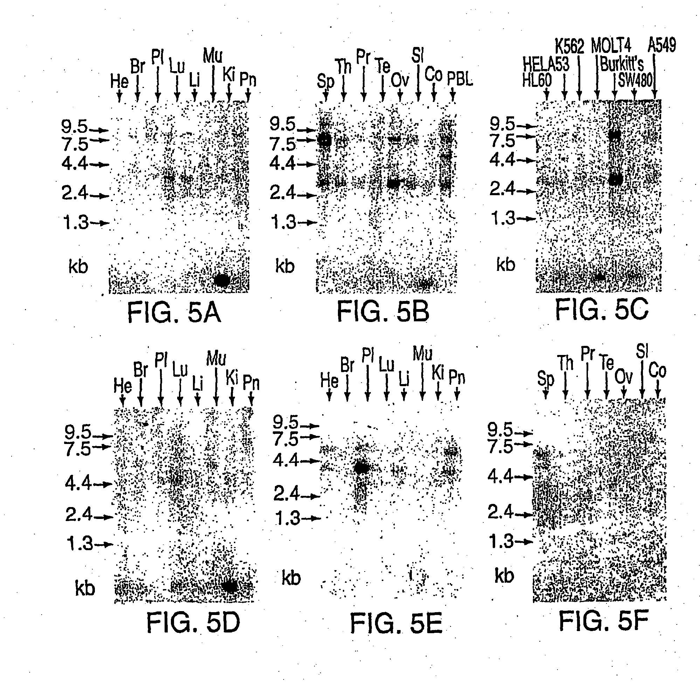

Figures 5A-5F depict mRNA blot analyses of Human DTLRs. Human multiple tissue blots (He, heart; Br, brain; P1, placenta; Lu, lung; Li, liver; Mu, muscle; Ki, kidney; Pn, Pancreas; Sp, spleen; Th, thymus; Pr, prostate; Te, testis; Ov, ovary, SI, small intestine; Co, colon; PBL, peripheral blood lymphocytes) and cancer cell line (promyelocytic leukemia, HL60; cervical cancer, HELAS3; chronic myelogenous leukemia, K562; lymphoblastic leukemia, Molt4; colorectal adenocarcinoma, SW480; melanoma, G361; Burkitt's Lymphoma Raji, Burkitt's; colorectal adenocarcinoma, SW480; lung carcinoma, A549) containing approximately 2 µg of poly(A)⁺ RNA per lane were probed with radiolabeled cDNAs encoding DTLR1 (Figures 5A-5C), DTLR2 (Figure 5D), DTLR3 (Figure 5E), and DTLR4 (Figure 5F) as described. Blots were exposed to X-ray film for 2 days (Figures 5A-5C) or one week (Figure 5D-5F) at -70° C with intensifying screens. An anomalous 0.3 kB species appears in some lanes; hybridization experiments exclude a message encoding a DTLR cytoplasmic fragment.

### SUMMARY OF THE INVENTION

The present invention is directed to nine novel related mammalian receptors, e.g., primate, human, DNAX Toll receptor like molecular structures, designated DTLR2, DTLR3, DTLR4, DTLR5, DTLR7, DTLR8, DTLR9, and DTLR10, and their biological activities. It includes nucleic acids coding for the polypeptides themselves and methods for their production and use. The nucleic acids of the invention are characterized, in part, by their homology to cloned complementary DNA (cDNA) sequences enclosed herein.

In certain embodiments, the invention provides a composition of matter selected from the group of: a substantially pure or recombinant DTLR2 protein or peptide exhibiting identity over a length of at least about 12 amino acids to SEQ ID NO: 4; a natural sequence DTLR2 of SEQ ID NO: 4; a fusion protein comprising DTLR2 sequence; a substantially pure or recombinant DTLR3 protein or peptide exhibiting identity over a length of at least about 12 amino acids to SEQ ID NO: 6; a natural sequence DTLR3 of SEQ ID NO: 6; a fusion protein comprising DTLR3 sequence; a substantially pure or recombinant DTLR4 protein or peptide exhibiting identity over a length of at least about 12 amino acids to SEQ ID NO: 26; a natural sequence DTLR4 of SEQ ID NO: 26; a fusion protein comprising DTLR4 sequence; a substantially pure or recombinant DTLR5 protein or peptide exhibiting identity over a length of at least about 12 amino acids to SEQ ID NO: 10; a natural sequence DTLR5 of SEQ ID NO: 10; a fusion protein comprising DTLR5 sequence; a substantially pure or recombinant DTLR6 protein or peptide exhibiting identity over a length of at least about 12 amino acids to SEQ ID NO: 12, 28, or 30; a natural sequence DTLR6 of SEQ ID NO: 12, 28, or 30; a fusion protein comprising DTLR6 sequence; a substantially pure or recombinant DTLR7 protein or peptide exhibiting identity over a length of at least about 12 amino acids to SEQ ID NO: 16, 18, or 37; a natural sequence DTLR7 of SEQ ID NO: 16, 18, or 37; a fusion protein comprising DTLR7 sequence; a substantially pure or recombinant DTLR8 protein or peptide exhibiting identity over a length of at least about 12 amino acids to SEQ ID NO: 32 or 39; a natural sequence DTLR8 of SEQ ID NO: 32 or 39; a fusion protein comprising DTLR8 sequence; a substantially pure or recombinant DTLR9 protein or peptide exhibiting identity over a length of at least about 12 amino acids to SEQ ID NO: 22 or 41; a natural sequence DTLR9 of SEQ ID NO: 22 or 41; a fusion protein comprising DTLR9 sequence; a substantially pure or recombinant DTLR10 protein or peptide exhibiting identity over a length of at least about 12 amino acids to SEQ ID NO: 34, 43, or 45; a natural sequence DTLR10 of SEQ ID NO: 34, 43, or 45; and a fusion protein comprising DTLR10 sequence. Preferably, the substantially pure or isolated protein comprises a segment exhibiting sequence identity to a corresponding portion of a DTLR2, DTLR3, DTLR4, DTLR5, DTLR6, DTLR7, DTLR8, DTLR9, or DTLR10, wherein said identity is over at least about 15 amino acids; preferably about 19 amino acids; or more preferably about 25 amino acids. In specific embodiments, the composition of matter: is DTLR2, which comprises a mature sequence of Table 2; or lacks a post-translational modification; is DTLR3, which comprises a mature.sequence of Table 3; or lacks a post-translational modification; is DTLR4, which: comprises a mature sequence of Table 4; or lacks a post-translational modification; is DTLR5, which: comprises the complete sequence of Table 5; or lacks a post-translational; is DTLR6, which comprises a mature sequence of Table 6; or lacks a post-translational modification; is DTLR7, which comprises a mature sequence of Table 7; or lacks a post-translational modification; is DTLR8, which: comprises a mature sequence of Table 8; or lacks a post-translational modification; is DTLR9, which: comprises the complete sequence of Table 9; or lacks a post-translational; is DTLR10, which comprises a mature sequence of Table 10; or lacks a post-translational modification; or the composition of matter may be a protein or peptide which: is from a warm blooded animal selected from a mammal, including a primate, such as a human; comprises at least one polypeptide segment of SEQ ID NO: 4, 6, 26, 10, 12, 28, 30, 16, 18, 32, 22, or 34; exhibits a plurality of portions exhibiting said identity; is a natural allelic variant of DTLR2, DTLR3, DTLR4, DTLR5, DTLR6, DTLR7, DTLR8, DTLR9, or DTLR10; has a length. at least about 30 amino acids; exhibits at least two non-overlapping epitopes which are specific for a primate DTLR2, DTLR3, DTLR4, DTLR5, DTLR6, DTLR7, DTLR8, DTLR9, or DTLR10; exhibits sequence identity over a length of at least about 35 amino acids to a primate DTLR2, DTLR3, DTLR4, DTLR5, DTLR6, DTLR7, DTLR8, DTLR9. or DTLR10; further exhibits at least two non-overlapping epitopes which are specific for a primate DTLR2, DTLR3, DTLR4, DTLR5, DTLR6, DTLR7, DTLR8, DTLR9, or DTLR10; exhibits identity over a length of at least about 20 amino acids to a rodent DTLR6; is glycosylated; has a molecular weight of at least 100 kD with natural glycosylation; is a synthetic polypeptide; is attached to a solid substrate; is conjugated to another chemical moiety; is a 5-fold or less substitution from natural sequence; or is a deletion or insertion variant from a natural sequence.

Other embodiments include a composition comprising: a sterile DTLR2 protein or peptide; or the DTLR2 protein or peptide and a carrier, wherein the carrier is: an aqueous compound, including water, saline, and/or buffer; and/or formulated for oral, rectal, nasal, topical, or parenteral administration; a sterile DTLR3 protein or peptide; or the DTLR3 protein or peptide and a carrier, wherein the carrier is: an aqueous compound, including water, saline, and/or buffer; and/or formulated for oral, rectal, nasal, topical, or parenteral administration; a sterile DTLR4 protein or peptide; or the DTLR4 protein or peptide and a carrier, wherein the carrier is: an aqueous compound, including water, saline, and/or buffer; and/or formulated for oral, rectal, nasal, topical, or parenteral administration; a sterile DTLR5 protein or peptide; or the DTLR5 protein or peptide and a carrier, wherein the carrier is: an aqueous compound, including water, saline, and/or buffer; and/or formulated for oral, rectal, nasal, topical, or parenteral administration; a sterile DTLR6 protein or peptide; or the DTLR6 protein or peptide and a carrier, wherein the carrier is: an aqueous compound, including water, saline, and/or buffer; and/or formulated for oral, rectal, nasal, topical, or parenteral administration; a sterile DTLR7 protein or peptide; or the DTLR7 protein or peptide and a carrier, wherein the carrier is: an aqueous compound, including water, saline, and/or buffer; and/or formulated for oral, rectal, nasal, topical, or parenteral administration; a sterile DTLR8 protein or peptide; or the DTLR8 protein or peptide and a carrier, wherein the carrier is: an aqueous compound, including water, saline, and/or buffer; and/or formulated for oral, rectal, nasal, topical, or parenteral administration; a sterile DTLR9 protein or peptide; or the DTLR9 protein or peptide and a carrier, wherein the carrier is: an aqueous compound, including water, saline, and/or buffer; and/or formulated for oral, rectal, nasal, topical, or parenteral administration; a sterile DTLR10 protein or peptide; or the DTLR10 protein or peptide and a carrier, wherein the carrier is: an aqueous compound, including water, saline, and/or buffer; and/or formulated for oral, rectal, nasal, topical, or parenteral administration.

In certain fusion protein embodiments, the invention provides a fusion protein comprising: mature protein sequence of Table 2, 3, 4, 5, 6, 7, 8, 9, or 10; a detection or purification tag, including a FLAG, His6, or Ig sequence; or sequence of another receptor protein.

Various kit embodiments include a kit comprising a DTLR protein or polypeptide, and: a compartment comprising the protein or polypeptide; and/or instructions for use or disposal of reagents in the kit.

Binding compound embodiments include those comprising an antigen binding site from an antibody, which specifically binds to a natural DTLR2, DTLR3, DTLR4, DTLR5, DTLR6, DTLR7, DTLR8, DTLR9, or DTLR10 protein, wherein: the protein is a primate protein; the binding compound is an Fv, Fab, or Fab2 fragment; the binding compound is conjugated to another chemical moiety; or the antibody: is raised against a peptide sequence of a mature polypeptide of Table 2, 3, 4, 5, 6, 7, 8, 9, or 10; is raised against a mature DTLR2, DTLR3, DTLR4, DTLR5, DTLR6, DTLR7, DTLR8, DTLR9, or DTLR10; is raised to a purified human DTLR2, DTLR3, DTLR4, DTLR5, DTLR6, DTLR7, DTLR8, DTLR9, or DTLR10; is immunoselected; is a polyclonal antibody; binds to a denatured DTLR2, DTLR3, DTLR4, DTLR5, DTLR6, DTLR7, DTLR8, DTLR9, or DTLR10; exhibits a Kd to antigen of at least 30 µM; is attached to a solid substrate, including a bead or plastic membrane; is in a sterile composition; or is detectably labeled, including a radioactive or fluorescent label. A binding composition kit often comprises the binding compound, and: a compartment comprising said binding compound; and/or instructions for use or disposal of reagents in the kit. Often the kit is capable of making a qualitative or quantitative analysis.

Methods are provided, e.g., of making an antibody, comprising immunizing an immune system with an immunogenic amount of a primate DTLR2, DTLR3, DTLR4, DTLR5, DTLR6, DTLR7, DTLR8, DTLR9, or DTLR10, thereby causing said antibody to be produced; or producing an antigen:antibody complex, comprising contacting such an antibody with a mammalian DTLR2, DTLR3, DTLR4, DTLR5, DTLR6, DTLR7, DTLR8, DTLR9, or DTLR10 protein or peptide, thereby allowing said complex to form.

Other compositions include a composition comprising: a sterile binding compound, or the binding compound and a carrier, wherein the carrier is: an aqueous compound, including water, saline, and/or buffer; and/or formulated for oral, rectal, nasal, topical, or parenteral administration.

Nucleic acid embodiments include an isolated or recombinant nucleic acid encoding a DTLR2-10 protein or peptide or fusion protein, wherein: the DTLR is from a mammal; or the nucleic acid: encodes an antigenic peptide sequence of Table 2, 3, 4, 5, 6, 7, 8, 9, or 10; encodes a plurality of antigenic peptide sequences of Table 2, 3, 4, 5, 6, 7, 8, 9, or 10; comprises at least 17 contiguous nucleotides from Table 2, 3, 4, 5, 6, 7, 8, 9, or 10; exhibits at least about 80% identity to a natural cDNA encoding said segment; is an expression vector; further comprises an origin of replication; is from a natural source; comprises a detectable label; comprises synthetic nucleotide sequence; is less than 6 kb, preferably less than 3 kb; is from a mammal, including a primate; comprises a natural full length coding sequence; is a hybridization probe for a gene encoding said DTLR; or is a PCR primer, PCR product, or mutagenesis primer. A cell, tissue, or organ comprising such a recombinant nucleic acid is also provided. Preferably, the cell is: a prokaryotic cell; a eukaryotic cell; a bacterial cell; a yeast cell; an insect cell; a mammalian cell; a mouse cell; a primate cell; or a human cell. Kits are provided comprising such nucleic acids, and: a compartment comprising said nucleic acid; a compartment further comprising a primate DTLR2, DTLR3, DTLR4, or DTLR5 protein or polypeptide; and/or instructions for use or disposal of reagents in the kit. Often, the kit is capable of making a qualitative or quantitative analysis.

Other embodiments include a nucleic acid which: hybridizes under wash conditions of 30°C and less than 2M salt to SEQ ID NO: 3; hybridizes under wash conditions of 30° C and less than 2 M salt to SEQ ID NO: 5; hybridizes under wash conditions of 30°C and less than 2M salt to SEQ ID NO: 7; hybridizes under wash conditions of 30°C and less than 2 M salt to SEQ ID NO: 9; hybridizes under wash conditions of 30° C and less than 2 M salt to SEQ ID NO: 11, 13, 27, or 29; hybridizes under wash conditions of 30° C and less than 2 M salt to SEQ ID NO: 15, 17, or 36; hybridizes under wash conditions of 30° C and less than 2 M salt to SEQ ID NO: 19, 31, or 38; hybridizes under wash conditions of 30° C and less than 2 M salt to SEQ ID NO: 21 or 40; hybridizes under wash conditions of 30° C and less than 2 M salt to SEQ ID NO: 23, 33, 42, or 44; exhibits at least about 85% identity over a stretch of at least about 30 nucleotides to a primate DTLR2; exhibits at least about 85% identity over a stretch of at least about 30 nucleotides to a primate DTLR3; exhibits at least about 85% identity over a stretch of at least about 30 nucleotides to a primate DTLR4; or exhibits at least about 85% identity over a stretch of at least about 30 nucleotides to a primate DTLR5. Preferably, such nucleic acid will have such properties, wherein: wash conditions are at 45° C and/or 500 mM salt; or the identity is at least 90% and/or the stretch is at least 55 nucleotides.

More preferably, the wash conditions are at 55° C and/or 150 mM salt; or the identity is at least 95% and/or the stretch is at least 75 nucleotides.

Also provided are methods of producing a ligand:receptor complex, comprising contacting a substantially pure primate DTLR2, DTLR3, DTLR4, DTLR5, DTLR6, DTLR7, DTLR8, DTLR9, or DTLR10, including a recombinant or synthetically produced protein, with candidate Toll ligand; thereby allowing said complex to form:

The invention also provides a method of modulating physiology or development of a cell or tissue culture cells comprising contacting the cell with an agonist or antagonist of a mammalian DTLR2, DTLR3, DTLR4, DTLR5, DTLR6, DTLR7, DTLR8, DTLR9, or DTLR10. Preferably, the cell is a pDC2 cell with the agonist or antagonist of DTLR10.

### DETAILED DESCRIPTION OF THE PREFERRED EMBODIMENTS

### OUTLINE

I. General
II. Activities
III. Nucleic acids
   A. encoding fragments, sequence, probes
   B. mutations, chimeras, fusions
   C. making nucleic acids
   D. vectors, cells comprising
IV. Proteins, Peptides
   A. fragments, sequence, immunogens, antigens
   B. muteins
   C. agonists/antagonists, functional equivalents
   D. making proteins
V. Making nucleic acids, proteins
   A. synthetic
   B. recombinant
   C. natural sources
VI. Antibodies
   A. polyclonals
   B. monoclonal
   C. fragments; Kd
   D. anti-idiotypic antibodies
   E. hybridoma cell lines
VII. Kits and Methods to quantify DTLRs 2-10
   A. ELISA
   B. assay mRNA encoding
   C. qualitative/quantitative
   D. kits
VIII. Therapeutic compositions, methods
   A. combination compositions
   B. unit dose
   C. administration
IX. Ligands

### I. General

The present invention provides the amino acid sequence and DNA sequence of mammalian, herein primate DNAX Toll like receptor molecules (DTLR) having particular defined properties, both structural and biological. These have been designated herein as DTLR2, DTLR3, DTLR4, DTLR5, DTLR6, DTLR7, DTLR8, DTLR9, and DTLR10, respectively, and increase the number of members of the human Toll like receptor family from 1 to 10. Various cDNAs encoding these molecules were obtained from primate, e.g., human, cDNA sequence libraries. Other primate or other mammalian counterparts would also be desired.

Some of the standard methods applicable are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and periodic supplements) Current Protocols in Molecular Biology, Greene/Wiley, New York; each of which is incorporated herein by reference.

A complete nucleotide (SEQ ID NO: 1) and corresponding amino acid sequence (SEQ ID NO: 2) of a human DTLR1 coding segment is shown in Table 1. See also Nomura, et al. (1994) DNA Res. 1:27-35. A complete nucleotide (SEQ ID NO: 3) and corresponding amino acid sequence (SEQ ID NO: 4) of a human DTLR2 coding segment is shown in Table 2. A complete nucleotide (SEQ ID NO: 5) and corresponding amino acid sequence (SEQ ID NO: 6) of a human DTLR3 coding segment is shown in Table 3. A complete nucleotide (SEQ ID NO: 7) and corresponding amino acid sequence (SEQ ID NO: 8) of a human DTLR4 coding segment is shown in Table 4; see also SEQ ID NO: 25 and 26. A partial nucleotide (SEQ ID NO: 9) and corresponding amino acid sequence (SEQ ID NO: 10) of a human DTLR5 coding segment is shown in Table 5. A complete nucleotide (SEQ ID NO: 11) and corresponding amino acid sequence (SEQ ID NO: 12) of a human DTLR6 coding segment is shown in Table 6, along with partial sequence of a mouse DTLR6 (SEQ ID NO: 13, 14, 27, 28, 29, and 30). Partial nucleotide (SEQ ID NO: 15 and 17) and corresponding amino acid sequence (SEQ ID NO: 16 and 18) of a human DTLR7 coding segment is shown in Table 7; full length sequence is provided in SEQ ID NO: 36 and 37. Partial nucleotide (SEQ ID NO: 19) and corresponding amino acid sequence (SEQ ID NO: 20) of a human DTLR8 coding segment is shown in Table 8, with supplementary sequence (SEQ ID NO: 31, 32, 38, and 39) . Partial nucleotide (SEQ ID NO: 21) and corresponding amino acid sequence (SEQ ID NO: 22) of a human DTLR9 coding segment is shown in Table 9; see also SEQ ID NO: 40 and 41. Partial nucleotide (SEQ ID NO: 23) and corresponding amino acid sequence (SEQ ID NO: 24) of a human DTLR10 coding segment is shown in Table 10, along with supplementary sequence (SEQ ID NO: 33, 34, 42, and 43) and rodent, e.g., mouse, sequence (SEQ ID NO: 35, 44, and 45).

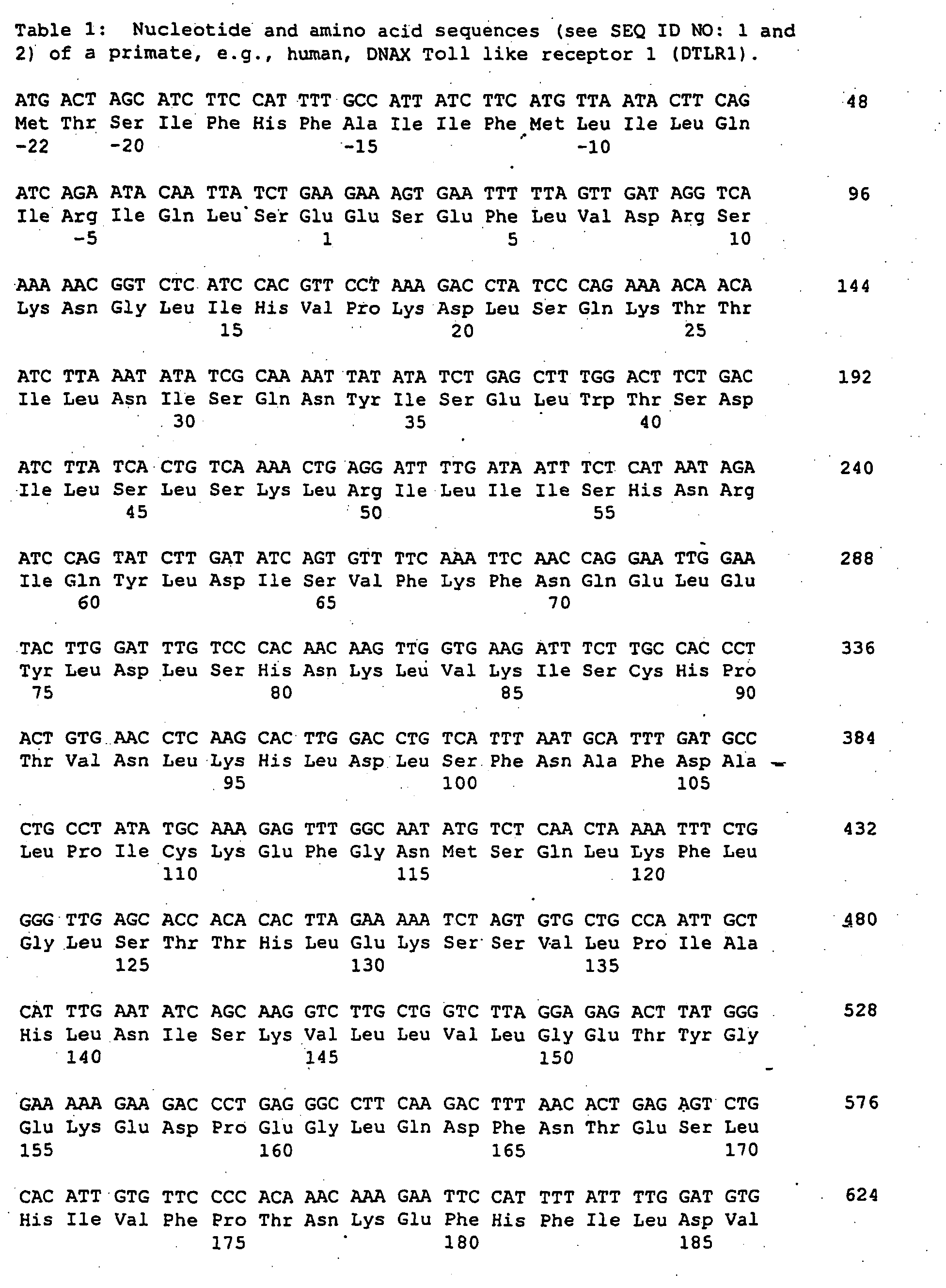

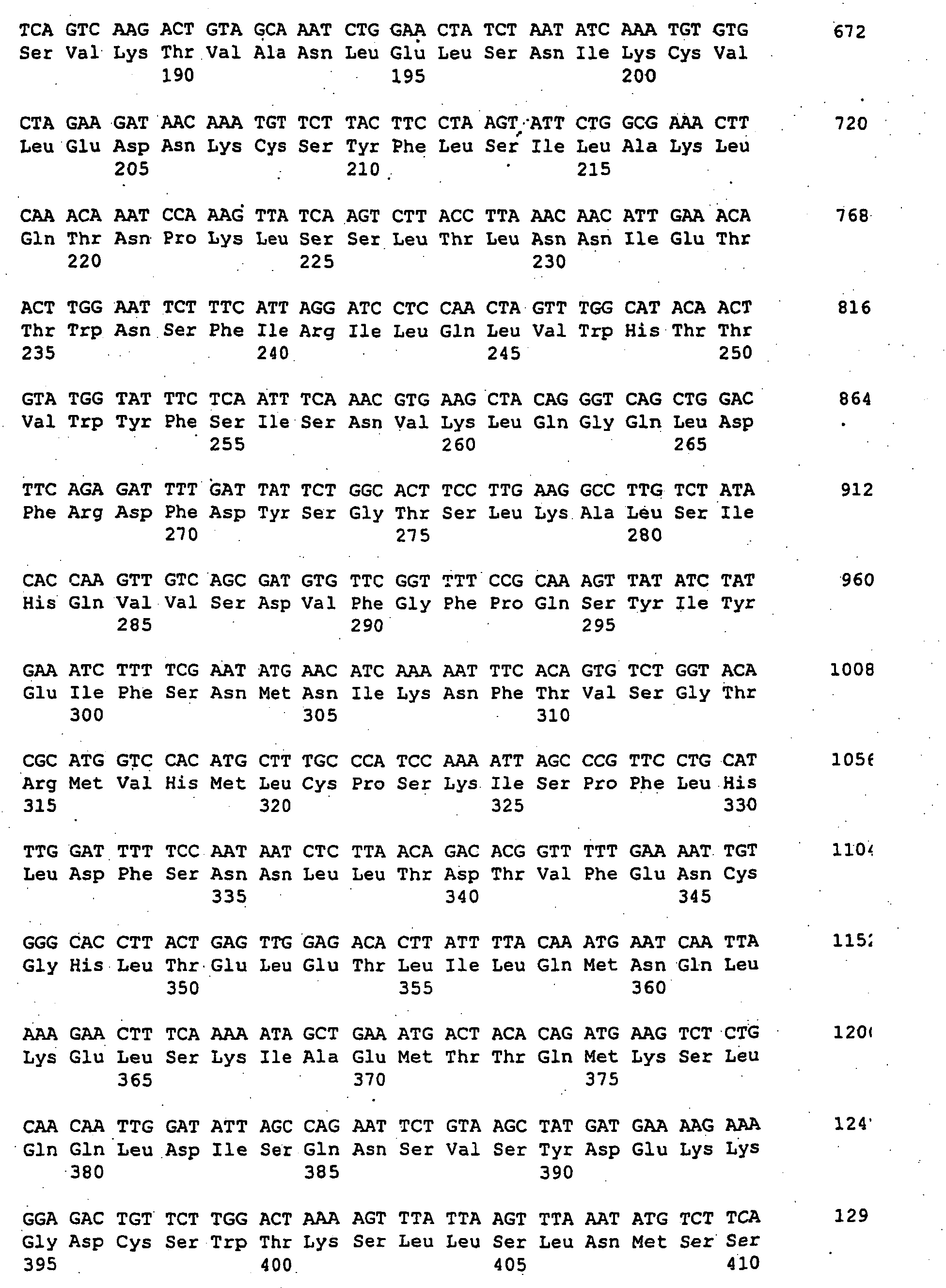

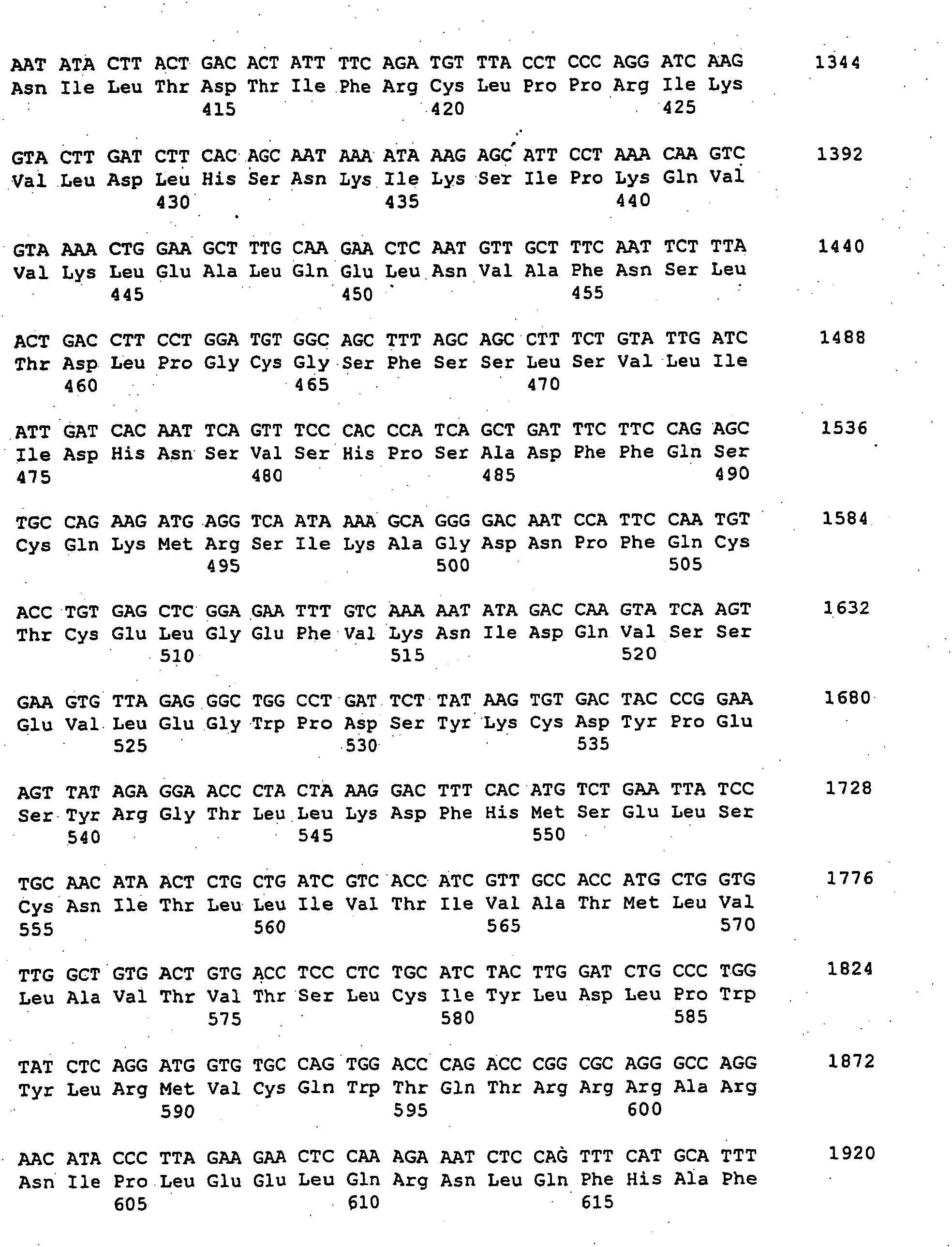

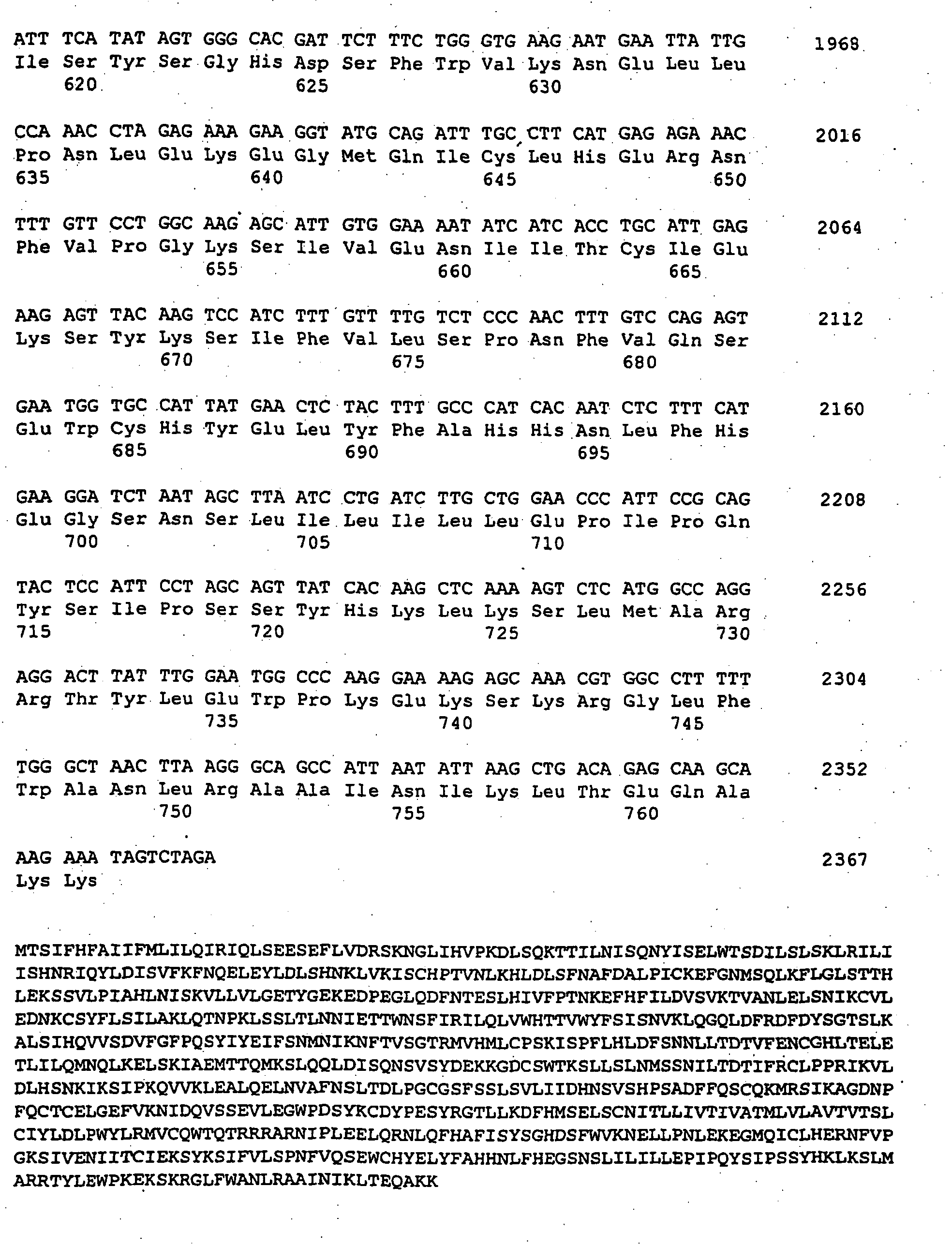

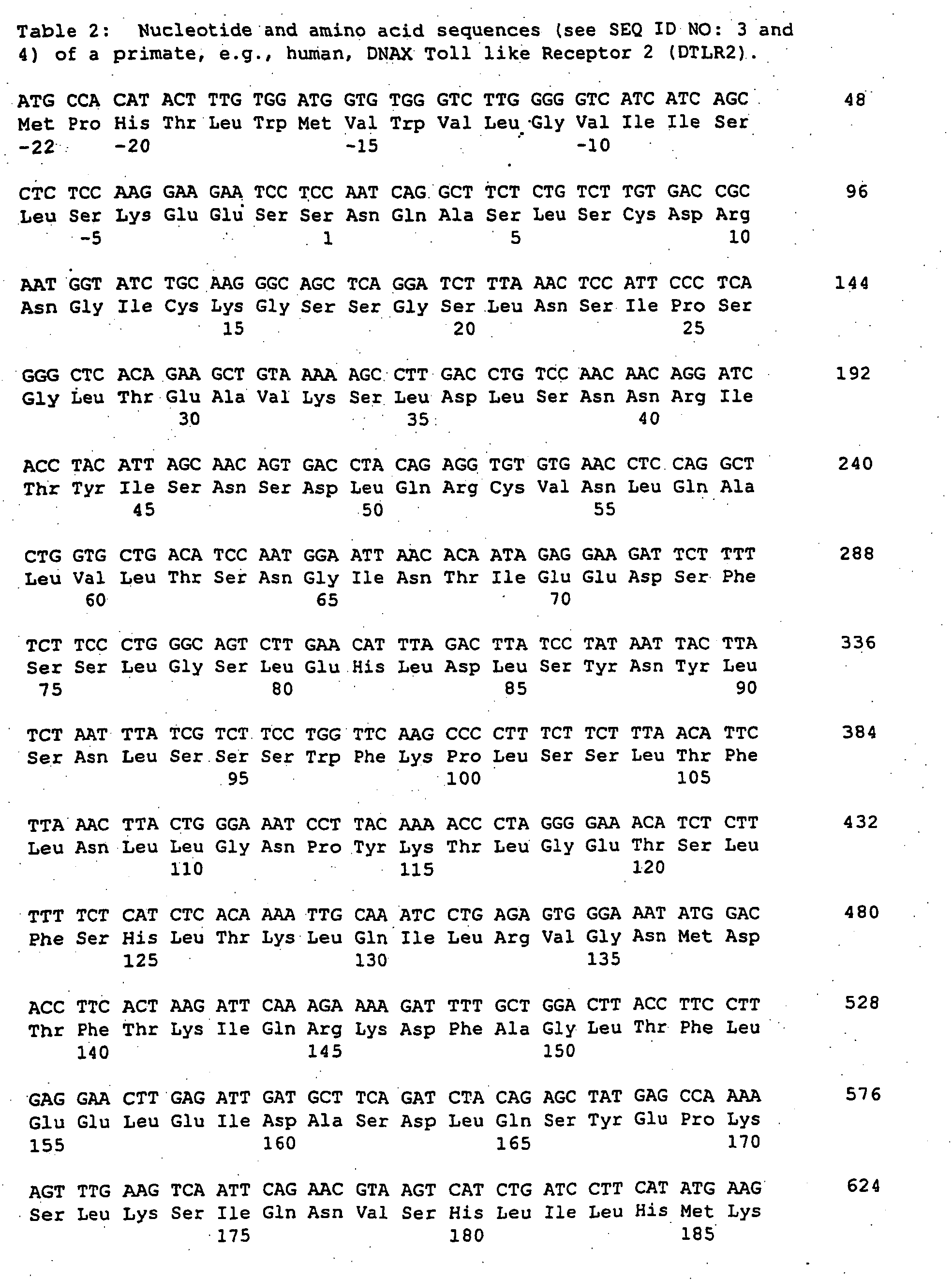

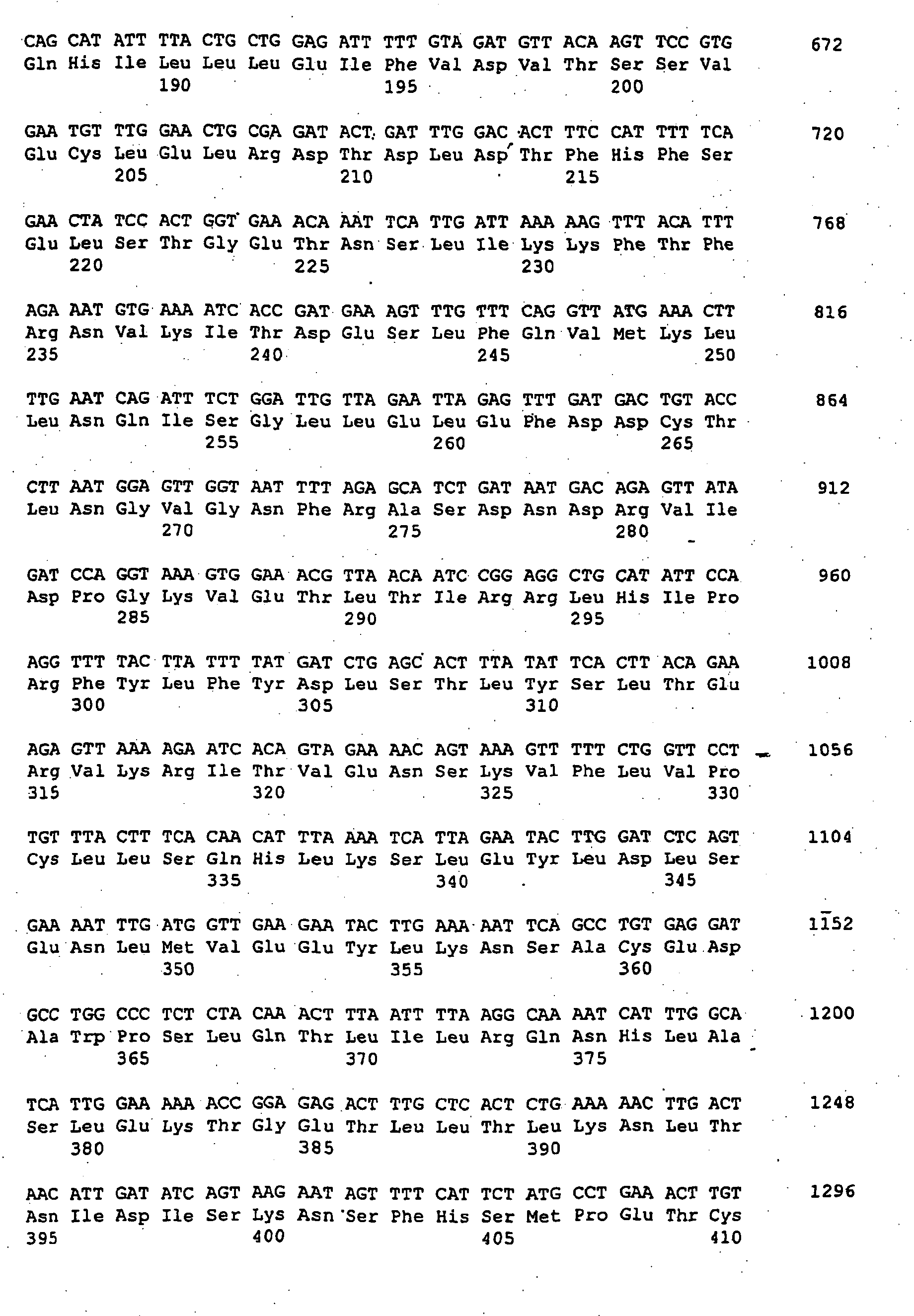

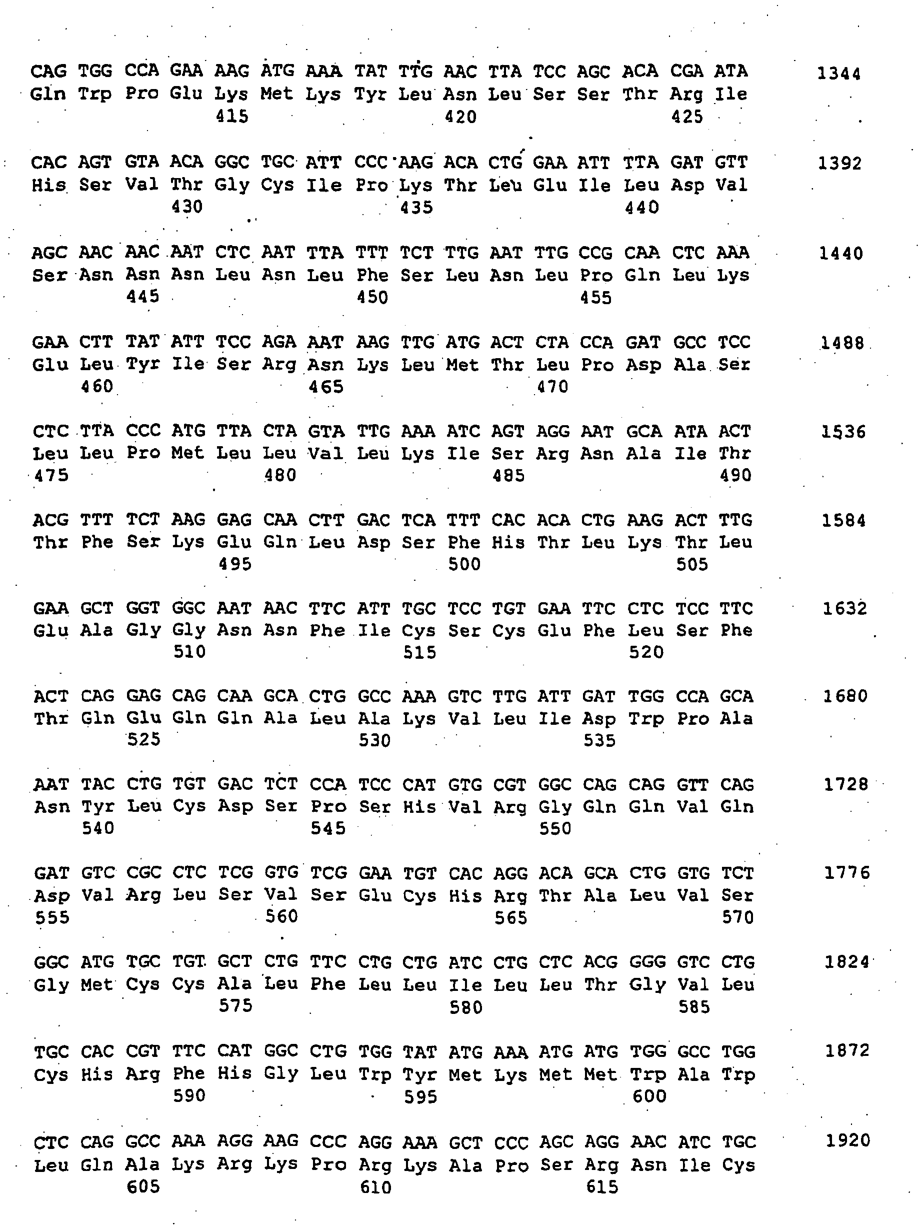

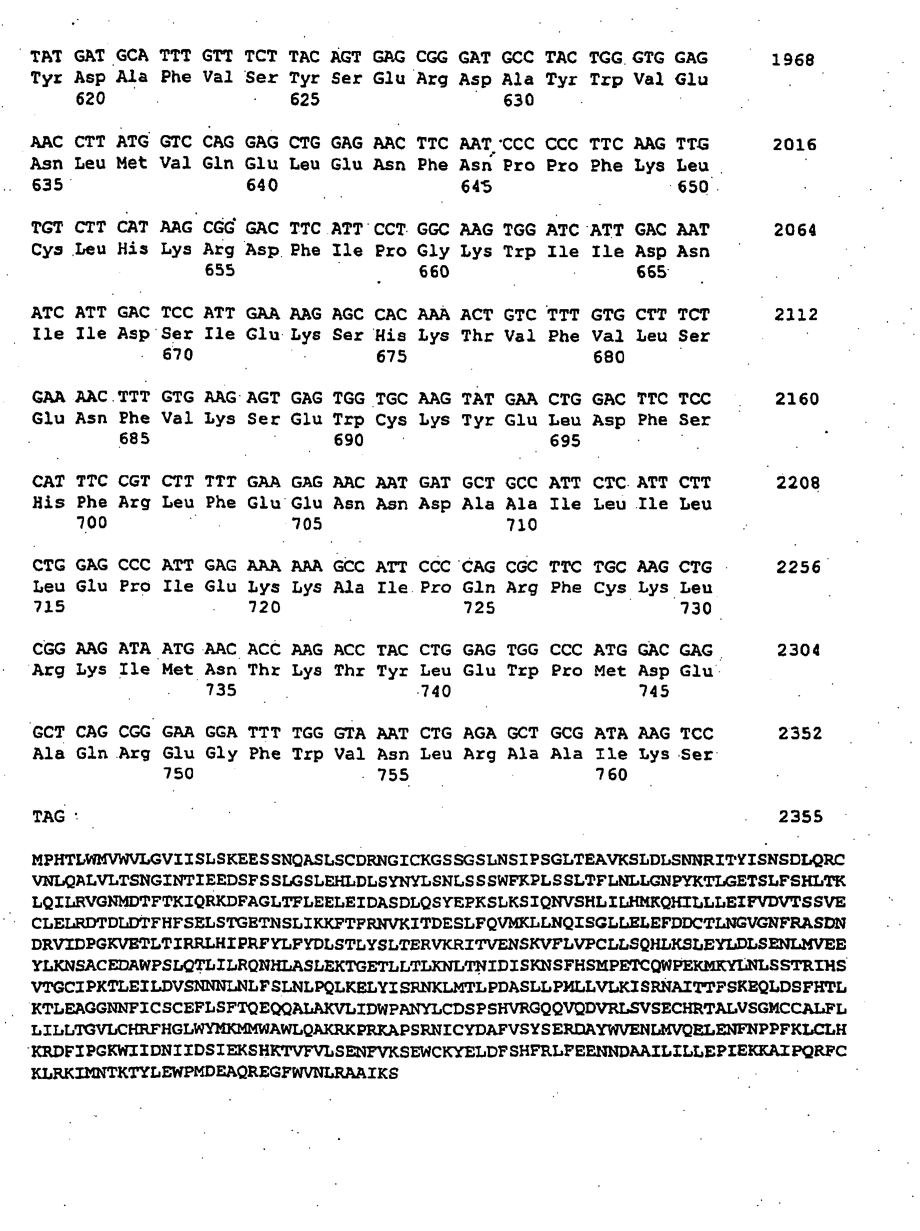

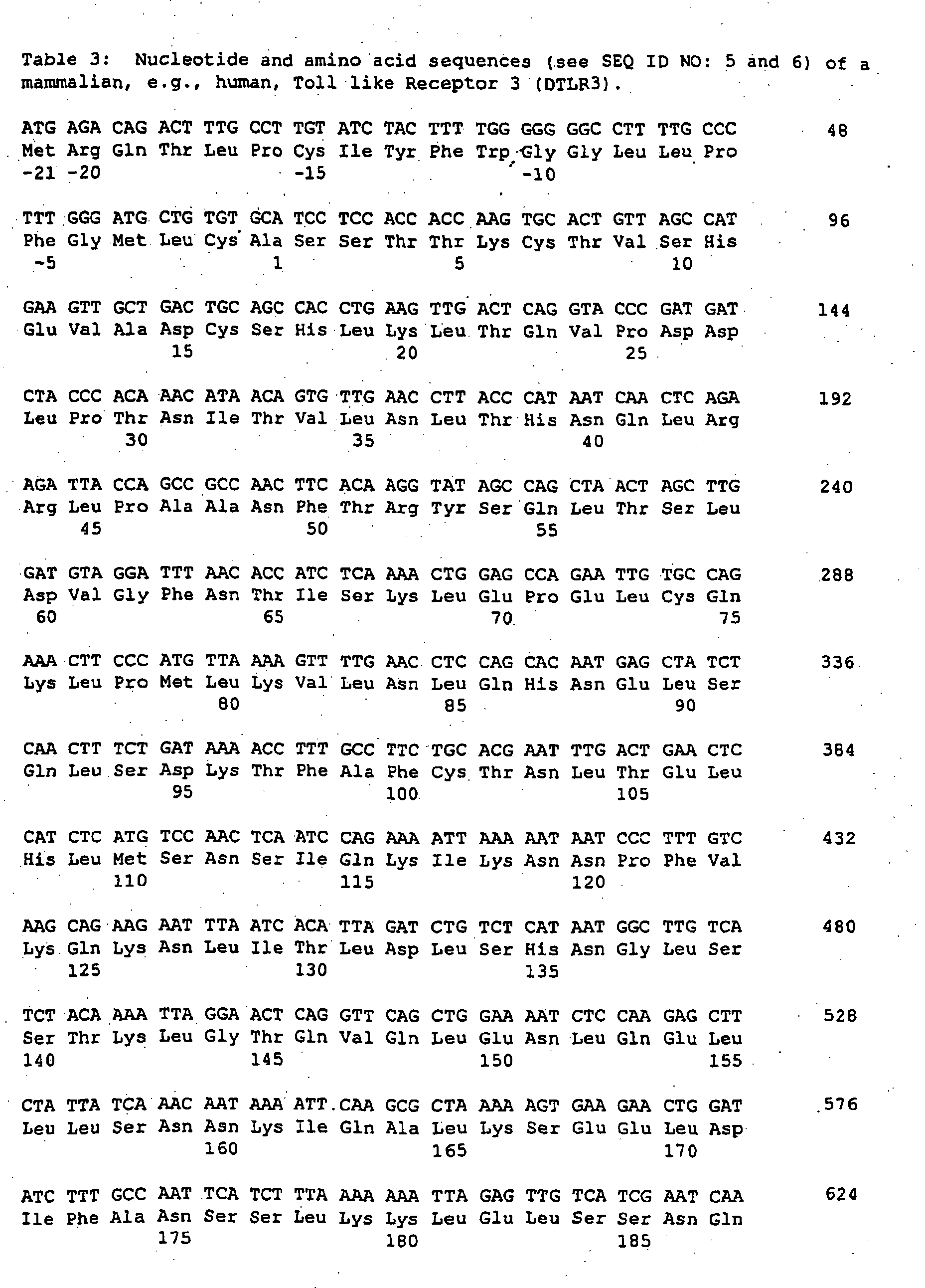

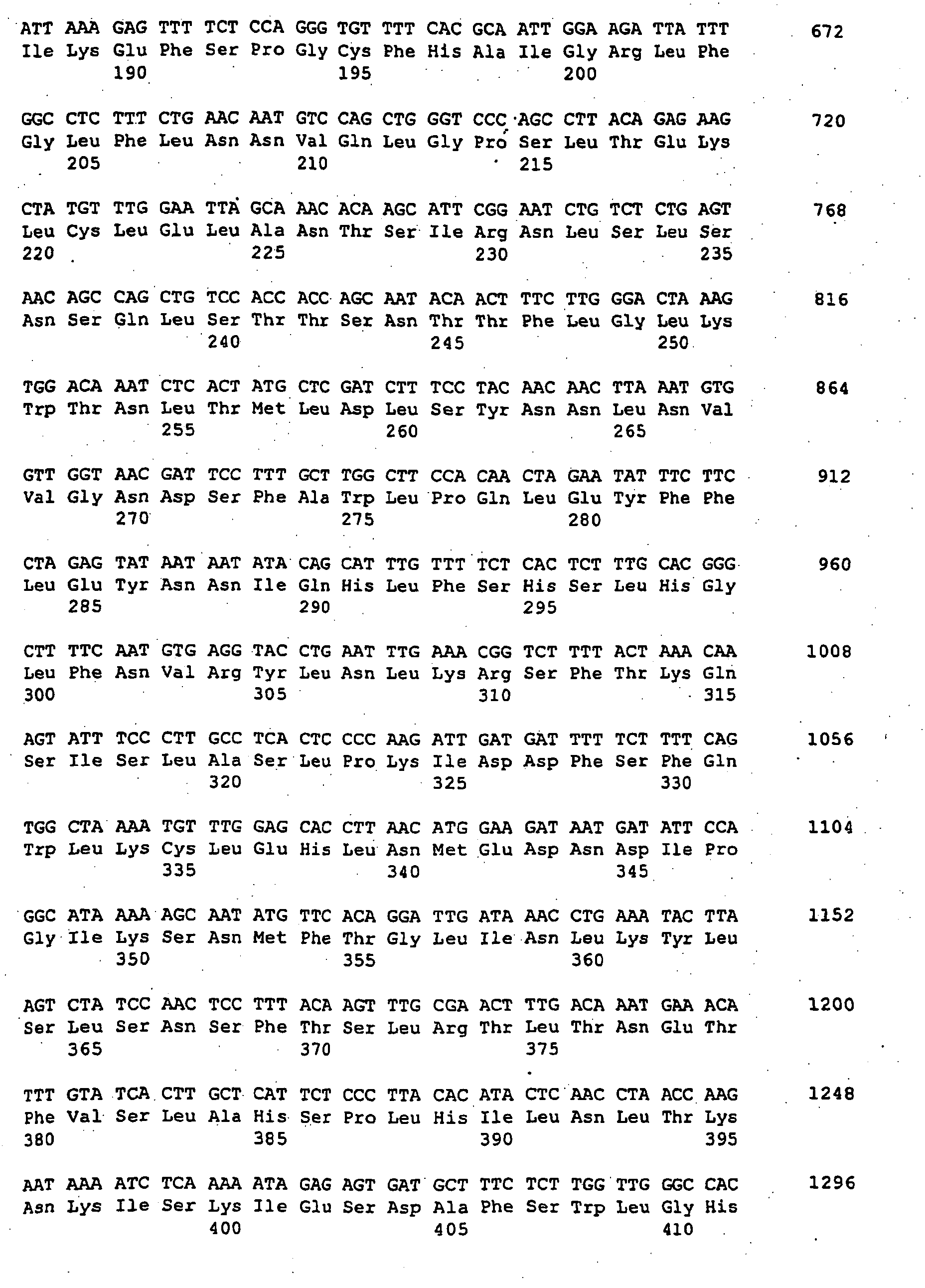

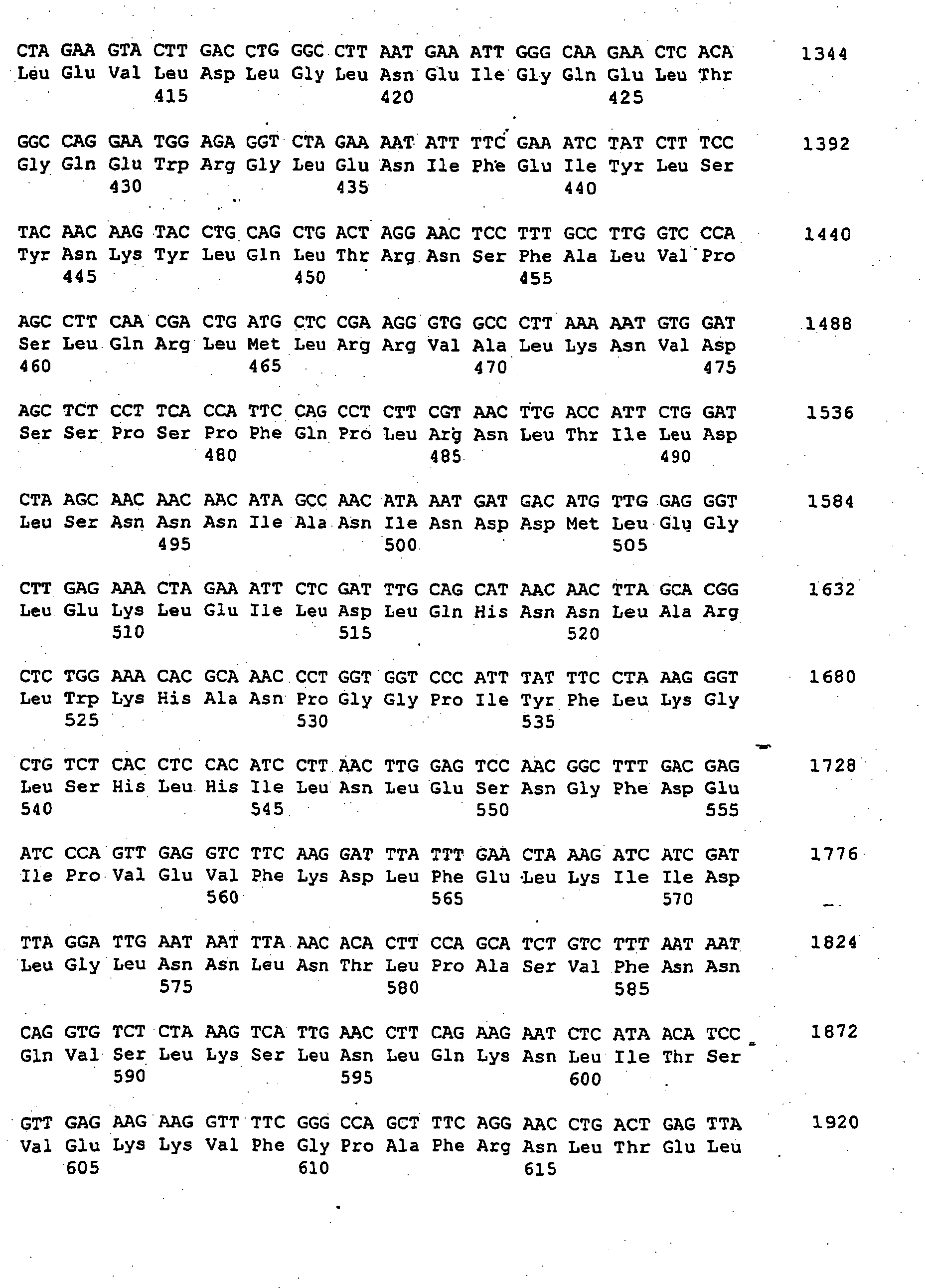

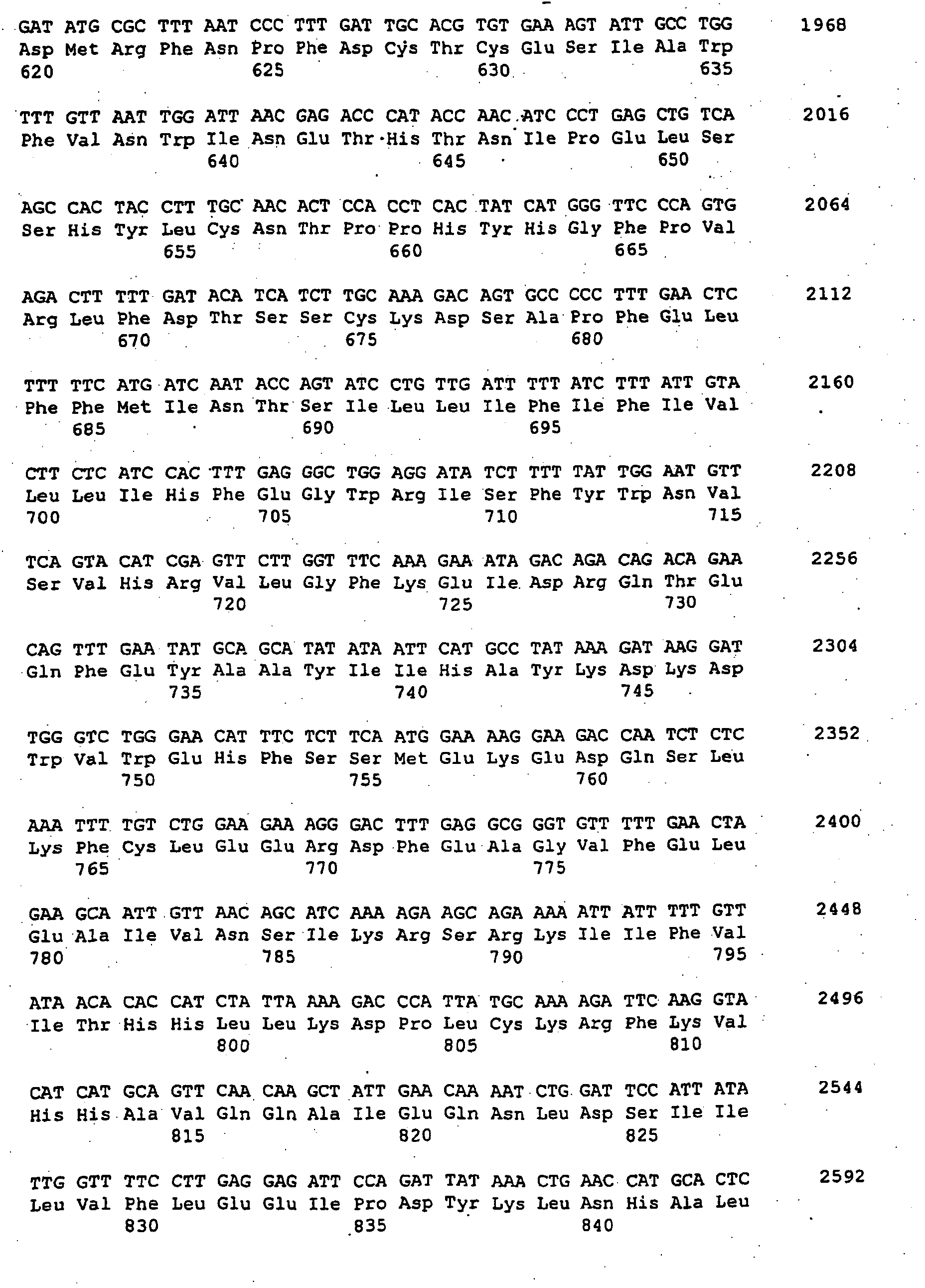

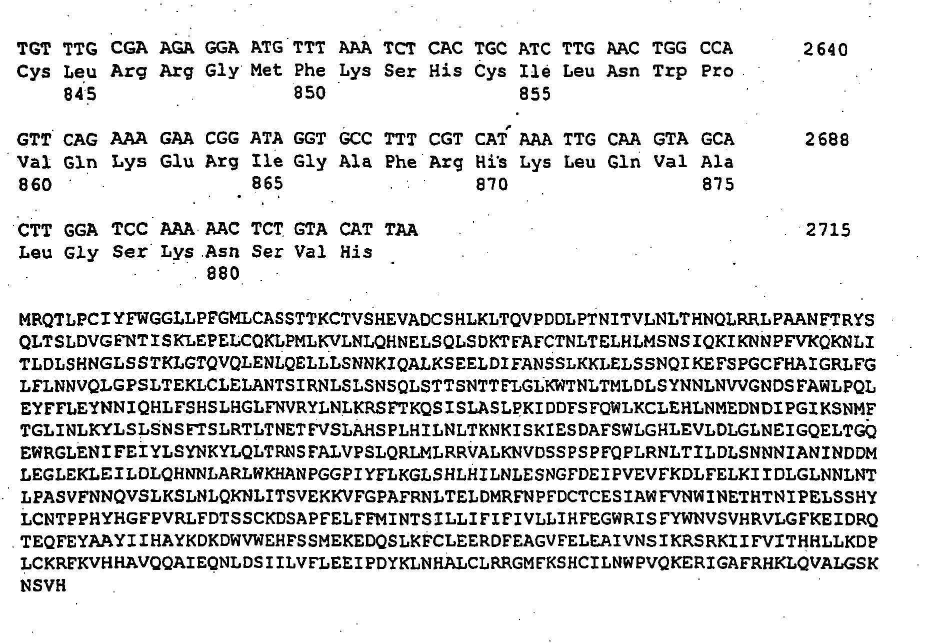

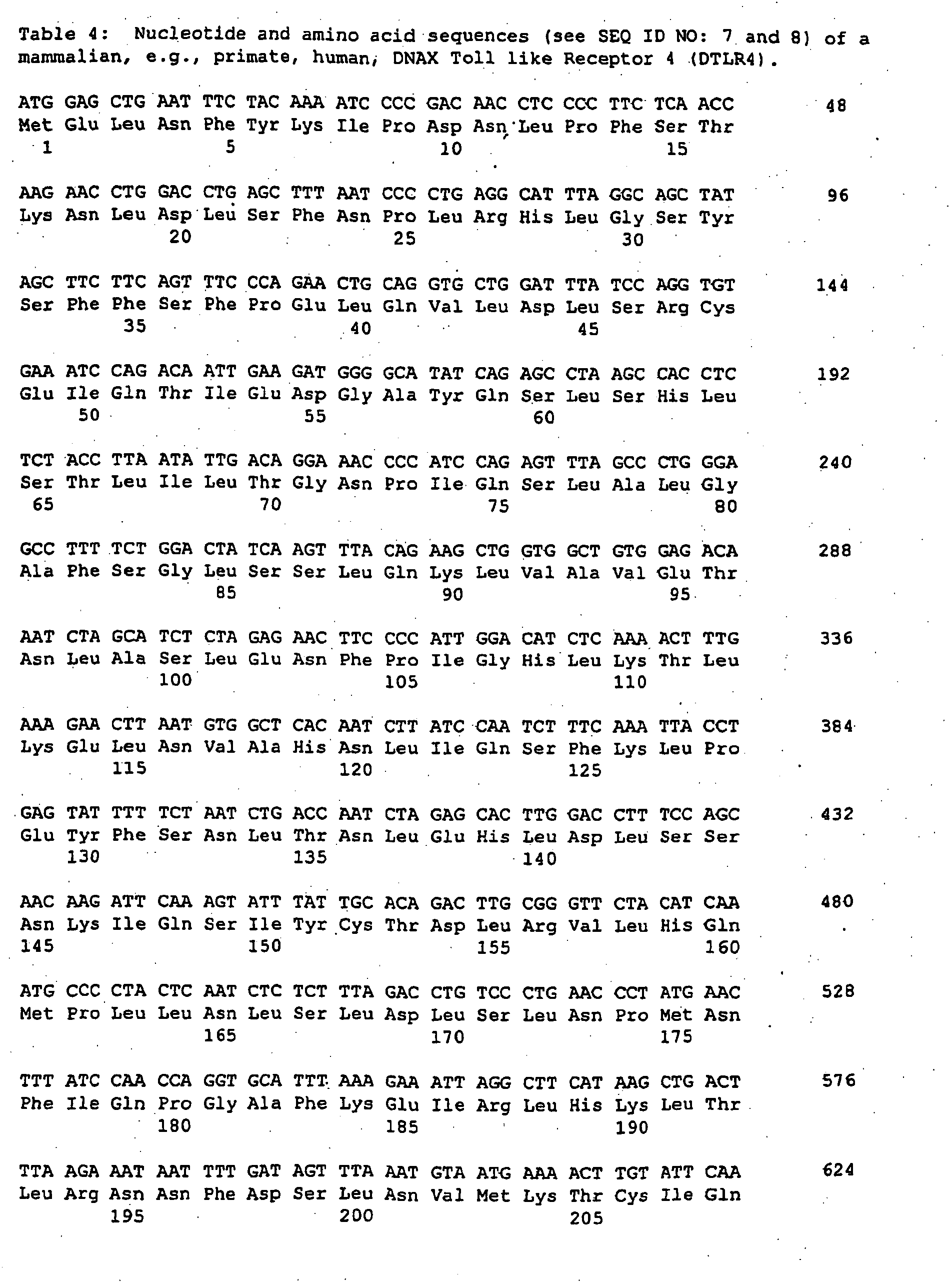

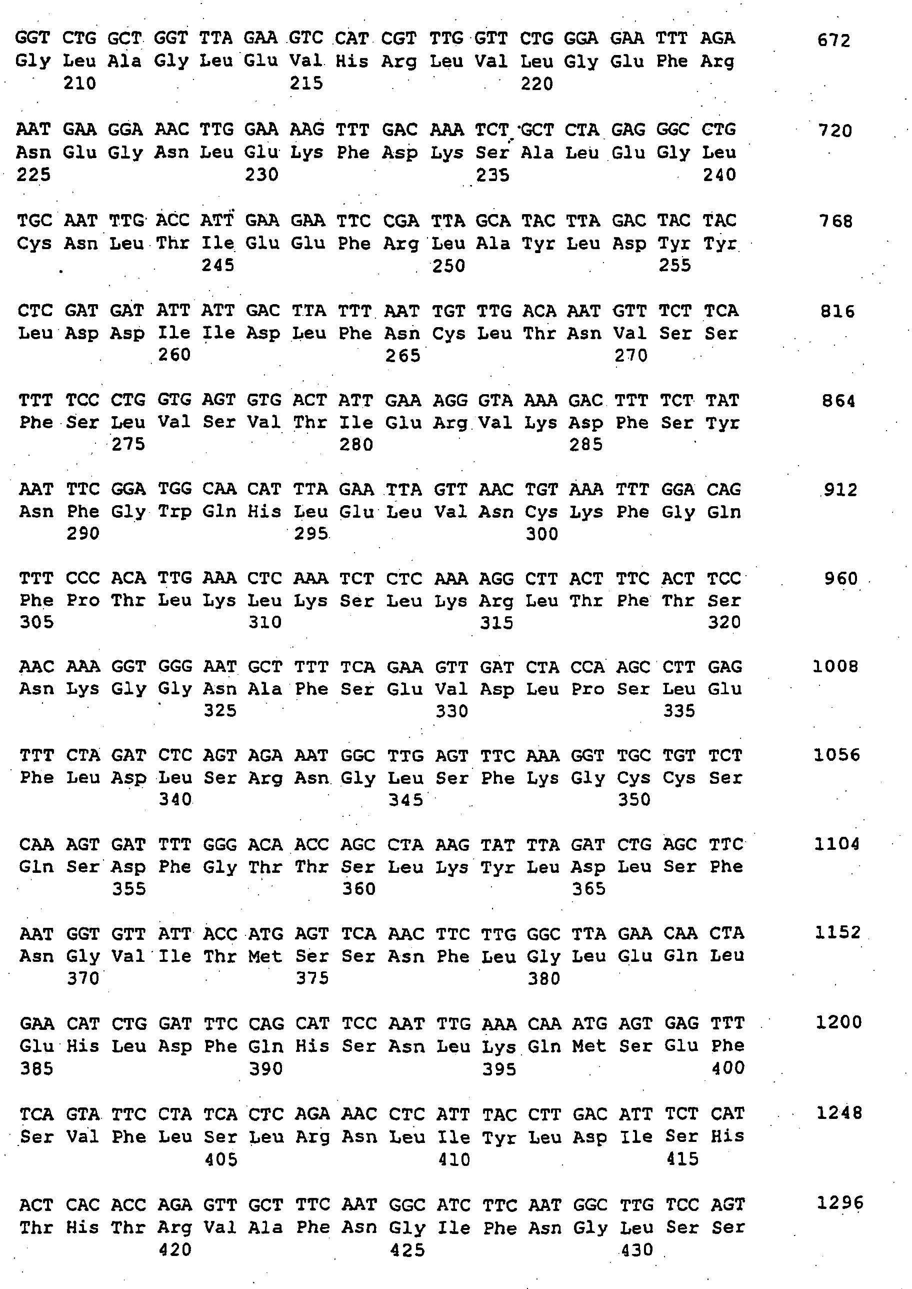

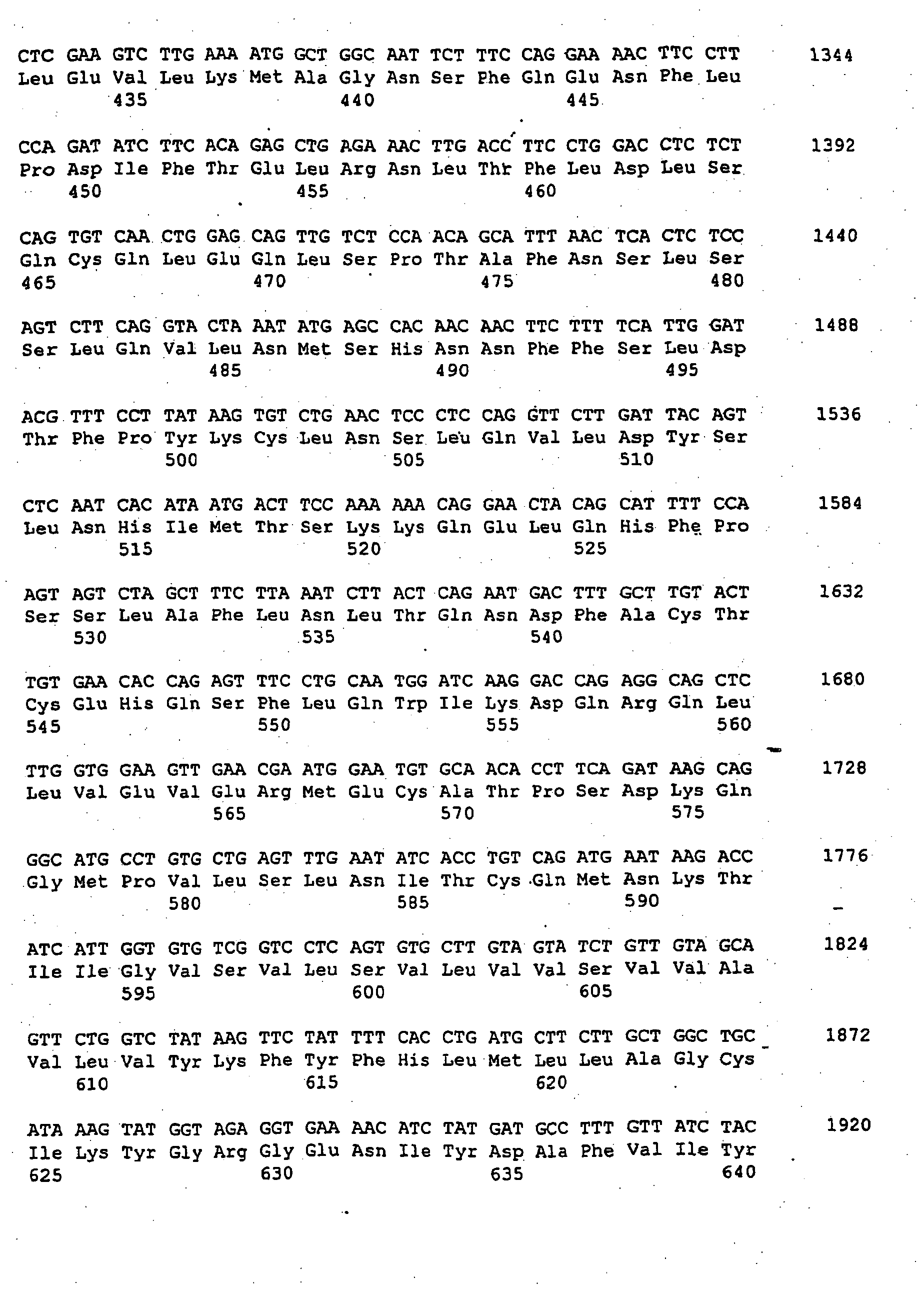

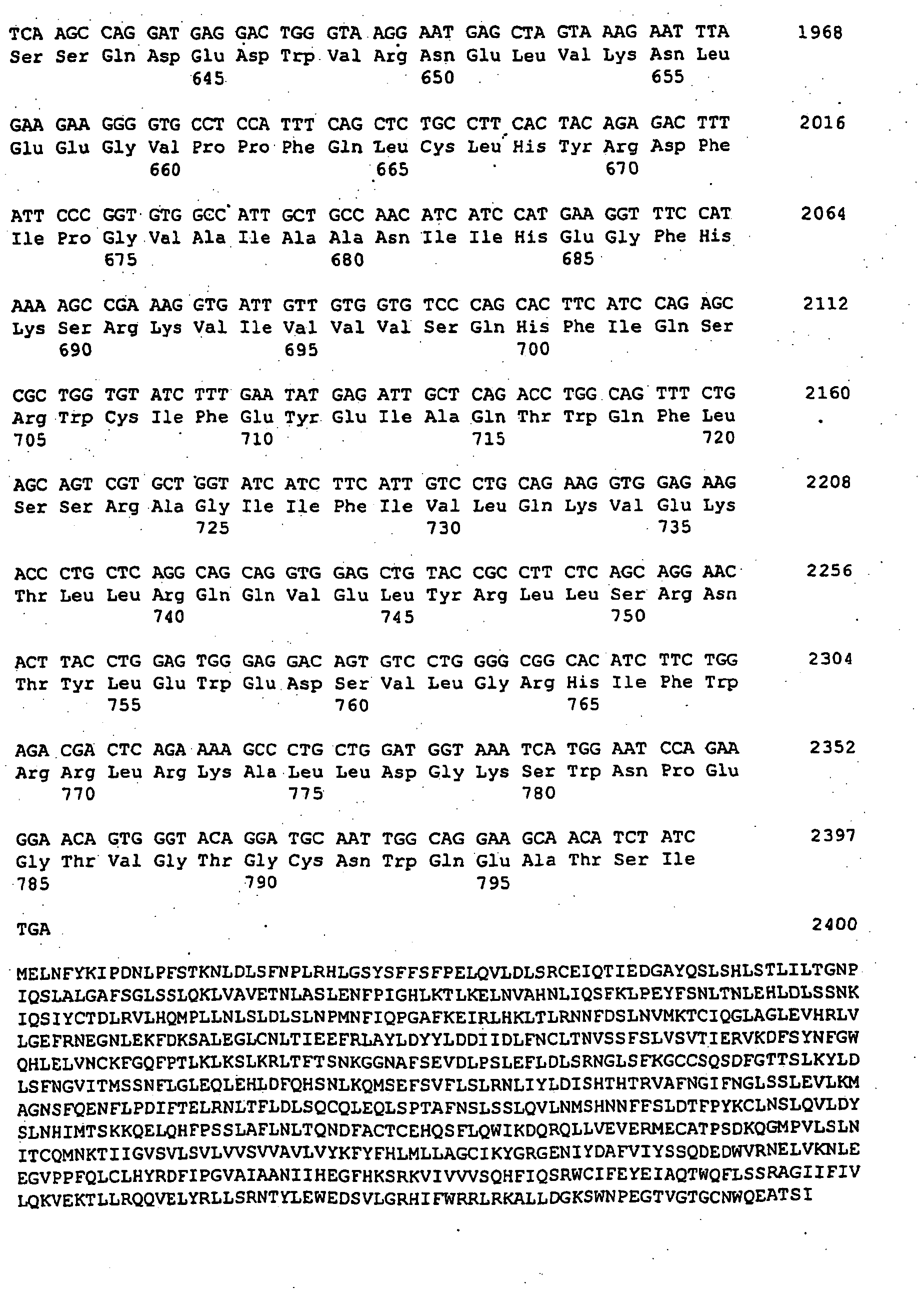

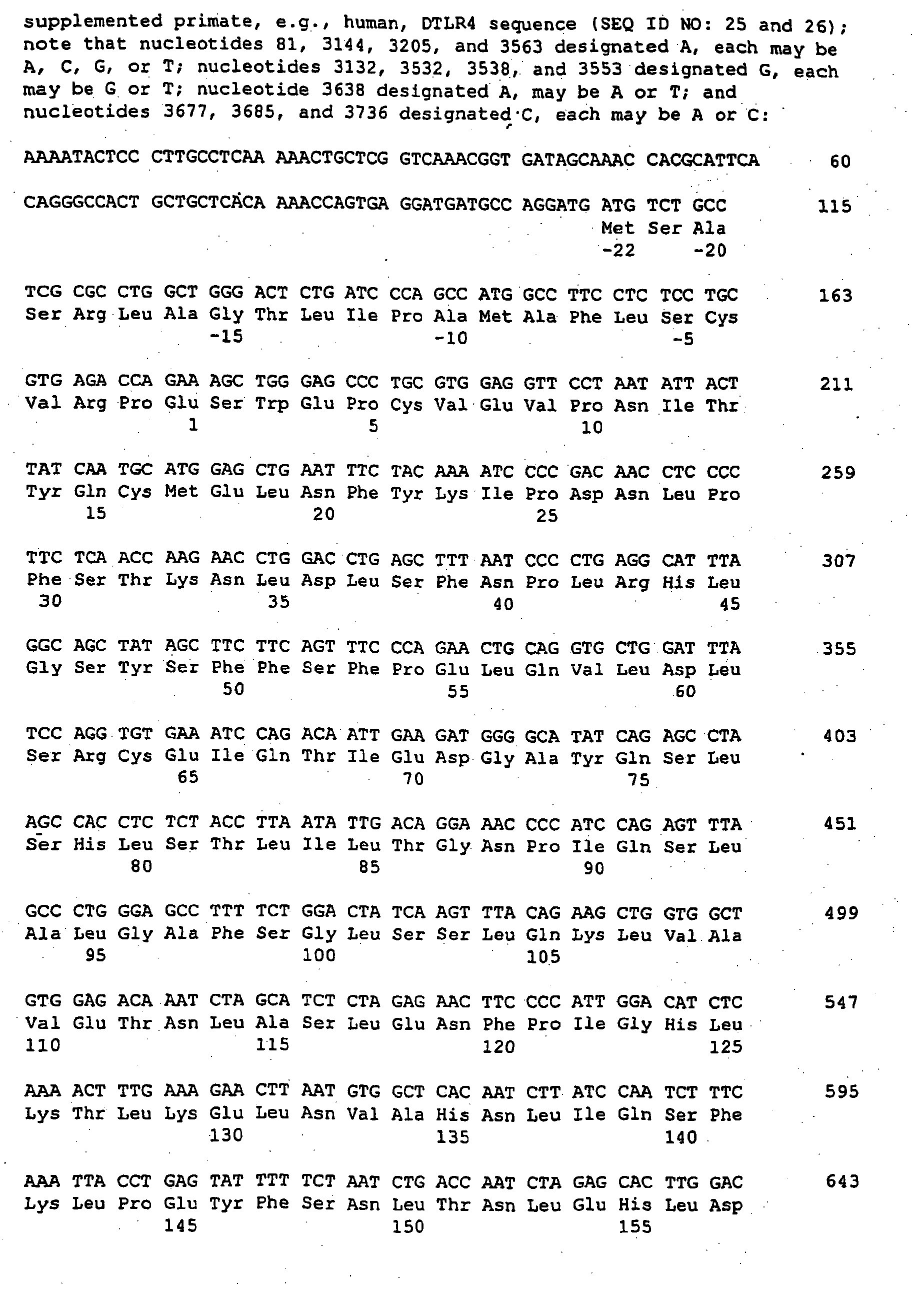

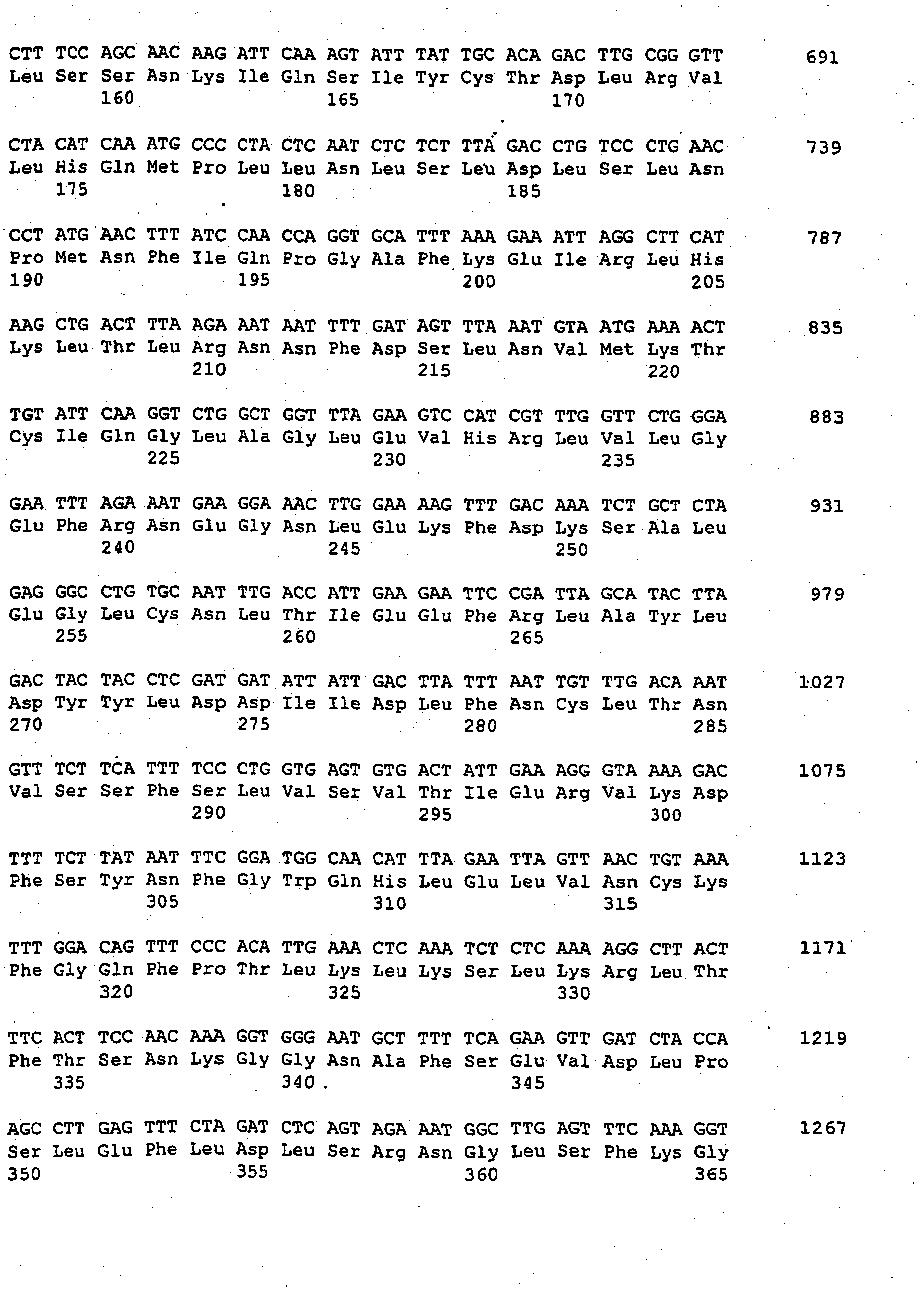

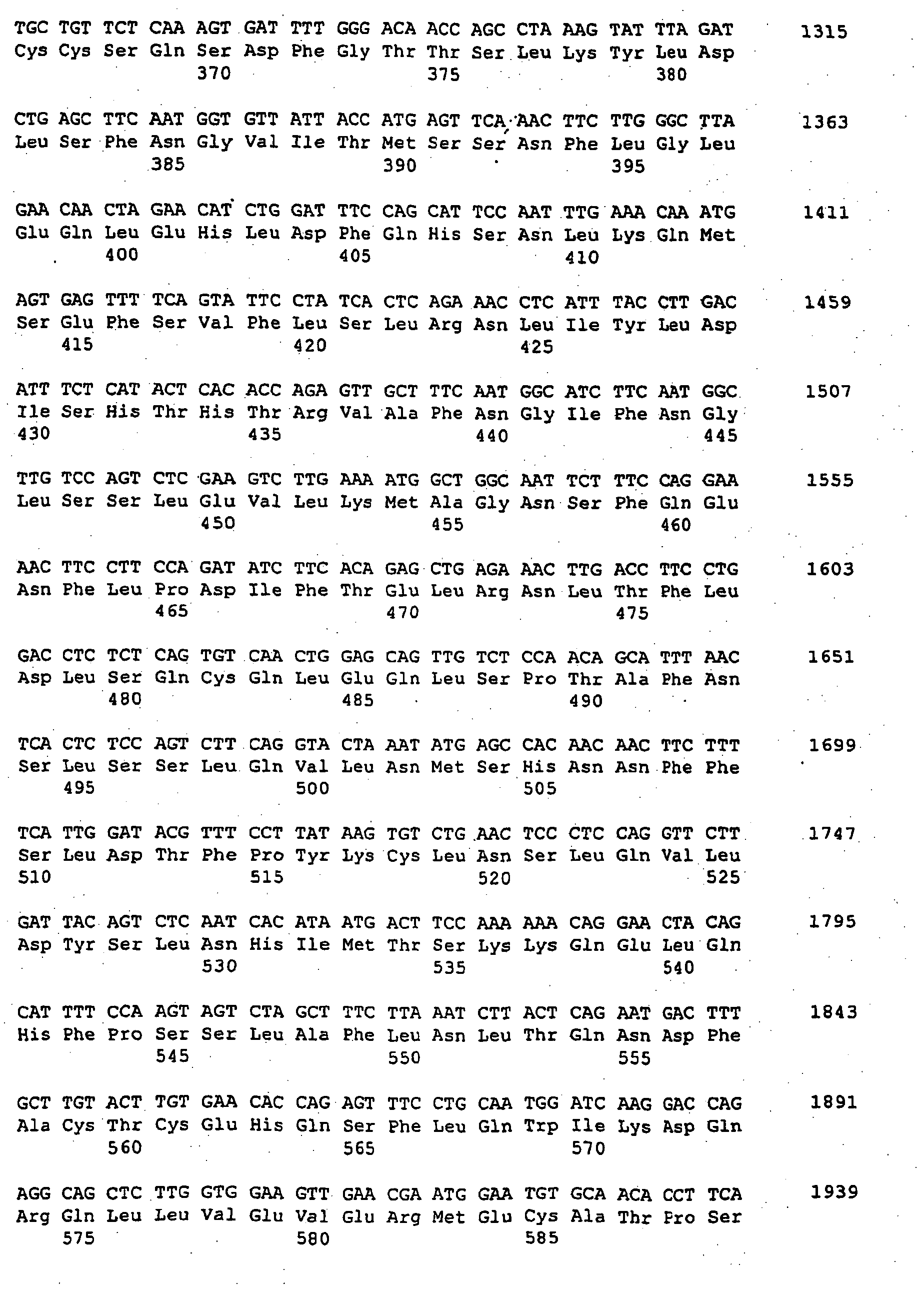

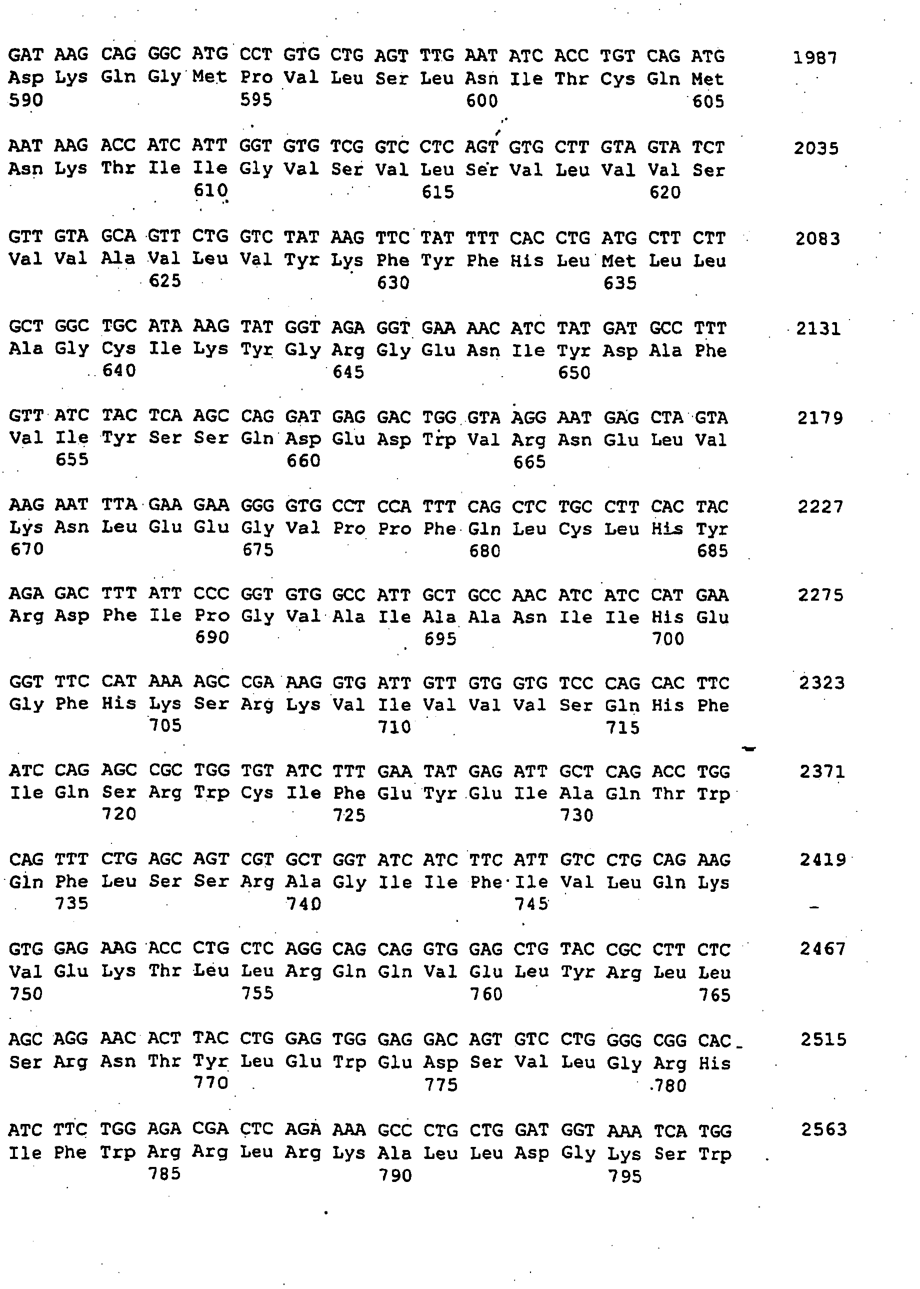

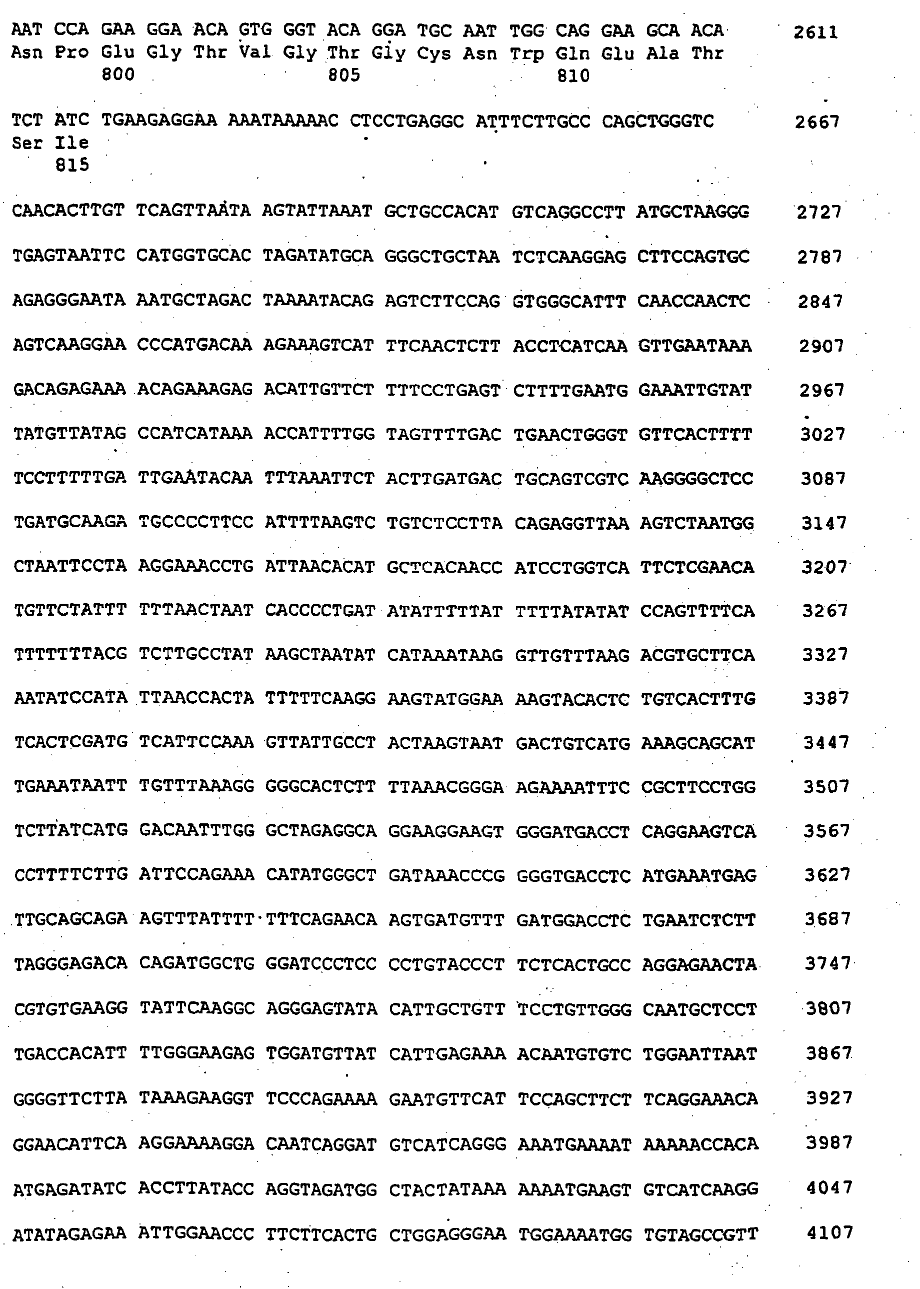

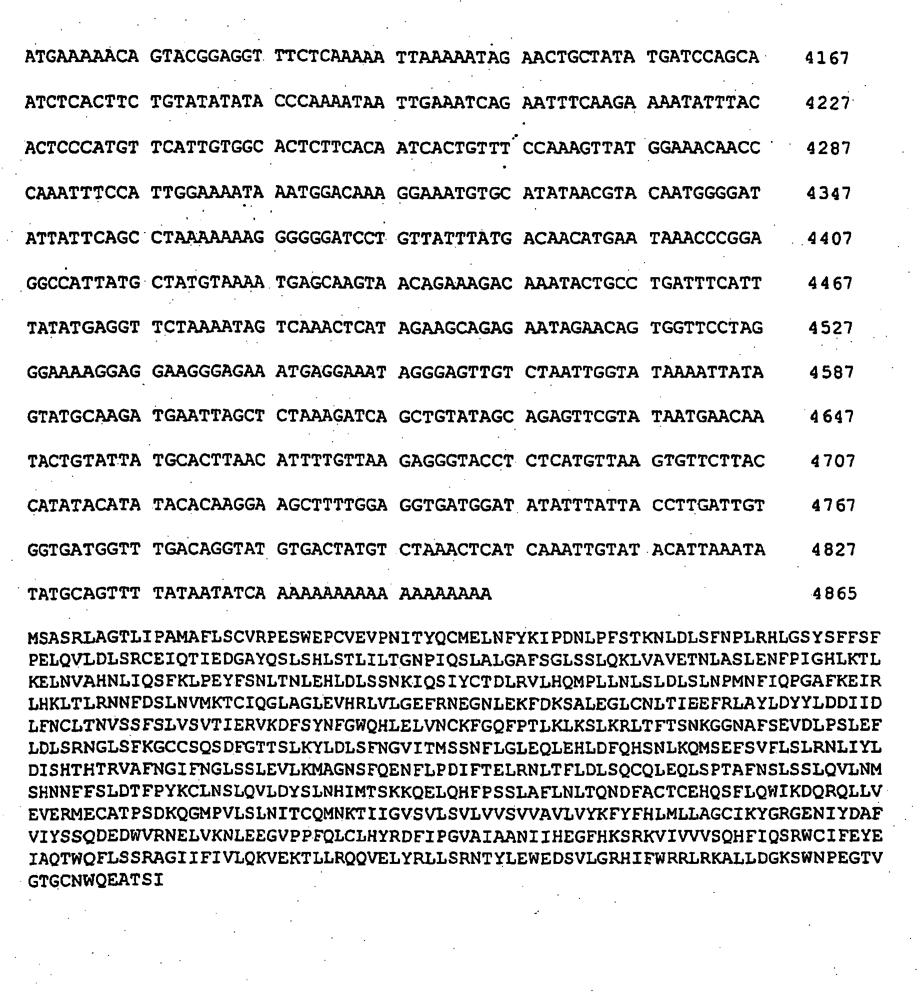

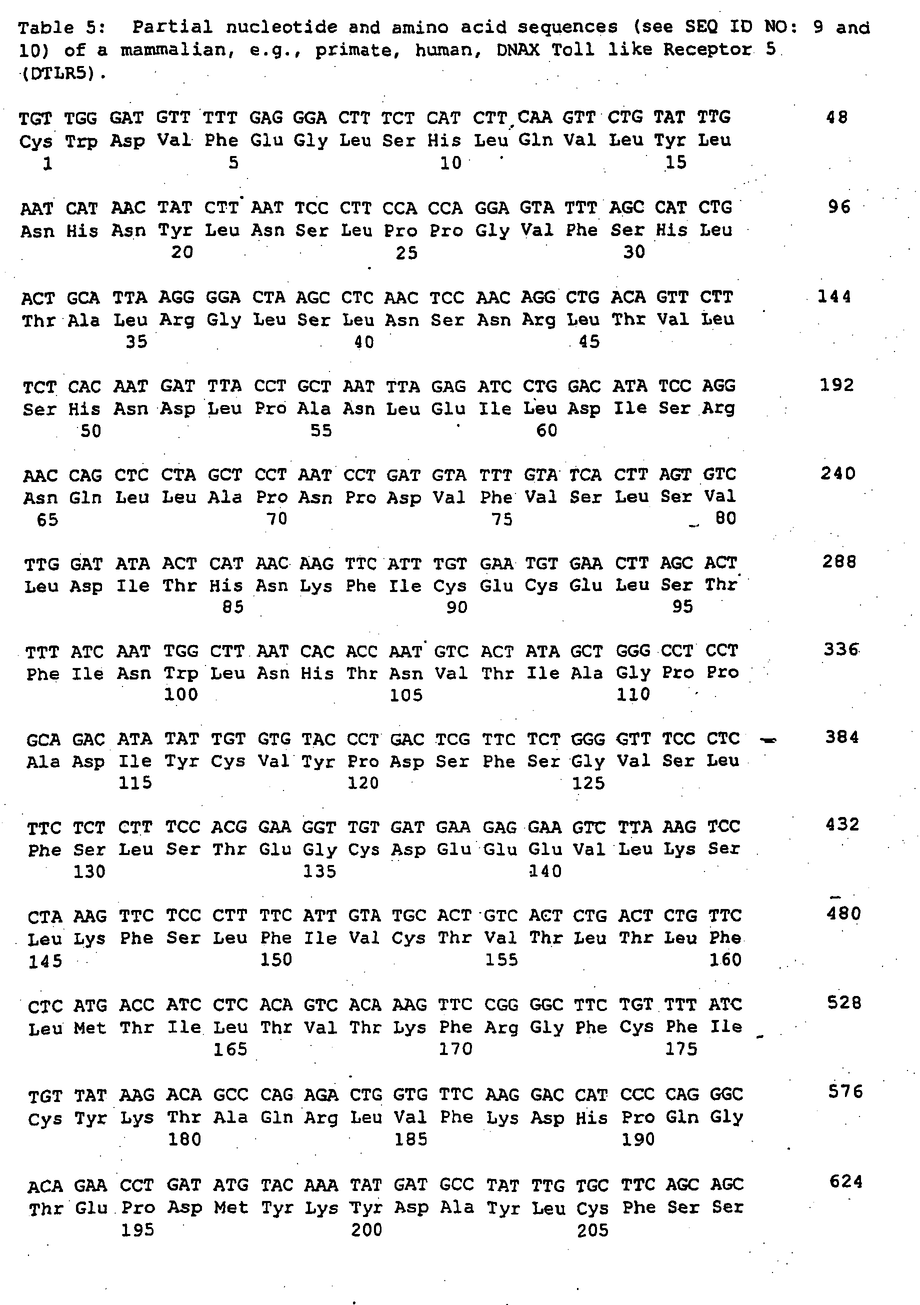

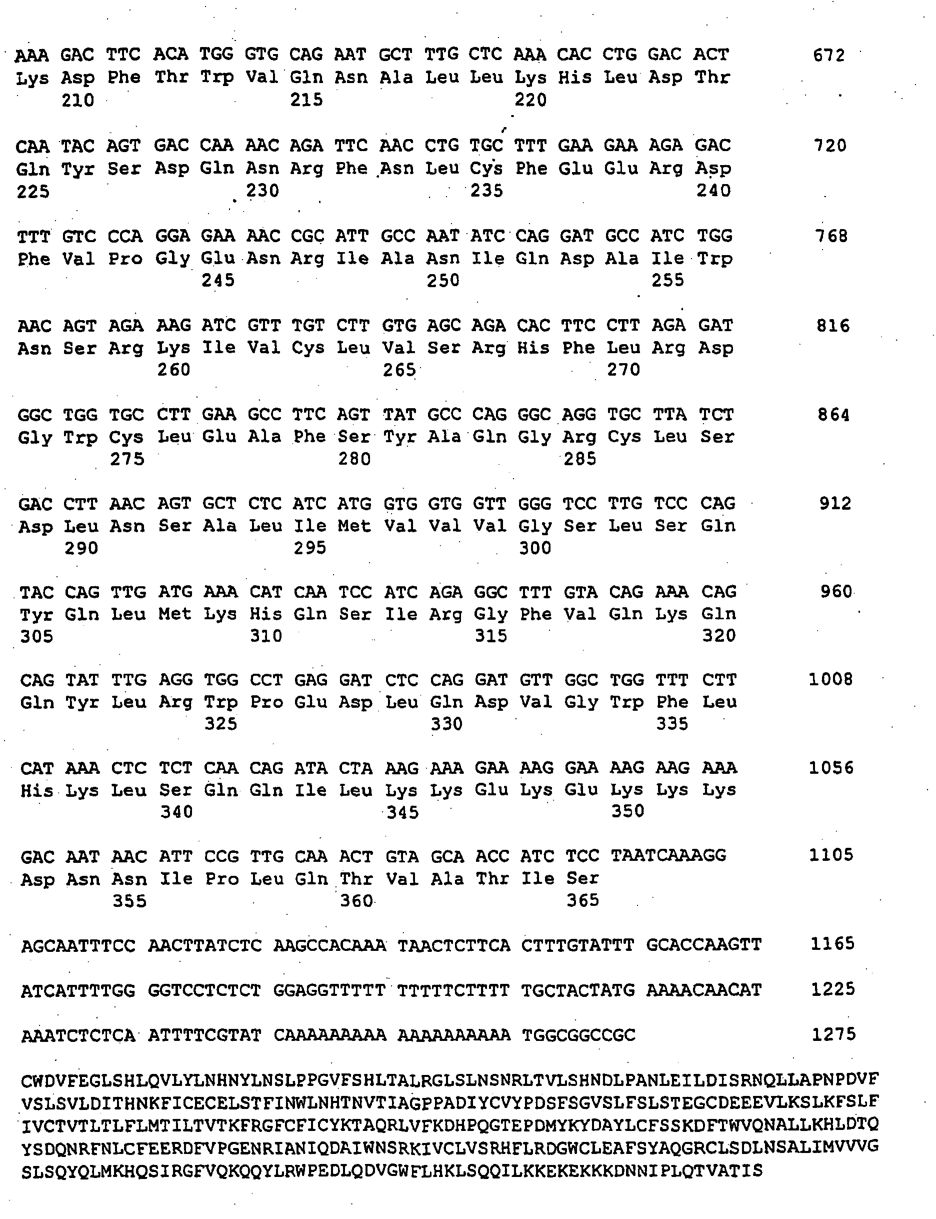

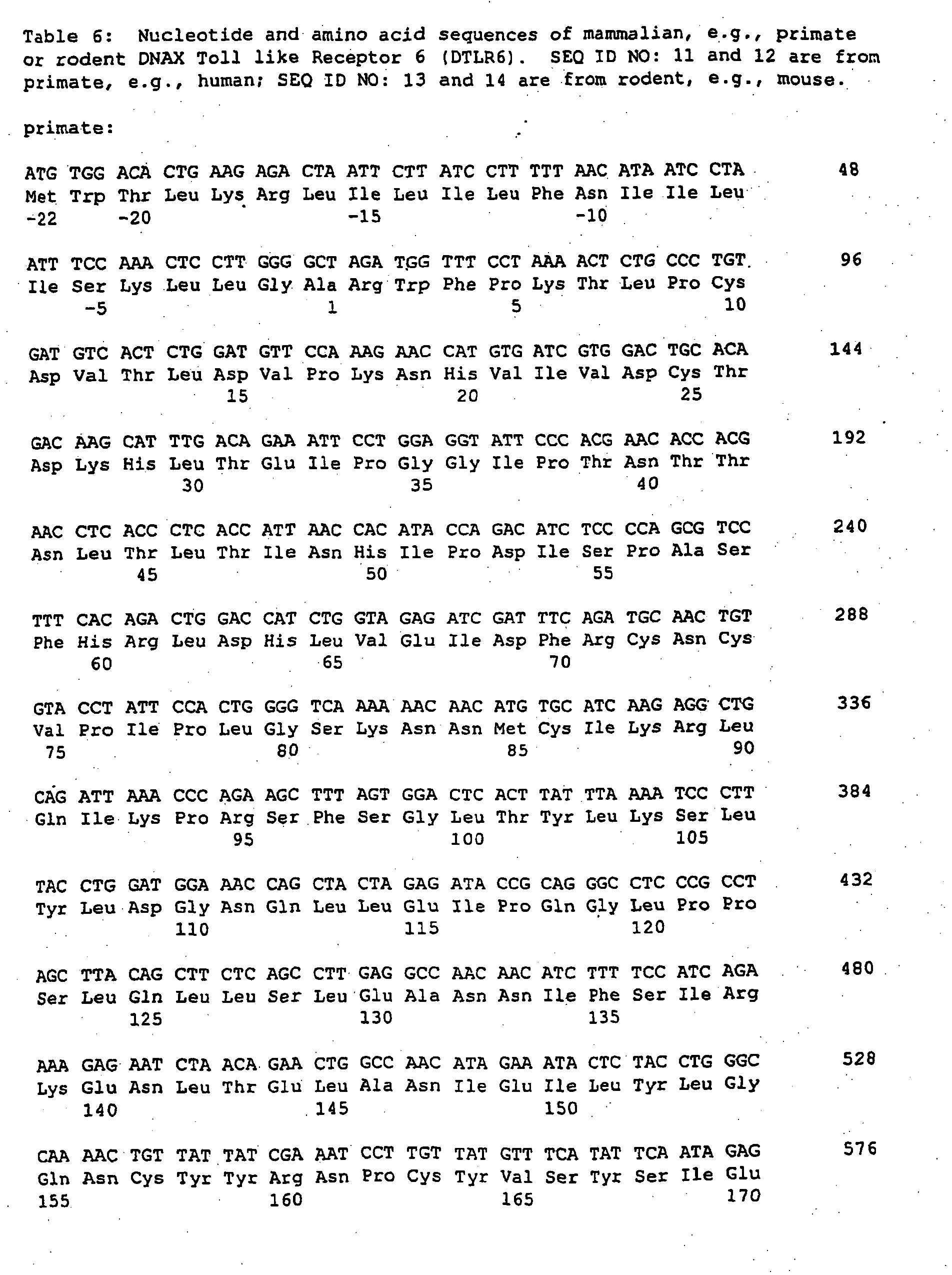

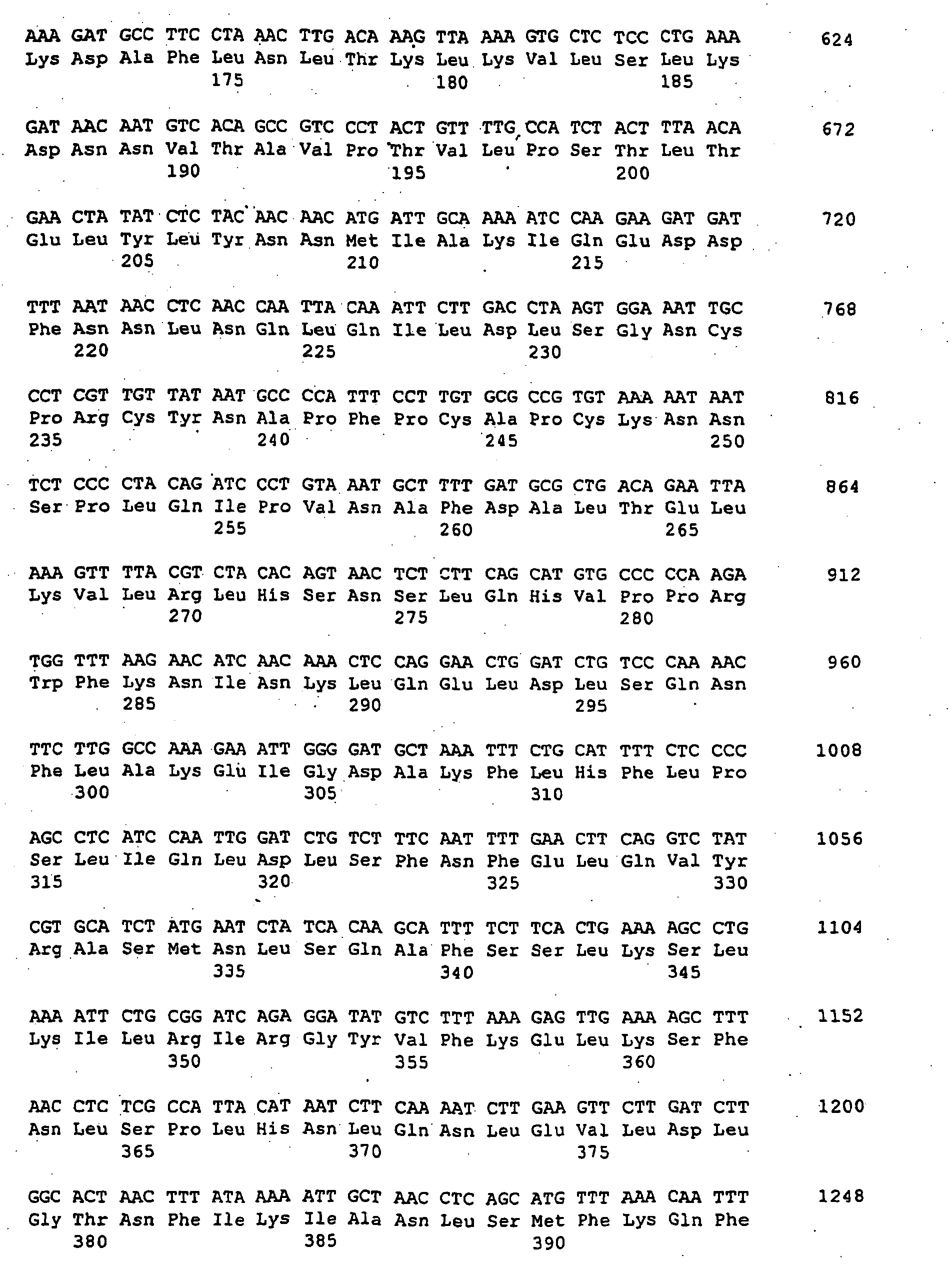

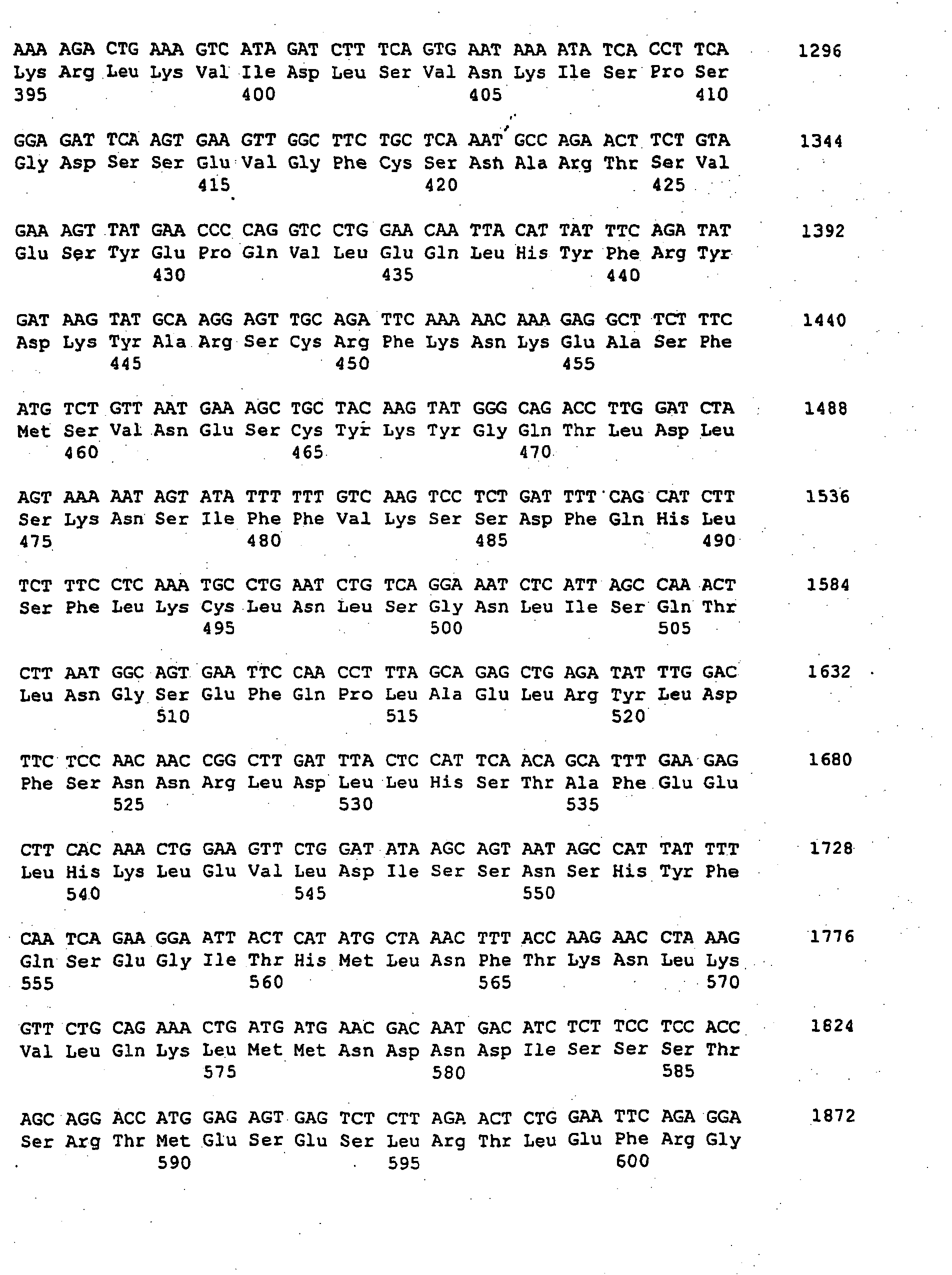

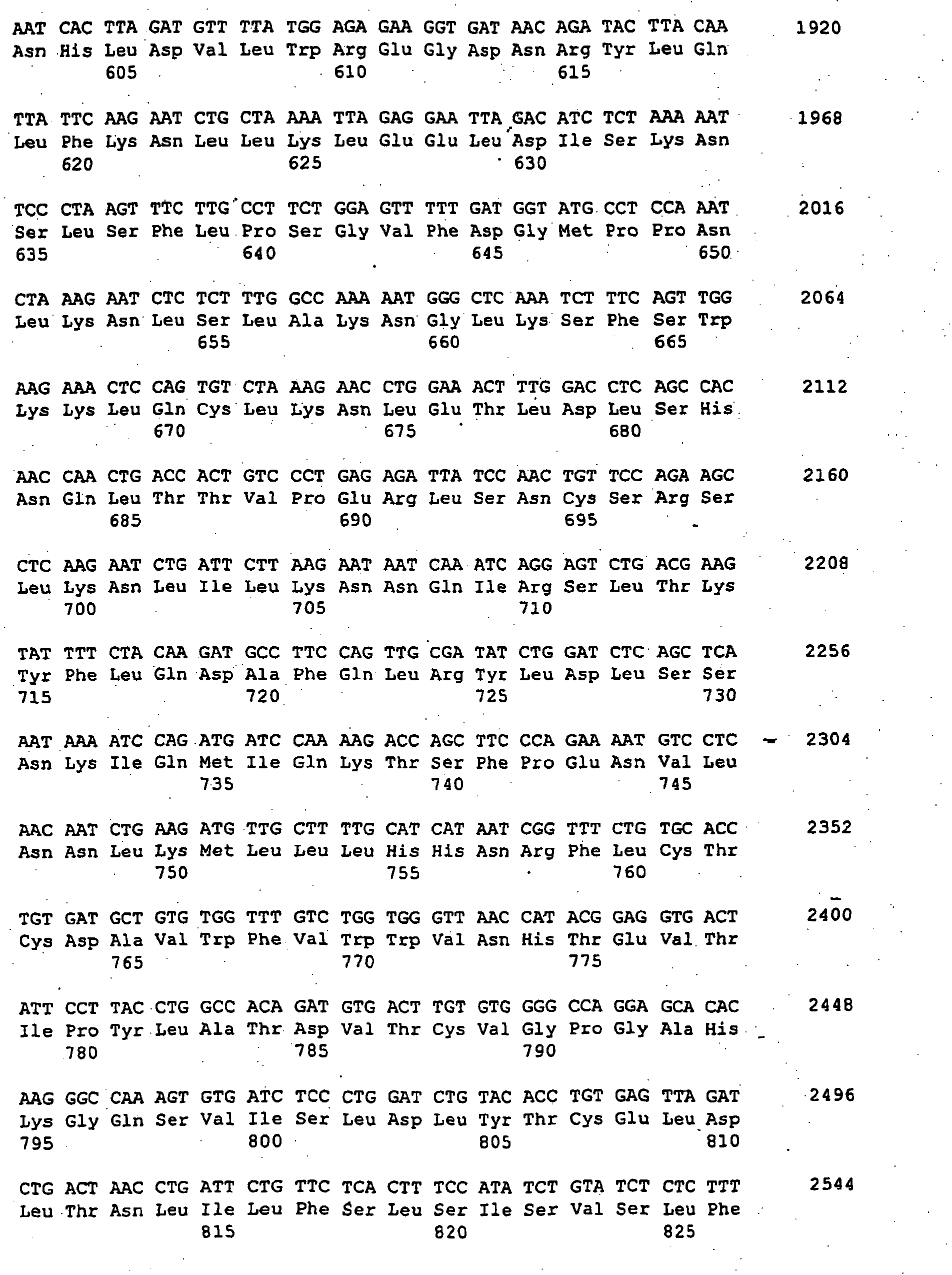

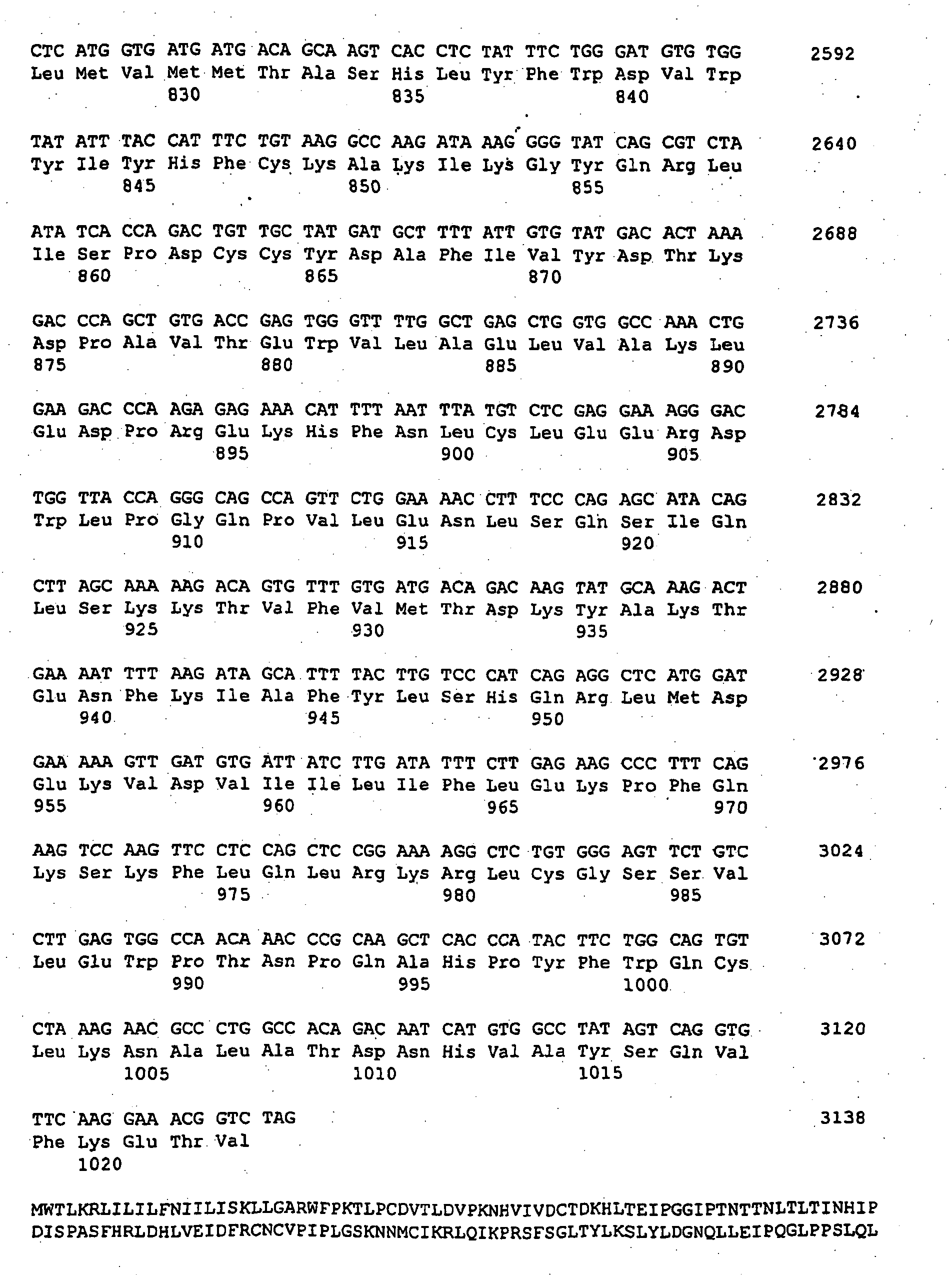

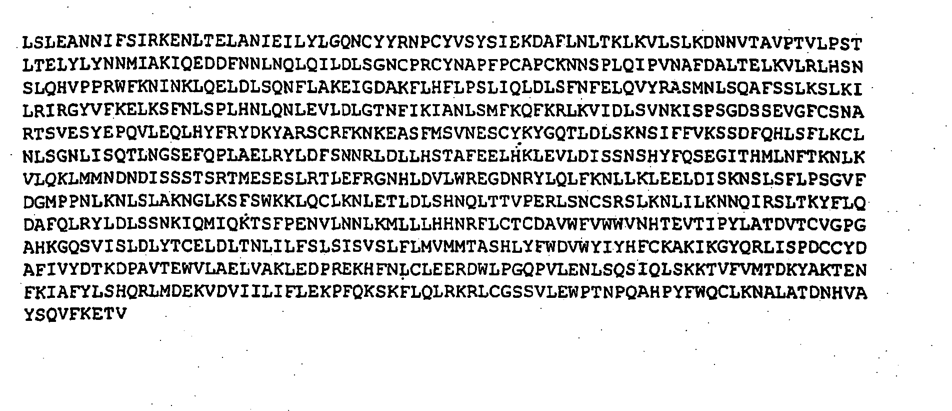

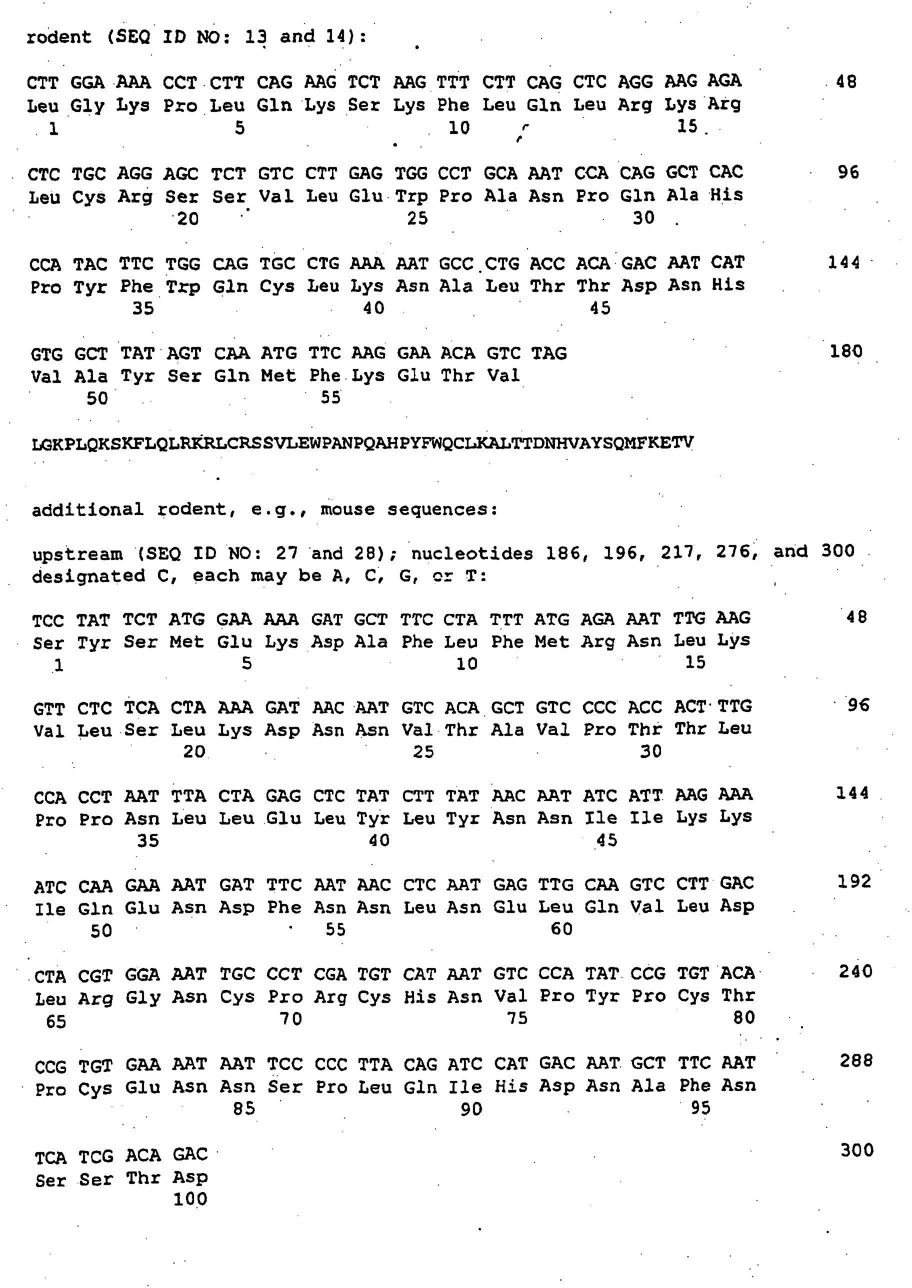

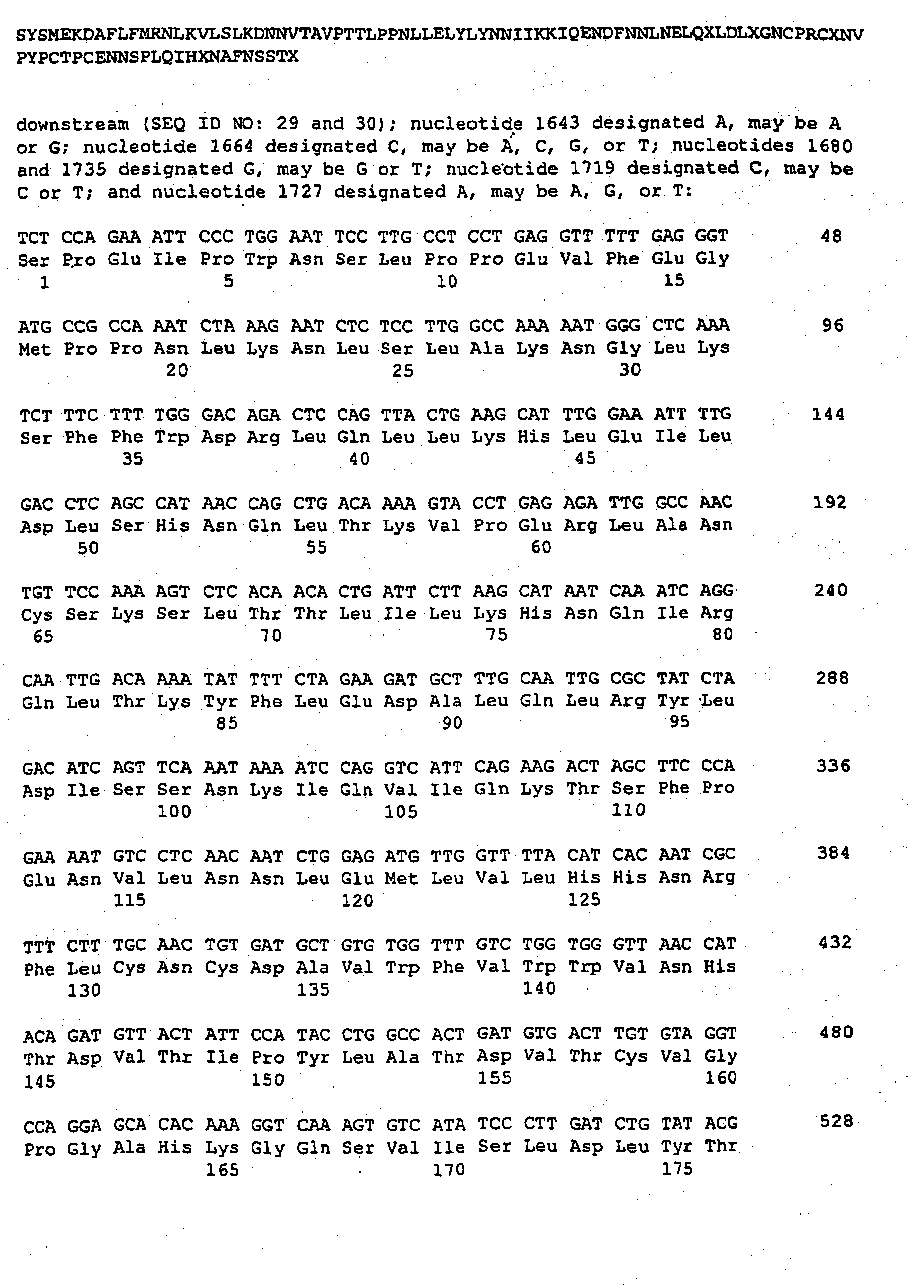

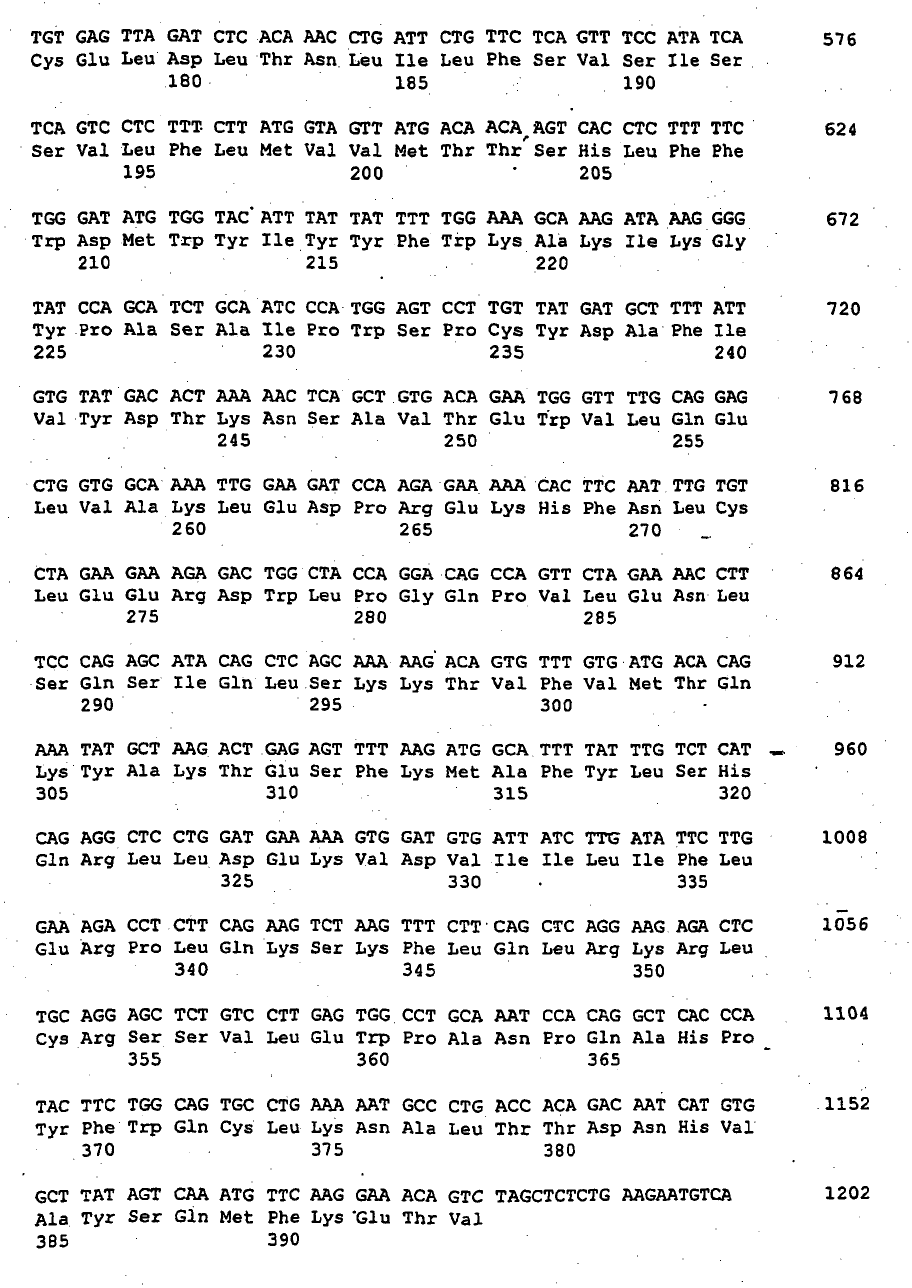

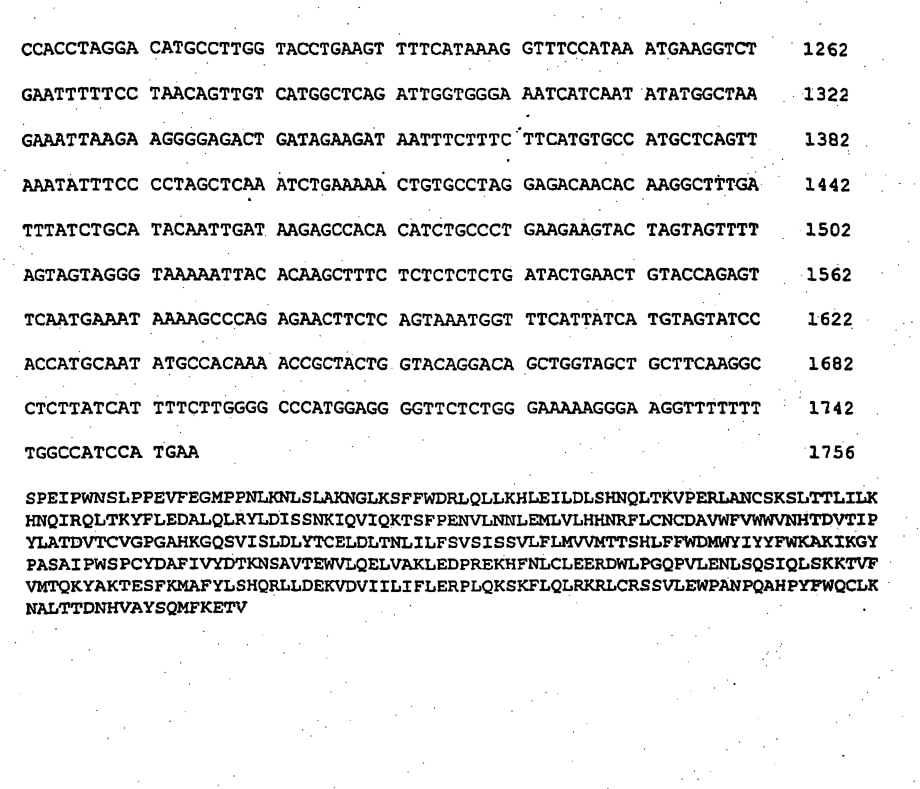

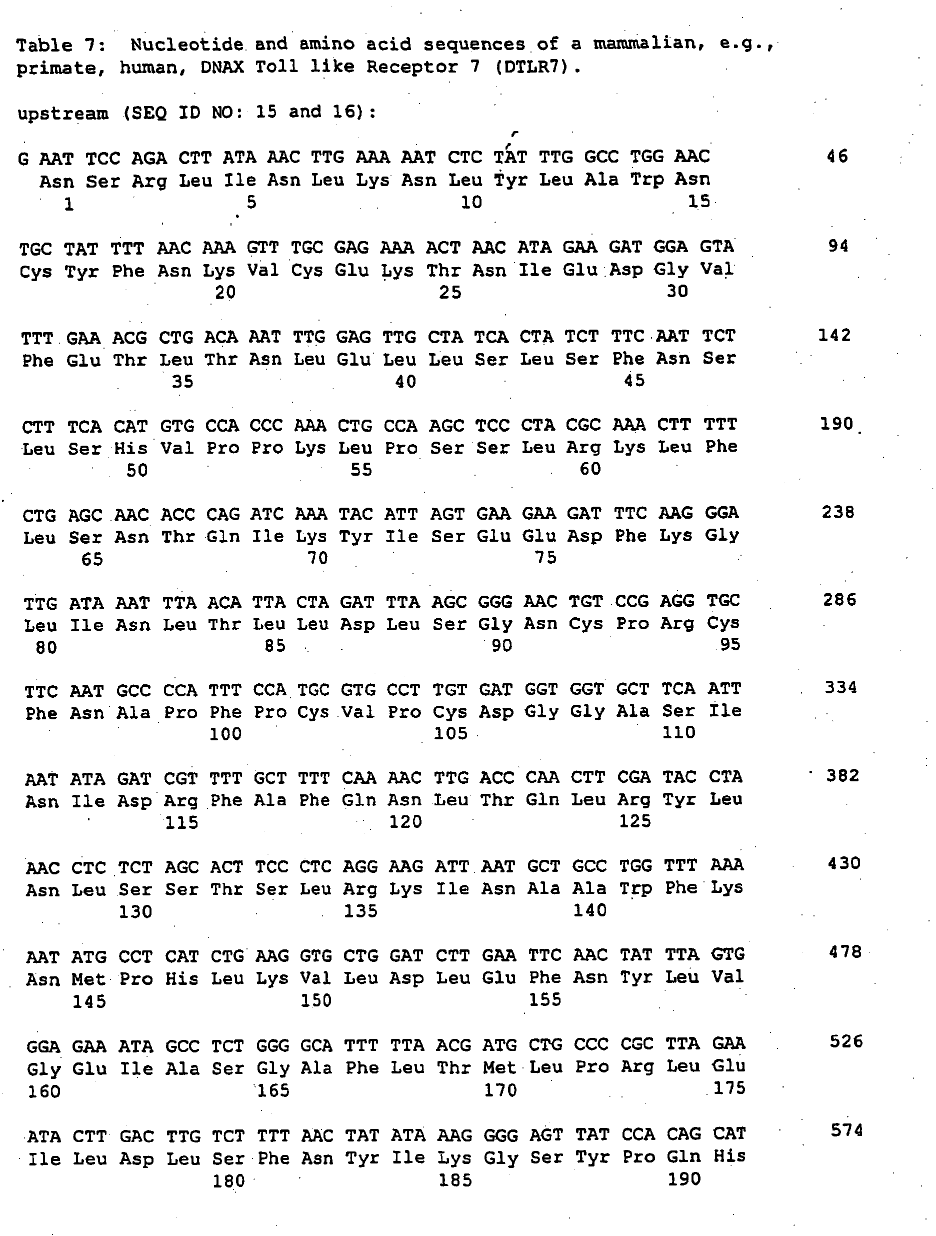

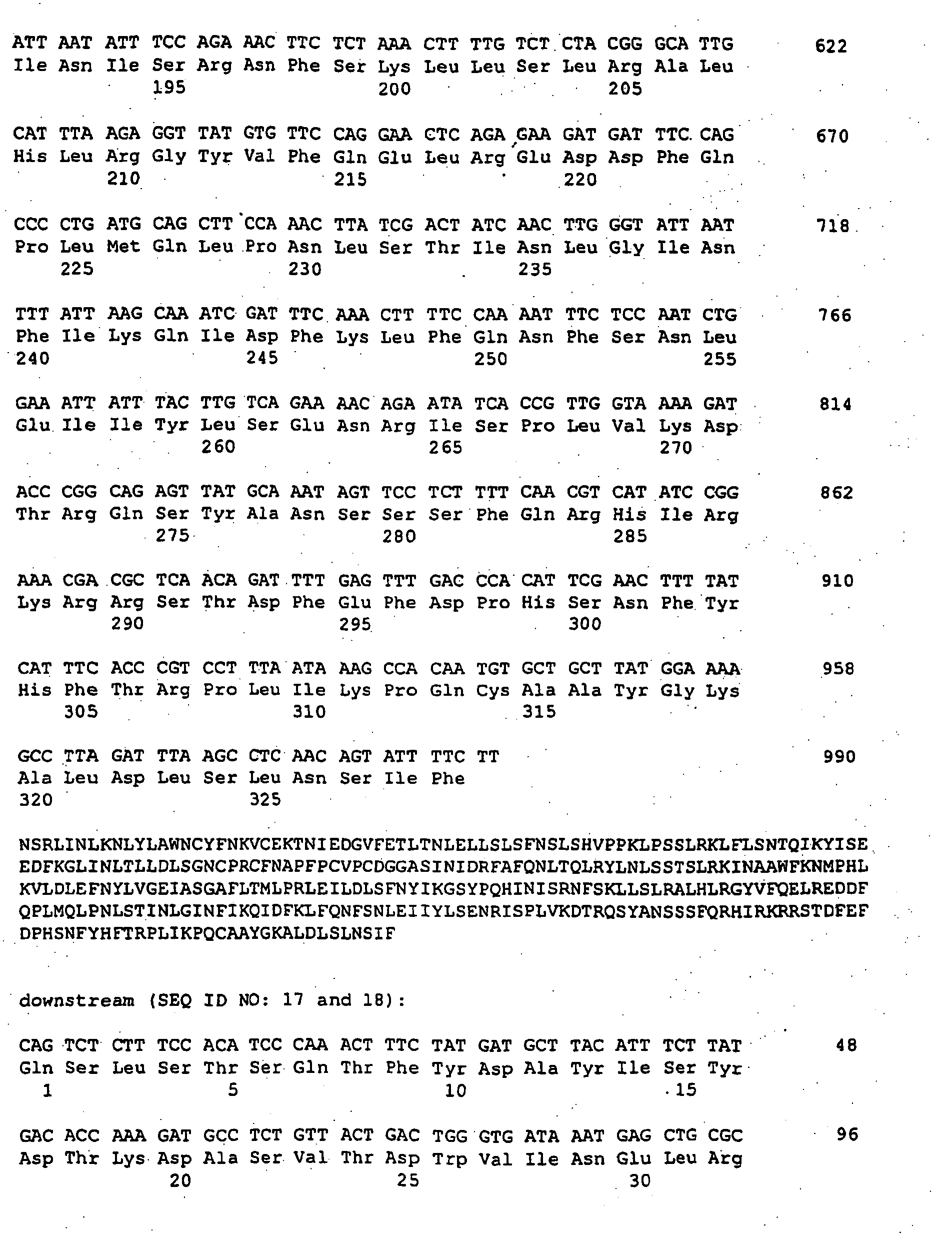

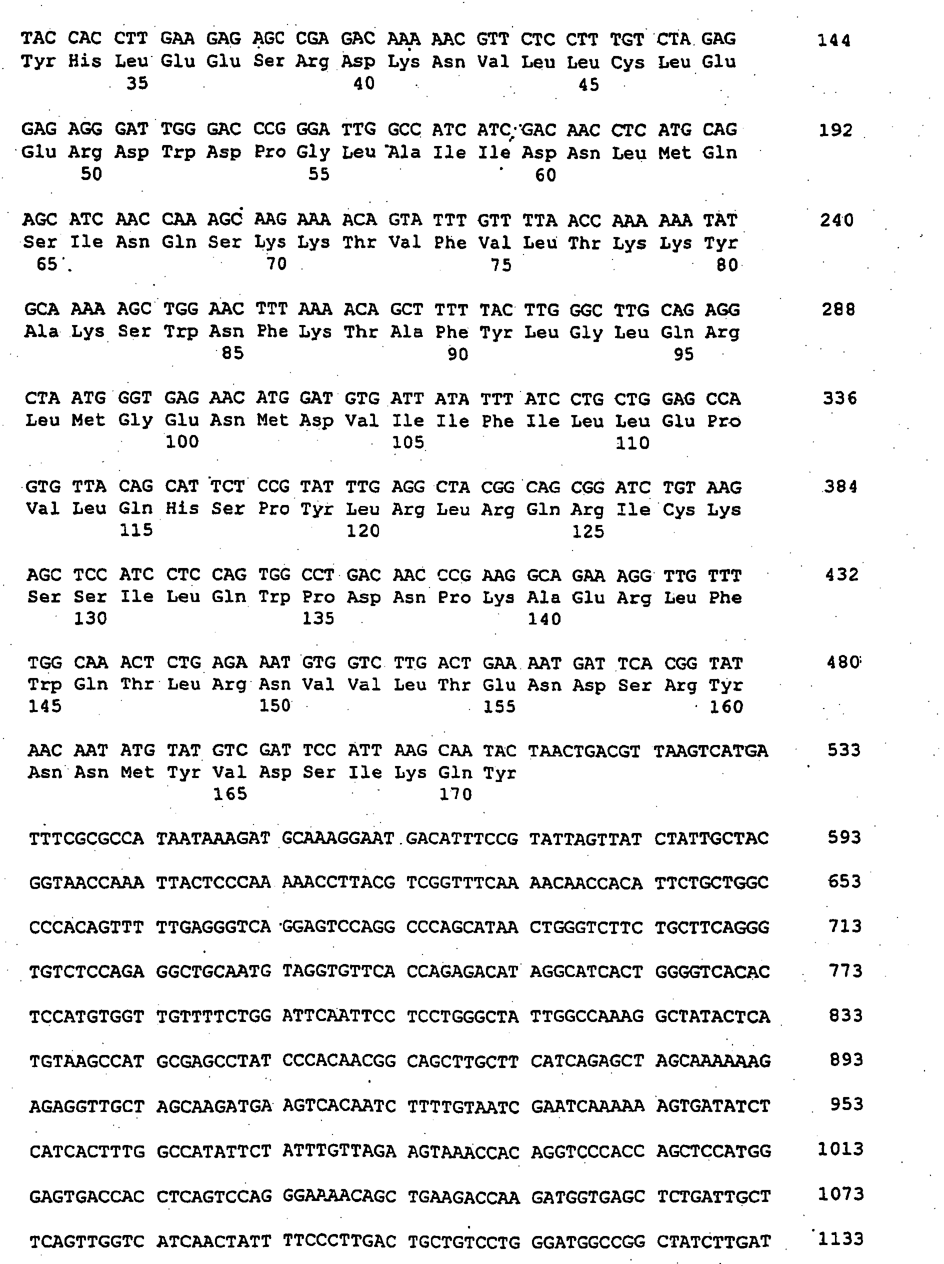

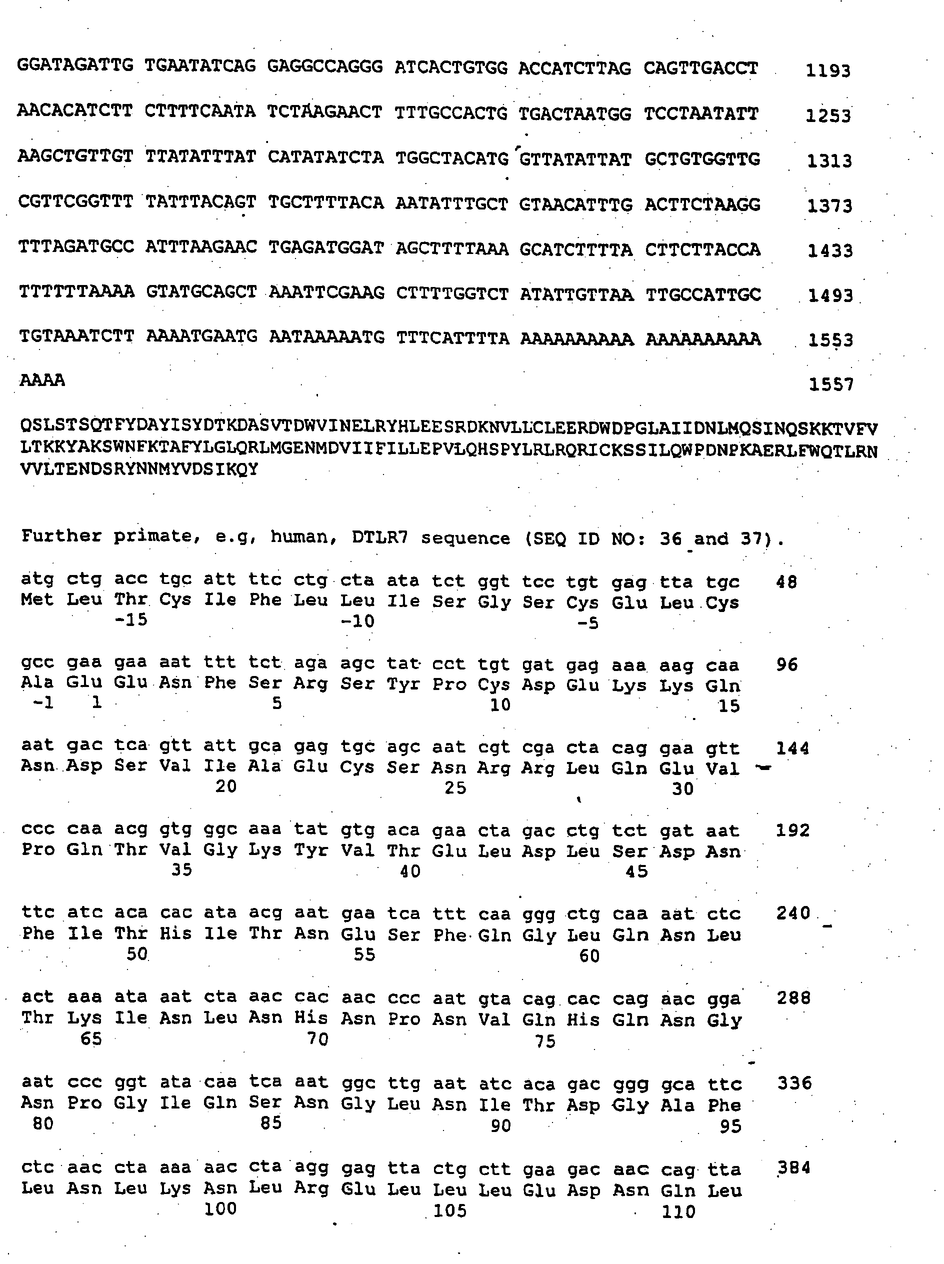

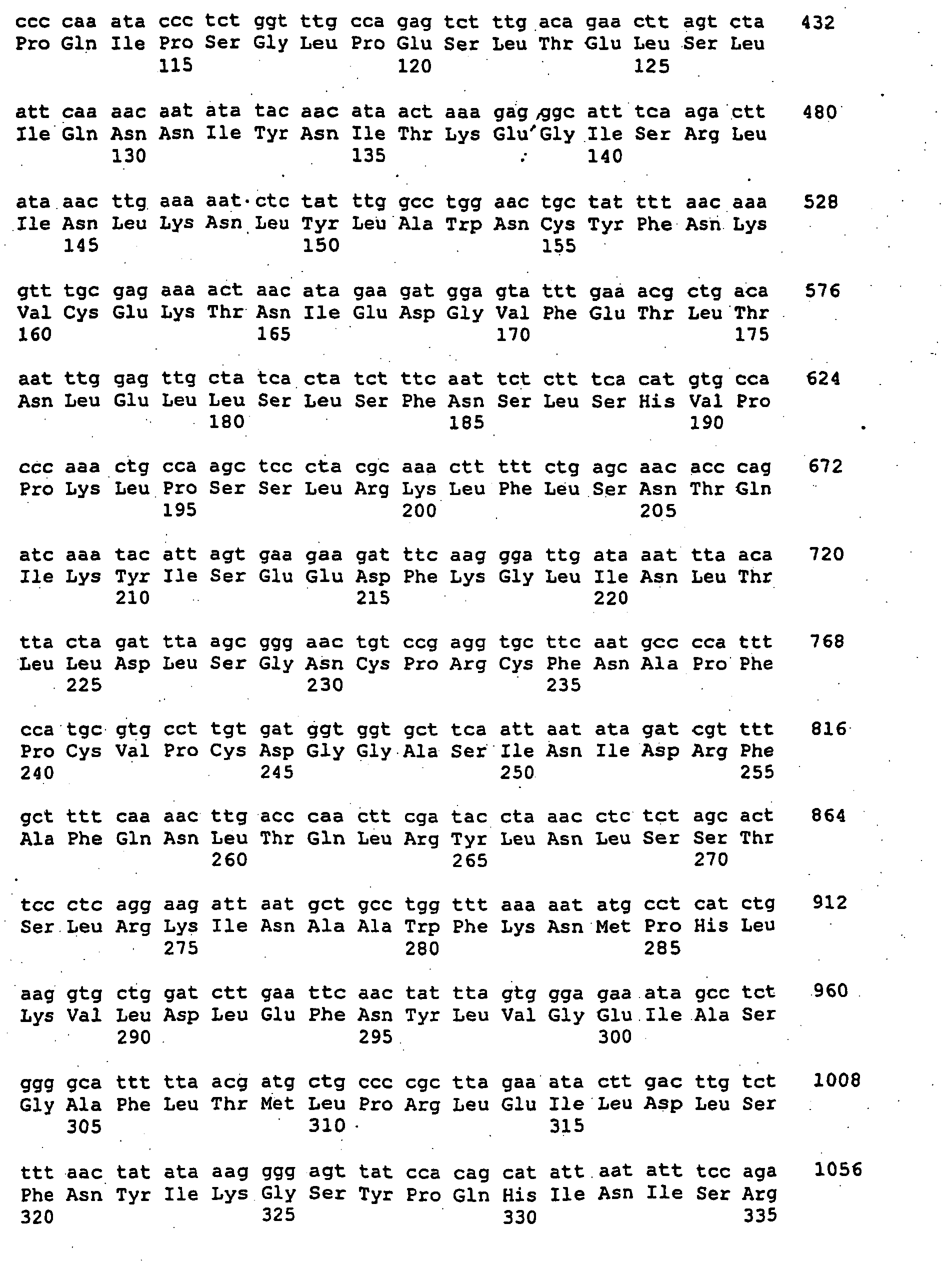

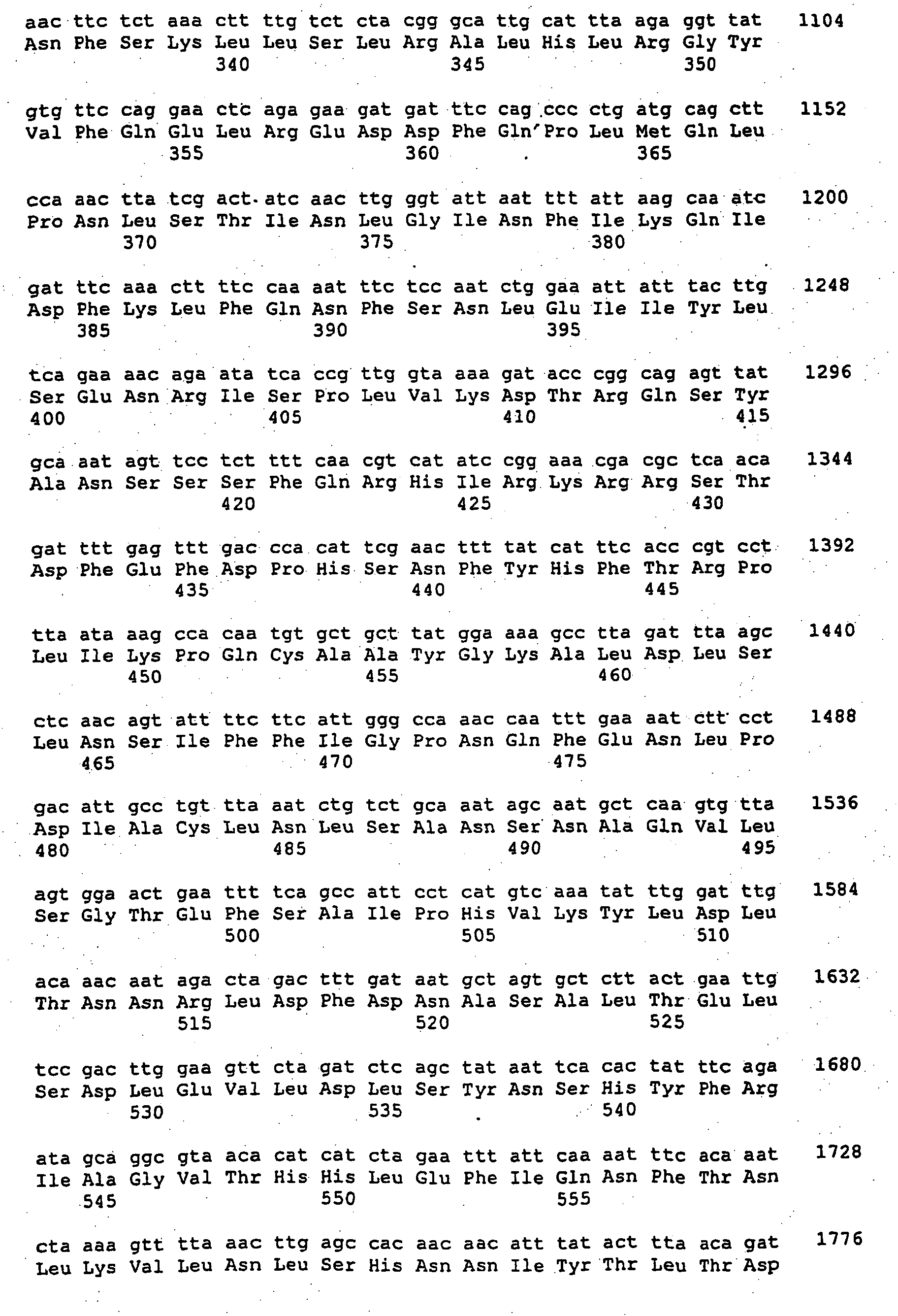

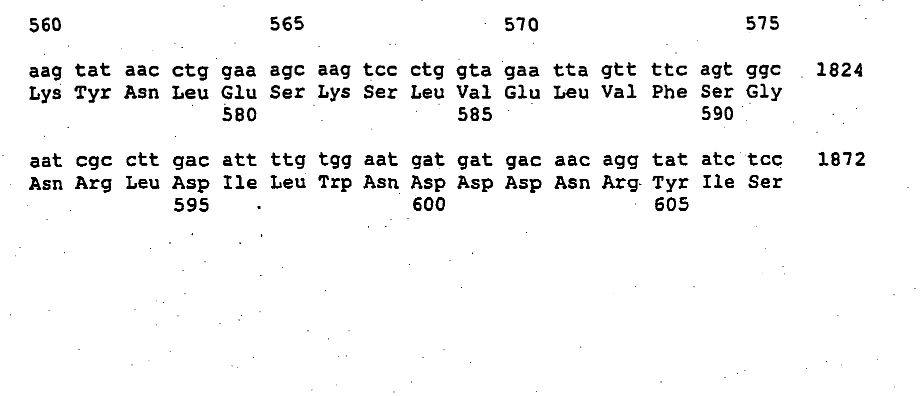

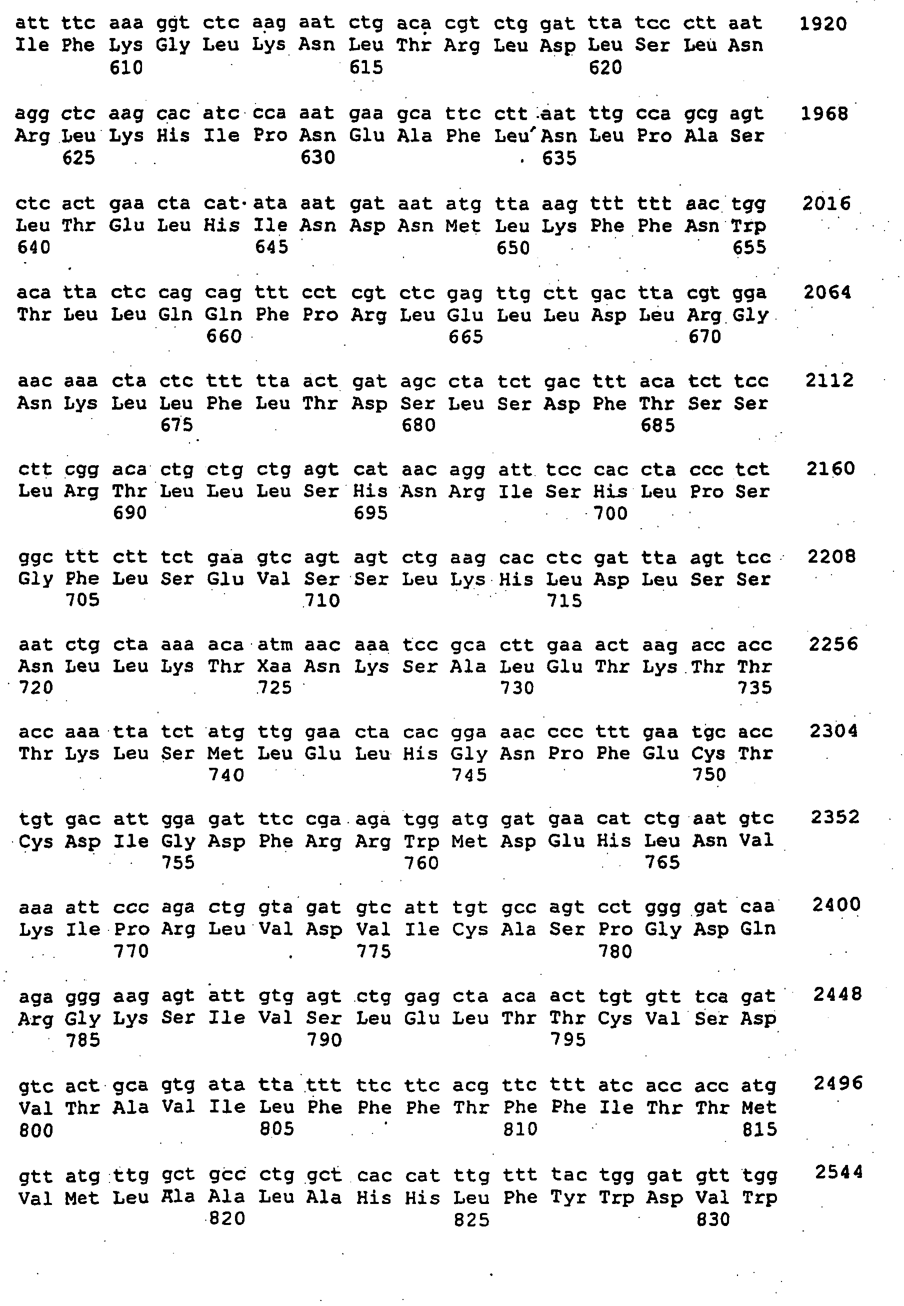

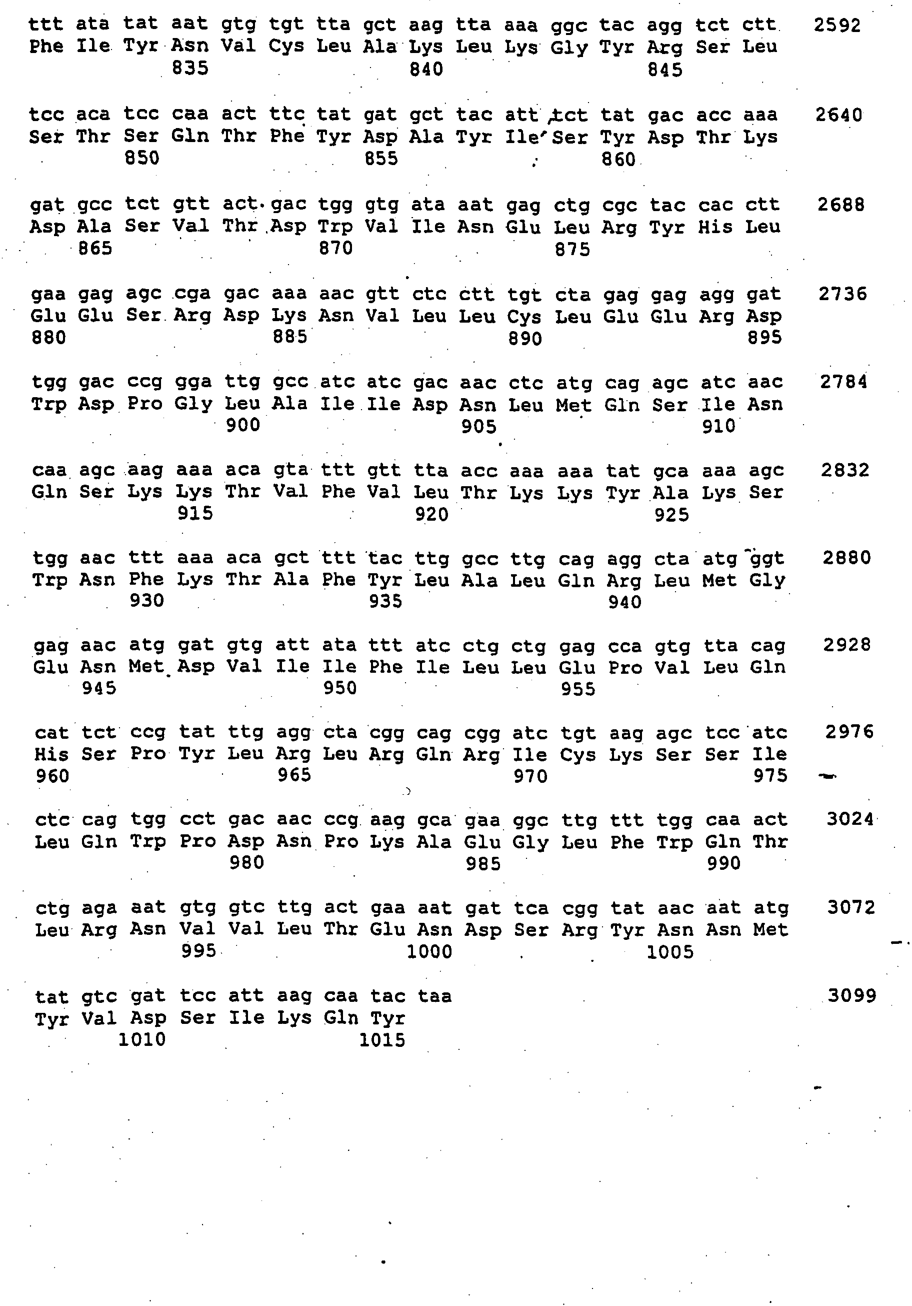

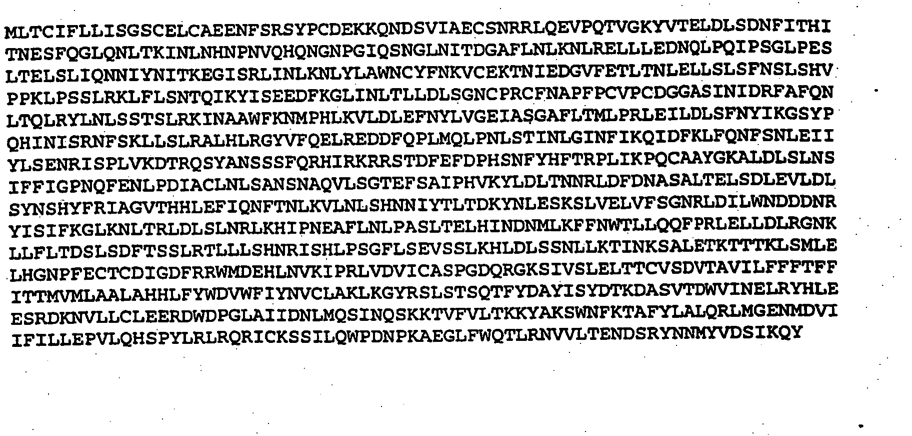

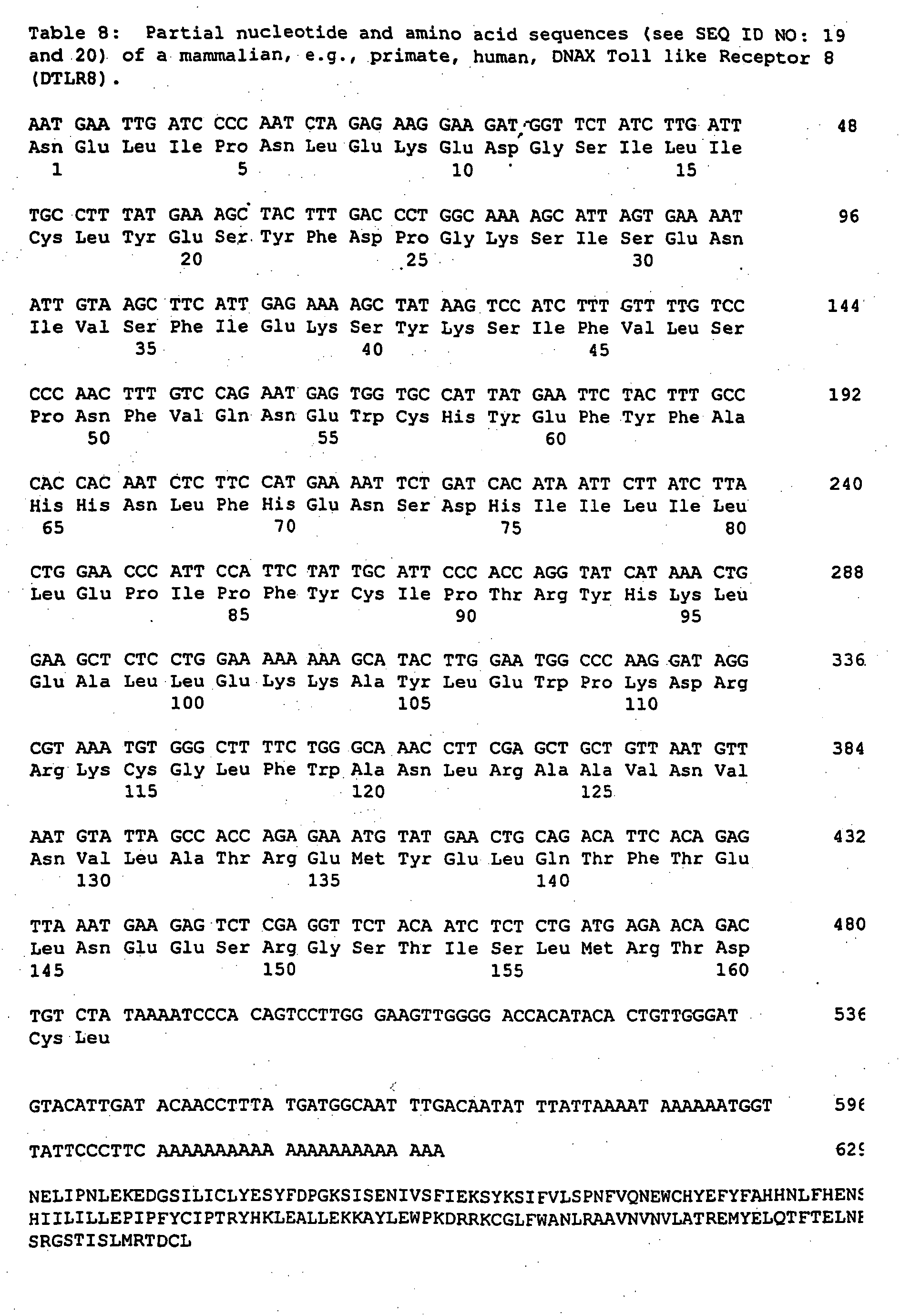

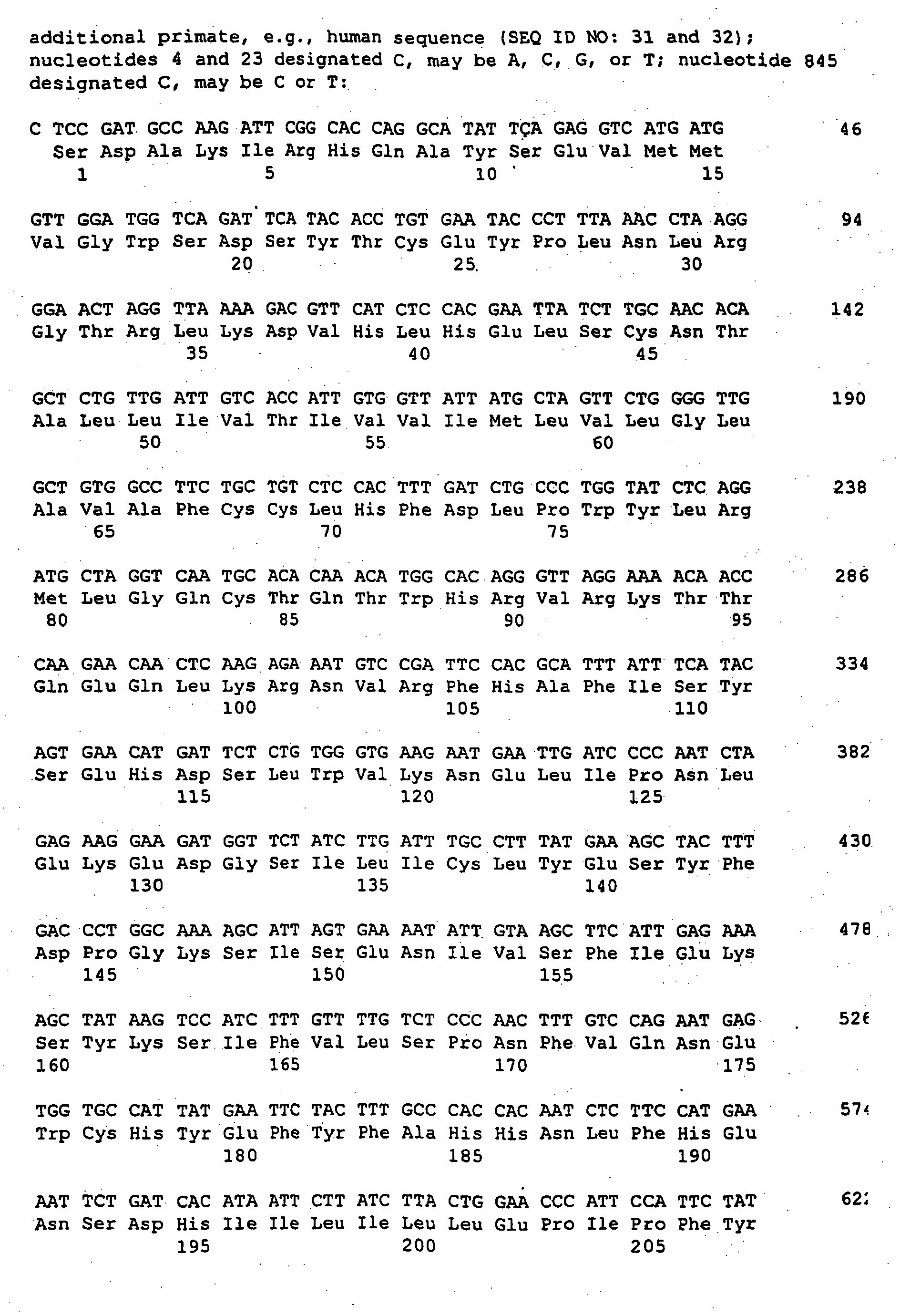

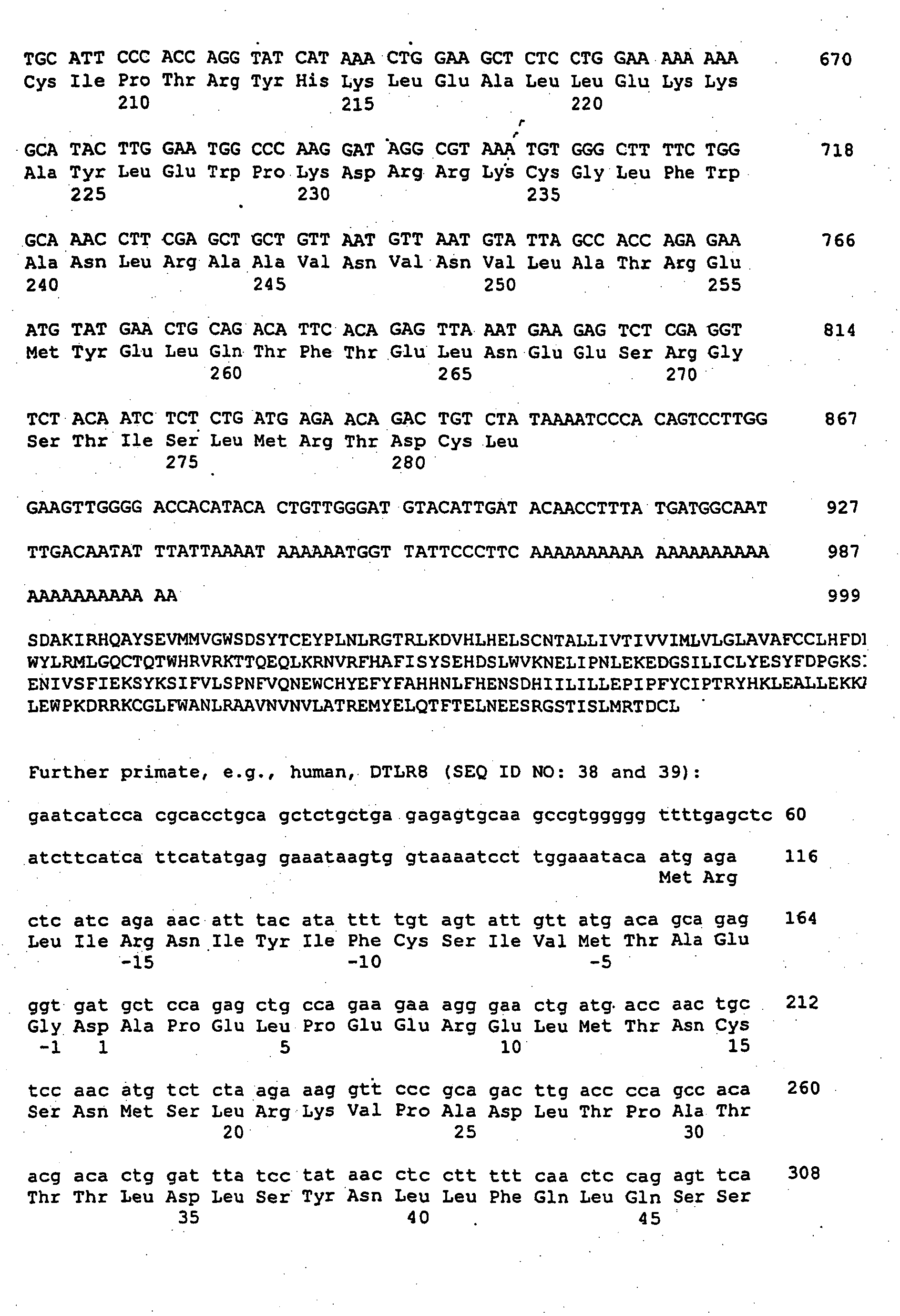

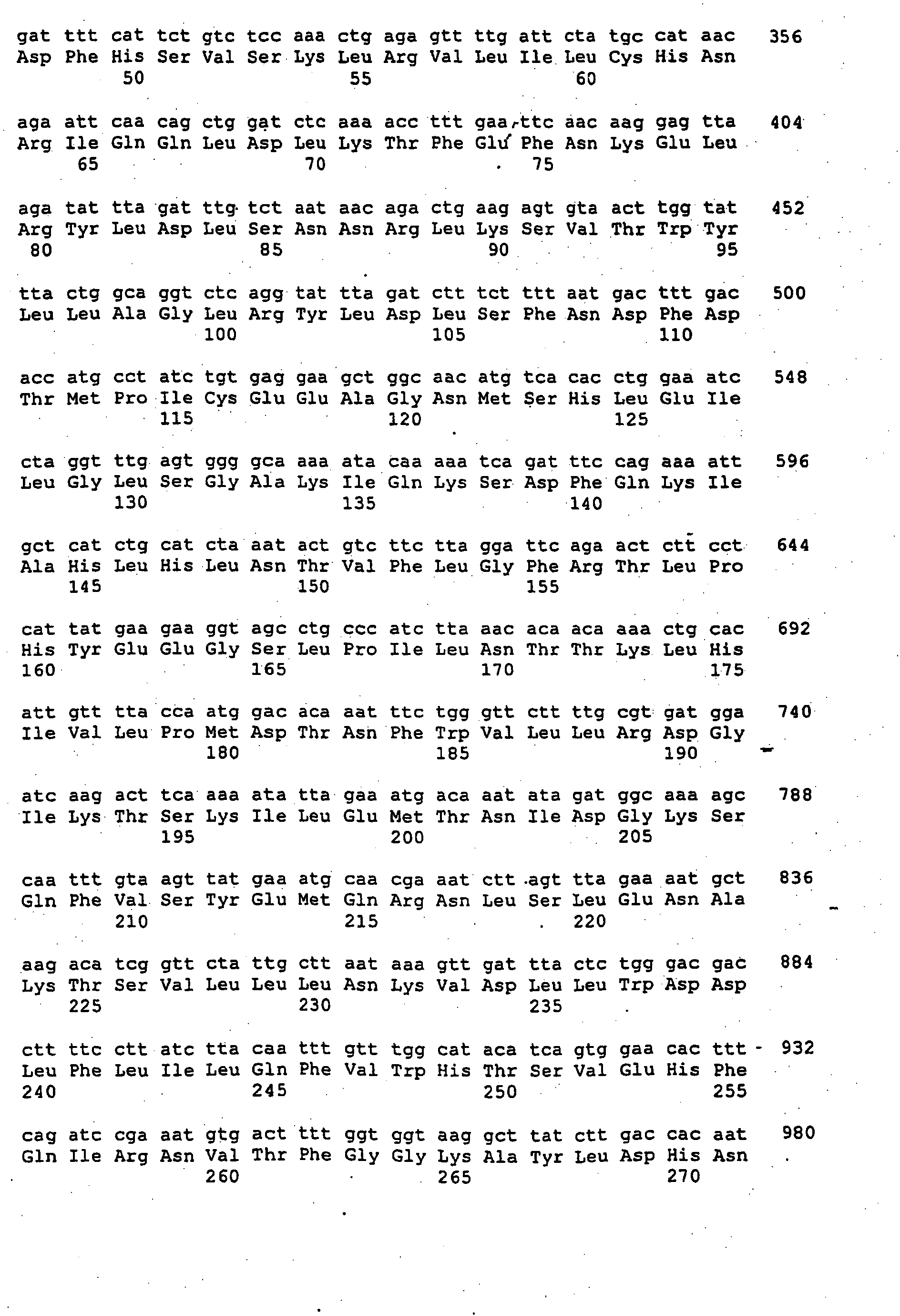

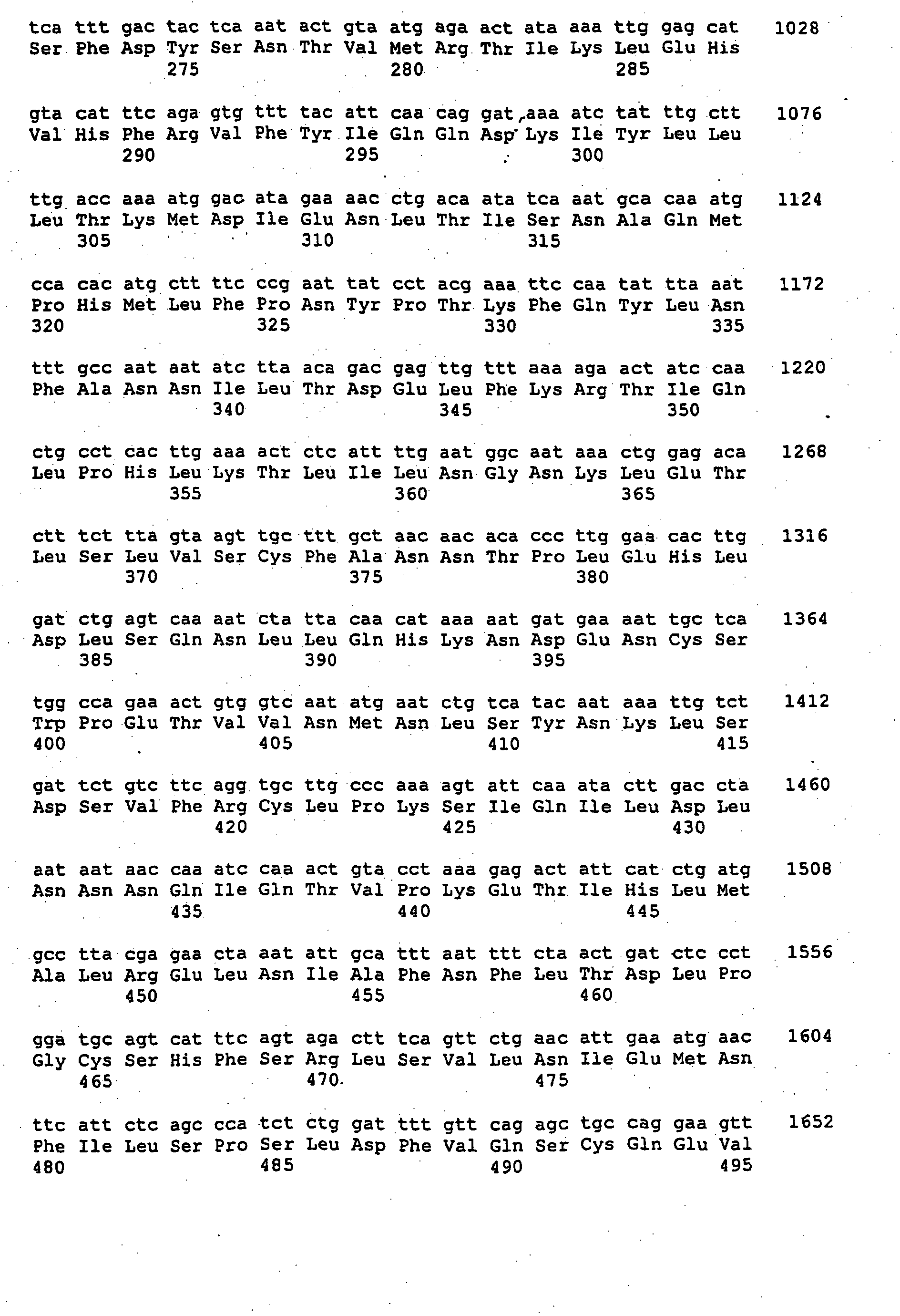

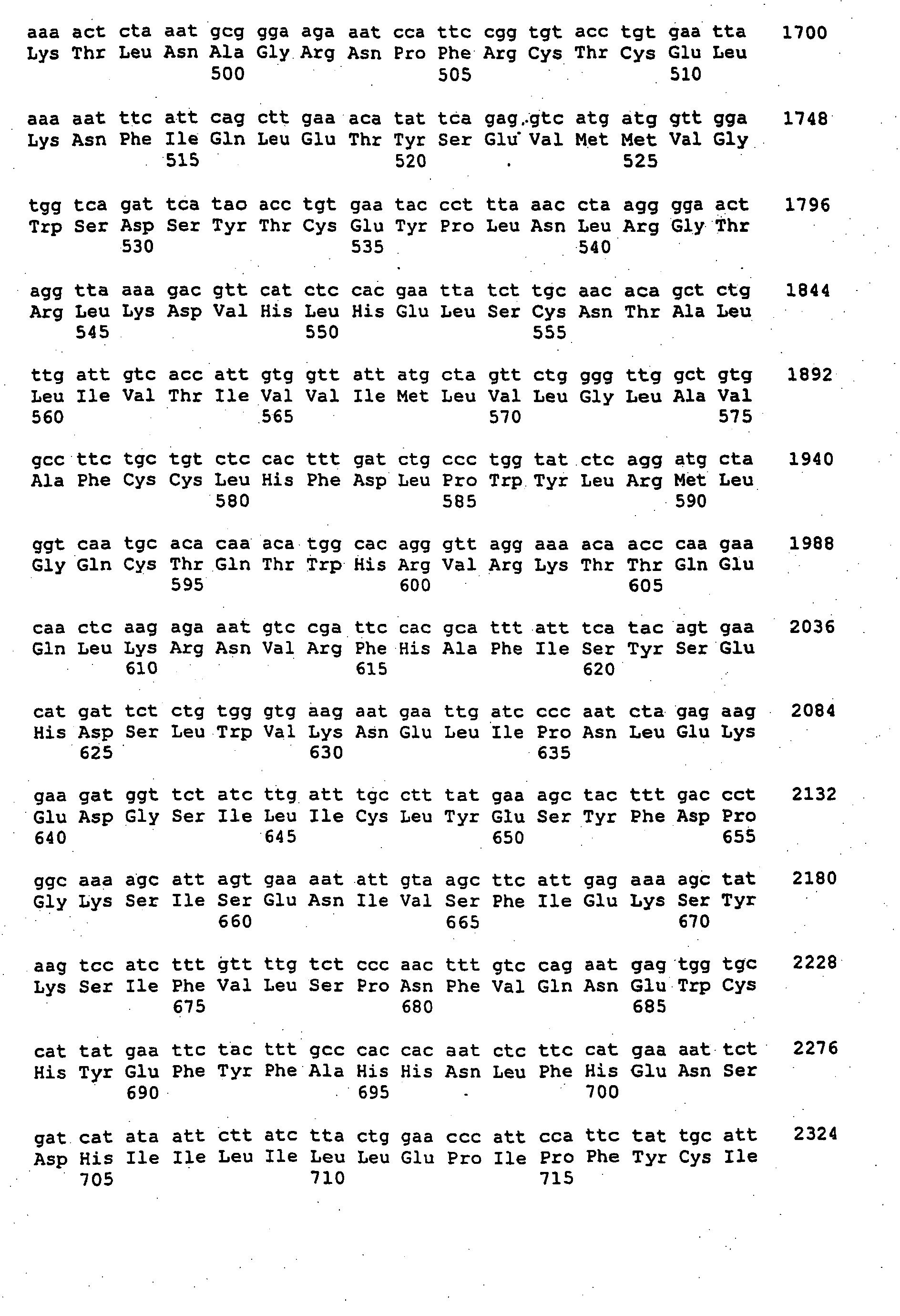

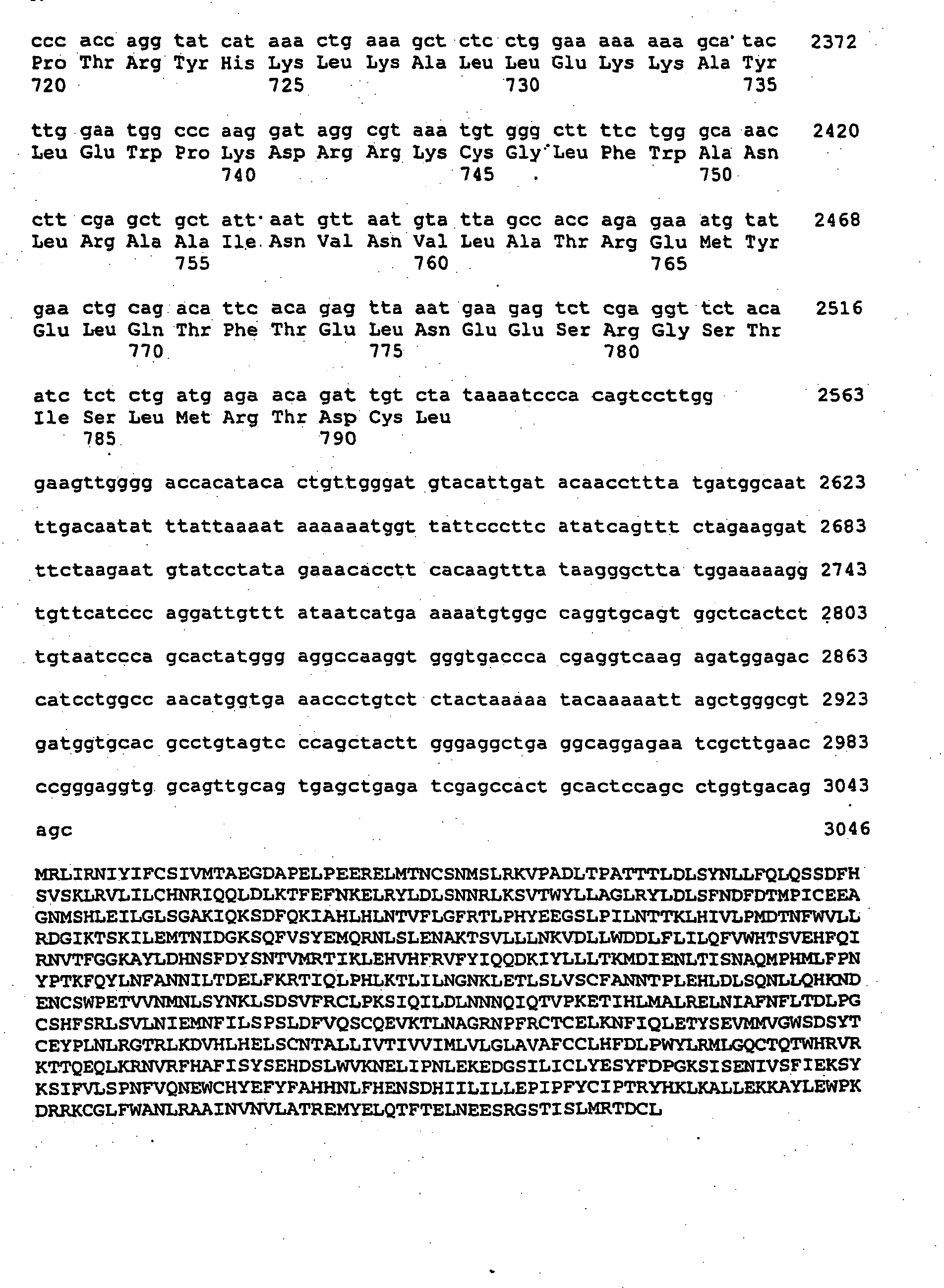

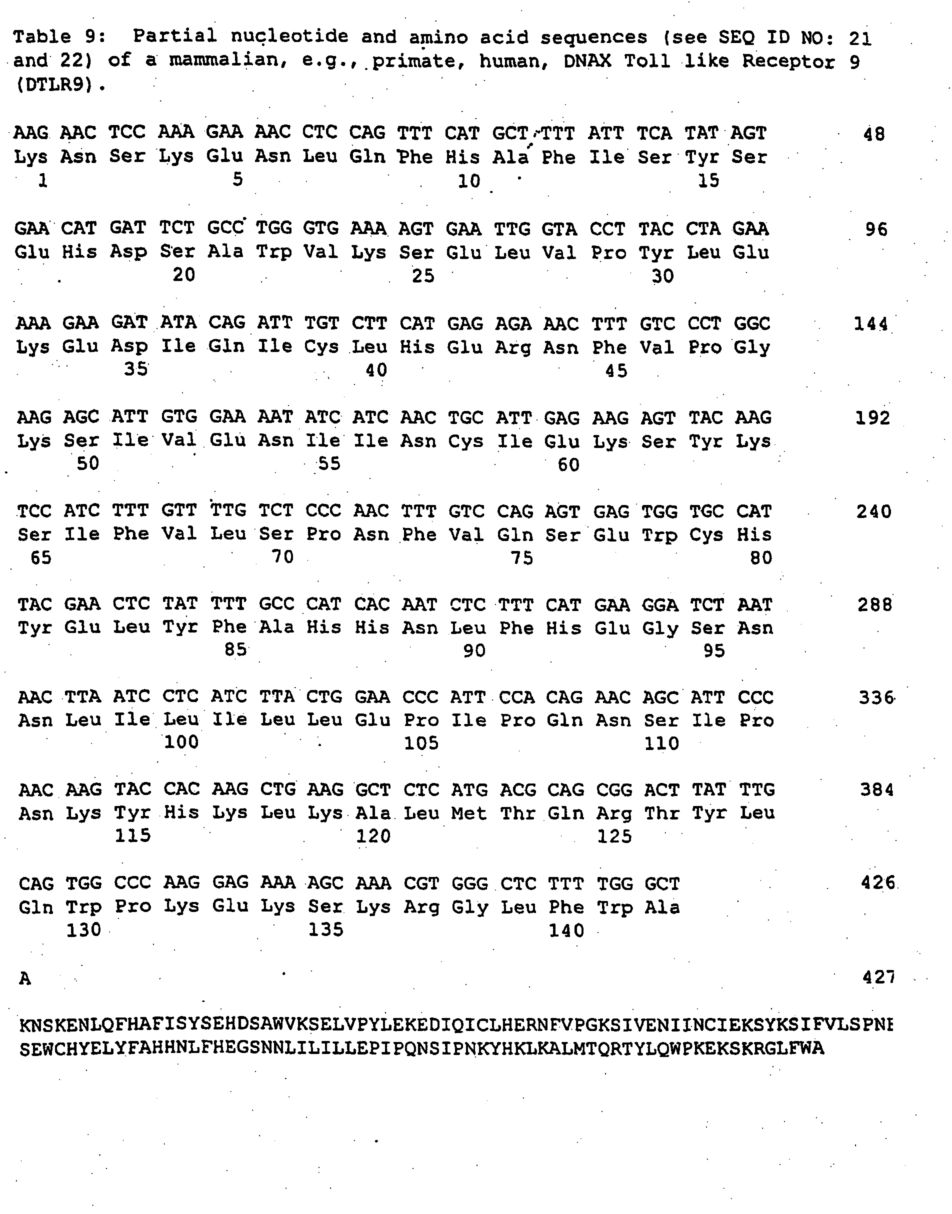

Further primate, e.g., human DTLR9 (SEQ ID NO: 40 and 41):

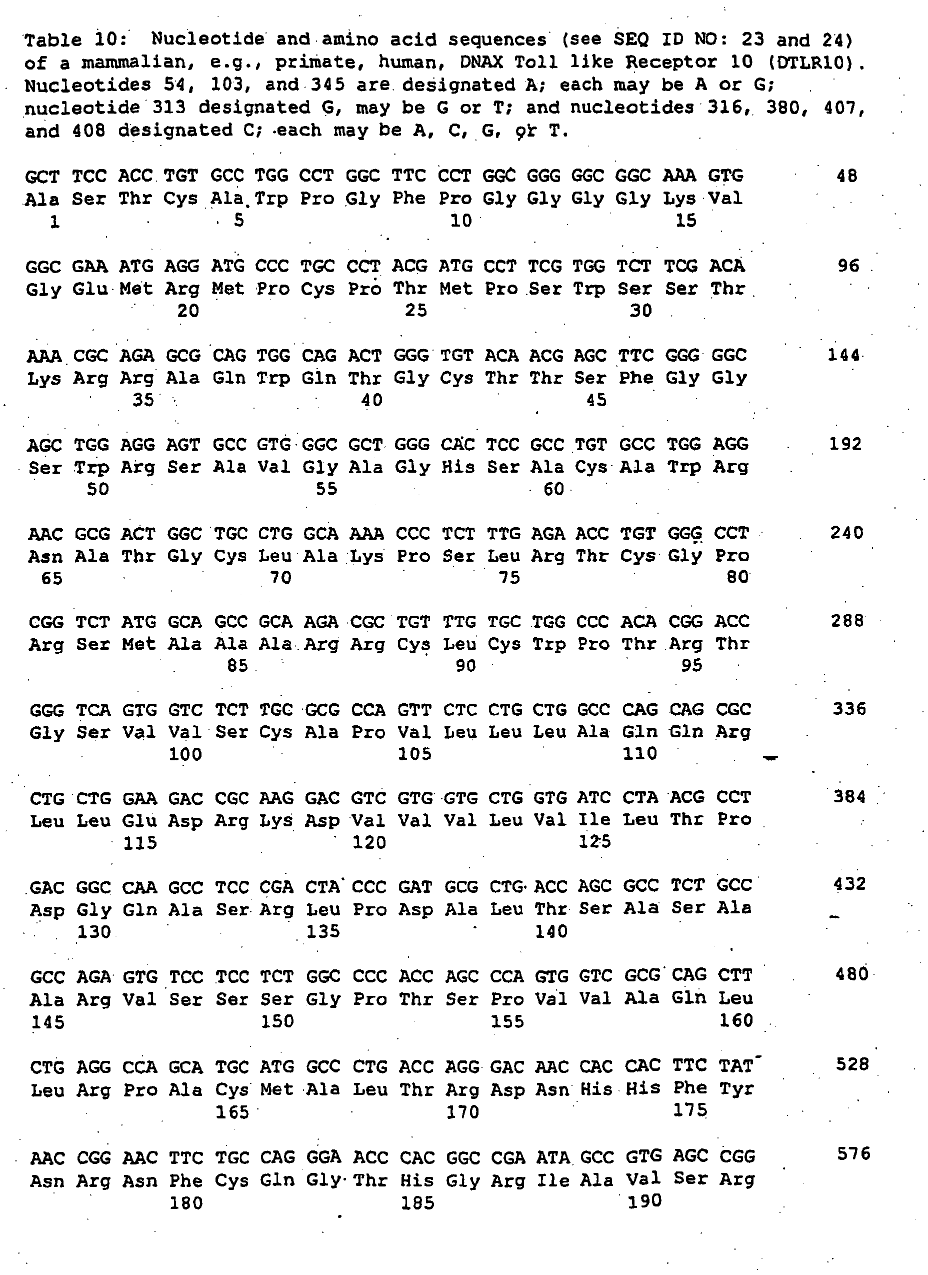

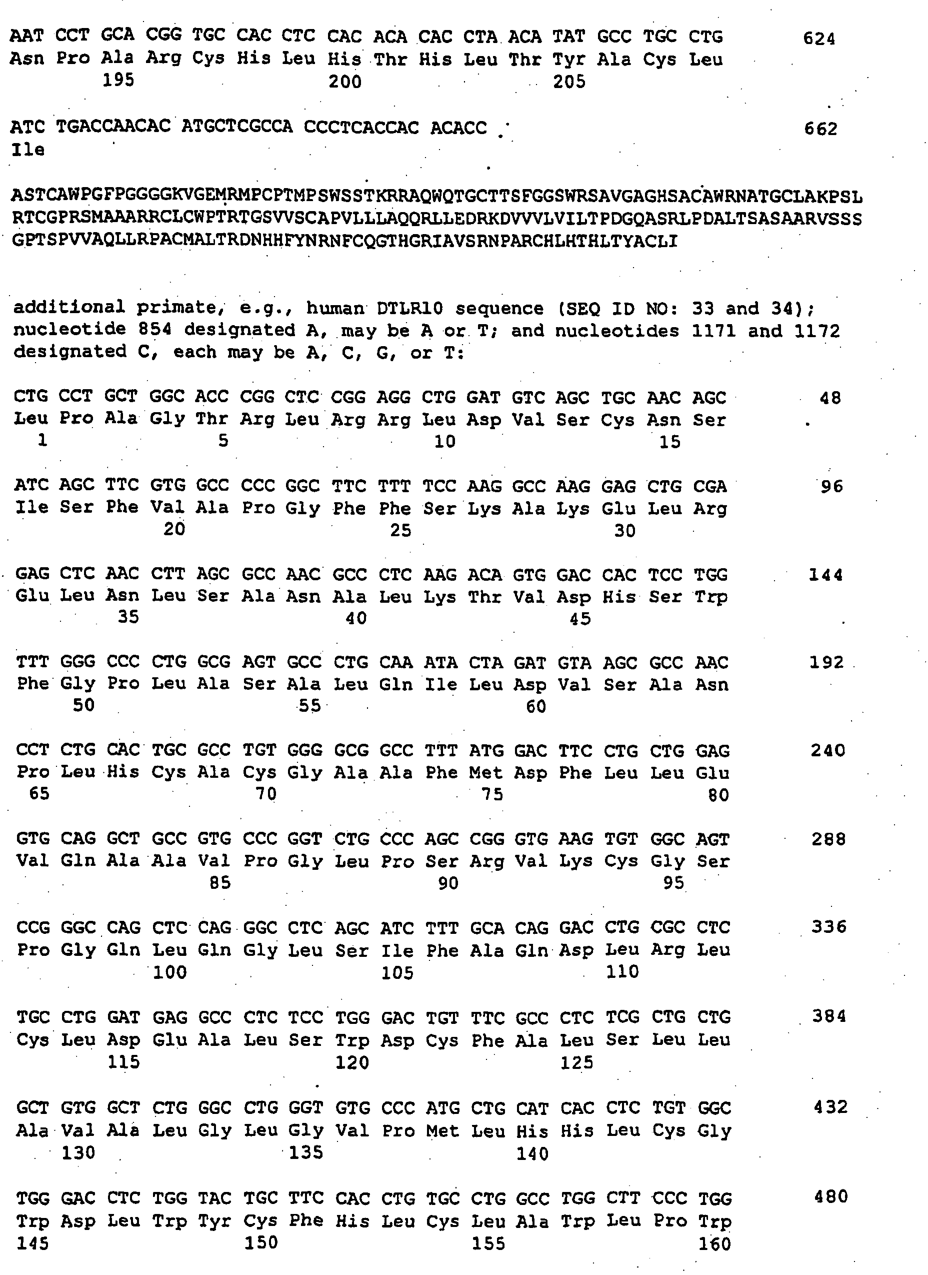

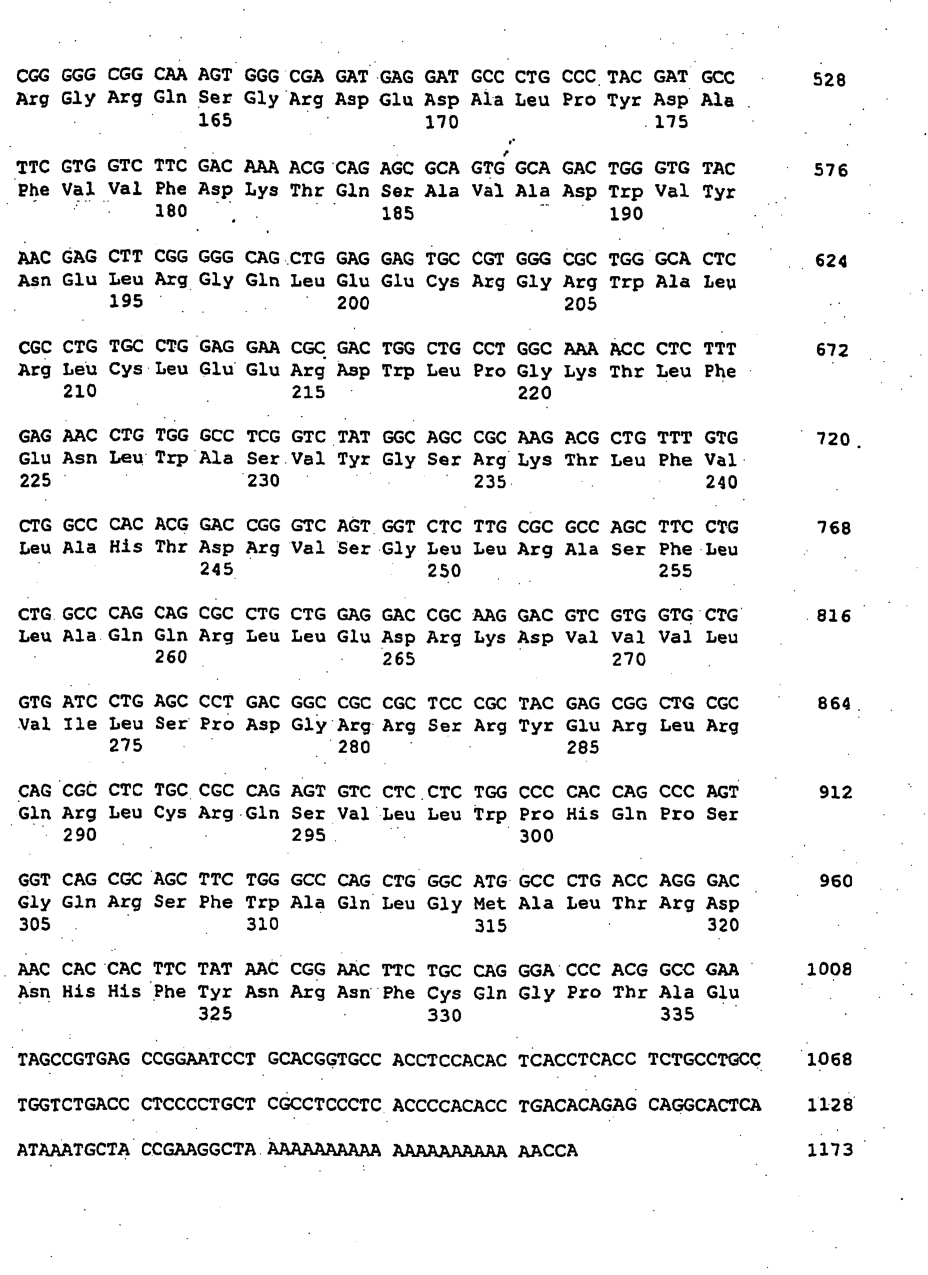

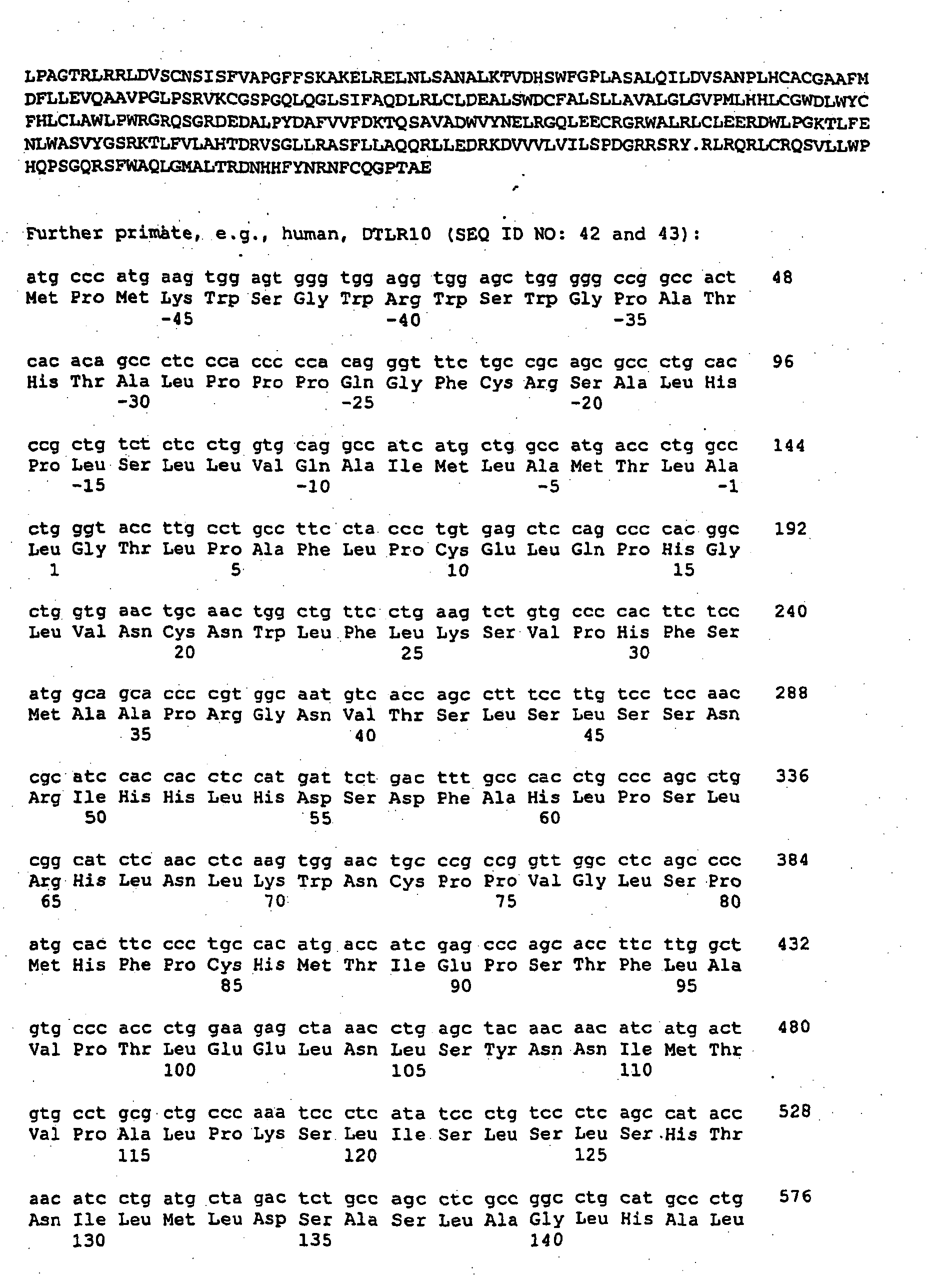

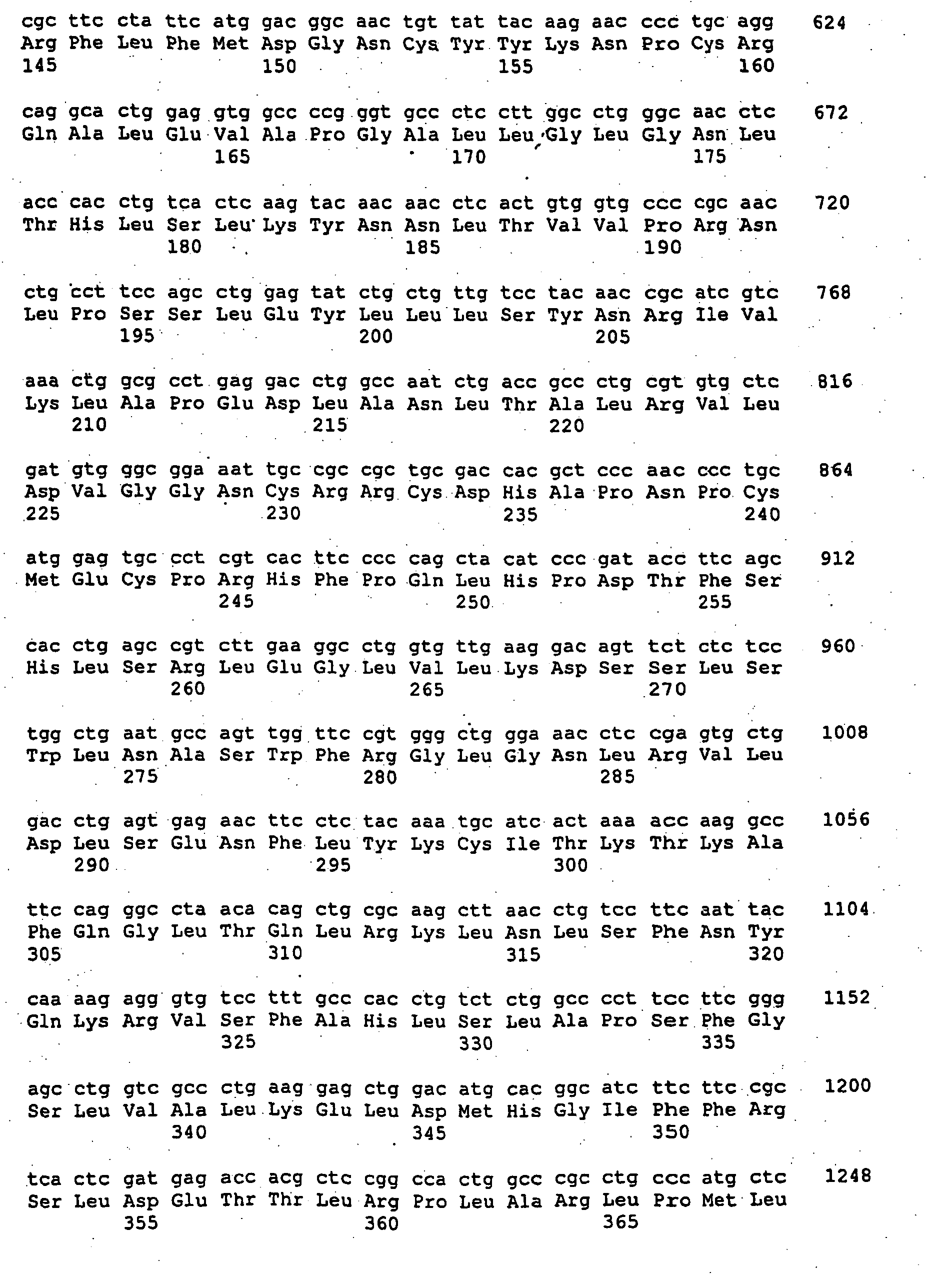

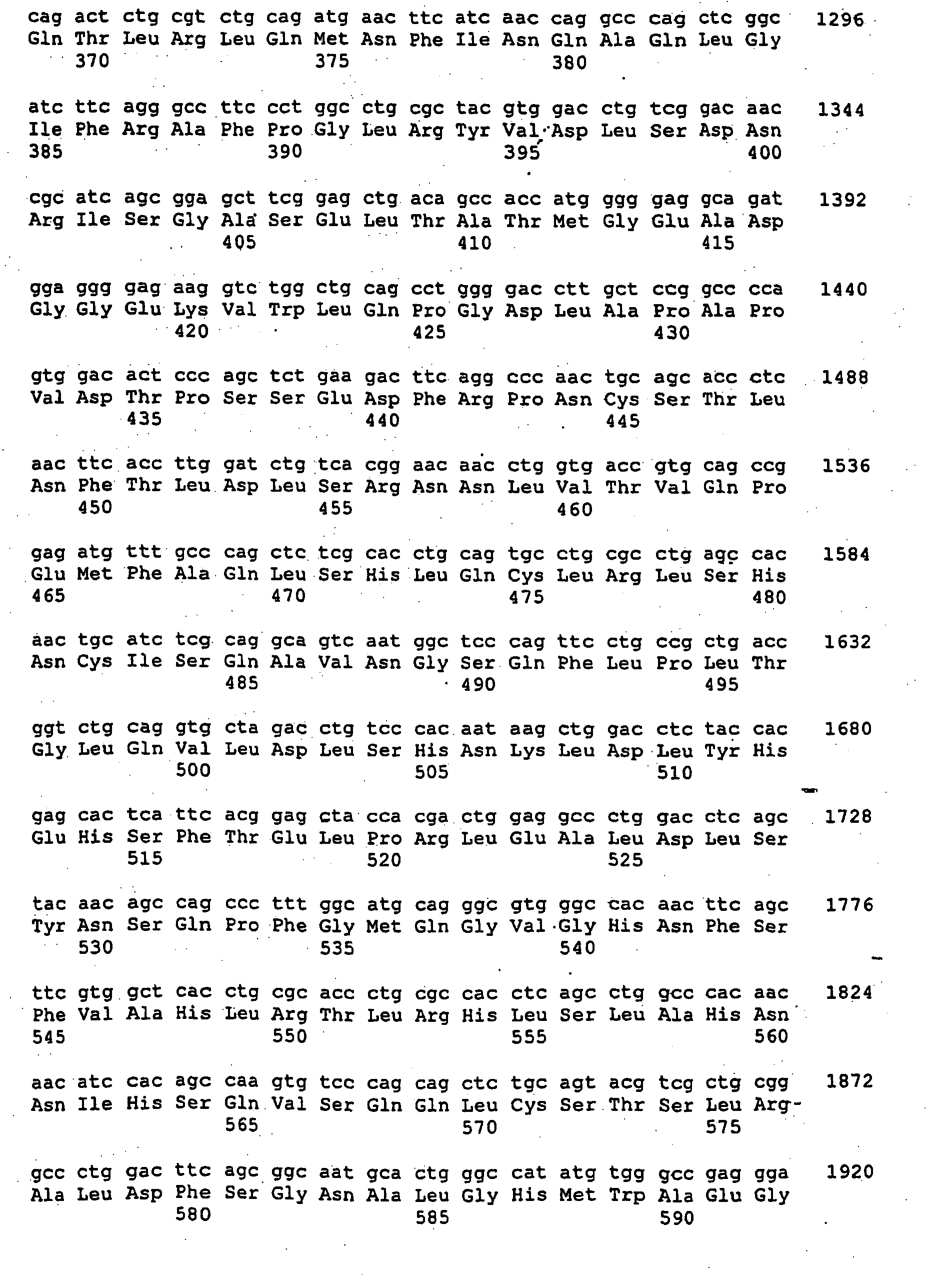

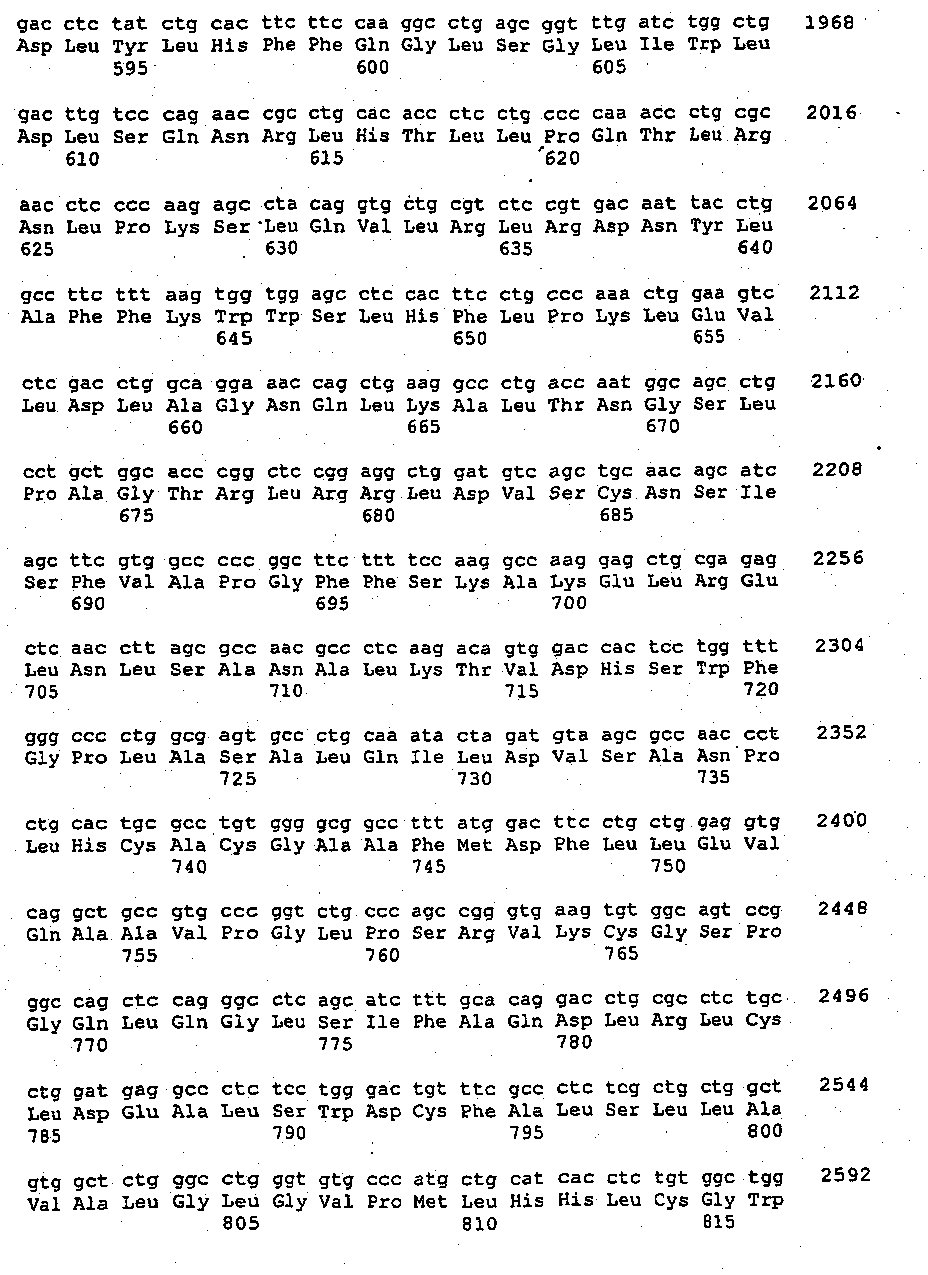

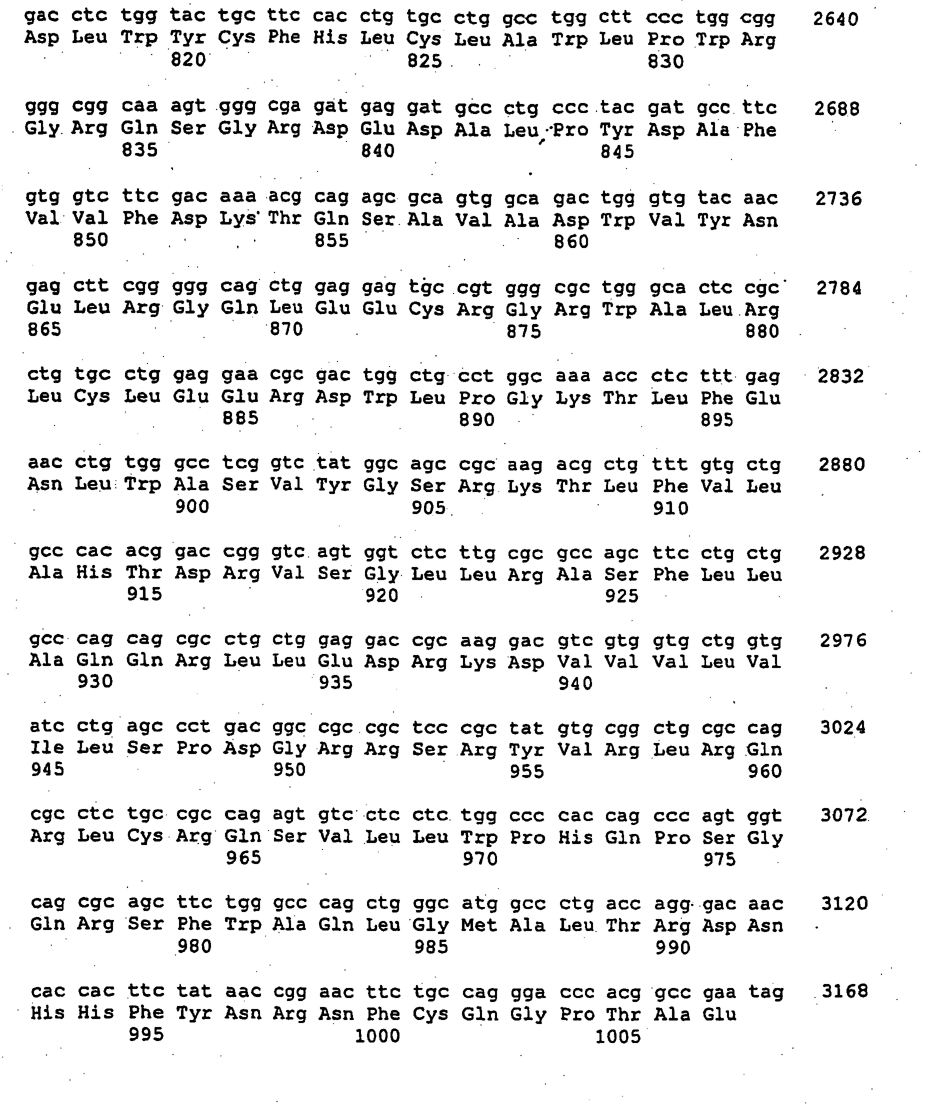

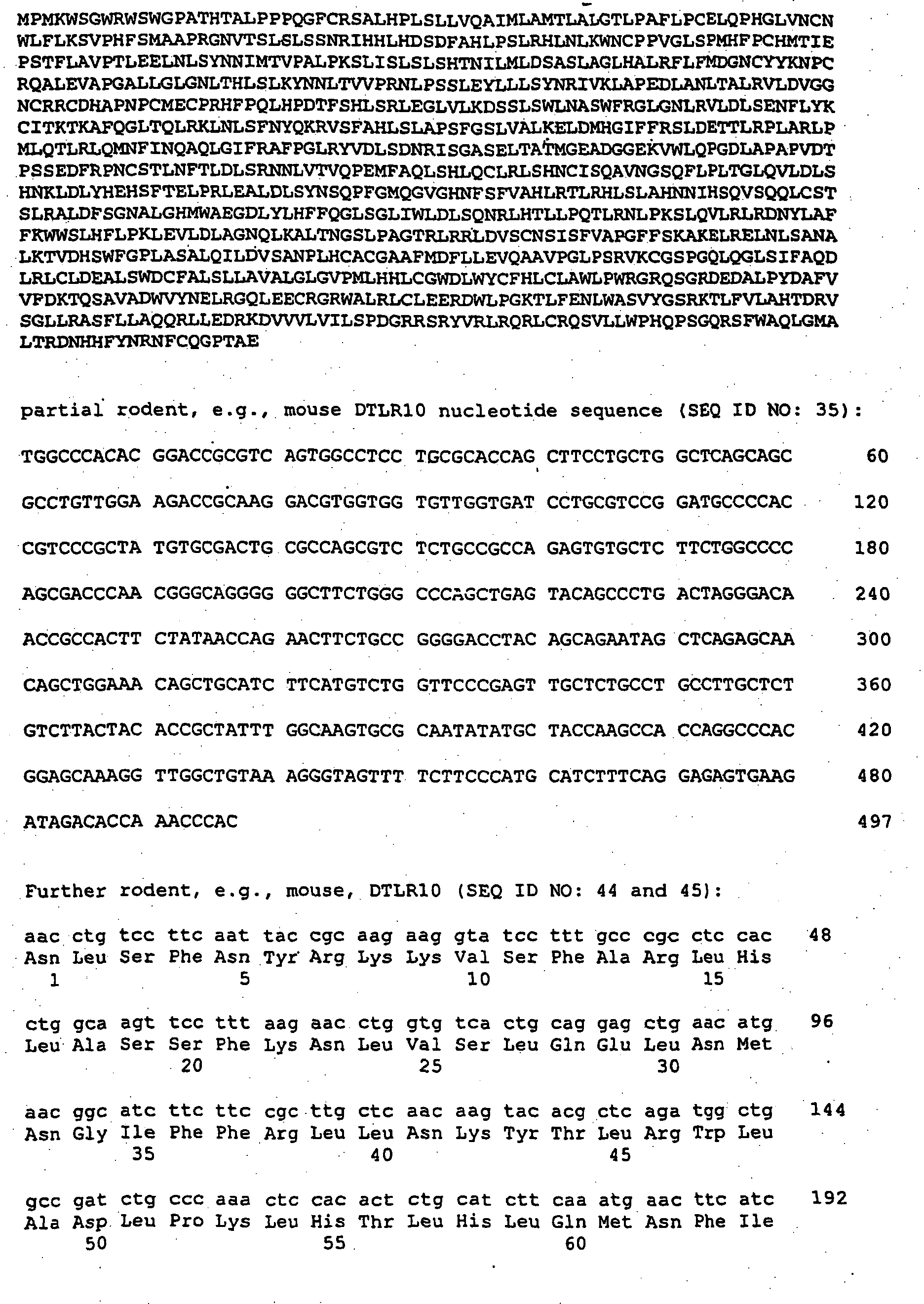

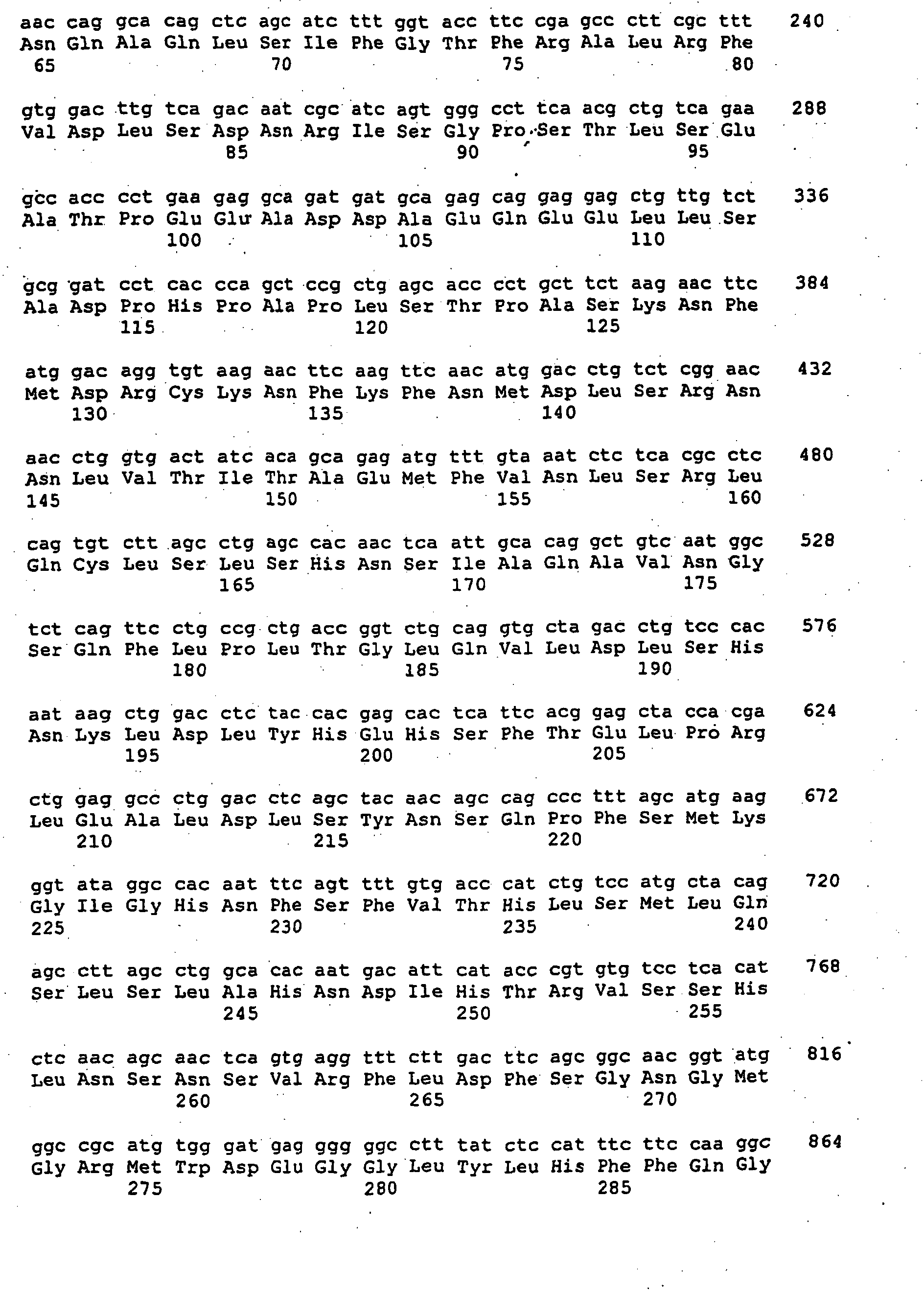

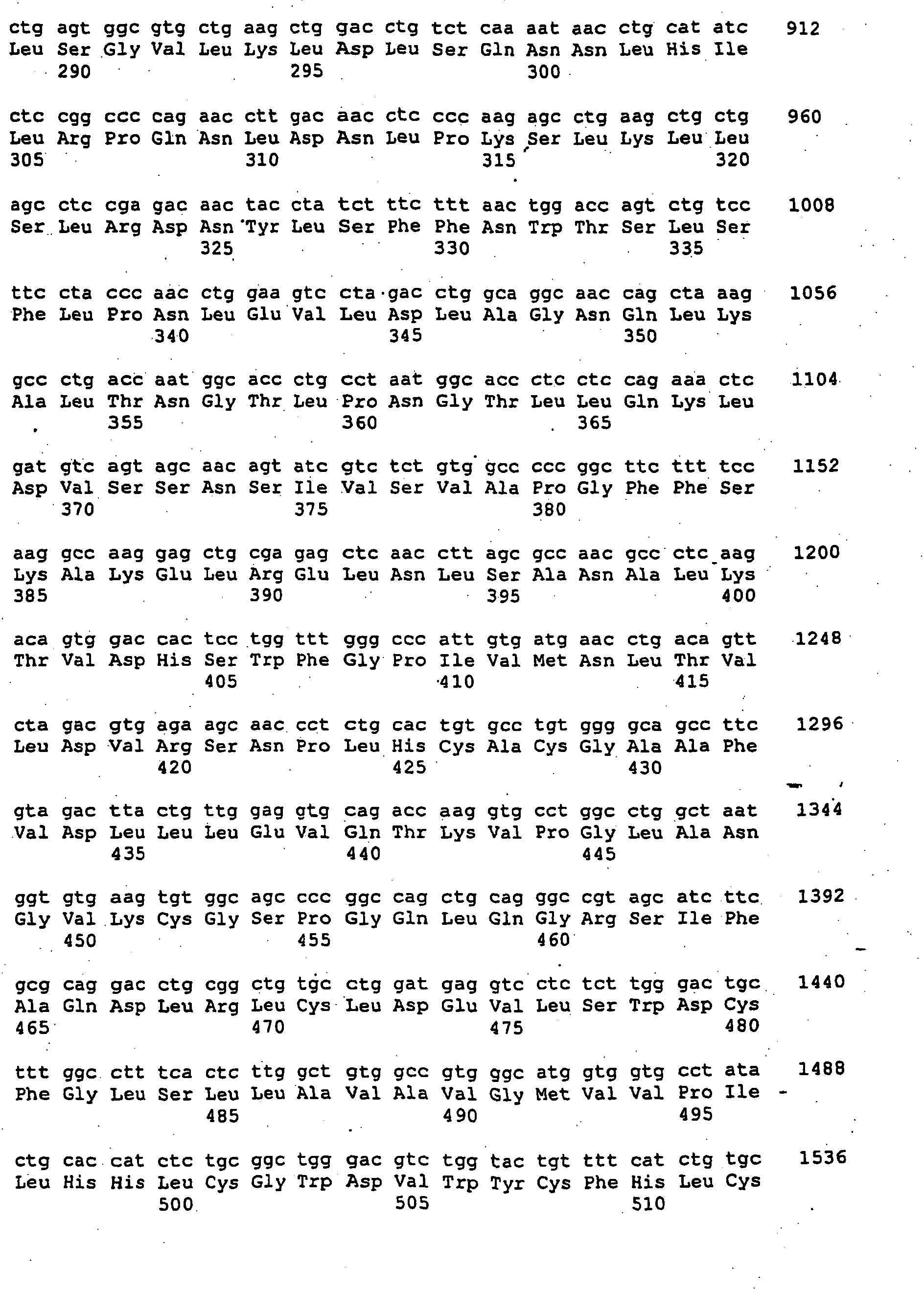

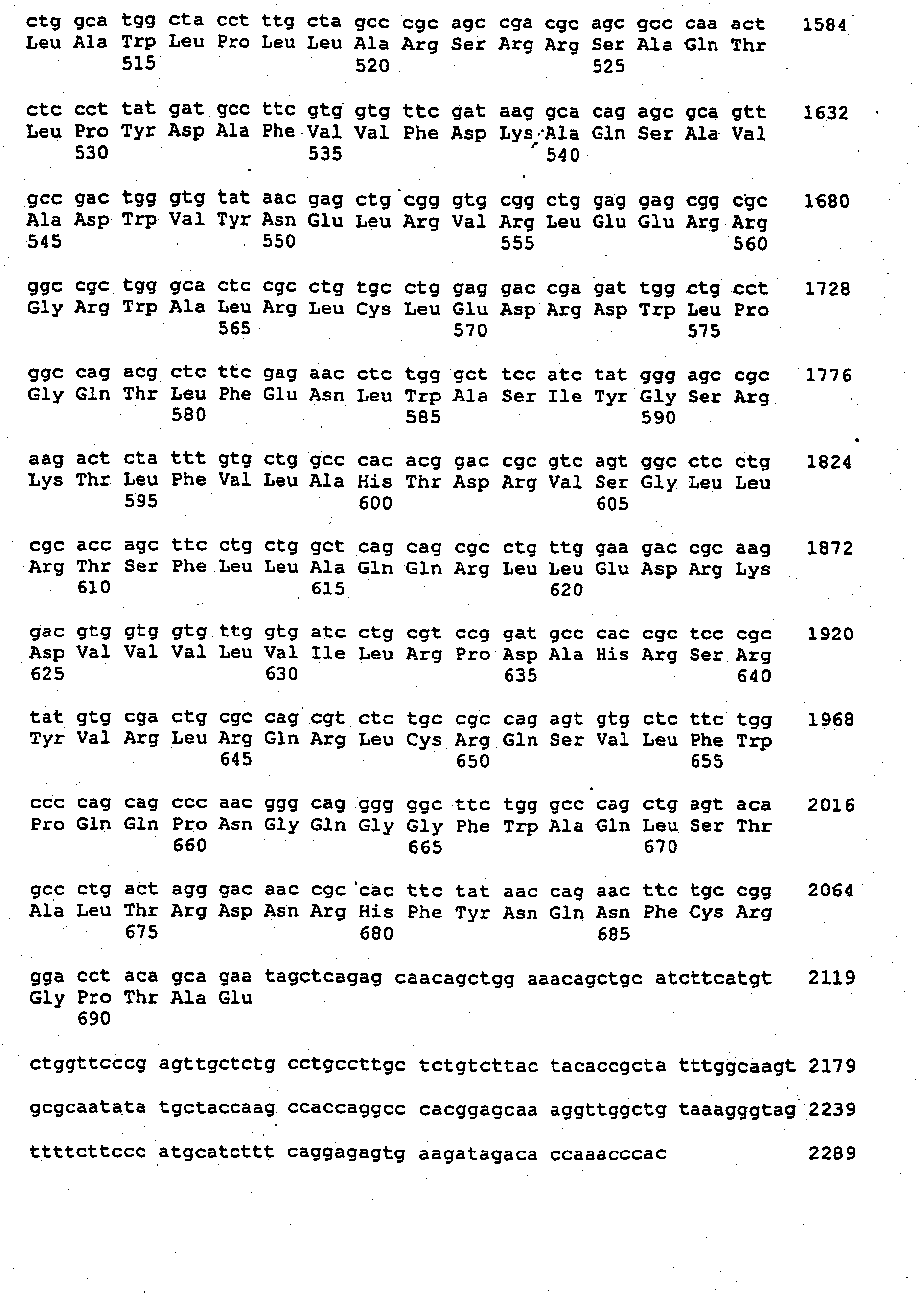

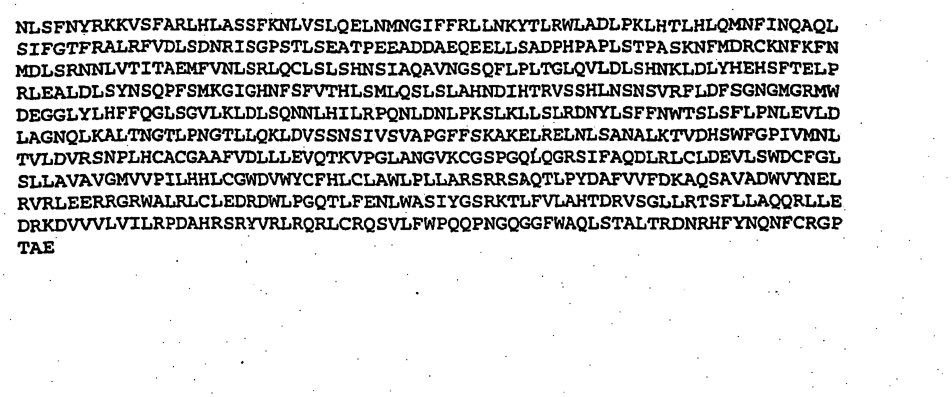

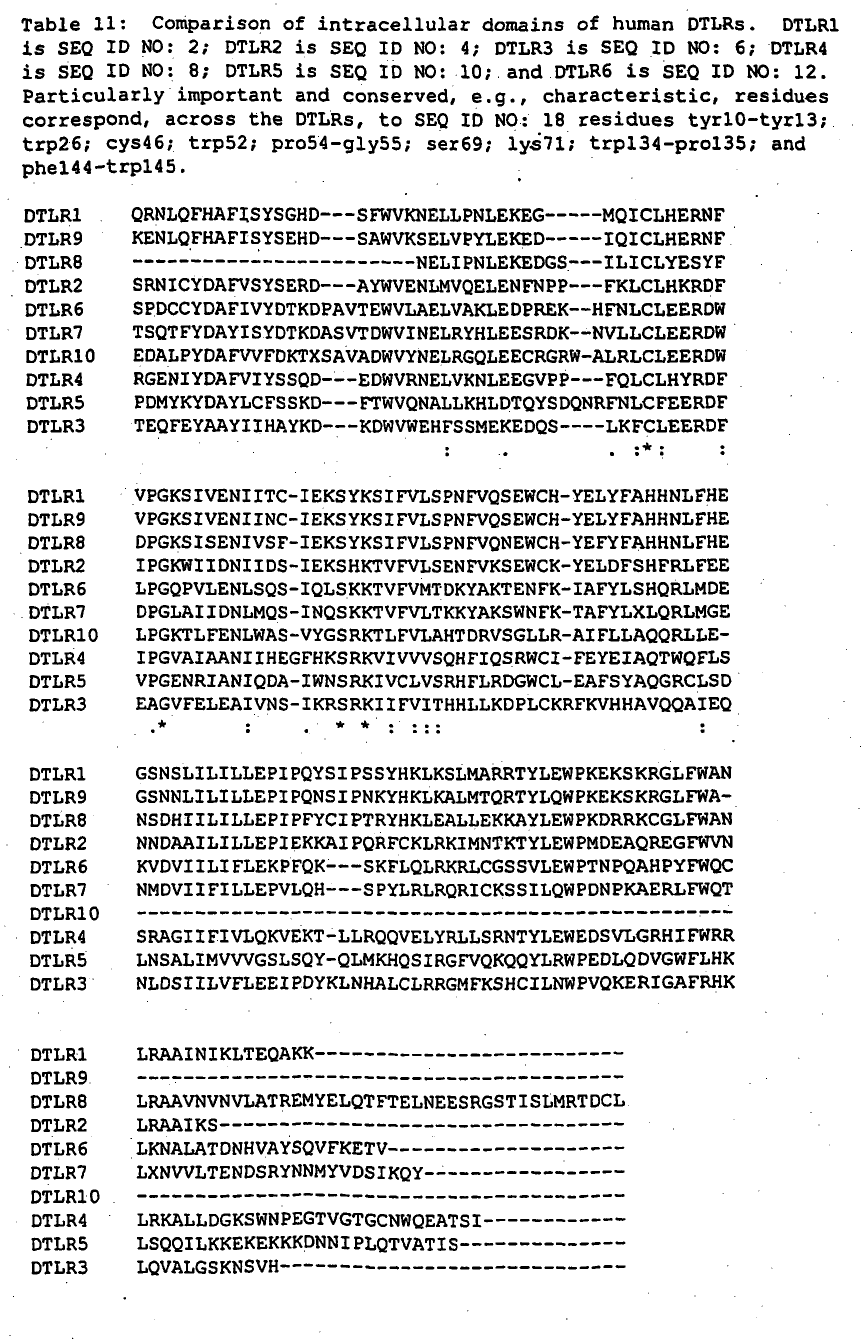

aagaatttgg actcatatca agatgctctg aagaagaaca accctttagg atagccactg 60

caacatc atg acc aaa gac aaa gaa cct att gtt aaa agc ttc cat ttt 109
Met Thr Lys Asp Lys Glu Pro Ile Val Lys Ser Phe His Phe
-30 -25 -20

gtt tgc ctt atg atc ata ata gtt gga acc aga atc cag ttc tcc gac 157
Val Cys Leu Met Ile Ile Ile Val Gly Thr Arg Ile Gln Phe Ser Asp
-15 -10 -5

gga aat gaa ttt gca gta gac aag tca aaa aga ggt ctt att cat gtt 205
Gly Asn Glu Phe Ala Val Asp Lys Ser Lys Arg Gly Leu Ile His Val
-1 1 5 10 15

cca aaa gac cta ccg ctg aaa acc aaa gtc tta gat atg tct cag aac 253
Pro Lys Asp Leu Pro Leu Lys Thr Lys Val Leu Asp Met Ser Gln Asn
20 25 30

tac atc gct gag ctt cag gtc tct gac atg agc ttt cta tca gag ttg 301
Tyr Ile Ala Glu Leu Gln Val Ser Asp Met Ser Phe Leu Ser Glu Leu
35 40 45

aca gtt ttg aga ctt tcc cat aac aga atc cag cta ctt gat tta agt 349
Thr Val Leu Arg Leu Ser His Asn Arg Ile Gln Leu Leu Asp Leu Ser
50 55 60

gtt ttc aag ttc aac cag gat tta gaa tat ttg gat tta tct cat aat 397
Val Phe Lys Phe Asn Gln Asp Leu Glu Tyr Leu Asp Leu Ser His Asn
65 70 75

cag ttg caa aag ata tcc tgc cat cct att gtg agt ttc agg cat tta 445
Gln Leu Gln Lys Ile Ser Cys His Pro Ile Val Ser Phe Arg His Leu
80 85 90 95

gat ctc tca ttc aat gat ttc aag gcc ctg ccc atc tgt aag gaa ttt 493
Asp Leu Ser Phe Asn Asp Phe Lys Ala Leu Pro Ile Cys Lys Glu Phe
100 105 110

ggc aac tta tca caa ctg aat ttc ttg gga ttg agt gct atg aag ctg 541
Gly Asn Leu Ser Gln Leu Asn Phe Leu Gly Leu Ser Ala Met Lys Leu
115 120 125

caa aaa tta gat ttg ctg cca att gct cac ttg cat cta agt tat atc 589
Gln Lys Leu Asp Leu Leu Pro Ile Ala His Leu His Leu Ser Tyr Ile
130 135 140

ctt ctg gat tta aga aat tat tat ata aaa gaa aat gag aca gaa agt 637
Leu Leu Asp Leu Arg Asn Tyr Tyr Ile Lys Glu Asn Glu Thr Glu Ser
145 150 155

cta caa att ctg aat gca aaa acc ctt cac ctt gtt ttt cac cca act 685
Leu Gln Ile Leu Asn Ala Lys Thr Leu His Leu Val Phe His Pro Thr
160 165 170 175

agt tta ttc gct atc caa gtg aac ata tca gtt aat act tta ggg tgc 733
Ser Leu Phe Ala Ile Gln Val Asn Ile Ser Val Asn Thr Leu Gly Cys
180 185 190

tta caa ctg act aat att aaa ttg aat gat gac aac tgt caa gtt ttc 781
Leu Gln Leu Thr Asn Ile Lys Leu Asn Asp Asp Asn Cys Gln Val Phe
195 200 205

att aaa ttt tta tca gaa ctc acc aga ggt cca acc tta ctg aat ttt 829
Ile Lys Phe Leu Ser Glu Leu Thr Arg Gly Pro Thr Leu Leu Asn Phe
210 215 220

acc ctc aac cac ata gaa acg act tgg aaa tgc ctg gtc aga gtc ttt 877
Thr Leu Asn His Ile Glu Thr Thr Trp Lys Cys Leu Val Arg Val Phe
225 230 235

caa ttt ctt tgg ccc aaa cct gtg gaa tat ctc aat att tac aat tta 925
Gln Phe Leu Trp Pro Lys Pro Val Glu Tyr Leu Asn Ile Tyr Asn Leu
240 245 250 255

aca ata att gaa agc att cgt gaa gaa gat ttt act tat tct aaa acg 973
Thr Ile Ile Glu Ser Ile Arg Glu Glu Asp Phe Thr Tyr Ser Lys Thr
260 265 270

aca ttg aaa gca ttg aca ata gaa cat atc acg aac caa gtt ttt ctg 1021
Thr Leu Lys Ala Leu Thr Ile Glu His Ile Thr Asn Gln Val Phe Leu
275 280 285

ttt tca cag aca gct ttg tac acc gtg ttt tct gag atg aac att atg 1069
Phe Ser Gln Thr Ala Leu Tyr Thr Val Phe Ser Glu Met Asn Ile Met
290 295 300

atg tta acc att tca gat aca cct ttt ata cac atg ctg tgt cct cat 1117
Met Leu Thr Ile Ser Asp Thr Pro Phe Ile His Met Leu Cys Pro His
305 310 315

gca cca agc aca ttc aag ttt ttg aac ttt acc cag aac gtt ttc aca 1165
Ala Pro Ser Thr Phe Lys Phe Leu Asn Phe Thr Gln Asn Val Phe Thr
320 325 330 335

gat agt att ttt gaa aaa tgt tcc acg tta gtt aaa ttg gag aca ctt 1213
Asp Ser Ile Phe Glu Lys Cys Ser Thr Leu Val Lys Leu Glu Thr Leu
340 345 350

atc tta caa aag aat gga tta aaa gac ctt ttc aaa gta ggt ctc atg 1261
Ile Leu Gln Lys Asn Gly Leu Lys Asp Leu Phe Lys Val Gly Leu Met
355 360 365

acg aag gat atg cct tct ttg gaa ata ctg gat gtt agc tgg aat tct 1309
Thr Lys Asp Met Pro Ser Leu Glu Ile Leu Asp Val Ser Trp Asn Ser
370 375 380

ttg gaa tct ggt aga cat aaa gaa aac tgc act tgg gtt gag agt ata 1357
Leu Glu Ser Gly Arg His Lys Glu Asn Cys Thr Trp Val Glu Ser Ile
385 390 395

gtg gtg tta aat ttg tct tca aat atg ctt act gac tct gtt ttc aga 1405
Val Val Leu Asn Leu Ser Ser Asn Met Leu Thr Asp Ser Val Phe Arg
400 405 410 415

tgt tta cct ccc agg atc aag gta ctt gat ctt cac agc aat aaa ata 1453
Cys Leu Pro Pro Arg Ile Lys Val Leu Asp Leu His Ser Asn Lys Ile
420 425 430

aag agc gtt cct aaa caa gtc gta aaa ctg gaa gct ttg caa gaa ctc 1501
Lys Ser Val Pro Lys Gln Val Val Lys Leu Glu Ala Leu Gln Glu Leu
435 440 445

aat gtt gct ttc aat tct tta act gac ctt cct gga tgt ggc agc ttt 1549
Asn Val Ala Phe Asn Ser Leu Thr Asp Leu Pro Gly Cys Gly Ser Phe
450 455 460

agc agc ctt tct gta ttg atc att gat cac aat tca gtt tcc cac cca 1597
Ser Ser Leu Ser Val Leu Ile Ile Asp His Asn Ser Val Ser His Pro
465 470 475

tcg gct gat ttc ttc cag agc tgc cag aag atg agg tca ata aaa gca 1645
Ser Ala Asp Phe Phe Gln Ser Cys Gln Lys Met Arg Ser Ile Lys Ala
480 485 490 495

ggg gac aat cca ttc caa tgt acc tgt gag cta aga gaa ttt gtc aaa 1693
Gly Asp Asn Pro Phe Gln Cys Thr Cys Glu Leu Arg Glu Phe Val Lys
500 505 510

aat ata gac caa gta tca agt gaa gtg tta gag ggc tgg cct gat tct 1741
Asn Ile Asp Gln Val Ser Ser Glu Val Leu Glu Gly Trp Pro Asp Ser
515 520 525

tat aag tgt gac tac cca gaa agt tat aga gga agc cca cta aag gac 1789
Tyr Lys Cys Asp Tyr Pro Glu Ser Tyr Arg Gly Ser Pro Leu Lys Asp
530 535 540

ttt cac atg tct gaa tta tcc tgc aac ata act ctg ctg atc gtc acc 1837
Phe His Met Ser Glu Leu Ser Cys Asn Ile Thr Leu Leu Ile Val Thr
545 550 555

atc ggt gcc acc atg ctg gtg ttg gct gtg act gtg acc tcc ctc tgc 1885
Ile Gly Ala Thr Met Leu Val Leu Ala Val Thr Val Thr Ser Leu Cys
560 565 570 575

atc tac ttg gat ctg ccc tgg tat ctc agg atg gtg tgc cag tgg acc 1933
Ile Tyr Leu Asp Leu Pro Trp Tyr Leu Arg Met Val Cys Gln Trp Thr
580 585 590

cag act cgg cgc agg gcc agg aac ata ccc tta gaa gaa ctc caa aga 1981
Gln Thr Arg Arg Arg Ala Arg Asn Ile Pro Leu Glu Glu Leu Gln Arg
595 600 605

aac ctc cag ttt cat gct ttt att tca tat agt gaa cat gat tct gcc 2029
Asn Leu Gln Phe His Ala Phe Ile Ser Tyr Ser Glu His Asp Ser Ala
610 615 620

tgg gtg aaa agt gaa ttg gta cct tac cta gaa aaa gaa gat ata cag 2077
Trp Val Lys Ser Glu Leu Val Pro Tyr Leu Glu Lys Glu Asp Ile Gln
625 630 635

att tgt ctt cat gag agg aac ttt gtc cct ggc aag agc att gtg gaa 2125
Ile Cys Leu His Glu Arg Asn Phe Val Pro Gly Lys Ser Ile Val Glu
640 645 650 655

aat atc atc aac tgc att gag aag agt tac aag tcc atc ttt gtt ttg 2173
Asn Ile Ile Asn Cys Ile Glu Lys Ser Tyr Lys Ser Ile Phe Val Leu
660 665 670

tct ccc aac ttt gtc cag agt gag tgg tgc cat tac gaa ctc tat ttt 2221
Ser Pro Asn Phe Val Gln Ser Glu Trp Cys His Tyr Glu Leu Tyr Phe
675 680 685

gcc cat cac aat ctc ttt cat gaa gga tct aat aac tta atc ctc atc 2269
Ala His His Asn Leu Phe His Glu Gly Ser Asn Asn Leu Ile Leu Ile
690 695 700

tta ctg gaa ccc att cca cag aac agc att ccc aac aag tac cac aag 2317
Leu Leu Glu Pro Ile Pro Gln Asn Ser Ile Pro Asn Lys Tyr His Lys
705 710 715

ctg aag gct ctc atg acg cag cgg act tat ttg cag tgg ccc aag gag 2365
Leu Lys Ala Leu Met Thr Gln Arg Thr Tyr Leu Gln Trp Pro Lys Glu
720 725 730 735

aaa agc aaa cgt ggg ctc ttt tgg gct aac att aga gcc gct ttt aat 2413
Lys Ser Lys Arg Gly Leu Phe Trp Ala Asn Ile Arg Ala Ala Phe Asn
740 745 750

atg aaa tta aca cta gtc act gaa aac aat gat gtg aaa tct 2455
Met Lys Leu Thr Leu Val Thr Glu Asn Asn Asp Val Lys Ser
755 760 765

taaaaaaatt taggaaattc aacttaagaa accattattt acttggatga tggtgaatag 2515

tacagtcgta agtnactgtc tggaggtgcc tccattatcc tcatgccttc aggaaagact 2575

taacaaaaac aatgtttcat ctggggaact gagctaggcg gtgaggttag cctgccagtt 2635

agagacagcc cagtctcttc tggtttaatc attatgtttc aaattgaaac agtctctttt 2695

gagtaaatgc tcagtttttc agctcctctc cactctgctt tcccaaatgg attctgttgg 2755

tgaag 2760

MTKDKEPIVKSFHFVCLMIIIVGTRIQFSDGNEFAVDKSKRGLIHVPKDLPLKTKVLDMSQNYIAELQV
SDMSFLSELTVLRLSHNRIQLLDLSVFKFNQDLEYLDLSHNQLQKISCHPIVSFRHLDLSFNDFKALPI
CKEFGNLSQLNFLGLSAMKLQKLDLLPIAHLHLSYILLDLRNYYIKENETESLQILNAKTLHLVFHPTS
LFAIQVNISVNTLGCLQLTNIKLNDDNCQVFIKFLSELTRGPTLLNFTLNHIETTWKCLVRVFQFLWPK
PVEYLNIYNLTIIESIREEDFTYSKTTLKALTIEHITNQVFLFSQTALYTVFSEMNIMMLTISDTPFIH
MLCPHAPSTFKFLNFTQNVFTDSIFEKCSTLVKLETLILQKNGLKDLFKVGLMTKDMPSLEILDVSWNS
LESGRHKENCTWVESIVVLNLSSNMLTDSVFRCLPPRIKVLDLHSNKIKSVPKQVVKLEALQELNVAFN
SLTDLPGCGSFSSLSVLIIDHNSVSHPSADFFQSCQKMRSIKAGDNPFQCTCELREFVKNIDQVSSEVL
EGWPDSYKCDYPESYRGSPLKDFHMSELSCNITLLIVTIGATMLVLAVTVTSLCIYLDLPWYLRMVCQW
TQTRRRARNIPLEELQRNLQFHAFISYSEHDSAWVKSELVPYLEKEDIQICLHERNFVPGKSIVENIIN
CIEKSYKSIFVLSPNFVQSEWCHYELYFAHHNLFHEGSNNLILILLEPIPQNSIPNKYHKLKALMTQRT
YLQWPKEKSKRGLFWANIRAAFNMKLTLVTENNDVKS

Transmembrane segments correspond approximately to 802-818 (791-823) of primate DTLR7 SEQ ID NO: 37; 559-575 (550-586) of DTLR8 SEQ ID NO: 39; 553-569 (549-582) of DTLR9 SEQ ID NO: 41; 796-810 (790-814) of DTLR10 SEQ ID NO: 43; and 481-497 (475-503) of DTLR10 SEQ ID NO: 45.

As used herein, the term DNAX Toll like receptor 2 (DTLR2) shall be used to describe a protein comprising a protein or peptide segment having or sharing the amino acid sequence shown in Table 2, or a substantial fragment thereof. Similarly, with a DTLR3 and Table 3; DTLR4 and Table 4; DTLR5 and Table 5; DTLR6 and Table 6; DTLR7 and Table 7; DTLR8 and Table 8; DTLR9 and Table 9; and DTLR10 and Table 10. Rodent, e.g., mouse, DTLR11 sequence is provided, e.g., in EST AA739083; DTLR13 in ESTAI019567; DTLR14 in ESTs AI390330 and AA244663.

The invention also includes a protein variations of the respective DTLR allele whose sequence is provided, e.g., a mutein agonist or antagonist. Typically, such agonists or antagonists will exhibit less than about 10% sequence differences, and thus will often have between 1-and 11-fold substitutions, e.g., 2-, 3-, 5-, 7-fold, and others. It also encompasses allelic and other variants, e.g., natural polymorphic, of the protein described. Typically, it will bind to its corresponding biological receptor with high affinity, e.g., at least about 100 nM, usually better than about 30 nM, preferably better than about 10 nM, and more preferably at better than about 3 nM. The term shall also be used herein to refer to related naturally occurring forms, e.g., alleles, polymorphic variants, and metabolic variants of the mammalian protein.

This invention also encompasses proteins or peptides having substantial amino acid sequence identity with the amino acid sequence in Table 2. It will include sequence variants with relatively few substitutions, e.g., preferably less than about 3-5. Similar features apply to the other DTLR sequences provided in Tables 3, 4, 5, 6, 7, 8, 9, or 10.

A substantial polypeptide " fragment" , or "segment", is a stretch of amino acid residues of at least about 8 amino acids, generally at least 10 amino acids, more generally at least 12 amino acids, often at least 14 amino acids, more often at least 16 amino acids, typically at least 18 amino acids, more typically at least 20 amino acids, usually at least 22 amino acids, more usually at least 24 amino acids, preferably at least 26 amino acids, more preferably at least 28 amino acids, and, in particularly preferred embodiments, at least about 30 or more amino acids. Sequences of segments of different proteins can be compared to one another over appropriate length stretches.

Amino acid sequence homology, or sequence identity, is determined by optimizing residue matches, if necessary, by introducing gaps as required. See, e.g., Needleham, et al., (1970) J. Mol. Biol. 48:443-453; Sankoff, et al., (1983) chapter one in Time Warps, String Edits, and Macromolecules: The Theory and Practice of Sequence Comparison, Addison-Wesley, Reading, MA; and software packages from IntelliGenetics, Mountain View, CA; the University of Wisconsin Genetics Computer Group (GCG), Madison, WI; and the NCBI (NIH); each of which is incorporated herein by reference. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Homologous amino acid sequences are intended to include natural allelic and interspecies variations in the cytokine sequence. Typical homologous proteins or peptides will have from 50-100% homology (if gaps can be introduced), to 60-100% homology (if conservative substitutions are included) with an amino acid sequence segment of Table 2, 3, 4, 5, 6, 7, 8, 9, or 10. Homology measures will be at least about 70%, generally at least 76%, more generally at least 81%, often at least 85%, more often at least 88%, typically at least 90%, more typically at least 92%, usually at least 94%, more usually at least 95%, preferably at least 96%, and more preferably at least 97%, and in particularly preferred embodiments, at least 98% or more. The degree of homology will vary with the length of the compared segments. Homologous proteins or peptides, such as the allelic variants, will share most biological activities with the embodiments described in Table 2, 3, 4, 6, 7, 8, 9, or 10. Particularly interesting regions of comparison, at the amino acid or nucleotide levels, correspond to those within each of the blocks 1-10, or intrablock regions, corresponding to those indicated in Figures 2A-2B.

As used herein, the term "biological activity" is used to describe, without limitation, effects on inflammatory responses, innate immunity, and/or morphogenic development by respective ligands. For example, these receptors should, like IL-1 receptors, mediate phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738. The receptors exhibit biological activities much like regulatable enzymes, regulated by ligand binding.
However, the enzyme turnover number is more close to an enzyme than a receptor complex. Moreover, the numbers of occupied receptors necessary to induce such enzymatic activity is less than most receptor systems, and may number closer to dozens per cell, in contrast to most receptors which will trigger at numbers in the thousands per cell. The receptors, or portions thereof, may be useful as phosphate labeling enzymes to label general or specific substrates.

The terms ligand, agonist, antagonist, and analog of, e.g., a DTLR, include molecules that modulate the characteristic cellular responses to Toll ligand like proteins, as well as molecules possessing the more standard structural binding competition features of ligand-receptor interactions, e.g., where the receptor is a natural receptor or an antibody. The cellular responses likely are mediated through binding of various Toll ligands to cellular receptors related to, but possibly distinct from, the type I or type II IL-1 receptors. See, e.g., Belvin and Anderson (1996) Ann. Rev. Cell Dev. Biol. 12:393-416; Morisato and Anderson (1995) Ann. Rev. Genetics 29:371-3991 and Hultmark (1994) Nature 367:116-117.

Also, a ligand is a molecule which serves either as a natural ligand to which said receptor, or an analog thereof, binds, or a molecule which is a functional analog of the natural ligand. The functional analog may be a ligand with structural modifications, or may be a wholly unrelated molecule which has a molecular shape which interacts with the appropriate ligand binding determinants. The ligands may serve as agonists or antagonists, see, e.g., Goodman, et al. (eds. 1990) Goodman & Gilman's: The Pharmacological Bases of Therapeutics, Pergamon Press, New York.

Rational drug design may also be based upon structural studies of the molecular shapes of a receptor or antibody and other effectors or ligands. Effectors may be other proteins which mediate other functions in response to ligand binding, or other proteins which normally interact with the receptor. One means for determining which sites interact with specific other proteins is a physical structure determination, e.g., x-ray crystallography or 2 dimensional NMR techniques.
These will provide guidance as to which amino acid residues form molecular contact regions. For a detailed description of protein structural determination, see, e.g., Blundell and Johnson (1976) Protein Crystallography, Academic Press, New York, which is hereby incorporated herein by reference.

### II. Activities

The Toll like receptor proteins will have a number of different biological activities, e.g., in phosphate metabolism, being added to or removed from specific substrates, typically proteins. Such will generally result in modulation of an inflammatory function, other innate immunity response, or a morphological effect. The DTLR2, 3, 4, 5, 6, 7, 8, 9, or 10 proteins are homologous to other Toll like receptor proteins, but each have structural differences. For example, a human DTLR2 gene coding sequence probably has about 70% identity with the nucleotide coding sequence of mouse DTLR2. At the amino acid level, there is also likely to be reasonable identity.

The biological activities of the DTLRs will be related to addition or removal of phosphate moieties to substrates, typically in a specific manner, but occasionally in a non specific manner. Substrates may be identified, or conditions for enzymatic activity may be assayed by standard methods, e.g., as described in Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738.

### III. Nucleic Acids

This invention contemplates use of isolated nucleic acid or fragments, e.g., which encode these or closely related proteins, or fragments thereof, e.g., to encode a corresponding polypeptide, preferably one which is biologically active. In addition, this invention covers isolated or recombinant DNA which encodes such proteins or polypeptides having characteristic sequences of the respective DTLRs, individually or as a group. Typically, the nucleic acid is capable of hybridizing, under appropriate conditions, with a nucleic acid sequence segment shown in Tables 2-10, but preferably not with a corresponding segment of Table 1. Said biologically active protein or polypeptide can be a full length protein, or fragment, and will typically have a segment of amino acid sequence highly homologous to one shown in Tables 2-10. Further, this invention covers the use of isolated or recombinant nucleic acid, or fragments thereof, which encode proteins having fragments which are equivalent to the DTLR2-10 proteins. The isolated nucleic acids can have the respective regulatory sequences in the 5' and 3' flanks, e.g., promoters, enhancers, poly-A addition signals, and others from the natural gene.

An "isolated" nucleic acid is a nucleic acid, e.g., an RNA, DNA, or a mixed polymer, which is substantially pure, e.g., separated from other components which naturally accompany a native sequence, such as ribosomes, polymerases, and flanking genomic sequences from the originating species. The term embraces a nucleic acid sequence which has been removed from its naturally occurring environment, and includes recombinant or cloned DNA isolates, which are thereby distinguishable from naturally occurring compositions, and chemically synthesized analogs or analogs biologically synthesized by heterologous systems. A substantially pure molecule includes isolated forms of the molecule, either completely or substantially pure.

An isolated nucleic acid will generally be a homogeneous composition of molecules, but will, in some embodiments, contain heterogeneity, preferably minor.
This heterogeneity is typically found at the polymer ends or portions not critical to a desired biological function or activity.

A "recombinant" nucleic acid is typically defined either by its method of production or its structure. In reference to its method of production, e.g., a product made by a process, the process is use of recombinant nucleic acid techniques, e.g., involving human intervention in the nucleotide sequence. Typically this intervention involves in vitro manipulation, although under certain circumstances it may involve more classical animal breeding techniques. Alternatively, it can be a nucleic acid made by generating a sequence comprising fusion of two fragments which are not naturally contiguous to each other, but is meant to exclude products of nature, e.g., naturally occurring mutants as found in their natural state. Thus, for example, products made by transforming cells with any unnaturally occurring vector is encompassed, as are nucleic acids comprising sequence derived using any synthetic oligonucleotide process. Such a process is often done to replace a codon with a redundant codon encoding the same or a conservative amino acid, while typically introducing or removing a restriction enzyme sequence recognition site. Alternatively, the process is performed to join together nucleic acid segments of desired functions to generate a single genetic entity comprising a desired combination of functions not found in the commonly available natural forms, e.g., encoding a fusion protein. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. This will include a dimeric repeat. Specifically included are synthetic nucleic acids which, by genetic code redundancy, encode equivalent polypeptides to fragments of DTLR2-5 and fusions of sequences from various different related molecules, e.g., other IL-1 receptor family members.

A "fragment" in a nucleic acid context is a contiguous segment of at least about 17 nucleotides, generally at least 21 nucleotides, more generally at least 25 nucleotides, ordinarily at least 30 nucleotides, more ordinarily at least 35 nucleotides, often at least 39 nucleotides, more often at least 45 nucleotides, typically at least 50 nucleotides, more typically at least 55 nucleotides, usually at least 60 nucleotides, more usually at least 66 nucleotides, preferably at least 72 nucleotides, more preferably at least 79 nucleotides, and in particularly preferred embodiments will be at least 85 or more nucleotides. Typically, fragments of different genetic sequences can be compared to one another over appropriate length stretches, particularly defined segments such as the domains described below.

A nucleic acid which codes for a DTLR2-10 will be particularly useful to identify genes, mRNA, and cDNA species which code for itself or closely related proteins, as well as DNAs which code for polymorphic, allelic, or other genetic variants, e.g., from different individuals or related species. Preferred probes for such screens are those regions of the interleukin which are conserved between different polymorphic variants or which contain nucleotides which lack specificity, and will preferably be full length or nearly so. In other situations, polymorphic variant specific sequences will be more useful.

This invention further covers recombinant nucleic acid molecules and fragments having a nucleic acid sequence identical to or highly homologous to the isolated DNA set forth herein. In particular, the sequences will often be operably linked to DNA segments which control transcription, translation, and DNA replication. These additional segments typically assist in expression of the desired nucleic acid segment.

Homologous, or highly identical, nucleic acid sequences, when compared to one another or Table 2-10 sequences, exhibit significant similarity. The standards for homology in nucleic acids are either measures for homology generally used in the art by sequence comparison or based upon hybridization conditions. Comparative hybridization conditions are described in greater detail below.

Substantial identity in the nucleic acid sequence comparison context means either that the segments, or their complementary strands, when compared, are identical when optimally aligned, with appropriate nucleotide insertions or deletions, in at least about 60% of the nucleotides, generally at least 66%, ordinarily at least 71%, often at least 76%, more often at least 80%, usually at least 84%, more usually at least 88%, typically at least 91%, more typically at least about 93%, preferably at least about 95%, more preferably at least about 96 to 98% or more, and in particular embodiments, as high at about 99% or more of the nucleotides, including, e.g., segments encoding structural domains such as the segments described below. Alternatively, substantial identity will exist when the segments will hybridize under selective hybridization conditions, to a strand or its complement, typically using a sequence derived from Tables 2-10. Typically, selective hybridization will occur when there is at least about 55% homology over a stretch of at least about 14 nucleotides, more typically at least about 65%, preferably at least about 75%, and more preferably at least about 90%. See, Kanehisa (1984) Nucl. Acids Res. 12:203-213, which is incorporated herein by reference.
The length of homology comparison, as described, may be over longer stretches, and in certain embodiments will be over a stretch of at least about 17 nucleotides, generally at least about 20 nucleotides, ordinarily at least about 24 nucleotides, usually at least about 28 nucleotides, typically at least about 32 nucleotides, more typically at least about 40 nucleotides, preferably at least about 50 nucleotides; and more preferably at least about 75 to 100 or more nucleotides.

Stringent conditions, in referring to homology in the hybridization context, will be stringent combined conditions of salt, temperature, organic solvents, and other parameters typically controlled in hybridization reactions. Stringent temperature conditions will usually include temperatures in excess of about 30° C, more usually in excess of about 37° C, typically in excess of about 45° C, more typically in excess of about 55° C, preferably in excess of about 65° C, and more preferably in excess of about 70° C. Stringent salt conditions will ordinarily be less than about 500 mM, usually less than about 400 mM, more usually less than about 300 mM, typically less than about 200 mM, preferably less than about 100 mM, and more preferably less than about 80 mM, even down to less than about 20 mM. However, the combination of parameters is much more important than the measure of any single parameter. See, e.g., Wetmur and Davidson (1968) J. Mol. Biol. 31:349-370, which is hereby incorporated herein by reference.

Alternatively, for sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optical alignment of sequences for comparison can be conducted, e.g., by the local homology algorithm of Smith and Waterman (1981) Adv. Appl. Math. 2:482, by the homology alignment algorithm of Needlman and Wunsch (1970) J. Mol. Biol. 48:443, by the search for similarity method of Pearson and Lipman (1988) Proc. Nat'l Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra).

One example of a useful algorithm is PILEUP. PILEUP creates a multiple sequence alignment from a group of related sequences using progressive, pairwise alignments to show relationship and percent sequence identity. It also plots a tree or dendrogram showing the clustering relationships used to create the alignment. PILEUP uses a simplification of the progressive alignment method of Feng and Doolittle (1987) J. Mol. Evol. 35:351-360. The method used is similar to the method described by Higgins and Sharp (1989) CABIOS 5:151-153. The program can align up to 300 sequences, each of a maximum length of 5,000 nucleotides or amino acids. The multiple alignment. procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. For example, a reference sequence can be compared to other test sequences to determine the percent sequence identity relationship using the following parameters: default gap weight (3.00), default gap length weight (0.10), and weighted end gaps.

Another example of algorithm that is suitable for determining percent sequence identity and sequence similarity is the BLAST algorithm, which is described Altschul, et al. (1990) J. Mol. Biol. 215:403-410. Software for performing BLAST analyses is publicly available through the National Center for Biotechnology Information (http:www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul, et al., supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff and Henikoff (1989) Proc. Nat'l Acad. Sci. USA 89:10915) alignments (B) of 50, expectation (E) of 10, M=5, N=4, and a comparison of both strands.

In addition to calculating percent sequence identity, the BLAST algorithm also performs a statistical analysis of the similarity between two sequences (see, e.g., Karlin and Altschul (1993) Proc. Nat'l Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

A further indication that two nucleic acid sequences of polypeptides are substantially identical is that the polypeptide encoded by the first nucleic acid is immunologically cross reactive with the polypeptide encoded by the second nucleic acid, as described below. Thus, a polypeptide is typically substantially identical to a second polypeptide, e.g., where the two peptides differ only by conservative substitutions. Another indication that two nucleic acid sequences are substantially identical is that the two molecules hybridize to each other under stringent conditions, as described below.

The isolated DNA can be readily modified by nucleotide substitutions, nucleotide deletions, nucleotide insertions, and inversions of nucleotide stretches. These modifications result in novel DNA sequences which encode this protein or its derivatives. These modified sequences can be used to produce mutant proteins (muteins) or to enhance the expression of variant species. Enhanced expression may involve gene amplification, increased transcription, increased translation, and other mechanisms. Such mutant DTLR-like derivatives include predetermined or site-specific mutations of the protein or its fragments, including silent mutations using genetic code degeneracy. "Mutant DTLR" as used herein encompasses a polypeptide otherwise falling within the homology definition of the DTLR as set forth above, but having an amino acid sequence which differs from that of other DTLR-like proteins as found in nature, whether by way of deletion, substitution, or insertion. In particular, "site specific mutant DTLR" encompasses a protein having substantial homology with a protein of Tables 2-10, and typically shares most of the biological activities or effects of the forms disclosed herein.

Although site specific mutation sites are predetermined, mutants need not be site specific. Mammalian DTLR mutagenesis can be achieved by making amino acid insertions or deletions in the gene, coupled with expression. Substitutions, deletions, insertions, or any combinations may be generated to arrive at a final construct. Insertions include amino- or carboxy- terminal fusions. Random mutagenesis can be conducted at a target codon and the expressed mammalian DTLR mutants can then be screened for the desired activity. Methods for making substitution mutations at predetermined sites in DNA having a known sequence are well known in the art, e.g., by M13 primer mutagenesis. See also Sambrook, et al. (1989) and Ausubel, et al. (1987 and periodic Supplements).

The mutations in the DNA normally should not place coding sequences out of reading frames and preferably will not create complementary regions that could hybridize to produce secondary mRNA structure such as loops or hairpins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Polymerase chain reaction (PCR) techniques can often be applied in mutagenesis. Alternatively, mutagenesis primers are commonly used methods for generating defined mutations at predetermined sites. See, e.g., Innis, et al. (eds. 1990) PCR Protocols: A Guide to Methods and Applications Academic Press, San Diego, CA; and Dieffenbach and Dveksler (eds. 1995) PCR Primer: A Laboratory Manual Cold Spring Harbor Press, CSH, NY.

### IV. Proteins, Peptides

As described above, the present invention encompasses primate DTLR2-10, e.g., whose sequences are disclosed in Tables 2-10, and described above. Allelic and other variants are also contemplated, including, e.g., fusion proteins combining portions of such sequences with others, including epitope tags and functional domains.

The present invention also provides recombinant proteins, e.g., heterologous fusion proteins using segments from these rodent proteins. A heterologous fusion protein is a fusion of proteins or segments which are naturally not normally fused in the same manner.
Thus, the fusion product of a DTLR with an IL-1 receptor is a continuous protein molecule having sequences fused in a typical peptide linkage, typically made as a single translation product and exhibiting properties, e.g., sequence or antigenicity, derived from each source peptide. A similar concept applies to heterologous nucleic acid sequences.

In addition, new constructs may be made from combining similar functional or structural domains from other related proteins, e.g., IL-1 receptors or other DTLRs, including species variants. For example, ligand-binding or other segments may be "swapped" between different new fusion polypeptides or fragments. See, e.g., Cunningham, et al. (1989) Science 243:1330-1336; and O'Dowd, et al. (1988) J. Biol. Chem. 263:15985-15992, each of which is incorporated herein by reference. Thus, new chimeric polypeptides exhibiting new combinations of specificities will result from the functional linkage of receptor-binding specificities. For example, the ligand binding domains from other related receptor molecules may be added or substituted for other domains of this or related proteins. The resulting protein will often have hybrid function and properties. For example, a fusion protein may include a targeting domain which may serve to provide sequestering of the fusion protein to a particular subcellular organelle.

Candidate fusion partners and sequences can be selected from various sequence data bases, e.g., GenBank, c/o IntelliGenetics, Mountain View, CA; and BCG, University of Wisconsin Biotechnology Computing Group, Madison, WI, which are each incorporated herein by reference.

The present invention particularly provides muteins which bind Toll ligands, and/or which are affected in signal transduction. Structural alignment of human DTLR1-10 with other members of the IL-1 family show conserved features/residues. See, e.g., Figure 3A. Alignment of the human DTLR sequences with other members of the IL-1 family indicates various structural and functionally shared features. See also, Bazan, et al. (1996) Nature 379:591; Lodi, et al. (1994) Science 263:1762-1766; Sayle and Milner-White (1995) TIBS 20:374-376; and Gronenberg, et al. (1991) Protein Engineering 4:263-269.

The IL-1α and IL-1β ligands bind an IL-1 receptor type I as the primary receptor and this complex then forms a high affinity receptor complex with the IL-1 receptor type III. Such receptor subunits are probably shared with the new IL-1 family members.

Similar variations in other species counterparts of DTLR2-10 sequences, e.g., in the corresponding regions, should provide similar interactions with ligand or substrate. Substitutions with either mouse sequences or human sequences are particularly preferred. Conversely, conservative substitutions away from the ligand binding interaction regions will probably preserve most signaling activities.

"Derivatives" of the primate DTLR2-10 include amino acid sequence mutants, glycosylation variants, metabolic derivatives and covalent or aggregative conjugates with other chemical moieties. Covalent derivatives can be prepared by linkage of functionalities to groups which are found in the DTLR amino acid side chains or at the N- or C- termini, e.g., by means which are well known in the art. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties including C3 to C18 normal alkyl, thereby forming alkanoyl aroyl species.

In particular, glycosylation alterations are included, e.g., made by modifying the glycosylation patterns of a polypeptide during its synthesis and processing, or in further processing steps. Particularly preferred means for accomplishing this are by exposing the polypeptide to glycosylating enzymes derived from cells which normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine.

A major group of derivatives are covalent conjugates of the receptors or fragments thereof with other proteins of polypeptides. These derivatives can be synthesized in recombinant culture such as N- or C-terminal fusions or by the use of agents known in the art for their usefulness in cross-linking proteins through reactive side groups. Preferred derivatization sites with cross-linking agents are at free amino groups, carbohydrate moieties, and cysteine residues.

Fusion polypeptides between the receptors and other homologous or heterologous proteins are also provided. Homologous polypeptides may be fusions between different receptors, resulting in, for instance, a hybrid protein exhibiting binding specificity for multiple different Toll ligands, or a receptor which may have broadened or weakened specificity of substrate effect. Likewise, heterologous fusions may be constructed which would exhibit a combination of properties or activities of the derivative proteins. Typical examples are fusions of a reporter polypeptide, e.g., luciferase, with a segment or domain of a receptor, e.g., a ligand-binding segment, so that the presence or location of a desired ligand may be easily determined. See, e.g., Dull, et al., U.S. Patent No. 4,859,609, which is hereby incorporated herein by reference. Other gene fusion partners include glutathione-S-transferase (GST), bacterial β-galactosidase, trpE, Protein A, β-lactamase, alpha amylase, alcohol dehydrogenase, and yeast alpha mating factor. See, e.g., Godowski, et al. (1988) Science 241:812-816.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Such polypeptides may also have amino acid residues which have been chemically modified by phosphorylation, sulfonation, biotinylation, or the addition or removal of other moieties, particularly those which have molecular shapes similar to phosphate groups. In some embodiments, the modifications will be useful labeling reagents, or serve as purification targets, e.g., affinity ligands.

Fusion proteins will typically be made by either recombinant nucleic acid methods or by synthetic polypeptide methods. Techniques for nucleic acid manipulation and expression are described generally, for example, in Sambrook, et al. (1989) Molecular Cloning: A Laboratory Manual (2d ed.), Vols. 1-3, Cold Spring Harbor Laboratory, and Ausubel, et al. (eds. 1987 and periodic supplements) Current Protocols in Molecular Biology, Greene/Wiley, New York, which are each incorporated herein by reference. Techniques for synthesis of polypeptides are described, for example, in Merrifield (1963) J. Amer. Chem. Soc. 85:2149-2156; Merrifield (1986) Science 232: 341-347; and Atherton, et al. (1989) Solid Phase Peptide Synthesis: A Practical Approach, IRL Press, Oxford; each of which is incorporated herein by reference. See also Dawson, et al. (1994) Science 266:776-779 for methods to make larger polypeptides.

This invention also contemplates the use of derivatives of a DTLR2-10 other than variations in amino acid sequence or glycosylation. Such derivatives may involve covalent or aggregative association with chemical moieties. These derivatives generally fall into three classes: (1) salts, (2) side chain and terminal residue covalent modifications, and (3) adsorption complexes, for example with cell membranes. Such covalent or aggregative derivatives are useful as immunogens, as reagents in immunoassays, or in purification methods such as for affinity purification of a receptor or other binding molecule, e.g., an antibody. For example, a Toll ligand can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of a DTLR receptor, antibodies, or other similar molecules. The ligand can also be labeled with a detectable group, for example radioiodinated by the chloramine T procedure, covalently bound to rare earth chelates, or conjugated to another fluorescent moiety for use in diagnostic assays.

A DTLR of this invention can be used as an immunogen for the production of antisera or antibodies specific, e.g., capable of distinguishing between other IL-1 receptor family members, for the DTLR or various fragments thereof. The purified DTLR can be used to screen monoclonal antibodies or antigen-binding fragments prepared by immunization with various forms of impure preparations containing the protein. In particular, the term "antibodies" also encompasses antigen binding fragments of natural antibodies, e.g., Fab, Fab2, Fv, etc. The purified DTLR can also be used as a reagent to detect antibodies generated in response to the presence of elevated levels of expression, or immunological disorders which lead to antibody production to the endogenous receptor. Additionally, DTLR fragments may also serve as immunogens to produce the antibodies of the present invention, as described immediately below. For example, this invention contemplates antibodies having binding affinity to or being raised against the amino acid sequences shown in Tables 2-10, fragments thereof, or various homologous peptides. In particular, this invention contemplates antibodies having binding affinity to, or having been raised against, specific fragments which are predicted to be, or actually are, exposed at the exterior protein surface of the native DTLR.

The blocking of physiological response to the receptor ligands may result from the inhibition of binding of the ligand to the receptor, likely through competitive inhibition. Thus, in vitro assays of the present invention will often use antibodies or antigen binding segments of these antibodies, or fragments attached to solid phase substrates. These assays will also allow for the diagnostic determination of the effects of either ligand binding region mutations and modifications, or other mutations and modifications, e.g., which affect signaling or enzymatic function.

This invention also contemplates the use of competitive drug screening assays, e.g., where neutralizing antibodies to the receptor or fragments compete with a test compound for binding to a ligand or other antibody. In this manner, the neutralizing antibodies or fragments can be used to detect the presence of a polypeptide which shares one or more binding sites to a receptor and can also be used to occupy binding sites on a receptor that might otherwise bind a ligand.

### V. Making Nucleic Acids and Protein

DNA which encodes the protein or fragments thereof can be obtained by chemical synthesis, screening cDNA libraries, or by screening genomic libraries prepared from a wide variety of cell lines or tissue samples. Natural sequences can be isolated using standard methods and the sequences provided herein, e.g., in Tables 2-10. Other species counterparts can be identified by hybridization techniques, or by various PCR techniques, combined with or by searching in sequence databases, e.g., GenBank.

This DNA can be expressed in a wide variety of host cells for the synthesis of a full-length receptor or fragments which can in turn, for example, be used to generate polyclonal or monoclonal antibodies; for binding studies; for construction and expression of modified ligand binding or kinase/phosphatase domains; and for structure/function studies. Variants or fragments can be expressed in host cells that are transformed or transfected with appropriate expression vectors. These molecules can be substantially free of protein or cellular contaminants, other than those derived from the recombinant host, and therefore are particularly useful in pharmaceutical compositions when combined with a pharmaceutically acceptable carrier and/or diluent. The protein, or portions thereof, may be expressed as fusions with other proteins.

Expression vectors are typically self-replicating DNA or RNA constructs containing the desired receptor gene or its fragments, usually operably linked to suitable genetic control elements that are recognized in a suitable host cell. These control elements are capable of effecting expression within a suitable host. The specific type of control elements necessary to effect expression will depend upon the eventual host cell used. Generally, the genetic control elements can include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention include those which contain DNA which encodes a protein, as described, or a fragment thereof encoding a biologically active equivalent polypeptide. The DNA can be under the control of a viral promoter and can encode a selection marker. This invention further contemplates use of such expression vectors which are capable of expressing eukaryotic cDNA coding for such a protein in a prokaryotic or eukaryotic host, where the vector is compatible with the host and where the eukaryotic cDNA coding for the receptor is inserted into the vector such that growth of the host containing the vector expresses the cDNA in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the protein or its fragments in various hosts using vectors that do not contain a replication origin that is recognized by the host cell. It is also possible to use vectors that cause integration of the protein encoding portion or its fragments into the host DNA by recombination.

Vectors, as used herein, comprise plasmids, viruses, bacteriophage, integratable DNA fragments, and other vehicles which enable the integration of DNA fragments into the genome of the host. Expression vectors are specialized vectors which contain genetic control elements that effect expression of operably linked genes. Plasmids are the most commonly used form of vector but all other forms of vectors which serve an equivalent function and which are, or become, known in the art are suitable for use herein. See, e.g., Pouwels, et al. (1985 and Supplements) Cloning Vectors: A Laboratory Manual, Elsevier, N.Y., and Rodriquez, et al. (eds.) Vectors: A Survey of Molecular Cloning Vectors and Their Uses, Buttersworth, Boston, 1988, which are incorporated herein by reference.

Transformed cells are cells, preferably mammalian, that have been transformed or transfected with receptor vectors constructed using recombinant DNA techniques. Transformed host cells usually express the desired protein or its fragments, but for purposes of cloning, amplifying, and manipulating its DNA, do not need to express the subject protein. This invention further contemplates culturing transformed cells in a nutrient medium, thus permitting the receptor to accumulate in the cell membrane. The protein can be recovered, either from the culture or, in certain instances, from the culture medium.

For purposes of this invention, nucleic sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include prokaryotes, lower eukaryotes, and higher eukaryotes. Prokaryotes include both gram negative and gram positive organisms, e.g., E. coli and B. subtilis. Lower eukaryotes include yeasts, e.g., S. cerevisiae and Pichia, and species of the genus Dictyostelium. Higher eukaryotes include established tissue culture cell lines from animal cells, both of non-mammalian origin, e.g., insect cells, and birds, and of mammalian origin, e.g., human, primates, and rodents.

Prokaryotic host-vector systems include a wide variety of vectors for many different species. As used herein, E. coli and its vectors will be used generically to include equivalent vectors used in other prokaryotes. A representative vector for amplifying DNA is pBR322 or many of its derivatives. Vectors that can be used to express the receptor or its fragments include, but are not limited to, such vectors as those containing the lac promoter (pUC-series); trp promoter (pBR322-trp); Ipp promoter (the pIN-series); lambda-pP or pR promoters (pOTS); or hybrid promoters such as ptac (pDR540). See Brosius; et al. (1988) "Expression Vectors Employing Lambda-, trp-, lac-, and Ipp-derived Promoters", in Vectors: A Survey of Molecular Cloning Vectors and Their Uses, (eds. Rodriguez and Denhardt), Buttersworth, Boston, Chapter 10, pp. 205-236, which is incorporated herein by reference.

Lower eukaryotes, e.g., yeasts and Dictyostelium, may be transformed with DTLR sequence containing vectors. For purposes of this invention, the most common lower eukaryotic host is the baker's yeast, Saccharomyces cerevisiae. It will be used to generically represent lower eukaryotes although a number of other strains and species are also available. Yeast vectors typically consist of a replication origin (unless of the integrating type), a selection gene, a promoter, DNA encoding the receptor or its fragments, and sequences for translation termination, polyadenylation, and transcription termination. Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-replicating high copy number (such as the YEp-series) ; integrating types (such as the YIp-series), or mini-chromosomes (such as the YCp-series).

Higher eukaryotic tissue culture cells are normally the preferred host cells for expression of the functionally active interleukin protein. In principle, any higher eukaryotic tissue culture cell line is workable, e.g., insect baculovirus expression systems, whether from an invertebrate or vertebrate source. However, mammalian cells are preferred. Transformation or transfection and propagation of such cells has become a routine procedure. Examples of useful cell lines include HeLa cells, Chinese hamster ovary (CHO) cell lines, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also usually contain a selection gene or amplification gene. Suitable expression vectors may be plasmids, viruses, or retroviruses carrying promoters derived, e.g., from such sources as from adenovirus, SV40, parvoviruses, vaccinia virus, or cytomegalovirus. Representative examples of suitable expression vectors include pCDNA1; pCD, see Okayama, et al. (1985) Mol. Cell Biol. 5:1136-1142; pMClneo PolyA, see Thomas, et al. (1987) Cell 51:503-512; and a baculovirus vector such as pAC 373 or pAC 610.

For secreted proteins, an open reading frame usually encodes a polypeptide that consists of a mature or secreted product covalently linked at its N-terminus to a signal peptide. The signal peptide is cleaved prior to secretion of the mature, or active, polypeptide. The cleavage site can be predicted with a high degree of accuracy from empirical rules, e.g., von-Heijne (1986) Nucleic Acids Research 14:4683-4690, and the precise amino acid composition of the signal peptide does not appear to be critical to its function, e.g., Randall, et al. (1989) Science 243:1156-1159; Kaiser, et al. (1987) Science 235:312-317.

It will often be desired to express these polypeptides in a system which provides a specific or defined glycosylation pattern. In this case, the usual pattern will be that provided naturally by the expression system. However, the pattern will be modifiable by exposing the polypeptide, e.g., an unglycosylated form, to appropriate glycosylating proteins introduced into a heterologous expression system. For example, the receptor gene may be co-transformed with one or more genes encoding mammalian or other glycosylating enzymes. Using this approach, certain mammalian glycosylation patterns will be achievable in prokaryote or other cells.

The source of DTLR can be a eukaryotic or prokaryotic host expressing recombinant DTLR, such as is described above. The source can also be a cell line such as mouse Swiss 3T3 fibroblasts, but other mammalian cell lines are also contemplated by this invention, with the preferred cell line being from the human species.

Now that the sequences are known, the primate DTLRs, fragments, or derivatives thereof can be prepared by conventional processes for synthesizing peptides. These include processes such as are described in Stewart and Young (1984) Solid Phase Peptide Synthesis, Pierce Chemical Co., Rockford, IL; Bodanszky and Bodanszky (1984) The Practice of Peptide Synthesis, Springer-Verlag, New York; and Bodanszky (1984) The Principles of Peptide Synthesis, Springer-Verlag, New York; all of each which are incorporated herein by reference. For example, an azide process, an acid chloride process, an acid anhydride process, a mixed anhydride process, an active ester process (e.g., p-nitrophenyl ester, N-hydroxysuccinimide ester, or cyanomethyl ester), a carbodiimidazole process, an oxidative-reductive process, or a dicyclohexylcarbodiimide (DCCD)/additive process can be used. Solid phase and solution phase syntheses are both applicable to the foregoing processes. Similar techniques can be used with partial DTLR sequences.

The DTLR proteins, fragments, or derivatives are suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino acid. Amino groups that are not being used in the coupling reaction typically must be protected to prevent coupling at an incorrect location.

If a solid phase synthesis is adopted, the C-terminal amino acid is bound to an insoluble carrier or support through its carboxyl group. The insoluble carrier is not particularly limited as long as it has a binding capability to a reactive carboxyl group. Examples of such insoluble carriers include halomethyl resins, such as chloromethyl resin or bromomethyl resin, hydroxymethyl resins, phenol resins, tert-alkyloxycarbonylhydrazidated resins, and the like.

An amino group-protected amino acid is bound in sequence through condensation of its activated carboxyl group and the reactive amino group of the previously formed peptide or chain, to synthesize the peptide step by step. After synthesizing the complete sequence, the peptide is split off from the insoluble carrier to produce the peptide. This solid-phase approach is generally described by Merrifield, et al. (1963) in J. Am. Chem. Soc. 85:2149-2156, which is incorporated herein by reference.

The prepared protein and fragments thereof can be isolated and purified from the reaction mixture by means of peptide separation, for example, by extraction, precipitation, electrophoresis, various forms of chromatography, and the like. The receptors of this invention can be obtained in varying degrees of purity depending upon desired uses. Purification can be accomplished by use of the protein purification techniques disclosed herein, see below, or by the use of the antibodies herein described in methods of immunoabsorbant affinity chromatography. This immunoabsorbant affinity chromatography is carried out by first linking the antibodies to a solid support and then contacting the linked antibodies with solubilized lysates of appropriate cells, lysates of other cells expressing the receptor, or lysates or supernatants of cells producing the protein as a result of DNA techniques, see below.

Generally, the purified protein will be at least about 40% pure, ordinarily at least about 50% pure, usually at least about 60% pure, typically at least about 70% pure, more typically at least about 80% pure, preferable at least about 90% pure and more preferably at least about 95% pure, and in particular embodiments, 97%-99% or more. Purity will usually be on a weight basis, but can also be on a molar basis. Different assays will be applied as appropriate.

### VI. Antibodies

Antibodies can be raised to the various mammalian, e.g., primate DTLR proteins and fragments thereof, both in naturally occurring native forms and in their recombinant forms, the difference being that antibodies to the active receptor are more likely to recognize epitopes which are only present in the native conformations. Denatured antigen detection can also be useful in, e.g., Western analysis. Anti-idiotypic antibodies are also contemplated, which would be useful as agonists or antagonists of a natural receptor or an antibody.

Preferred antibodies will exhibit properties of both affinity and selectivity. High affinity is generally preferred, while selectivity will allow distinction between various embodiment subsets. In particular, it will be desirable to possess antibody preparations characterized to bind, e.g., various specific combinations of related members while not binding others. Such various combinatorial subsets are specifically enabled, e.g., these reagents may be generated or selected using standard methods of immunoaffinity, selection, etc.

Antibodies, including binding fragments and single chain versions, against predetermined fragments of the protein can be raised by immunization of animals with conjugates of the fragments with immunogenic proteins. Monoclonal antibodies are prepared from cells secreting the desired antibody. These antibodies can be screened for binding to normal or defective protein, or screened for agonistic or antagonistic activity. These monoclonal antibodies will usually bind with at least a K_{D} of about 1 mM, more usually at least about 300 µM, typically at least about 100µM, more typically at least about 30 µM, preferably at least about 10 µM, and more preferably at least about 3 µM or better.

The antibodies, including antigen binding fragments, of this invention can have significant diagnostic or therapeutic value. They can be potent antagonists that bind to the receptor and inhibit binding to ligand or inhibit the ability of the receptor to elicit a biological response, e.g., act on its substrate. They also can be useful as non-neutralizing antibodies and can be coupled to toxins or radionuclides to bind producing cells, or cells localized to the source of the interleukin. Further, these antibodies can be conjugated to drugs or other therapeutic agents, either directly or indirectly by means of a linker.

The antibodies of this invention can also be useful in diagnostic applications. As capture or non-neutralizing antibodies, they might bind to the receptor without inhibiting ligand or substrate binding. As neutralizing antibodies, they can be useful in competitive binding assays. They will also be useful in detecting or quantifying ligand. They may be used as reagents for Western blot analysis, or for immunoprecipitation or immunopurification of the respective protein.

Protein fragments may be joined to other materials, particularly polypeptides, as fused or covalently joined polypeptides to be used as immunogens. Mammalian DTLR and its fragments may be fused or covalently linked to a variety of immunogens, such as keyhole limpet hemocyanin, bovine serum albumin, tetanus toxoid, etc. See Microbiology, Hoeber Medical Division, Harper and Row, 1969; Landsteiner (1962) Specificity of Serological Reactions, Dover Publications, New York; and Williams, et al. (1967) Methods in Immunology and Immunochemistry, Vol. 1, Academic Press, New York; each of which are incorporated herein by reference, for descriptions of methods of preparing polyclonal antisera. A typical method involves hyperimmunization of an animal with an antigen. The blood of the animal is then collected shortly after the repeated immunizations and the gamma globulin is isolated.

In some instances, it is desirable to prepare monoclonal antibodies from various mammalian hosts, such as mice, rodents, primates, humans, etc. Description of techniques for preparing such monoclonal antibodies may be found in, e.g., Stites, et al. (eds.) Basic and Clinical Immunology (4th ed.), Lange Medical Publications, Los Altos, CA, and references cited therein; Harlow and Lane (1988.) Antibodies: A Laboratory Manual, CSH Press; Goding (1986) Monoclonal Antibodies: Principles and Practice (2d ed) Academic Press,. New York; and particularly in Kohler and Milstein (1975) in Nature 256: .495-497, which discusses one method of generating monoclonal antibodies. Each of these references is incorporated herein by reference. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 296:1275-1281; and Ward, et al. (1989) Nature 341:544-546, each of which is hereby incorporated herein by reference. The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents, teaching the use of such labels include U.S. Patent Nos. 3,817,837; 3,850,752; 3,939,350; 3,996,345; 4,277,437; 4,275,149; and 4,366,241. Also, recombinant or chimeric immunoglobulins may be produced, see Cabilly, U.S. Patent No. 4,816,567; or made in transgenic mice, see Mendez, et al. (1997) Nature Genetics 15:146-156. These references are incorporated herein by reference.

The antibodies of this invention can also be used for affinity chromatography in isolating the DTLRs. Columns can be prepared where the antibodies are linked to a solid support, e.g., particles, such as agarose, Sephadex, or the like, where a cell lysate may be passed through the column, the column washed, followed by increasing concentrations of a mild denaturant, whereby the purified protein will be released. The protein may be used to purify antibody.

The antibodies may also be used to screen expression libraries for particular expression products. Usually the antibodies used in such a procedure will be labeled with a moiety allowing easy detection of presence of antigen by antibody binding.

Antibodies raised against a DTLR will also be used to raise anti-idiotypic antibodies. These will be useful in detecting or diagnosing various immunological conditions related to expression of the protein or cells which express the protein. They also will be useful as agonists or antagonists of the ligand, which may be competitive inhibitors or substitutes for naturally occurring ligands.

A DTLR protein that specifically binds to or that is specifically immunoreactive with an antibody generated against a defined immunogen, such as an immunogen consisting of the amino acid sequence of SEQ ID NO: 4, 6, 8, 10, 12, 14, 16, 18, 20, 22, or 24, is typically determined in an immunoassay. The immunoassay typically uses a polyclonal antiserum which was raised, e.g., to a protein of SEQ ID NO: 4, 6, 8, 10, 12, 14, 16, 18, 20, 22, or 24. This antiserum is selected to have low crossreactivity against other IL-1R family members, e.g., DTLR1, preferably from the same species, and any such crossreactivity is removed by immunoabsorption prior to use in the immunoassay.

In order to produce antisera for use in an immunoassay, the protein of SEQ ID NO: 4, 6, 8, 10, 12, 14, 16, 18, 20, 22, or 24, or a combination thereof, is isolated as described herein. For example, recombinant protein may be produced in a mammalian cell line. An appropriate host, e.g., an inbred strain of mice such as Balb/c, is immunized with the selected protein, typically using a standard adjuvant, such as Freund's adjuvant, and a standard mouse immunization protocol (see Harlow and Lane, supra). Alternatively, a synthetic peptide derived from the sequences disclosed herein and conjugated to a carrier protein can be used an immunogen. Polyclonal sera are collected and titered against the immunogen protein in an immunoassay, e.g., a solid phase immunoassay with the immunogen immobilized on a solid support. Polyclonal antisera with a titer of 10⁴ or greater are selected and tested for their cross reactivity against other IL-1R family members, e.g., mouse DTLRs or human DTLR1, using a competitive binding immunoassay such as the one described in Harlow and Lane, supra, at pages 570-573. Preferably at least two DTLR family members are used in this determination in conjunction with either or some of the human DTLR2-10. These IL-1R family members can be produced as recombinant proteins and isolated using standard molecular biology and protein chemistry techniques as described herein.

Immunoassays in the competitive binding format can be used for the crossreactivity determinations. For example, the proteins of SEQ ID NO: 4, 6, 8, 10, 12, 14, 16, 18, 20, 22, and/or 24, or various fragments thereof, can be immobilized to a solid support. Proteins added to the assay compete with the binding of the antisera to the immobilized antigen. The ability of the above proteins to compete with the binding of the antisera to the immobilized protein is compared to the protein of SEQ ID NO: 4, 6, 8, 10, 12, 14, 16, 18, 20, 22, and/or 24. The percent crossreactivity for the above proteins is calculated, using standard calculations. Those antisera with less than 10% crossreactivity with each of the proteins listed above are selected and pooled. The cross-reacting antibodies are then removed from the pooled antisera by immunoabsorption with the above-listed proteins.

The immunoabsorbed and pooled antisera are then used in a competitive binding immunoassay as described above to compare a second protein to the immunogen protein (e.g., the IL-1R like protein of SEQ ID NO: 4, 6, 8, 10, 12, 14, 16, 18, 20, 22, and/or 24). In order to make this comparison, the two proteins are each assayed at a wide range of concentrations and the amount of each protein required to inhibit 50% of the binding of the antisera to the immobilized protein is determined. If the amount of the second protein required is less than twice the amount of the protein of the selected protein or proteins that is required, then the second protein is said to specifically bind to an antibody generated to the immunogen.

It is understood that these DTLR proteins are members of a family of homologous proteins that comprise at least 10 so far identified genes. For a particular gene product, such as the DTLR2-10, the term refers not only to the amino acid sequences disclosed herein, but also to other proteins that are allelic, non-allelic or species variants. It also understood that the terms include nonnatural mutations introduced by deliberate mutation using conventional recombinant technology such as single site mutation, or by excising short sections of DNA encoding the respective proteins, or by substituting new amino acids, or adding new amino acids. Such minor alterations must substantially maintain the immunoidentity of the original molecule and/or its biological activity. Thus, these alterations include proteins that are specifically immunoreactive with a designated naturally occurring IL-1R related protein, for example, the DTLR proteins shown in SEQ ID NO: 2, 4, 6, 8, 10, 12, 14, 16, 18, 20, 22, or 24. The biological properties of the altered proteins can be determined by expressing the protein in an appropriate cell line and measuring the appropriate effect upon lymphocytes. Particular protein modifications considered minor would include conservative substitution of amino acids with similar chemical properties, as described above for the IL-1R family as a whole. By aligning a protein optimally with the protein of DTLR2-10 and by using the conventional immunoassays described herein to determine immunoidentity, one can determine the protein compositions of the invention.

### VII. Kits and quantitation

Both naturally occurring and recombinant forms of the IL-1R like molecules of this invention are particularly useful in kits and assay methods. For example, these methods would also be applied to screening for binding activity, e.g., ligands for these proteins. Several methods of automating assays have been developed in recent years so as to permit screening of tens of thousands of compounds per year. See, e.g., a BIOMEK automated workstation, Beckman Instruments, Palo Alto, California, and Fodor, et al. (1991) Science 251:767-773, which is incorporated herein by reference. The latter describes means for testing binding by a plurality of defined polymers synthesized on a solid substrate. The development of suitable assays to screen for a ligand or agonist/antagonist homologous proteins can be greatly facilitated by the availability of large amounts of purified, soluble DTLRs in an active state such as is provided by this invention.

Purified DTLR can be coated directly onto plates for use in the aforementioned ligand screening techniques. However, non-neutralizing antibodies to these proteins can be used as capture antibodies to immobilize the respective receptor on the.solid phase, useful, e.g., in diagnostic uses.

This invention also contemplates use of DTLR2-10, fragments thereof, peptides, and their fusion products in a variety of diagnostic kits and methods for detecting the presence of the protein or its ligand. Alternatively, or additionally, antibodies against the molecules may be incorporated into the kits and methods. Typically the kit will have a compartment containing either a defined DTLR peptide or gene segment or a reagent which recognizes one or the other. Typically, recognition reagents, in the case of peptide, would be a receptor or antibody, or in the case of a gene segment, would usually be a hybridization probe.

A preferred kit for determining the concentration of, e.g., DTLR4, a sample would typically comprise a labeled compound, e.g., ligand or antibody, having known binding affinity for DTLR4, a source of DTLR4 (naturally occurring or recombinant) as a positive control, and a means for separating the bound from free labeled compound, for example a solid phase for immobilizing the DTLR4 in the test sample. Compartments containing reagents, and instructions, will normally be provided.

Antibodies, including antigen binding fragments, specific for mammalian DTLR or a peptide fragment, or receptor fragments are useful in diagnostic applications to detect the presence of elevated levels of ligand and/or its fragments. Diagnostic assays may be homogeneous (without a separation step between free reagent and antibody-antigen complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA) and the like. For example, unlabeled antibodies can be employed by using a second antibody which is labeled and which recognizes the antibody to DTLR4 or to a particular fragment thereof. These assays have also been extensively discussed in the literature. See, e.g., Harlow and Lane (1988) Antibodies: A Laboratory Manual, CSH., and Coligan (ed. 1991 and periodic supplements) Current Protocols In Immunology Greene/Wiley, New York.

Anti-idiotypic antibodies may have similar use to serve as agonists or antagonists of DTLR4. These should be useful as therapeutic reagents under appropriate circumstances.

Frequently, the reagents for diagnostic assays are supplied in kits, so as to optimize the sensitivity of the assay. For the subject invention, depending upon the nature of the assay, the protocol, and the label, either labeled or unlabeled antibody, or labeled ligand is provided. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent, and will contain instructions for proper use and disposal of reagents. Desirably, the reagents are provided as a dry lyophilized powder, where the reagents may be reconstituted in an aqueous medium having appropriate concentrations for performing the assay.

The aforementioned constituents of the diagnostic assays may be used without modification or may be modified in a variety of ways. For example, labeling may be achieved by covalently or non-covalently joining a moiety which directly or indirectly provides a detectable signal. In any of these assays, a test compound, DTLR, or antibodies thereto can be labeled either directly or indirectly. Possibilities for direct labeling include label groups: radiolabels such as ¹²⁵I, enzymes (U.S. Pat. No. 3,645,090) such as peroxidase and alkaline phosphatase, and fluorescent labels (U.S. Pat. No. 3,940,475) capable of monitoring the change in fluorescence intensity, wavelength shift, or fluorescence polarization. Both of the patents are incorporated herein by reference. Possibilities for indirect labeling include biotinylation of one constituent followed by binding to avidin coupled to one of the above label groups.

There are also numerous methods of separating the bound from the free ligand, or alternatively the bound from the free test compound. The DTLR can be immobilized on various matrixes followed by washing. Suitable matrices include plastic such as an ELISA plate, filters, and beads. Methods of immobilizing the receptor to a matrix include, without limitation, direct adhesion to plastic, use of a capture antibody, chemical coupling, and biotin-avidin. The last step in this approach involves the precipitation of antibody/antigen complex by any of several methods including those utilizing, e.g., an organic solvent such as polyethylene glycol or a salt such as ammonium sulfate. Other suitable separation techniques include, without limitation, the fluorescein antibody magnetizable particle method described in Rattle, et al. (1984) Clin. Chem. 30(9) :1457-1461, and the double antibody magnetic particle separation as described in U.S. Pat. No. 4,659,678, each of which is incorporated herein by reference.

The methods for linking protein or fragments to various labels have been extensively reported in the literature and do not require detailed discussion here. Many of the techniques involve the use of activated carboxyl groups 'either through the use of carbodiimide or active esters to form peptide bonds, the formation of thioethers by reaction of a mercapto group with an activated halogen such as chloroacetyl, or an activated olefin such as maleimide, for linkage, or the like.
Fusion proteins will also find use in these applications.

Another diagnostic aspect of this invention involves use of oligonucleotide or polynucleotide sequences taken from the sequence of a DTLR. These sequences can be used as probes for detecting levels of the respective DTLR in patients suspected of having an immunological disorder. The preparation of both RNA and DNA nucleotide sequences, the labeling of the sequences, and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly ³²P. However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes, DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel anti-sense RNA may be carried out in any conventional techniques such as nucleic acid hybridization, plus and minus screening, recombinational probing, hybrid released translation (HRT), and hybrid arrested translation (HART). This also includes amplification techniques such as polymerase chain reaction (PCR).

Diagnostic kits which also test for the qualitative or quantitative presence of other markers are also contemplated. Diagnosis or prognosis may depend on the combination of multiple indications used as markers. Thus, kits may test for combinations of markers. See, e.g., Viallet, et al. (1989) Progress in Growth Factor Res. 1:89-97.

### VIII. Therapeutic Utility

This invention provides reagents with significant therapeutic value. The DTLRs (naturally occurring or recombinant), fragments thereof, mutein receptors, and antibodies, along with compounds identified as having binding affinity to the receptors or antibodies, should be useful in the treatment of conditions exhibiting abnormal expression of the receptors of their ligands. Such abnormality will typically be manifested by immunological disorders. Additionally, this invention should provide therapeutic value in various diseases or disorders associated with abnormal expression or abnormal triggering of response to the ligand. The Toll ligands have been suggested to be involved in morphologic development, e.g., dorso-ventral polarity determination, and immune responses, particularly the primitive innate responses. See, e.g., Sun, et al. (1991) Eur. J. Biochem. 196:247-254; Hultmark (1994) Nature 367:116-117.

Recombinant DTLRs, muteins, agonist or antagonist antibodies thereto, or antibodies can be purified and then administered to a patient. These reagents can be combined for therapeutic use with additional active ingredients, e.g., in conventional pharmaceutically acceptable carriers or diluents, along with physiologically innocuous stabilizers and excipients. These combinations can be sterile, e.g., filtered, and placed into dosage forms as by lyophilization in dosage vials or storage in stabilized aqueous preparations. This invention also contemplates use of antibodies or binding fragments thereof which are not complement binding.

Ligand screening using DTLR or fragments thereof can be performed to identify molecules having binding affinity to the receptors. Subsequent biological assays can then be utilized to determine if a putative ligand can provide competitive binding, which can block intrinsic stimulating activity. Receptor fragments can be used as a blocker or antagonist in that it blocks the activity of ligand. Likewise, a compound having intrinsic stimulating activity can activate the receptor and is thus an agonist in that it simulates the activity of ligand, e.g., inducing signaling. This invention further contemplates the therapeutic use of antibodies to DTLRs as antagonists.

The quantities of reagents necessary for effective therapy will depend upon many different factors, including means of administration, target site, physiological state of the patient, and other medicants administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds. 1990) Goodman and Gilman's: The Pharmacological Bases of Therapeutics, 8th Ed., Pergamon Press; and Remington's Pharmaceutical Sciences, (current edition), Mack Publishing Co., Easton, Penn.; each of which is hereby incorporated herein by reference. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Because of the likely high affinity binding, or turnover numbers, between a putative ligand and its receptors, low dosages of these reagents would be initially expected to be effective. And the signaling pathway suggests extremely low amounts of ligand may have effect. Thus, dosage ranges would ordinarily be expected to be in amounts lower than 1 mM concentrations, typically less than about 10 µM concentrations, usually less than about 100 nM; preferably less than about 10 pM (picomolar), and most preferably less than about 1 fM (femtomolar), with an appropriate carrier. Slow release formulations, or slow release apparatus will often be utilized for continuous administration.

DTLRs, fragments thereof, and antibodies or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in any conventional dosage formulation. While it is possible for the active ingredient to be administered alone, it is preferable to present it as a pharmaceutical formulation. Formulations comprise at least one active ingredient, as defined above, together with one or more acceptable carriers thereof. Each carrier must be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration.. The formulations may conveniently be presented in unit dosage form and may be prepared by any methods well known in the art of pharmacy. See, e.g., Gilman, et al. (eds. 1990) Goodman and Gilman's: The Pharmacological Bases of Therapeutics, 8th Ed., Pergamon Press; and Remington's Pharmaceutical Sciences (current edition), Mack Publishing Co., Easton, Penn.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. The therapy of this invention may be combined with or used in association with other therapeutic agents, particularly agonists or antagonists of other IL-1 family members.

### IX. Ligands

The description of the Toll receptors herein provide means to identify ligands, as described above. Such ligand should bind specifically to the respective receptor with reasonably high affinity. Various constructs are made available which allow either labeling of the receptor to detect its ligand. For example, directly labeling DTLR, fusing onto it markers for secondary labeling, e.g., FLAG or other epitope tags, etc., will allow detection of receptor. This can be histological, as an affinity method for biochemical purification, or labeling or selection in an expression cloning approach. A two-hybrid selection system may also be applied making appropriate constructs with the available DTLR sequences. See, e.g., Fields and Song (1989) Nature 340:245-246.

Generally, descriptions of DTLRs will be analogously applicable to individual specific embodiments directed to DTLR2, DTLR3, DTLR4, DTLR5, DTLR6, DTLR7, DTLR8, DTLR9, and/or DTLR10 reagents and compositions.

The invention includes the following aspects:
Aspect A - A composition of matter selected from the group consisting of:
   a) a substantially pure or recombinant DTLR2 protein or peptide exhibiting identity over a length of at least about 12 amino acids to SEQ ID NO:4;
   b) a natural sequence DTLR2 of SEQ ID NO:4;
   c) a fusion protein comprising DTLR2 sequence;
   d) a substantially pure or recombinant DTLR3 protein or peptide exhibiting identity over a length of at least about 12 amino acids to SEQ ID NO:6;
   e) a natural sequence DTLR3 of SEQ ID NO:6;
   f) a fusion protein comprising DTLR3 sequence;
   g) a substantially pure or recombinant DTLR4 protein or peptide exhibiting identity over a length of at least about 12 amino acids to SEQ ID NO:8;
   h) a natural sequence DTLR4 of SEQ ID NO:8;
   i) a fusion protein comprising DTLR4 sequence;
   j) a substantially pure or recombinant DTLR5 protein or peptide exhibiting identity over a length of at least about 12 amino acids to SEQ ID NO:10;
   k) a natural sequence DTLR5 comprising SEQ ID NO:10;
   l) a fusion protein comprising DTLR5 sequence;
   m) a substantially pure or recombinant DTLR6 protein or peptide exhibiting identity over a length of at least about 12 amino acids to SEQ ID NO:12, 28 or 30;
   n) a natural sequence DTLR6 comprising SEQ ID NO:12, 28 or 30;
   o) a fusion protein comprising DTLR6 sequence;
   p) a substantially pure or recombinant DTLR7 protein or peptide exhibiting identity over a length of at least about 12 amino acids to SEQ ID NO: 16, 18, or 37;
   q) a natural sequence DTLR7 comprising SEQ ID NO:16, 18, or 37;
   r) a fusion protein comprising DTLR7 sequence;
   s) a substantially pure or recombinant DTLR8 protein or peptide exhibiting identity over a length of at least about 12 amino acids to SEQ ID NO:32 or 39;
   t) a natural sequence DTLR8 comprising SEQ ID NO:32 or 39;
   u) a fusion protein comprising DTLR8 sequence;
   v) a substantially pure or recombinant DTLR9 protein or peptide exhibiting identity over a length of at least about 12 amino acids to SEQ ID NO:22 or 41;
   w) a natural sequence DTLR9 comprising SEQ ID NO:22 or 41;
   x) a fusion protein comprising DTLR9 sequence;
   y) a substantially pure or recombinant DTLR10 protein or peptide exhibiting identity over a length of at least about 12 amino acids to SEQ ID NO:34, 43 or 45;
   z) a natural sequence DTLR10 comprising SEQ ID NO:34, 43 or 45;
   zz) a fusion protein comprising DTLR10 sequence.
Aspect B - A substantially pure or isolated protein comprising a segment exhibiting sequence identity to a corresponding portion of a:
   a) DTRL2 of Aspect A, and said identity is over at least:
      a) about 15 amino acids;
      b) about 19 amino acids; or
      c) about 25 amino acids;
   b) DTLR3 of Aspect A, and said identity is over at least:
      a) about 15 amino acids;
      b) about 19 amino acids; or
      c) about 25 amino acids;
   c) DTLR4 of Aspect A, and said identity is over at least:
      a) about 15 amino acids;
      b) about 19 amino acids; or
      c) about 25 amino acids;
   d) DTLR5 of Aspect A, and said identity is over at least:
      a) about 15 amino acids;
      b) about 19 amino acids; or
      c) about 25 amino acids;
   e) DTLR6 of Aspect A, and said identity is over at least:
      a) about 15 amino acids;
      b) about 19 amino acids; or
      c) about 25 amino acids;
   f) DTLR7 of Aspect A, and said identity is over at least:
      a) about 15 amino acids;
      b) about 19 amino acids; or
      c) about 25 amino acids;
   g) DTLR8 of Aspect A, and said identity is over at least:
      a) about 15 amino acids;
      b) about 19 amino acids; or
      c) about 25 amino acids;
   h) DTLR9 of Aspect A, and said identity is over at least:
      a) about 15 amino acids;
      b) about 19 amino acids; or
      c) about 25 amino acids;
   i) DTLR10 of Aspect A, and said identity is over at least:
      a) about 15 amino acids;
      b) about 19 amino acids; or
      c) about 25 amino acids;

### Aspect C - The composition of matter of Aspect A, wherein said:

a) DTLR2:
   i) comprises a mature sequence of Table 2; or
   ii) lacks post-translational modification;
b) DTLR3:
   i) comprises a mature sequence of Table 3; or
   ii) lacks post-translational modification;
   c) DTLR4:
      i) comprises a mature sequence of Table 4; or
      ii) lacks post-translational modification;
   d) DTLR5:
      i) comprises a mature sequence of Table 5; or
      ii) lacks post-translational modification;
   e) DTLR6:
      i) comprises a mature sequence of Table 6; or
      ii) lacks post-translational modification;
   f) DTLR7:
      i) comprises a mature sequence of Table 7; or
      ii) lacks post-translational modification;
   g) DTLR8:
      i) comprises a mature sequence of Table 8; or
      ii) lacks post-translational modification;
   h) DTLR9:
      i) comprises a mature sequence of Table 9; or
      ii) lacks post-translational modification;
   i) DTLR10:
      i) comprises a mature sequence of Table 10; or
      ii) lacks post-translational modification;
   j) protein or peptide:
      i) is from a warm blooded animal selected from a mammal, including a primate, such as a human;
      ii) comprises at least one polypeptide segment of SEQ ID NO:4, 6, 26, 10, 12, 28, 30, 16, 18, 37, 39, 32, 22, 34, 43, or 45;
      iii) exhibits a plurality of said segments of identity;
      iv) is a natural allelic variant of DTLR2, DTLR3, DTLR4, DTLR5, DTLR6, DTLR7, DTLR8, DTLR9 or DTLR10;
      v) has a length at least about 30 amino acids;
      vi) exhibits at least two non-overlapping epitopes which are specific for a primate DTLR2, DTLR3, DTLR4, DTLR5, DTLR6, DTLR7, DTLR8, DTLR9 or DTLR10;
      vii) exhibits sequence identity over a length of at least about 35 amino acids to a primate DTLR2, DTLR3, DTLR4, DTLR5, DTLR6, DTLR7, DTLR8, DTLR9 or DTLR10;
      viii) further exhibits at least two non-overlapping epitopes which are specific for a primate DTLR2, DTLR3, DTLR4, DTLR5, DTLR6, DTLR7, DTLR8, DTLR9 or DTLR10;
      ix) exhibits identity over a length of at least about 20 amino acids to a rodent DTLR6;
      x) is glycosylated;
      xi) has a molecular weight of at least 100 kD with natural glycosylation;
      xii) is a synthetic polypeptide;
      xiii) is attached to a solid substrate;
      xiv) is conjugated to another chemical moiety;
      xv) is a 5-fold or less substitution from natural sequence; or
      xvi) is a deletion or insertion variant from a natural sequence.

Aspect D - A composition comprising:
a) a sterile DTLR2 protein or peptide of Aspect A;
b) said DTLR2 protein or peptide of Aspect A and a carrier, wherein said carrier is:
   i) an aqueous compound, including water, saline, and/or buffer; and/or;
   ii) formulated for oral, rectal, nasal, topical, or parenteral administration;
c) a sterile DTLR3 protein or peptide of Aspect A;
d) said DTLR3 protein or peptide of Aspect A and a carrier, wherein said carrier is:
   i) an aqueous compound, including water, saline, and/or buffer; and/or;
   ii) formulated for oral, rectal, nasal, topical, or parenteral administration;
e) a sterile DTLR4 protein or peptide of Aspect A;
f) said DTLR4 protein or peptide of Aspect A and a carrier, wherein said carrier is:
   i) an aqueous compound, including water, saline, and/or buffer; and/or;
   ii) formulated for oral, rectal, nasal, topical, or parenteral administration;
g) a sterile DTLR5 protein or peptide of Aspect A;
h) said DTLR5 protein or peptide of Aspect A and a carrier, wherein said carrier is:
   i) an aqueous compound, including water, saline, and/or buffer; and/or;
   ii) formulated for oral, rectal, nasal, topical, or parenteral administration;
i) a sterile DTLR6 protein or peptide of Aspect A;
j) said DTLR6 protein or peptide of Aspect A and a carrier, wherein said carrier is:
   i) an aqueous compound, including water, saline, and/or buffer; and/or;
   ii) formulated for oral, rectal, nasal, topical, or parenteral administration;
k) a sterile DTLR7 protein or peptide of Aspect A;
l) said DTLR7 protein or peptide of Aspect A and a carrier, wherein said carrier is:
   i) an aqueous compound, including water, saline, and/or buffer; and/or;
   ii) formulated for oral, rectal, nasal, topical, or parenteral administration;
m) a sterile DTLR8 protein or peptide of Aspect A;
n) said DTLR8 protein or peptide of Aspect A and a carrier, wherein said carrier is:
   i) an aqueous compound, including water, saline, and/or buffer; and/or;
   ii) formulated for oral, rectal, nasal, topical, or parenteral administration;
o) a sterile DTLR9 protein or peptide of Aspect A;
p) said DTLR9 protein or peptide of Aspect A and a carrier, wherein said carrier is:
   i) an aqueous compound, including water, saline, and/or buffer; and/or;
   ii) formulated for oral, rectal, nasal, topical, or parenteral administration;
q) a sterile DTLR10 protein or peptide of Aspect A;
r) said DTLR10 protein or peptide of Aspect A and a carrier, wherein said carrier is:
   i) an aqueous compound, including water, saline, and/or buffer; and/or;
   ii) formulated for oral, rectal, nasal, topical, or parenteral administration;

Aspect E- The fusion protein of Aspect A, comprising:
a) mature protein comprising sequence of Table 2, 3, 4, 5, 6, 7, 8, 9, or 10;
b) a detection or purification tag, including a FLAG, His6, or Ig sequence; or
c) sequence of another receptor protein.

Aspect F - A kit comprising a protein or polypeptide of Aspect A, and:
a) a compartment comprising said protein or polypeptide; and/or
b) instructions for use or disposal of reagents in said kit.

Aspect G - A binding compound comprising an antigen binding site from an antibody, which specifically binds to a natural DTLR2, DTLR3, DTLR4, DTLRS, DTLR6, DTLR7, DTLR8, DTLR9, or DTLR10 protein of Aspect A, wherein:
a) said protein is a primate protein;
b) said binding compound is an Fv, Fab, or Fab2 fragment;
c) said binding compound is conjugated to another chemical moiety; or
d) said antibody:
   i) is raised against a peptide sequence of a mature polypeptide of Table 2, 3, 4, 5, 6, 7, 8, 9, or 10;
   ii) is raised against a mature DTLR2, DTLR3, DTLR4, DTLRS, DTLR6, DTLR7, DTLR8, DTLR9, or DTLR10;
   iii) is raised to a purified human DTLR2, DTLR3, DTLR4, DTLR5, DTLR6, DTLR7, DTLR8, DTLR9, or DTLR10;
   iv) is immunoselected;
   v) is a polyclonal antibody;
   vi) binds to a denatured DTLR2, DTLR3, DTLR4, DTLR5, DTLR6, DTLR7, DTLR8, DTLR9, or DTLR10;
   vii) exhibits a Kd to antigen of at least 30µM;
   viii) is attached to a solid substrate, including a bead or plastic membrane;
   ix) is in a sterile composition; or
   x) is detectably labeled, including a radioactive or fluorescent label.

Aspect H - A kit comprising said binding compound of Aspect G, and:
a) a compartment comprising said binding compound; and/or
b) instructions for use or disposal of reagents in said kit.

Aspect l - A method of:
A) making an antibody of Aspect G, comprising immunizing an immune system with an immunogenic amount of:
   a) a primate DTLR2;
   b) a primate DTLR3;
   c) a primate DTLR4;
   d) a primate DTLR5;
   e) a primate DTLR6;
   f) a primate DTLR7;
   g) a primate DTLR8;
   h) a primate DTLR9; or
   i) a primate DTLR10;
   thereby causing said antibody to be produced; or
B) producing an antigen: antibody complex, comprising contacting an antibody of Aspect G with:
   a) a mammalian DTLR2 protein or peptide;
   b) a mammalian DTLR3 protein or peptide;
   c) a mammalian DTLR4 protein or peptide;
   d) a mammalian DTLR5 protein or peptide;
   e) a mammalian DTLR6 protein or peptide;
   f) a mammalian DTLR7 protein or peptide;
   g) a mammalian DTLR8 protein or peptide;
   h) a mammalian DTLR9 protein or peptide; or
   i) a mammalian DTLR10 protein or peptide;
   thereby allowing said complex to form.

Aspect J - A composition comprising:
i) a sterile binding compound of Aspect G, or
ii) said binding compound of Aspect G and a carrier,
   wherein said carrier is:
   i) an aqueous compound, including water, saline, and/or buffer; and/or
   ii) formulated for oral, rectal, nasal, topical, or parenteral administration.

Aspect K - An isolated or recombinant nucleic acid encoding a protein or peptide or fusion protein of Aspect A, wherein:
a) said DTLR is from a mammal; or
b) said nucleic acid:
   i) encodes an antigenic peptide sequence of Table 2, 3, 4, 5, 6, 7, 8, 9, or 10;
   ii) encodes a plurality of antigenic peptide sequences of 2, 3, 4, 5, 6, 7, 8, 9, or 10;
   iii) exhibits at least about 80% identity to a natural cDNA encoding said segment;
   iv) is an expression vector;
   v) further comprises an origin of replication;
   vi) is from a natural source;
   vii) comprises a detectable label;
   viii) comprises synthetic nucleotide sequence;
   ix) is less than 6 kb, preferably less than 3 kb;
   x) is from a mammal, including a primate;
   xi) comprises a natural full length coding sequence;
   xii) is a hybridization probe for a gene encoding said DTLR;
   xiii) comprises at least 17 contiguous nucleotides from Table 2, 3, 4, 5, 6, 7, 8, 9, or 10;
   xiv) comprises a plurality of nonoverlapping segments of least 17 contiguous nucleotides from Table 2, 3, 4, 5, 6, 7, 8, 9, or 10; or
   xv) is a PCR primer, PCR product, or mutagenesis primer.

Aspect L - A cell, tissue, or organ comprising a recombinant nucleic acid of Aspect K.

Aspect M - The cell of Aspect L, wherein said cell is:
a) a prokaryotic cell;
b) a eukaryotic cell;
c) a bacterial cell;
d) a yeast cell;
e) an insect cell;
f) a mammalian cell;
g) a mouse cell;
h) a primate cell; or
i) a human cell.

Aspect N - A kit comprising said nucleic acid of Aspect K, and:
a) a compartment comprising said nucleic acid;
b) a compartment further comprising a primate DTLR2, DTLR3, DTLR4, DTLR5, DTLR6, DTLR7, DTLR8, DTLR9, or DTLR10 protein or polypeptide; and/or
c) instructions for use or disposal of reagents in said kit.

Aspect O - A method of:
A) making a polypeptide, comprising expressing said nucleic acid of Aspect K, thereby producing said polypeptide; or
B) making a duplex nucleic acid, comprising contacting said nucleic acid of Aspect K with a complementary nucleic acid, thereby allowing said duplex to form.

Aspect P - A nucleic acid which:
a) hybridizes under wash conditions of 30° C and less than 2M salt to SEQ ID NO: 3;
b) hybridizes under wash conditions of 30° C and less than 2 M salt to SEQ ID NO: 5;
c) hybridizes under wash conditions of 30° C and less than 2M salt to SEQ ID NO: 25;
d) hybridizes under wash conditions of 30° C and less than 2 M salt to SEQ ID NO: 9;
e) hybridizes under wash conditions of 30° C and less than 2M salt to SEQ ID NO: 11, 27, or 29;
f) hybridizes under wash conditions of 30° C and less than 2 M salt to SEQ ID NO: 15, 17, or 36;
g) hybridizes under wash conditions of 30° C and less than 2M salt to SEQ ID NO: 31 or 38;
h) hybridizes under wash conditions of 30° C and less than 2 M salt to SEQ ID NO: 21 or 40;
i) hybridizes under wash conditions of 30° C and less than 2 M salt to SEQ ID NO: 33, 35, 42, or 44;
j) exhibits at least about 85% identity over a stretch of at least about 30 nucleotides to a primate DTLR2;
k) exhibits at least about 85% identity over a stretch of at least about 30 nucleotides to a primate DTLR3;
l) exhibits at least about 85% identity over a stretch of at least about 30 nucleotides to a primate DTLR4;
m) exhibits at least about 85% identity over a stretch of at least about 30 nucleotides to a primate DTLR5;
n) exhibits at least about 85% identity over a stretch of at least about 30 nucleotides to a primate DTLR6;
o) exhibits at least about 85% identity over a stretch of at least about 30 nucleotides to a primate DTLR7;
p) exhibits at least about 85% identity over a stretch of at least about 30 nucleotides to a primate DTLR8;
q) exhibits at least about 85% identity over a stretch of at least about 30 nucleotides to a primate DTLR9; or
r) exhibits at least about 85% identity over a stretch of at least about 30 nucleotides to a primate DTLR10.

Aspect Q - The nucleic acid of Aspect P, wherein:
a) said wash conditions are at 45° C and/or 500 mM salt; or
b) said identity is at least 90% and/or said stretch is at least 55 nucleotides.

Aspect R - The nucleic acid of Aspect Q, wherein:
a) said wash conditions are at 55° C and/or 150 mM salt; or
b) said identity is at least 95% and/or said stretch is at least 75 nucleotides.

Aspect S - A method of producing a ligand: receptor complex, comprising contacting:
a) a substantially pure primate DTLR2, including a recombinant or synthetically produced protein, with candidate Toll ligand;
b) a substantially pure primate DTLR3, including a recombinant or synthetically produced protein, with candidate Toll ligand;
c) a substantially pure primate DTLR4, including a recombinant or synthetically produced protein, with candidate Toll ligand;
d) a substantially pure primate DTLR5, including a recombinant or synthetically produced protein, with candidate Toll ligand;
e) a substantially pure primate DTLR6, including a recombinant or synthetically produced protein, with candidate Toll ligand;
f) a substantially pure primate DTLR7, including a recombinant or synthetically produced protein, with candidate Toll ligand;
g) a substantially pure primate DTLR8, including a recombinant or synthetically produced protein, with candidate Toll ligand;
h) a substantially pure primate DTLR9, including a recombinant or synthetically produced protein, with candidate Toll ligand;
i) a substantially pure primate DTLR10, including a recombinant or synthetically produced protein, with candidate Toll ligand;
   thereby allowing said complex to form.

Aspect T - A method of modulating physiology or development of a cell or tissue culture cells comprising contacting said cell with an agonist or antagonist of a mammalian DTLR2, DTLR3, DTLR4, DTLR5, DTLR6, DTLR7, DTLR8, DTLR9, or DTLR10.

Aspect U - The method of Aspect T, wherein said agonist or antagonist is of DTLR10, and said cell is a pDC2 cell.

The broad scope of this invention is best understood with reference to the following examples, which are not intended to limit the inventions to the specific embodiments.

### EXAMPLES

### I. General Methods

Some of the standard methods are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. See, e.g., Ausubel, et al. (1987 and periodic supplements); Coligan, et al. (ed. 1996) and periodic supplements, Current Protocols In Protein Science Greene/Wiley, New York; Deutscher (1990) "Guide to Protein Purification" in Methods in Enzymology, vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1989) Chemische Industrie 12:69-70; Hochuli (1990) "Purification of Recombinant Proteins with Metal Chelate Absorbent" in Setlow (ed.) Genetic Engineering,Principle and Methods 12:87-98, Plenum Press, N.Y.; and Crowe, et al. (1992) QIAexpress: The High Level Expression and Protein Purification System QUIAGEN, Inc., Chatsworth, CA.

Standard immunological techniques and assays are described, e.g., in Hertzenberg, et al. (eds. 1996) Weir's Handbook of Experimental Immunology vols. 1-4, Blackwell Science; Coligan (1991) Current Protocols in Immunology Wiley/Greene, NY; and Methods in Enzymology volumes. 70, 73, 74, 84, 92, 93, 108, 116, 121, 132, 150, 162, and 163.

Assays for vascular biological activities are well known in the art. They will cover angiogenic and angiostatic activities in tumor, or other tissues, e.g., arterial smooth muscle proliferation (see, e.g., Koyoma, et al. (1996) Cell 87:1069-1078), monocyte adhesion to vascular epithelium (see McEvoy, et al. (1997) J. Exp. Med. 185:2069-2077), etc. See also Ross (1993) Nature 362:801-809; Rekhter and Gordon (1995) Am. J. Pathol. 147:668-677; Thyberg, et al. (1990) Atherosclerosis 10:966-990; and Gumbiner (1996) Cell 84:345-357.

Assays for neural cell biological activities are described, e.g., in Wouterlood (ed. 1995) Neuroscience Protocols modules 10, Elsevier; Methods in Neurosciences Academic Press; and Neuromethods Humana Press, Tptowa, NJ. Methodology of developmental systems is described, e.g., in Meisami (ed.) Handbook of Human Growth and Developmental Biology CRC Press; and Chrispeels (ed.) Molecular Techniques and Approaches in Developmental Biology Interscience.

Computer sequence analysis is performed, e.g., using available software programs, including those from the GCG (U. Wisconsin) and GenBank sources. Public sequence databases were also used, e.g., from GenBank, NCBI, EMBO, and others. Determination of transmembrane and other important motifs may be predicted using such bioinformatics tools.

Many techniques applicable to IL-10 receptors may be applied to DTLRs, as described, e.g., in USSN 08/110,683 (IL-10 receptor), which is incorporated herein by reference for all purposes.

### II. Novel Family of Human Receptors

Abbreviations: DTLR, DNAX Toll-like receptor; IL-1R, interleukin-1 receptor; TH, Toll homology; LRR, leucine-rich repeat; EST, expressed sequence tag; STS, sequence tagged site; FISH, fluorescence in situ hybridization.

The discovery of sequence homology between the cytoplasmic domains of Drosophila Toll and human interleukin-1 (IL-1) receptors has sown the conviction that both molecules trigger related signaling pathways tied to the nuclear translocation of Rel-type transcription factors. This conserved signaling scheme governs an evolutionarily ancient immune response in both insects and vertebrates. We report the molecular cloning of a novel class of putative human receptors with a protein architecture that is closely similar to Drosophila Toll in both intra- and extra-cellular segments. Five human Toll-like receptors, designated DTLRs 1-5, are likely the direct homologs of the fly molecule, and as such could constitute an important and unrecognized component of innate immunity in humans; intriguingly, the evolutionary retention of DTLRs in vertebrates may indicate another role, akin to Toll in the dorso-ventralization of the Drosophila embryo, as regulators of early morphogenetic patterning. Multiple tissue mRNA blots indicate markedly different patterns of expression for the human DTLRs. Using fluorescence in situ hybridization and Sequence-Tagged Site database analyses, we also show that the cognate DTLR genes reside on chromosomes 4 (DTLRs 1, 2, and 3), 9 (DTLR4), and 1 (DTLR5). Structure prediction of the aligned Toll-homology (TH) domains from varied insect and human DTLRs, vertebrate IL-1 receptors, and MyD88 factors, and plant disease resistance proteins, recognizes a parallel β/α fold with an acidic active site; a similar structure notably recurs in a class of response regulators broadly involved in transducing sensory information in bacteria.

The seeds of the morphogenetic gulf that so dramatically separates flies from humans are planted in familiar embryonic shapes and patterns, but give rise to very different cell complexities. DeRobertis and Sasai (1996) Nature 380:37-40; and Arendt and Nubler-Jung (1997) Mech. Develop. 61:7-21. This divergence of developmental plans between insects and vertebrates is choreographed by remarkably similar signaling pathways, underscoring a greater conservation of protein networks and biochemical mechanisms from unequal gene repertoires. Miklos and Rubin (1996) Cell 86:521-529; and Chothia (1994) Develop. 1994 Suppl., 27-33. A powerful way to chart the evolutionary design of these regulatory pathways is by inferring their likely molecular components (and biological functions) through interspecies comparisons of protein sequences and structures. Miklos and Rubin (1996) Cell 86:521-529; Chothia (1994) Develop. 1994 Suppl., 27-33 (3-5); and Banfi, et al. (1996) Nature Genet. 13:167-174.

A universally critical step in embryonic development is the specification of body axes, either born from innate asymmetries or triggered by external cues. DeRobertis and Sasai (1996) Nature 380:37-40; and Arendt and Nübler-Jung (1997) Mech. Develop. 61:7-21. As a model system, particular attention has been focused on the phylogenetic basis and cellular mechanisms of dorsoventral polarization. DeRobertis and Sasai (1996) Nature 380:37-40; and Arendt and Nübler-Jung (1997) Mech. Develop. 61:7-21. A prototype molecular strategy for this transformation has emerged from the Drosophila embryo, where the sequential action of a small number of genes results in a ventralizing gradient of the transcription factor Dorsal. St. Johnston and Nüsslein-Volhard (1992) Cell 68:201-219; and Morisato and Anderson (1995) Ann. Rev. Genet. 29:371-399.

This signaling pathway centers on Toll, a transmembrane receptor that transduces the binding of a maternally-secreted ventral factor, Spätzle, into the cytoplasmic engagement of Tube, an accessory molecule, and the activation of Pelle, a Ser/Thr·kinase that catalyzes the dissociation of Dorsal from the inhibitor Cactus and allows migration of Dorsal to ventral nuclei (Morisato and Anderson (1995) Ann. Rev. Genet. 29:371-399; and Belvin and Anderson (1996) Ann. Rev. Cell Develop. Biol. 12:393-416. The Toll pathway also controls the induction of potent antimicrobial factors in the adult fly (Lemaitre, et al. (1996) Cell 86:973-983); this role in Drosophila immune defense strengthens mechanistic parallels to IL-1 pathways that govern a host of immune and inflammatory responses in vertebrates. Belvin and Anderson (1996) Ann. Rev. Cell Develop. Biol. 12:393-416: and Wasserman (1993) Molec. Biol. Cell 4:767-771. A Toll-related cytoplasmic domain in IL-1 receptors directs the binding of a Pelle-like kinase, IRAK, and the activation of a latent NF-κB/I-κB complex that mirrors the embrace of Dorsal and Cactus. Belvin and Anderson (1996) Ann. Rev. Cell Develop. Biol. 12:393-416; and Wasserman (1993) Molec. Biol. Cell 4:767-771.

We describe the cloning and molecular characterization of four new Toll-like molecules in humans, designated DTLRs 2-5 (following Chiang and Beachy (1994) Mech. Develop. 47:225-239), that reveal a receptor family more closely tied to Drosophila Toll homologs than to vertebrate IL-1 receptors. The DTLR sequences are derived from human ESTs; these partial cDNAs were used to draw complete expression profiles in human tissues for the five DTLRs, map the chromosomal locations of cognate genes, and narrow the choice of cDNA libraries for full-length cDNA retrievals. Spurred by other efforts (Banfi, et al. (1996) Nature Genet. 13:167-174; and Wang, et al. (1996) J. Biol. Chem. 271:4468-4476), we are assembling, by structural conservation and molecular parsimony, a biological system in humans that is the counterpart of a compelling regulatory scheme in Drosophila. In addition, a biochemical mechanism driving Toll signaling is suggested by the proposed tertiary.fold of the Toll-homology (TH) domain, a core module shared by DTLRs, a broad family of IL-1 receptors, mammalian MyD88 factors and plant disease resistance proteins. Mitcham, et al. (1996) J. Biol. Chem. 271:5777-5783; and Hardiman, et al. (1996) Oncogene 13:2467-2475. We propose that a signaling route coupling morphogenesis and primitive immunity in insects, plants, and animals (Belvin and Anderson (1996) Ann. Rev. Cell Develop. Biol. 12:393-416; and Wilson, et al. (1997) Curr. Biol. 7:175-178) may have roots in bacterial two-component pathways.

### Computational Analysis.

Human sequences related to insect DTLRs were identified from the EST database (dbEST) at the National Center for Biotechnology Information (NCBI) using the BLAST server (Altschul, et al. (1994) Nature Genet. 6:119-129). More sensitive pattern- and profile-based methods (Bork and Gibson (1996) Meth. Enzymol. 266:162-184) were used to isolate the signaling domains of the DTLR family that are shared with vertebrate and plant proteins present in nonredundant databases. The progressive alignment of DTLR intra- or extracellular domain sequences was carried out by ClustalW (Thompson, et al. (1994) Nucleic Acids Res. 22:4673-4680); this program also calculated the branching order of aligned sequences by the Neighbor-Joining algorithm (5000 bootstrap replications provided confidence values for the tree groupings).

Conserved alignment patterns, discerned at several degrees of stringency, were drawn by the Consensus program (internet URL http://www.bork.embl-heidelberg.de/Alignment/ consensus.html). The PRINTS library of protein fingerprints (http://www.biochem.ucl.ac.uk/bsm/dbbrowser/PRINTS/PRINTS.html) (Attwood, et al. (1997) Nucleic Acids Res. 25:212-217) reliably identified the, myriad leucine-rich repeats (LRRs) present in the extracellular segments of DTLRs with a compound motif (PRINTS code Leurichrpt) that flexibly matches N- and C-terminal features of divergent LRRs. Two prediction algorithms whose three-state accuracy is above 72% were used to derive a consensus secondary structure for the intracellular domain alignment, as a bridge to fold recognition efforts (Fischer, et al. (1996) FASEB J. 10:126-136) . Both the neural network program PHD (Rost and Sander (1994) Proteins 19:55-72) and the statistical prediction method DSC (King and Sternberg (1996) Protein Sci. 5:2298-2310) have internet servers (URLs http://www.embl-heidelberg.de/predictprotein/phd_pred.html and http://bonsai.lif.icnet. uk/bmm/dsc/dsc_read_align.html, respectively). The intracellular region encodes the THD region discussed, e.g., in Hardiman, et al. (1996) Oncogene 13:2467-2475; and Rock, et al. (1998) Proc. Nat'l Acad. Sci. USA 95:588-593; each of which is incorporated herein by reference. This domain is very important in the mechanism of signaling by the receptors, which transfers a phosphate group to a substrate.

### Cloning of full-length human DTLR cDNAs.

PCR primers derived from the Toll-like Humrsc786 sequence (GenBank accession code D13637) (Nomura, et al. (1994) DNA Res. 1:27-35) were used to probe a human erythroleukemic, TF-1 cell line-derived cDNA library (Kitamura, et al. (1989) Blood 73:375-380) to yield the DTLR1 cDNA sequence. The remaining DTLR sequences were flagged from dbEST. and the relevant EST clones obtained from the I.M.A.G.E. consortium (Lennon, et al. (1996) Genomics 33:151-152) via Research Genetics (Huntsville, AL) : CloneID#'s 80633 and 117262 (DTLR2), 144675 (DTLR3), 202057 (DTLR4) and 277229 (DTLR5). Full length cDNAs for human DTLRs 2-4 were cloned by DNA hybridization screening of λgt10 phage, human adult lung, placenta, and fetal liver 5'-Stretch Plus cDNA libraries (Clontech), respectively; the DTLR5 sequence is derived from a human multiple-sclerosis plaque EST. All positive clones were sequenced and aligned to identify individual DTLR ORFs: DTLR1 (2366 bp clone, 786 aa ORF), DTLR2 (2600 bp, 784 aa), DTLR3 (3029 bp, 904 aa), DTLR4 (3811 bp, 879 aa) and DTLR5 (1275 bp, 370 aa). Similar methods are used for DTLRs 6-10. Probes for DTLR3 and DTLR4 hybridizations were generated by PCR using human placenta (Stratagene) and adult liver (Clontech) cDNA libraries as templates, respectively; primer pairs were derived from the respective EST sequences. PCR reactions were conducted using T. aquaticus Taqplus DNA polymerase (Stratagene) under the following conditions: 1 x (94° C, 2 min) 30 x (55° C, 20 sec; 72° C 30 sec; 94° C 20 sec) , 1 x (72° C, 8 min). For DTLR2 full-length cDNA screening, a 900 bp fragment generated by EcoRI/XbaI digestion of the first EST clone (ID# 80633) was used as a probe.

### mRNA blots and chromosomal localization.

Human multiple tissue (Cat# 1, 2) and cancer cell line blots (Cat# 7757-1), containing approximately 2 µg of poly(A)⁺ RNA per lane, were purchased from Clontech (Palo Alto, CA). For DTLRs 1-4, the isolated full-length cDNAs served as probes, for DTLR5 the EST clone (ID #277229) plasmid insert was used. Briefly, the probes were radiolabeled with [α-³²P] dATP using the Amersham Rediprime random primer labeling kit (RPN1633). Prehybridization and hybridizations were performed at 65° C in 0.5 M Na₂HPO₄, 7% SDS, 0.5 M EDTA (pH 8.0). All stringency washes were conducted at 65°C with two initial washes in 2 x SSC, 0.1% SDS for 40 min followed by a subsequent wash in 0.1 x SSC, 0.1% SDS for 20 min. Membranes were then exposed at -70° C to X-Ray film (Kodak) in the presence of intensifying screens. More detailed studies by cDNA library Southerns (14) were performed with selected human DTLR·clones to examine their expression in hemopoietic cell subsets.

Human chromosomal mapping was conducted by the method of fluorescence in situ hybridization (FISH) as described in Heng and Tsui (1994) Meth. Molec. Biol. 33:109-122, using the various full-length (DTLRs 2-4) or partial (DTLR5) cDNA clones as probes. These analyses were performed as a service by SeeDNA Biotech Inc. (Ontario, Canada). A search for human syndromes (or mouse defects in syntenic loci) associated with the mapped DTLR genes was conducted in the Dysmorphic Human-Mouse Homology Database by internet server (http://www.hgmp.mrc.ac.uk/DHMHD/ hum_chrome1.html). Similar methods nare applicable to DTLRs 6-10.

### Conserved architecture of insect and human DTLR ectodomains.

The Toll family in Drosophila comprises at least four distinct gene products: Toll, the prototype receptor involved in dorsoventral patterning of the fly embryo (Morisato and Anderson (1995) Ann. Rev. Genet. 29:371-399) and a second named '18 Wheeler' (18w) that may also be involved in early embryonic development (Chiang and Beachy (1994) Mech. Develop. 47:225-239; Eldon, et al. (1994) Develop. 120:885-899); two additional receptors are predicted by incomplete, Toll-like ORFs downstream of the male-specific-transcript (Mst) locus (GenBank code X67703) or encoded by the 'sequence-tagged-site' (STS) Dm2245 (GenBank code G01378) (Mitcham, et al. (1996) J. Biol. Chem. 271:5777-5783): The extracellular segments of Toll and 18w are distinctively composed of imperfect, -24 amino acid LRR motifs (Chiang and Beachy (1994) Mech. Develop. 47:225-239; and Eldon, et al. (1994) Develop. 120:885-899). Similar tandem arrays of LRRs commonly form the adhesive antennae of varied cell surface molecules and their generic tertiary structure is presumed to mimic the horseshoe-shaped cradle of a ribonuclease inhibitor fold, where seventeen LRRs show a repeating β/α-hairpin, 28 residue motif (Buchanan and Gay (1996) Prog. Biophys. Molec. Biol. 65:1-99). The specific recognition of Spätzle by Toll may follow a model proposed for the binding of cystine-knot fold glycoprotein hormones by the multi-LRR ectodomains of serpentine receptors, using the concave side of the curved β-sheet (Kajava, et al. (1995) Structure 3:867-877); intriguingly, the pattern of cysteines in Spätzle, and an orphan Drosophila ligand, Trunk, predict a similar cystine-knot tertiary structure (Belvin and Anderson (1996) Ann. Rev. Cell Develop. Biol. 12:393-416; and Casanova, et al. (1995) Genes Develop. 9:2539-2544) .

The 22 and 31 LRR ectodomains of Toll and 18w, respectively (the Mst ORF fragment displays 16 LRRs), are most closely related to the comparable 18, 19, 24, and 22 LRR arrays of DTLRs 1-4 (the incomplete DTLR5 chain presently includes four membrane-proximal LRRs) by sequence and pattern analysis (Altschul, et al. (1994) Nature Genet. 6:119-129; and Bork and Gibson (1996) Meth. Enzymol. 266:162-184) (Fig. 1). However, a striking difference in the human DTLR chains is the common loss of a ~90 residue cysteine-rich region that is variably embedded in the ectodomains of Toll, 18w and the Mst ORF (distanced four, six and two LRRs, respectively, from the membrane boundary). These cysteine clusters are bipartite, with distinct 'top' (ending an LRR) and 'bottom' (stacked atop an LRR) halves (Chiang and Beachy (1994) Mech. Develop. 47:225-239; Eldon, et al. (1994) Develop. 120:885-899; and Buchanan and Gay (1996) Prog. Biophys. Molec. Biol. 65:1-44); the 'top' module recurs in both Drosophila and human DTLRs as a conserved juxtamembrane spacer (Fig. 1). We suggest that the flexibly located cysteine clusters in Drosophila receptors (and other LRR proteins), when mated 'top' to 'bottom', form a compact module with paired termini that can be inserted between any pair of LRRs without altering the overall fold of DTLR ectodomains; analogous 'extruded' domains decorate the structures of other proteins (Russell (1994) Protein Engin. 7:1407-1410).

### Molecular design of the TH signaling domain.

Sequence comparison of Toll and IL-1 type-I (IL-1R1) receptors has disclosed a distant resemblance of a -200 amino acid cytoplasmic domain that presumably mediates signaling by similar Rel-type transcription factors. Belvin and Anderson (1996) Ann. Rev. Cell Develop. Biol. 12:393-416; and (Belvin and Anderson (1996) Ann. Rev. Cell Develop. Biol. 12:393-416; and Wasserman (1993) Molec. Biol. Cell 4:767-771). More recent additions to this functional paradigm include a pair of plant disease resistance proteins from tobacco and flax that feature an N-terminal TH module followed by nucleotide-binding (NTPase) and LRR segments (Wilson, et al. (1997) Curr. Biol. 7:175-178); by contrast, a 'death domain' precedes the TH chain of MyD88, an intracellular myeloid differentiation marker (Mitcham, et al. (1996) J. Biol. Chem. 271:5777-5783; and Hardiman, et al. (1996) Oncogene 13:2467-2475) (Fig. 1). New IL-1-type receptors include IL-1R3, an accessory signaling molecule, and orphan receptors IL-1R4 (also called ST2/Fit-1/T1), IL-1R5 (IL-1R-related protein), and IL-1R6 (IL-1R-related protein-2) (Mitcham, et al. (1996) J. Biol. Chem. 271:5777-5783;Hardiman, et al. (1996) Oncogene 13:2467-2475). With the new human DTLR sequences, we have sought a structural definition of this evolutionary thread by analyzing the conformation of the common TH module: ten blocks of conserved sequence comprising 128 amino acids form the minimal TH domain fold; gaps in the alignment mark the likely location of sequence and length-variable loops (Fig. 2A-2B).

Two prediction algorithms that take advantage of the patterns of conservation and variation in multiply aligned sequences, PHD (Rost and Sander (1994) Proteins 19:55-72) and DSC (King and Sternberg (1996) Protein Sci. 5:2298-2310), produced strong, concordant results for the TH signaling module (Fig. 2A-2B). Each block contains a discrete secondary structural element: the imprint of alternating β-strands (labeled A-E) and α-helices (numbered 1-5) is diagnostic of a β/α-class fold with α-helices on both faces of a parallel β-sheet. Hydrophobic β-strands A, C and D are predicted to form 'interior' staves in the β-sheet, while the shorter, amphipathic β-strands B and E resemble typical 'edge' units (Fig. 2A-2B). This assignment is consistent with a strand order of B-A-C-D-E in the core β-sheet (Fig. 2C); fold comparison ('mapping') and recognition ('threading') programs (Fischer, et al. (1996) FASEB J. 10:126-136) strongly return this doubly wound β/α topology. A surprising, functional prediction of this outline structure for the TH domain is that many of the conserved, charged residues in the multiple alignment map to the C-terminal end of the β-sheet: residue Asp16 (block numbering scheme - Fig. 2A-2B) at the end of βA, Arg39 and Asp40 following βB, Glu75 in the first turn of α3, and the more loosely conserved Glu/Asp residues in the βD-α4 loop, or after βE (Fig. 2A-2B). The location of four other conserved residues (Asp7, Glu28, and the Arg57-Arg/Lys58 pair) is compatible with a salt bridge network at the opposite, N-terminal end of the β-sheet (Fig. 2A-2B) . Alignment of the other DTLR embodiments exhibit similar features, and peptide segments comprising these feataures, e.g., 20 amino acid segments containing them, are particularly important.

Signaling function depends on the structural integrity of the TH domain. Inactivating mutations or deletions within the module boundaries (Fig. 2A-2B) have been catalogued for IL-1R1 and Toll. Heguy, et al. (1992) J. Biol. Chem. 267:2605-2609; Croston, et al. (1995) J. Biol. Chem. 270:16514-16517; Schneider, et al. (1991) Genes Develop. 5:797-807; Norris and Manley. (1992) Genes Develop. 6:1654-1667; Norris and Manley (1995) Genes Develop. 9:358-369; and Norris and Manley (1996) Genes Develop. 10:862-872. The human DTLR1-5 chains extending past the minimal TH domain (8, 0, 6, 22 and 18 residue lengths, respectively) are most closely similar to the stubby, 4 aa 'tail' of the Mst ORF. Toll and 18w display unrelated 102 and 207 residue tails (Fig. 2A-2B) that may negatively regulate the signaling of the fused TH domains. Norris and Manley (1995) Genes Develop. 9:358-369; and Norris and Manley (1996) Genes Develop. 10:B62-872.

The evolutionary relationship between the disparate proteins that carry the TH domain can best be discerned by a phylogenetic tree derived from the multiple alignment (Fig. 3). Four principal branches segregate the plant proteins, the MyD88 factors, IL-1 receptors, and Toll-like molecules; the latter branch clusters the Drosophila and human DTLRs.

### Chromosomal dispersal of human DTLR genes.

In order to investigate the genetic linkage of the nascent human DTLR gene family, we mapped the chromosomal loci of four of the five genes by FISH (Fig. 4). The DTLR1 gene has previously been charted by the human genome project: an STS database locus (dbSTS accession number G06709, corresponding to STS WI-7804 or SHGC-12827.) exists for the Humrsc786 cDNA (Nomura, et al. (1994) DNA Res. 1:27-35) and fixes the gene to chromosome 4 marker interval D4S1587-D92405 (50-56 cM) circa 4p14. This assignment has recently been corroborated by FISH analysis. Taguchi, et al. (1996) Genomics 32: 486-488. In the present work, we reliably assign the remaining DTLR genes to loci on chromosome 4q32 (DTLR2), 4q35 (DTLR3), 9q32-33 (DTLR4) and 1q33.3 (DTLRS). During the course of this work, an STS for the parent DTLR2 EST (cloneID # 80633) has been generated (dbSTS accession number T57791 for STS SHGC-33147) and maps to the chromosome 4 marker interval D4S424-D4S1548 (143-153 cM) at 4q32 -in accord with our findings. There is a -50 cM gap between DTLR2 and DTLR3 genes on the long arm of chromosome 4.

### DTLR genes are differentially expressed.

Both Toll and 18w have complex spatial and temporal patterns of expression in Drosophila that may point to functions beyond embryonic patterning. St. Johnston and Nüsslein-Volhard (1992) Cell 68:201-219; Morisato and Anderson (1995) Ann. Rev. Genet. 29:371-399; Belvin and Anderson (1996) Ann. Rev. Cell Develop. Biol. 12:393-416; Lemaitre, et al. (1996) Cell 86:973-983; Chiang and Beachy (1994) Mech. Develop. 47:225-239; and Eldon, et al. (1994) Develop. 120:885-899. We have examined the spatial distribution of DTLR transcripts by mRNA blot analysis with varied human tissue and cancer cell lines using radiolabeled DTLR cDNAs (Fig. 5). DTLR1 is found to be ubiquitously expressed, and at higher levels than the other receptors. Presumably reflecting alternative splicing, 'short' 3.0 kB and 'long' 8.0 kB DTLR1 transcript forms are present in ovary and spleen, respectively (Fig. 5, panels A and B). A cancer cell mRNA panel also shows the prominent overexpression of DTLR1 in a Burkitt's Lymphoma Raji cell line (Fig. 5, panel C). DTLR2 mRNA is less widely expressed than DTLR1, with a 4.0 kB species detected in lung and a 4.4 kB transcript evident in heart, brain and muscle. The tissue distribution pattern of DTLR3 echoes that of DTLR2 (Fig. 5, panel E). DTLR3 is also present as two major transcripts of approximately 4.0 and 6.0 kB in size, and the highest levels of expression are observed in placenta and pancreas. By contrast, DTLR4 and DTLR5 messages appear to be extremely tissue-specific. DTLR4 was detected only in placenta as a single transcript of -7.0 kB in size. A faint 4.0 kB signal was observed for DTLR5 in ovary and peripheral blood monocytes.

### Components of an evolutionarily ancient regulatory system.

The original molecular blueprints and divergent fates of signaling pathways can be reconstructed by comparative genomic approaches. Miklos and Rubin (1996) Cell 86:521-529; Chothia (1994) Develop. 1994 Suppl., 27-33; Banfi, et al. (1996) Nature Genet. 13:167-174; and Wang, et al. (1996) J. Biol. Chem. 271:4468-4476. We have used this logic to identify an emergent gene family in humans, encoding five receptor para logs at present, DTLRs 1-5, that are the direct evolutionary counterparts of a Drosophila gene family headed by Toll (Figs. 1-3). The conserved architecture of human and fly DTLRs, conserved LRR ectodomains and intracellular TH modules (Fig. 1), intimates that the robust pathway coupled to Toll in Drosophila (6, 7) survives in vertebrates. The best evidence borrows from a reiterated pathway: the manifold IL-1 system and its repertoire of receptor-fused TH domains, IRAK, NF-κB and I-κB homologs (Belvin and Anderson (1996) Ann. Rev. Cell Develop. Biol. 12:393-416; Wasserman (1993) Molec. Biol. Cell 4:767-771; Hardiman, et al. (1996) Oncogene 13:2467-2475; and Cao, et al. (1996) Science 271:1128-1131); a Tube-like factor has also been characterized. It is not known whether DTLRs can productively couple to the IL-1R signaling machinery, or instead, a parallel set of proteins is used. Differently from IL-1 receptors, the LRR cradle of human DTLRs is predicted to retain an affinity for Spätzle/Trunk-related cystine-knot factors; candidate DTLR ligands (called PENs) that fit this mold have been isolated.

Biochemical mechanisms of signal transduction can be gauged by the conservation of interacting protein folds in a pathway. Miklos and Rubin (1996) Cell 86:521-529; Chothia (1994) Develop. 1994 Suppl., 27-33. At present, the Toll signaling paradigm involves some molecules whose roles are narrowly defined by their structures, actions or fates: Pelle is a Ser/Thr kinase (phosphorylation), Dorsal is an NF-κB-like transcription factor (DNA-binding) and Cactus is an ankyrin-repeat inhibitor (Dorsal binding, degradation). Belvin and Anderson (1996) Ann. Rev. Cell Develop. Biol. 12:393-416. By contrast, the functions of the Toll TH domain and Tube remain enigmatic. Like other cytokine receptors (Heldin (1995) Cell 80:213-223), ligand-mediated dimerization of Toll appears to be the triggering event: free cysteines in the juxtamembrane region of Toll create constitutively active receptor pairs (Schneider, et al. (1991) Genes Develop. 5:797-807), and chimeric Torso-Toll receptors signal as dimers (Galindo, et al. (1995) Develop. 121:2209-2218); yet, severe truncations or wholesale loss of the Toll ectodomain results in promiscuous intracellular signaling (Norris and Manley (1995) Genes Develop. 9:358-369; and Winans and Hashimoto (1995) Molec. Biol. Cell 6:587-596), reminiscent of oncogenic receptors with catalytic domains (Heldin (1995) Cell 80:213-223). Tube is membrane-localized, engages the N-terminal (death) domain of Pelle and is phosphorylated, but neither Toll-Tube or Toll-Pelle interactions are registered by two-hybrid analysis (Galindo, et al. (1995) Develop. 121:2209-2218; and Groβhans, et al. (1994) Nature 372:563-566); this latter result suggests that the conformational 'state' of the Toll TH domain somehow affects factor recruitment. Norris and Manley (1996) Genes Develop. 10:862-872; and Galindo, et al. (1995) Develop. 121:2209-2218.

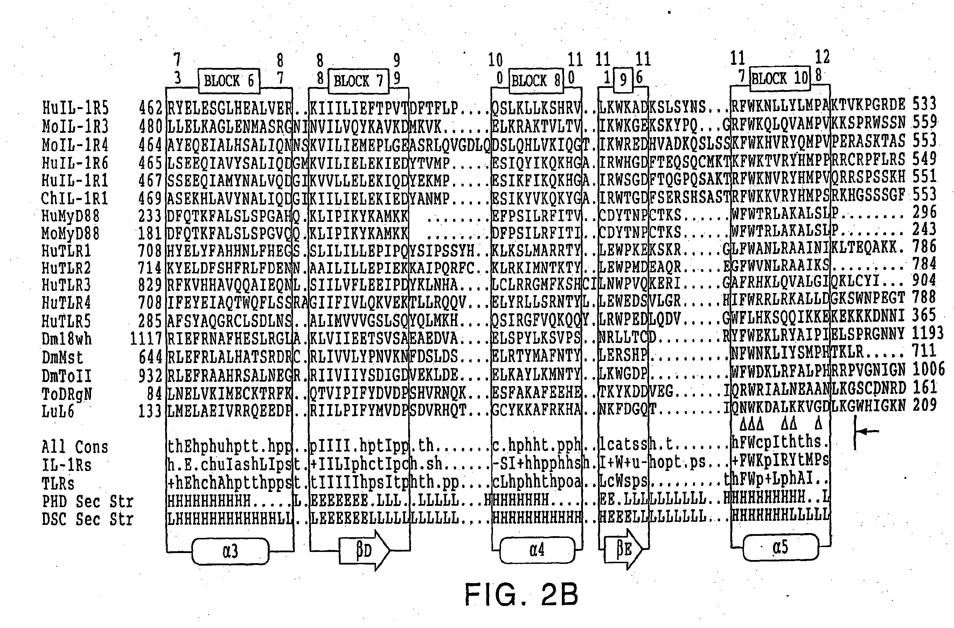

At the heart of these vexing issues is the structural nature of the Toll TH module. To address this question, we have taken advantage of the evolutionary diversity of TH sequences from insects, plants and vertebrates, incorporating the human DTLR chains, and extracted the minimal, conserved protein core for structure prediction and fold recognition (Fig. 2). The strongly predicted (β/α)₅ TH domain fold with its asymmetric cluster of acidic residues is topologically identical to the structures of response regulators in bacterial two-component signaling pathways (Volz (1993) Biochemistry 32:11741-11753; and Parkinson (1993) Cell 73:857-871) (Fig. 2A-2C). The prototype chemotaxis regulator CheY transiently binds a divalent cation in an 'aspartate pocket' at the C-end of the core β-sheet; this cation provides electrostatic stability and facilitates the activating phosphorylation of an invariant Asp. Volz (1993) Biochemistry 32:117 41-11753. Likewise, the TH domain may capture cations in its acidic nest, but activation, and downstream signaling, could depend on the specific binding of a negatively charged moiety: anionic ligands can overcome intensely negative binding-site potentials by locking into precise hydrogen-bond networks. Ledvina, et al. (1996) Proc. Natl. Acad. Sci. USA 93:6786-6791. Intriguingly, the TH domain may not simply act as a passive scaffold for the assembly of a Tube/Pelle complex for Toll, or homologous systems in plants and vertebrates, but instead actively participate as a true conformational trigger in the signal transducing machinery. Perhaps explaining the conditional binding of a Tube/Pelle complex, Toll dimerization could promote unmasking, by regulatory receptor tails (Norris and Manley (1995) Genes Develop. 9:358-369; Norris and Manley (1996) Genes Develop. 10:862-872), or binding by small molecule activators of the TH pocket. However, 'free' TH modules inside the cell (Norris and Manley (1995) Genes Develop. 9:358-369; Winans and Hashimoto (1995) Molec. Biol. Cell 6:587-596) could act as catalytic, CheY-like triggers by activating and docking with errant Tube/Pelle complexes.

### Morphogenetic receptors and immune defense.

The evolutionary link between insect and vertebrate immune systems is stamped in DNA: genes encoding antimicrobial factors in insects display upstream motifs similar to acute phase response elements known to bind NF-κB transcription factors in mammals. Hultmark (1993) Trends Genet. 9:178-183. Dorsal, and two Dorsal-related factors, Dif and Relish, help induce these defense proteins after bacterial challenge (Reichhart, et al. (1993) C. R. Acad. Sci. Paris 316:1218-1224; Ip, et al. (1993) Cell 75:753-763; and Dushay, et al. (1996) Proc. Natl. Acad. Sci. USA 93:10343-10347); Toll, or other DTLRs, likely modulate these rapid immune responses in adult Drosophila (Lemaitre, et al. (1996) Cell 86:973-983; and Rosetto, et al. (1995) Biochem. Biophys. Res. Commun. 209:111-116). These mechanistic parallels to the IL-1 inflammatory response in vertebrates are evidence of the functional versatility of the Toll signaling pathway, and suggest an ancient synergy between embryonic patterning and innate immunity (Belvin and Anderson (1996) Ann. Rev. Cell Develop. Biol. 12:393-416; Lemaitre, et al. (1996) Cell 86:973-983; Wasserman (1993) Molec. Biol. Cell 4:767-771; Wilson, et al. (1997) Curr. Biol. 7:175-178; Hultmark (1993) Trends Genet. 9:178-183; Reichhart, et al. (1993) C. R. Acad. Sci. Paris 316:1218-1229; Ip, et al. (1993) Cell 75:753-763; Dushay, et al. (1996) Proc. Natl. Acad. Sci. USA 93:10343-10347; Rosetto, et al. (1995) Biochem. Biophys. Res. Commun. 209:111-116; Medzhitov and Janeway (1997) Curr. Opin. Immunol. 9:4-9; and Medzhitov and Janeway (1997) Curr. Opin. Immunol. 9:4-9). The closer homology of insect and human DTLR proteins invites an even stronger overlap of biological functions that supersedes the purely immune parallels to IL-1 systems, and lends potential molecular regulators to dorso-ventral and other transformations of vertebrate embryos. DeRobertis and Sasai (1996) Nature 380:37-40; and Arendt and Nübler-Jung (1997) Mech. Develop. 61:7-21.

The present description of an emergent, robust receptor family in humans mirrors the recent discovery of the vertebrate Frizzled receptors for Wnt patterning factors. Wang, et al. (1996) J. Biol. Chem. 271:4468-4476. As numerous other cytokine-receptor systems have roles in early development (Lemaire and Kodjabachian (1996) Trends Genet. 12:525-531), perhaps the distinct cellular contexts of compact embryos and gangly adults simply result in familiar signaling pathways and their diffusible triggers having different biological outcomes at different times, e.g., morphogenesis versus immune defense for DTLRs. For insect, plant, and human Toll-related systems (Hardiman, et al. (1996) Oncogene 13:2467-2475; Wilson, et al. (1997) Curr. Biol. 7:175-178), these signals course through a regulatory TH domain that intriguingly resembles a bacterial transducing engine (Parkinson (1993) Cell 73:857-871).

In particular, the DTLR6 exhibits structural features which establish its membership in the family. Moreover, members of the family have been implicated in a number of significant developmental disease conditions and with function of the innate immune system. In particular, the DTLR6 has been mapped to the X chromosome to a location which is a hot spot for major developmental abnormalities. See, e.g., The Sanger Center: human X chromosome website http://www.sanger.ac.uk/HGP/ChrX/index.shtml; and the Baylor College of Medicine Human Genome Sequencing website http://gc.bcm.tmc.edu:8088/cgi-bin/seq/home.

The accession number for the deposited PAC is AC003046. This accession number contains sequence from two PACs: RPC-164K3 and RPC-263P4. These two PAC sequences mapped on human chromosome Xp22 at the Baylor web site between STS markers DXS704 and DXS7166. This region is a "hot spot" for severe developmental abnormalities.

### III. Amplification of DTLR fragment by PCR

Two appropriate primer sequences are selected (see Tables 1 through 10). RT-PCR is used on an appropriate mRNA sample selected for the presence of message to produce a partial or full length cDNA, e.g., a sample which expresses the gene. See, e.g., Innis, et al. (eds. 1990) PCR Protocols: A Guide to Methods and Applications Academic Press, San Diego, CA; and Dieffenbach and Dveksler (eds. 1995) PCR Primer: A Laboratory Manual Cold Spring Harbor Press, CSH, NY. Such will allow determination of a useful sequence to probe for a full length gene in a cDNA library. The DTLR6 is a contiguous sequence in the genome, which may suggest that the other DTLRs are also. Thus, PCR on genomic DNA may yield full length contiguous sequence, and chromosome walking methodology would then be applicable. Alternatively, sequence databases will contain sequence corresponding to portions of the described embodiments, or closely related forms, e.g., alternative splicing, etc. Expression cloning techniques also may be applied on cDNA libraries.

### IV. Tissue distribution of DTLRs

Message for each gene encoding these DTLRs has been detected. See Figures 5A-5F. Other cells and tissues will be assayed by appropriate technology, e.g., PCR, immunoassay, hybridization, or otherwise. Tissue and organ cDNA preparations are available, e.g., from Clontech, Mountain View, CA. Identification of sources of natural expression are useful, as described.

Southern Analysis: DNA (5 µg) from a primary amplifie cDNA library is digested with appropriate restriction enzymes to release the inserts, run on a 1% agarose gel and transferred to a nylon membrane (Schleicher and Schuell, Keene, NH).

Samples for human mRNA isolation would typically include, e.g.: peripheral blood mononuclear cells (monocytes, T cells, NK cells, granulocytes, B cells), resting (T100); peripheral blood mononuclear cells; activated with anti-CD3 for 2, 6, 12 h pooled (T101); T cell, TH0 clon Mot 72, resting (T102); T cell, TH0 clone Mot 72, activated with anti-CD28 and anti-CD3 for 3, 6, 12 h pooled (T103); T cell, TH0 clone Mot 72, anergic treated with specific peptide for 2, 7, 12 h pooled (T104); T cell, TH1 clone HY06, resting (T107); T cell, TH1 clone HY06, activated with anti-CD28 and anti-CD3 for 3, 6, 12 h pooled (T108); T cell, TH1 clone HY06, anergic treated with specific peptide for 2, 6, 12 h pooled (T109); T cell, TH2 clone HY935, resting (T110). T cell, TH2 clone HY935, activated with anti-CD28 and anti-CD3 for 2, 7, 12 h pooled (T111); T cells CD4+CD45RO= T cells polarized 27 days in anti-CD28, IL-4, and anti IFN-γ, TH2 polarized, activated with anti-CD3 and anti-CD28 4 h (T116); T cell tumor lines Jurkat and Hut78, resting (T117); T cell clones, pooled AD130.2, Tc783.12, Tc783.13, Tc783.58, Tc782.69, resting (T118); T cell random γδ T cell clones, resting (T119); Splenocytes, resting (B100); Splenocytes, activated with anti-CD40 and IL-4 (B101); B cell EBV lines pooled WT49, RSB, JY, CVIR, 721.221, RM3, HSY, resting (B102); B cell line JY, activated with PMA and ionomycin for 1, 6 h pooled (B103); NK 20 clones pooled, resting (K100); NK 20 clones pooled, activated with PMA and ionomycin for 6 h (K101); NKL clone, derived from peripheral blood of LGL leukemia patient, IL-2 treated (K106); NK cytotoxic clone 640-A30-1, resting (K107); hematopoietic precursor line TF1, activated with PMA and ionomycin for 1, 6 h pooled (C100); U937 premonocytic line, resting (M100); U937 premonocytic line, activated with PMA and ionomycin for 1, 6 h pooled (M101); elutriated monocytes, activated with LPS, IFNγ, anti-IL-10 for 1, 2, 6, 12, 24 h pooled (M102) ; elutriated monocytes, activated with LPS, IFNγ, IL-10 for 1, 2, 6, 12, 24 h pooled (M103) ; elutriated monocytes, activated with LPS, IFNγ, anti-IL-10 for 4, 16 h pooled (M106); elutriated monocytes, activated with LPS, IFNγ, IL-10 for 4, 16 h pooled (M107); elutriated monocytes, activated LPS for 1 h (M108) ; elutriated monocytes, activated LPS for 6 h (M109); DC 70% CD1a+, from CD34+ GM-CSF, TNFα 12 days, resting (D101); DC 70% CD1a+, from CD34+ GM-CSF, TNFα 12 days, activated with PMA and ionomycin for 1 hr (D102); DC 70% CD1a+, from CD34+ GM-CSF, TNFα 12 days, activated with PMA and ionomycin for 6 hr (D103); DC 95% CD1a+, from CD34+ GM-CSF, TNFα 12 days FACS sorted, activated with PMA and ionomycin for 1, 6 h pooled (D104); DC 95% CD14+, ex CD34+ GM-CSF, TNFα 12 days FACS sorted, activated with PMA and ionomycin 1, 6 hr pooled (D105); DC CD1a+ CD86+, from CD34+ GM-CSF, TNFα 12 days FACS sorted, activated with PMA and ionomycin for 1, 6 h pooled (D106); DC from monocytes GM-CSF, IL-4 5 days, resting (D107); DC from monocytes GM-CSF, IL-4 5 days, resting (D108); DC from monocytes GM-CSF, IL-4 5 days, activated LPS 4, 16 h pooled (D109); DC from monocytes GM-CSF, IL-4 5 days, activated TNFα, monocyte supe for 4, 16 h pooled (D110); leiomyoma L11 benign tumor (X101) ; normal myometrium M5 (O115); malignant leiomyosarcoma GS1 (X103); lung fibroblast sarcoma line MRC5, activated with PMA and ionomycin for 1, 6 h pooled (C101) ; kidney epithelial carcinoma cell line CHA, activated with PMA and ionomycin for 1, 6 h pooled (C102); kidney fetal 28 wk male (O100) ; lung fetal 28 wk male (O101) ; liver fetal 28 wk male (O102) ; heart fetal 28 wk male (O103) ; brain fetal 28 wk male (O104) ; gallbladder fetal 28 wk male (O106) ; small intestine fetal 28 wk male (O107) ; adipose tissue fetal 28 wk male (O108) ; ovary fetal 25 wk female (O109) ; uterus fetal 25 wk female (O110) ; testes fetal 28 wk male (O111) ; spleen fetal 28 wk male (O112) ; adult placenta 28 wk (O113) ; and tonsil inflamed, from 12 year old (X100).

Samples for mouse mRNA isolation can include, e.g.: resting mouse fibroblastic L cell line (C200); Braf:ER (Braf fusion to estrogen receptor) transfected cells, control (C201); T cells, TH1 polarized (Mel14 bright, CD4+ cells from spleen, polarized for 7 days with IFN-γ and anti IL-4; T200); T cells, TH2 polarized (Mel14 bright, CD4+ cells from spleen, polarized for 7 days with IL-4 and anti-IFN-γ; T201); T cells, highly TH1 polarized (see Openshaw, et al. (1995) J. Exp. Med. 182:1357-1367; activated with anti-CD3 for 2, 6, 16 h pooled; T202); T cells, highly TH2 polarized (see Openshaw, et al. (1995) J. Exp. Med. 182:1357-1367; activated with anti-CD3 for 2, 6, 16 h pooled; T203); CD44- CD25+ pre T cells, sorted from thymus (T204); TH1 T cell clone D1.1, resting for 3 weeks after last stimulation with antigen (T205); TH1 T cell clone D1.1, 10 µg/ml ConA stimulated 15 h (T206); TH2 T cell clone CDC35, resting for 3 weeks after last stimulation with antigen (T207); TH2 T cell clone CDC35, 10 µg/ml ConA stimulated 15 h (T208) ; Mel14+ naive T cells from spleen, resting (T209) ; Mel14+ T cells, polarized to Th1 with IFN-γ/IL-12/anti-IL-4 for 6, 12, 24 h pooled (T210); Mel14+ T cells, polarized to Th2 with IL-4/anti-IFN-γ for 6, 13, 24 h pooled (T211) ; unstimulated mature B cell leukemia cell line A20 (B200); unstimulated B cell line CH12 (B201); unstimulated large B cells from spleen (B202) ; B cells from total spleen, LPS activated (B203) ; metrizamide enriched dendritic cells from spleen, resting (D200); dendritic cells from bone marrow, resting (D201); monocyte cell line RAW 264.7 activated with LPS 4 h (M200); bone-marrow macrophages derived with GM and M-CSF (M201) ; macrophage cell line J774, resting (M202); macrophage cell line J774 + LPS + anti-IL-10 at 0.5, 1, 3, 6, 12 h pooled (M203) ; macrophage cell line J774 + LPS + IL-10 at 0.5, 1, 3, 5, 12 h pooled(M204); aerosol challenged mouse lung tissue, Th2 primers, aerosol OVA challenge 7, 14, 23 h pooled (see Garlisi, et al. (1995) Clinical Immunology and Immunopathology 75:75-83; X206); Nippostrongulus-infected lung tissue (see Coffman, et al. (1989) Science 2.45:308-310; X200); total adult lung, normal (O200); total lung, rag-1 (see Schwarz, et al. (1993) Immunodeficiency 4:249-252; O205); IL-10 K.O. spleen (see Kuhn, et al. (1991) Cell 75:263-274; X201) ; total adult spleen, normal (O201); total spleen, rag-1 (O207); IL-10 K.O. Peyer's patches (O202); total Peyer's patches, normal (O210) ; IL-10 K.O. mesenteric lymph nodes (X203) ; total mesenteric lymph nodes, normal (O211) ; IL-10 K.O. colon (X203); total colon, normal (O212); NOD mouse pancreas (see Makino, et al. (1980) Jikken Dobutsu 29:1-13; X205); total thymus, rag-1 (O208); total kidney, rag-1 (O209); total heart, rag-1 (O202); total brain, rag-1 (O203); total testes, rag-1 (O204); total liver, rag-1 (O206); rat normal joint tissue (O300); and rat arthritic joint tissue (X300).

The DTLR10 has been found to be highly expressed in precursor dendritic cell type 2 (pDC2). See, e.g., Rissoan, et al. (1999) Science 283:1183-1186; and Siegal, et al. (1999) Science 284:.1835-1837. However, it is not expressed on monocytes. The restricted expression of DTLR10 reinforces the suggestions of a role for the receptor in host immune defense. The pDC2 cells are natural interferon producing cells (NIPC), which produce large amounts of IFNα in response to Herpes simplex virus infection.

### V. Cloning of species counterparts of DTLRs

Various strategies are used to obtain species counterparts of these DTLRs, preferably from other primates. One method is by cross hybridization using closely related species DNA probes. It may be useful to go into evolutionarily similar species as intermediate steps. Another method is by using specific PCR primers based on the identification of blocks of similarity or difference between particular species, e.g., human, genes, e.g., areas of highly conserved or nonconserved polypeptide or nucleotide sequence: Alternatively, antibodies may be used for expression cloning.

### VI. Production of mammalian DTLR protein

An appropriate, e.g., GST, fusion construct is engineered for expression, e.g., in E. coli. For example, a mouse IGIF pGex plasmid is constructed and transformed into E. coli. Freshly transformed cells are grown in LB medium containing 50 µg/ml ampicillin and induced with IPTG (Sigma, St. Louis, MO). After overnight induction, the bacteria are harvested and the pellets containing the DTLR protein are isolated. The pellets are homogenized in TE buffer (50 mM Tris-base pH 8.0, 10 mM EDTA and 2 mM pefabloc) in 2 liters. This material is passed through a microfluidizer (Microfluidics, Newton, MA) three times. The fluidized supernatant is spun down on a Sorvall GS-3 rotor for 1 h at 13,000 rpm. The resulting supernatant containing the DTLR protein is filtered and passed over a glutathione-SEPHAROSE column equilibrated in 50 mM Tris-base pH 8.0. The fractions containing the DTLR-GST fusion protein are pooled and cleaved with thrombin (Enzyme Research Laboratories, Inc., South Bend, IN). The cleaved pool is then passed over a Q-SEPHAROSE column equilibrated in 50 mM Tris-base. Fractions containing DTLR are pooled and diluted in cold distilled H₂O, to lower the conductivity, and passed back over a fresh Q-Sepharose column, alone or in succession with an immunoaffinity antibody column.. Fractions containing the DTLR protein are pooled, aliquoted, and stored in the -70°C freezer.

Comparison of the CD spectrum with DTLR1 protein may suggest that the protein is correctly folded. See Hazuda, et al. (1969) J. Biol. Chem. 264:1689-1693.

### VII. Biological Assays with DTLRs

Biological assays will generally be directed to the ligand binding feature of the protein or to the kinase/phosphatase activity of the.receptor. The activity will typically be reversible, as are many other enzyme actions, and will mediate phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738.

The family of interleukin 1s contains molecules, each of which is an important mediator of inflammatory disease. For a comprehensive review, see Dinarello (1996) "Biologic basis for interleukin-1 in disease" Blood 87:2095-2147. There are suggestions that the various Toll ligands may play important roles in the initiation of disease, particularly inflammatory responses. The finding of novel proteins related to the IL-1 family furthers the identification of molecules that provide the molecular basis for initiation of disease and allow for the development of therapeutic strategies of increased range and efficacy.

### VIII. Preparation of antibodies specific for, e.g., DTLR4

Inbred Balb/c mice are immunized intraperitoneally with recombinant forms of the protein, e.g., purified DTLR4 or stable transfected NIH-3T3 cells. Animals are boosted at appropriate time points with protein, with or without additional adjuvant, to further stimulate antibody production. Serum is collected, or hybridomas produced with harvested spleens.

Alternatively, Balb/c mice are immunized with cells transformed with the gene or fragments thereof, either endogenous or exogenous cells, or with isolated membranes enriched for expression of the antigen. Serum is collected at the appropriate time, typically after numerous further administrations. Various gene therapy techniques may be useful, e.g., in producing protein in situ, for generating an immune response.

Monoclonal antibodies may be made. For example, splenocytes are fused with an appropriate fusion partner and hybridomas are selected in growth medium by standard procedures. Hybridoma supernatants are screened for the presence of antibodies which bind to the desired DTLR, e.g., by ELISA or other assay. Antibodies which specifically recognize specific DTLR embodiments may also be selected or prepared.

In another method, synthetic peptides or purified protein are presented to an immune system to generate monoclonal or polyclonal antibodies. See, e.g., Coligan (1991) Current Protocols in Immunology Wiley/Greene; and Harlow and Lane (1989) Antibodies: A Laboratory Manual Cold Spring Harbor Press. In appropriate situations, the binding reagent is either labeled as described above, e.g., fluorescence or otherwise, or immobilized to a substrate for panning methods. Nucleic acids may also be introduced into cells in an animal to produce the antigen, which serves to elicit an immune response. See, e.g., Wang, et al. (1993) Proc. Nat'l. Acad. Sci. 90:4156-4160; Barry, et al. (1994) BioTechniques 16:616-619; and Xiang, et al. (1995) Immunity 2: 129-135.

### IX. Production of fusion proteins with, e.g., DTLR5

Various fusion constructs are made with DTLR5. This portion of the gene is fused to an epitope tag, e.g., a FLAG tag, or to a two hybrid system construct. See, e.g., Fields and Song (1989) Nature 340:245-246.

The epitope tag may be used in an expression cloning procedure with detection with anti-FLAG antibodies to detect a binding partner, e.g.; ligand for the respective DTLR5. The two hybrid system may also be used to isolate proteins which specifically bind to DTLR5.

### X. Chromosomal mapping of DTLRs

Chromosome spreads are prepared. In situ hybridization is performed on chromosome preparations obtained from phytohemagglutinin-stimulated lymphocytes cultured for 72 h. 5-bromodeoxyuridine is added for the final seven hours of culture (60 µg/ml of medium), to ensure a posthybridization chromosomal banding of good quality.

An appropriate fragment, e.g., a PCR fragment, amplified with the help of primers on total B cell cDNA template, is cloned into an appropriate vector. The vector is labeled by nick-translation with ³H. The radiolabeled probe is hybridized to metaphase spreads as described in Mattei, et al. (1985) Hum. Genet. 69:327-331.

After coating with nuclear track emulsion (KODAK NTB₂), slides are exposed, e.g., for 18 days at 4° C. To avoid any slipping of silver grains during the banding procedure, chromosome spreads are first stained with buffered Giemsa solution and metaphase photographed. R-banding is then performed by the fluorochrome-photolysis-Giemsa (FPG) method and metaphases rephotographed before analysis.

Alternatively, FISH can be performed, as described above. The DTLR genes are located on different chromosomes. DTLR2 and DTLR3 are localized to human chromosome 4; DTLR4 is localized to human chromosome 9, and DTLR5 is localized to human chromosome 1. See Figures 4A-4D.

### XI. Structure activity relationship

Information on the criticality of particular residues is determined using standard procedures and analysis. Standard mutagenesis analysis is performed, e.g., by generating many different variants at determined positions, e.g., at the positions identified above, and evaluating biological activities of the variants. This may be performed to the extent of determining positions which modify activity, or to focus on specific positions to determine the residues which can be substituted to either retain, block, or modulate biological activity.

Alternatively, analysis of natural variants can indicate what positions tolerate natural mutations. This may result from populational analysis of variation among individuals, or across strains or species. Samples from selected individuals are analyzed, e.g., by PCR analysis and sequencing. This allows evaluation of population polymorphisms .

### XI. Isolation of a ligand for a DTLR

A DTLR can be used as a specific binding reagent to identify its binding partner, by taking advantage of its specificity of binding, much like an antibody would be used. A binding reagent is either labeled as described above, e.g., fluorescence or otherwise, or immobilized to a substrate for panning methods.

The binding composition is used to screen an expression library made from a cell line which expresses a binding partner, i.e., ligand, preferably membrane associated. Standard staining techniques are used to detect or sort surface expressed ligand, or surface expressing transformed cells are screened by panning. Screening of intracellular expression is performed by various staining or immunofluorescence procedures. See also McMahan, et al. (1991) EMBO J. 10:2821-2832.

For example, on day 0, precoat 2-chamber permanox slides with 1 ml per chamber of fibronectin, 10 ng/ml in PBS, for 30 min at room temperature. Rinse once with PBS. Then plate COS cells at 2-3 x 10⁵ cells per chamber in 1.5 ml of growth media. Incubate overnight at 37° C.

On day 1 for each sample, prepare 0.5 ml of a solution of 66 µg/ml DEAE-dextran, 66 µM chloroquine, and 4 µg DNA in serum free DME. For each set, a positive control is prepared, e.g., of DTLR-FLAG cDNA at 1 and 1/200 dilution, and a negative mock. Rinse cells with serum free DME. Add the DNA solution and incubate 5 hr at 37 °C. Remove the medium and add 0.5 ml 10% DMSO in DME for 2.5 min. Remove and wash once with DME. Add 1.5 ml growth medium and incubate overnight.

On day 2, change the medium. On days 3 or 4, the cells are fixed and stained. Rinse the cells twice with Hank's Buffered Saline Solution (HBSS) and fix in 4% paraformaldehyde (PFA)/glucose for 5 min. Wash 3X with HBSS. The slides may be stored at -80° C after all liquid is removed. For each chamber, 0.5 ml incubations are performed as follows. Add HBSS/saponin (0.1%) with 32 µl/ml of 1 M NaN₃ for 20 min. Cells are then washed with HBSS/saponin 1X. Add appropriate DTLR or DTLR/antibody complex to cells and incubate for 30 min. Wash cells twice with HBSS/saponin. If appropriate, add first antibody for 30 min. Add second antibody, e.g., Vector anti-mouse antibody, at 1/200 dilution, and incubate for 30 min. Prepare ELISA solution, e.g., Vector Elite ABC horseradish peroxidase solution, and preincubate for 30 min. Use, e.g., 1 drop of solution A (avidin) and 1 drop solution B (biotin) per 2.5 ml HBSS/saponin. Wash cells twice with HBSS/saponin. Add ABC HRP solution and incubate for 30 min. Wash cells twice with HBSS, second wash for 2 min, which closes cells. Then add Vector diaminobenzoic acid (DAB) for 5 to 10 min. Use 2 drops of buffer plus 4 drops DAB plus 2 drops of H₂O₂ per 5 ml of glass distilled water. Carefully remove chamber and rinse slide in water. Air dry for a few minutes, then add 1 drop of Crystal Mount and a cover slip. Bake for 5 min at 85-90° C.

Evaluate positive staining of pools and progressively subclone to isolation of single genes responsible for the binding.

Alternatively, DTLR reagents are used to affinity purify or sort out cells expressing a putative ligand. See, e.g., Sambrook, et al. or Ausubel, et al.

Another strategy is to screen for a membrane bound receptor by panning. The receptor cDNA is constructed as described above. The ligand can be immobilized and used to immobilize expressing cells. Immobilization may be achieved by use of appropriate antibodies which recognize, e.g., a FLAG sequence of a DTLR fusion construct, or by use of antibodies raised against the first antibodies. Recursive cycles of selection and amplification lead to enrichment of appropriate clones and eventual isolation of receptor expressing clones.

Phage expression libraries can be screened by mammalian DTLRs. Appropriate label techniques, e.g., anti-FLAG antibodies, will allow specific labeling of appropriate clones.

All citations herein are incorporated herein by reference to the same extent as if each individual publication or patent application was specifically and individually indicated to be incorporated by reference.

Many modifications and variations of this invention can be made without departing from its spirit and scope, as will be apparent to those skilled in the art. The specific embodiments described herein are offered by way of example only, and the invention is to be limited by the terms of the appended claims, along with the full scope of equivalents to which such claims are entitled; and the invention is not to be limited by the specific embodiments that have been presented herein by way of example.

Humans have two distinct types of dendritic cell (DC) precursors. Peripheral blood monocytes (pDC1) give rise to immature myeloid DCs after culturing with GMCSF and IL-4. These immature cells become mature myeloid DCs (DC1) after stimulation with CD40 ligand (CD40L). The CD4+CD3-CD11c plasmacytoid cells (pDC2) from blood or tonsils give rise to a distinct type of immature DC after culture with IL-3, and differentiate into mature DCs (DC2) after CD40L stimulation. Rissoan, et al. (1999) Science 283:1183-1186.

Siegal, et al. (1999) Science 284:1835-1837, show that pDC2 is the "Natural Interferon Producing Cell" (IPC). Interferons (IFNs) are the most important cytokines in antiviral immune responses. "Natural IFN-producing cells" (NIPCs) in human blood express CD4 and major histocompatibility complex class II proteins, but have not been isolated and further characterized because of their rarity, rapid apoptosis, and lack of lineage markers. Purified NIPCs are here shown to be the CD4(+)CD11c- type 2 dendritic cell precursors (pDC2s), which produce 200 to 1000 times more IFN than other blood cells after microbial challenge. pDC2s are thus an effector cell type of the immune system, critical for antiviral and antitumor immune responses. They are implicated as important cells in HIV infected patients

Toll-like receptor (TLR) molecules belong to the IL-1/Toll receptor family. Ligands for TLR2 and TLR4 have been identified, and their functions are related to the host immune response to microbial antigen or injury. Takeuchi, et al. (1999) Immunity 11:443-451; and Noshino, et al. (1999) J. Immunol. 162:3749-3752. The pattern of expression of TLRs seem to be restricted. Muzio, et al. (2000) J. Immunol. 164:5998-6004. With these findings that: i) TLR10 is highly expressed and restricted in pDC2s, and ii) pDC2 is the NIPC, it is likely that TLR10 will play an important role in the host's innate immune response.

SEQUENCE SUBMISSION

SEQ ID NO: 1 provides primate DTLR1 nucleotide sequence.
SEQ ID NO: 2 provides primate DTLR1 polypeptide sequence.
SEQ ID NO: 3 provides primate DTLR2 nucleotide sequence.
SEQ ID NO: 4 provides primate DTLR2 polypeptide sequence.
SEQ ID NO: 5 provides primate DTLR3 nucleotide sequence.
SEQ ID NO: 6 provides primate DTLR3 polypeptide sequence.
SEQ ID NO: 7 provides primate DTLR4 nucleotide sequence.
SEQ ID NO: 8 provides primate DTLR4 polypeptide sequence.
SEQ ID NO: 9 provides primate DTLR5 nucleotide sequence.
SEQ ID NO: 10 provides primate DTLR5 polypeptide sequence.
SEQ ID NO: 11 provides primate DTLR6 nucleotide sequence.
SEQ ID NO: 12 provides primate DTLR6 polypeptide sequence.
SEQ ID NO: 13 provides rodent DTLR6 nucleotide sequence.
SEQ ID NO: 14 provides rodent DTLR6 polypeptide sequence.
SEQ ID NO: 15 provides primate DTLR7 nucleotide sequence.
SEQ ID NO: 16 provides primate DTLR7 polypeptide sequence.
SEQ ID NO: 17 provides primate DTLR7 nucleotide sequence.
SEQ ID NO: 18 provides primate DTLR7 polypeptide sequence.
SEQ ID NO: 19 provides primate DTLR8 nucleotide sequence.
SEQ ID NO: 20 provides primate DTLR8 polypeptide sequence.
SEQ ID NO: 21 provides primate DTLR9 nucleotide sequence.
SEQ ID NO: 22 provides primate DTLR9 polypeptide sequence.
SEQ ID NO: 23 provides primate DTLR10 nucleotide sequence.
SEQ ID NO: 24 provides primate DTLR10 polypeptide sequence.
SEQ ID NO: 25 provides primate DTLR4 nucleotide sequence.
SEQ ID NO: 26 provides primate DTLR4 polypeptide sequence.
SEQ ID NO: 27 provides rodent DTLR6 nucleotide sequence.
SEQ ID NO: 28 provides rodent DTLR6 polypeptide sequence.
SEQ ID NO: 29 provides rodent DTLR6 nucleotide sequence.
SEQ ID NO: 30 provides rodent DTLR6 polypeptide sequence.
SEQ ID NO: 31 provides primate DTLR8 nucleotide sequence.
SEQ ID NO: 32 provides primate DTLR8 polypeptide sequence.
SEQ ID NO: 33 provides primate DTLR10 nucleotide sequence.
SEQ ID NO: 34 provides primate DTLR10 polypeptide sequence.
SEQ ID NO: 35 provides rodent DTLR10 nucleotide sequence.
SEQ ID NO: 36 provides primate DTLR7 nucleotide sequence.
SEQ ID NO: 37 provides primate DTLR7 polypeptide sequence.
SEQ ID NO: 38 provides primate DTLR8 nucleotide sequence.
SEQ ID NO: 39 provides primate DTLR8 polypeptide sequence.
SEQ ID NO: 40 provides primate DTLR9 nucleotide sequence.
SEQ ID NO: 41 provides primate DTLR9 polypeptide sequence.
SEQ ID NO: 42 provides primate DTLR10 nucleotide sequence.
SEQ ID NO: 43 provides primate DTLR10 polypeptide sequence.
SEQ ID NO: 44 provides rodent DTLR10 nucleotide sequence.
SEQ ID NO: 45 provides rodent DTLR10 polypeptide sequence.

## Claims

1. A composition of matter selected from the group consisting of:
a) a substantially pure or recombinant DTLR10 protein or peptide exhibiting identity over a length of at least about 12 amino acids to SEQ ID NO: 43 or 45;
b) a natural sequence DTLR10 comprising SEQ ID NO: 43 or 45; or
c) a fusion protein comprising DTLR 10 sequence.

2. A substantially pure or isolated protein comprising a segment exhibiting sequence identity to a corresponding portion of a:
a) DTLR 10 of Claim 1, and said identity is over at least:
a) about 15 amino acids;
b) about 19 amino acids; or
c) about 25 amino acids.

3. The composition of matter of Claim 1, wherein said:
a) DTLR10:
i) comprises a sequence of Table 10; or
ii) lacks post-translational modification; or
b) protein or peptide:
i) is from a warm blooded animal selected from a mammal, including a primate, such as a human;
ii) comprises at least one polypeptide segment of SEQ ID NO: 43 or 45;
iii) exhibits a plurality of said segments of identity;
iv) is a natural allelic variant of DTLR10;
v) has a length at least about 30 amino acids;
vi) exhibits at least two non-overlapping epitopes which are specific for a primate DTLR10;
vii) exhibits sequence identity over a length of at least about 35 amino acids to a primate DTLR10;
viii) further exhibits at least two non-overlapping epitopes which are specific for a primate DTLR10;
ix) is glycosylated;
x) has a molecular weight of a least 100 kD with natural glycosylation;
xi) is a synthetic polypeptide;
xii) is attached to a solid substrate;
xiii) is conjugated to another chemical moiety;
xiv) is a 5-fold or less substitution from natural sequence; or
xv) is a deletion or insertion variant from a natural sequence.

4. A composition comprising:
a) a sterile DTLR10 protein or peptide of Claim 1; or
b) said DTLR10 protein or peptide of Claim 1 and a carrier, wherein said carrier is:
i) an aqueous compound, including water, saline, and/or buffer; and/or
ii) formulated for oral, rectal, nasal, topical, or parenteral administration.

5. The fusion protein of Claim 1, comprising:
a) mature protein comprising sequence of Table 10;
b) a detection or purification tag, including a FLAG, His6, or lg sequence; or
c) sequence of another receptor protein.

6. A kit comprising a protein or polypeptide of Claim 1, and:
a) a compartment comprising said protein or polypeptide; and/or
b) instructions for use or disposal of reagents in said kit.

7. A binding compound comprising an antigen binding site from an antibody, which specifically binds to a natural DTLR10 protein of Claim 1, wherein:
a) said protein is a primate protein;
b) said binding compound is an Fv, Fab, or Fab2 fragment;
c) said binding compound is conjugated to another chemical moiety; or
d) said antibody:
i) is raised against a peptide sequence of a mature polypeptide of Table 10;
ii) is raised against a mature DTLR10;
iii) is raised to a purified human DTLR10;
iv) is immunoselected;
v) is a polyclonal antibody;
vi) binds to a denatured DTLR10;
vii) exhibits a Kd to antigen of at least 30 µM;
viii) is attached to a solid substrate, including a bead or plastic membrane;
ix) is in a sterile composition; or
x) is detectably labelled, including a radioactive or fluorescent label.

8. A kit comprising said binding compound of Claim 7, and:
a) a compartment comprising said binding compound; and/or
b) instructions for use or disposal of reagents in said kit.

9. A method of:
A) making an antibody of Claim 7, comprising immunizing an immune system with an immunogenic amount of:
a) a primate DTLR10;
thereby causing said antibody to be produced; or
B) producing an antigen:antibody complex, comprising contacting an antibody of Claim 7 with:
a) a mammalian DTLR10 protein or peptide;
thereby allowing said complex to form.

10. A composition comprising:
a) a sterile binding compound of Claim 7, or
b) said binding compound of Claim 7 and a carrier, wherein said carrier is:
i) an aqueous compound, including water, saline, and/or buffer; and/or
ii) formulated for oral, rectal, nasal, topical, or parenteral administration.

11. An isolated or recombinant nucleic acid encoding a protein or peptide or fusion protein of Claim 1, wherein:
a) said DTLR is from a mammal; or
b) said nucleic acid:
i) encodes an antigenic peptide sequence of Table 10;
ii) encodes a plurality of antigenic peptide sequences of Table 10;
iii) exhibits at least about 80% identity to a natural cDNA encoding said segment;
iv) is an expression vector;
v) further comprises an origin of replication;
vi) is from a natural source;
vii) comprises a detectable label;
viii) comprises synthetic nucleotide sequence;
ix) is less than 6 kb, preferably less than 3 kb;
x) is from a mammal, including a primate;
xi) comprises a natural full length coding sequence;
xii) is a hybridization probe for a gene encoding said DTLR;
xiii) comprises at least 17 contiguous nucleotides from Table 10;
xiv) comprises a plurality of non-overlapping segments of at least 17 contiguous nucleotides from Table 10; or
xv) is a PCR primer, PCR product, or mutagenesis primer.

12. A cell, tissue, or organ comprising a recombinant nucleic acid of Claim 11.

13. The cell of Claim 12, wherein said cell is:
a) a prokaryotic cell;
b) a eukaryotic cell;
c) a bacterial cell;
d) a yeast cell;
e) an insect cell;
f) a mammalian cell;
g) a mouse cell;
h) a primate cell; or
i) a human cell.

14. A kit comprising said nucleic acid of Claim 11, and:
a) a compartment comprising said nucleic acid;
b) a compartment further comprising a primate DTLR2, DTLR3, DTLR4, DTLR5, DTLR6, DTLR7, DTLR8, DTLR9 or DTLR10 protein or polypeptide; and/or
c) instructions for use or disposal of reagents in said kit.

15. A method of:
a) making a polypeptide, comprising expressing said nucleic acid of Claim 11, thereby producing said polypeptide; or
b) making a duplex nucleic acid, comprising contacting said nucleic acid of Claim 11 with a complementary nucleic acid, thereby allowing said duplex to form.

16. A nucleic acid which:
a) hybridizes under wash conditions of 30°C and less than 2 M salt to SEQ ID NO: 42 or 44; or
b) exhibits at least 85% identity over a stretch of at least 30 nucleotides to primate DTLR10.

17. The nucleic acid of Claim 16, wherein:
a) said wash conditions are at 45°C and/or 500 mM salt; or
b) said identity is at least 90% and/or said stretch is at least 55 nucleotides.

18. The nucleic acid of Claim 17, wherein:
a) said wash conditions are at 55°C and/or 150mM salt; or
b) said identity is at least 95% and/or said stretch is at least 75 nucleotides.

19. A method of producing a ligand:receptor complex, comprising contracting:
a) substantially pure primate DTLR10, including a recombinant or synthetically produced protein, with candidate Toll ligand, thereby allowing said complex to form.

20. A method of modulating physiology or development of a cell or tissue culture cells comprising contacting said cell with an agonist or antagonist of a mammalian DTLR10.

21. The method of Claim 20, wherein said agonist or antagonist is of DTLR10, and said cell is a pDC2 cell.


<110> Schering Corp.

<120> Human Receptor Proteins; Related Reagents and Methods

<130> DX0724KP

<140>
<141>

<160> 45

<170> PatentIn Ver. 2.0

<210> 1
<211> 2367
<212> DNA
<213> Unknown

<220>
<223> Description of Unknown Organism:primate; surmised
      Homo sapiens

<220>
<221> CDS
<222> (1)..(2358)

<220>
<221> mat_peptide
<222> (67)..(2358)

<400> 1
atg act agc atc ttc cat ttt gcc att atc ttc atg tta ata ctt cag      48
Met Thr Ser Ile Phe His Phe Ala Ile Ile Phe Met Leu Ile Leu Gln
        -20                 -15                 -10

atc aga ata caa tta tct gaa gaa agt gaa ttt tta gtt gat agg tca      96
Ile Arg Ile Gln Leu Ser Glu Glu Ser Glu Phe Leu Val Asp Arg Ser
     -5                  -1   1               5                  10

aaa aac ggt ctc atc cac gtt cct aaa gac cta tcc cag aaa aca aca      144
Lys Asn Gly Leu Ile His Val Pro Lys Asp Leu Ser Gln Lys Thr Thr
                 15                  20                  25

atc tta aat ata tcg caa aat tat ata tct gag ctt tgg act tct gac      192
Ile Leu Asn Ile Ser Gln Asn Tyr Ile Ser Glu Leu Trp Thr Ser Asp
             30                  35                  40

atc tta tca ctg tca aaa ctg agg att ttg ata att tct cat aat aga      240
Ile Leu Ser Leu Ser Lys Leu Arg Ile Leu Ile Ile Ser His Asn Arg
         45                  50                  55

atc cag tat ctt gat atc agt gtt ttc aaa ttc aac cag gaa ttg gaa      288
Ile Gln Tyr Leu Asp Ile Ser Val Phe Lys Phe Asn Gln Glu Leu Glu
     60                  65                  70

tac ttg gat ttg tcc cac aac aag ttg gtg aag att tct tgc cac cct      336
Tyr Leu Asp Leu Ser His Asn Lys Leu Val Lys Ile Ser Cys His Pro
 75                  80                  85                  90

act gtg aac ctc aag cac ttg gac ctg tca ttt aat gca ttt gat gcc 384
Thr Val Asn Leu Lys His Leu Asp Leu Ser Phe Asn Ala Phe Asp Ala
95 100 105

ctg cct ata tgc aaa gag ttt ggc aat atg tct caa cta aaa ttt ctg 432
Leu Pro Ile Cys Lys Glu Phe Gly Asn Met Ser Gln Leu Lys Phe Leu
110 115 120

ggg ttg agc acc aca cac tta gaa aaa tct agt gtg ctg cca att gct 480
Gly Leu Ser Thr Thr His Leu Glu Lys Ser Ser Val Leu Pro Ile Ala
125 130 135

cat ttg aat atc agc aag gtc ttg ctg gtc tta gga gag act tat ggg 528
His Leu Asn Ile Ser Lys Val Leu Leu Val Leu Gly Glu Thr Tyr Gly
140 145 150

gaa aaa gaa gac cct gag ggc ctt caa gac ttt aac act gag agt ctg 576
Glu Lys Glu Asp Pro Glu Gly Leu Gln Asp Phe Asn Thr Glu Ser Leu
155 160 165 170

cac att gtg ttc ccc aca aac aaa gaa ttc cat ttt att ttg gat gtg 624
His Ile Val Phe Pro Thr Asn Lys Glu Phe His Phe Ile Leu Asp Val
175 180 185

tca gtc aag act gta gca aat ctg gaa cta tct aat atc aaa tgt gtg 672
Ser Val Lys Thr Val Ala Asn Leu Glu Leu Ser Asn Ile Lys Cys Val
190 195 200

cta gaa gat aac aaa tgt tct tac ttc cta agt att ctg gcg aaa ctt 720
Leu Glu Asp Asn Lys Cys Ser Tyr Phe Leu Ser Ile Leu Ala Lys Leu
205 210 215

caa aca aat cca aag tta tca agt ctt acc tta aac aac att gaa aca 768
Gln Thr Asn Pro Lys Leu Ser Ser Leu Thr Leu Asn Asn Ile Glu Thr
220 225 230

act tgg aat tct ttc att agg atc ctc caa cta gtt tgg cat aca act 816
Thr Trp Asn Ser Phe Ile Arg Ile Leu Gln Leu Val Trp His Thr Thr
235 240 245 250

gta tgg tat ttc tca att tca aac gtg aag cta cag ggt cag ctg gac 864
Val Trp Tyr Phe Ser Ile Ser Asn Val Lys Leu Gln Gly Gln Leu Asp
255 260 265

ttc aga gat ttt gat tat tct ggc act tcc ttg aag gcc ttg tct ata 912
Phe Arg Asp Phe Asp Tyr Ser Gly Thr Ser Leu Lys Ala Leu Ser Ile
270 275 280

cac caa gtt gtc agc gat gtg ttc ggt ttt ccg caa agt tat atc tat 960
His Gln Val Val Ser Asp Val Phe Gly Phe Pro Gln Ser Tyr Ile Tyr
285 290 295

gaa atc ttt tcg aat atg aac atc aaa aat ttc aca gtg tct ggt aca 1008
Glu Ile Phe Ser Asn Met Asn Ile Lys Asn Phe Thr Val Ser Gly Thr
300 305 310

cgc atg gtc cac atg ctt tgc cca tcc aaa att agc ccg ttc ctg cat 1056
Arg Met Val His Met Leu Cys Pro Ser Lys Ile Ser Pro Phe Leu His

315                 320                 325                 330

ttg gat ttt tcc aat aat ctc tta aca gac acg gtt ttt gaa aat tgt     1104
Leu Asp Phe Ser Asn Asn Leu Leu Thr Asp Thr Val Phe Glu Asn Cys
                335                 340                 345

ggg cac ctt act gag ttg gag aca ctt att tta caa atg aat caa tta     1152
Gly His Leu Thr Glu Leu Glu Thr Leu Ile Leu Gln Met Asn Gln Leu
            350                 355                 360

aaa gaa ctt tca aaa ata gct gaa atg act aca cag atg aag tct ctg     1200
Lys Glu Leu Ser Lys Ile Ala Glu Met Thr Thr Gln Met Lys Ser Leu
        365                 370                 375

caa caa ttg gat att agc cag aat tct gta agc tat gat gaa aag aaa     1248
Gln Gln Leu Asp Ile Ser Gln Asn Ser Val Ser Tyr Asp Glu Lys Lys
    380                 385                 390

gga gac tgt tct tgg act aaa agt tta tta agt tta aat atg tct tca     1296
Gly Asp Cys Ser Trp Thr Lys Ser Leu Leu Ser Leu Asn Met Ser Ser
395                 400                 405                 410

aat ata ctt act gac act att ttc aga tgt tta cct ccc agg atc aag     1344
Asn Ile Leu Thr Asp Thr Ile Phe Arg Cys Leu Pro Pro Arg Ile Lys
                415                 420                 425

gta ctt gat ctt cac agc aat aaa ata aag agc att cct aaa caa gtc     1392
Val Leu Asp Leu His Ser Asn Lys Ile Lys Ser Ile Pro Lys Gln Val
            430                 435                 440

gta aaa ctg gaa gct ttg caa gaa ctc aat gtt gct ttc aat tct tta     1440
Val Lys Leu Glu Ala Leu Gln Glu Leu Asn Val Ala Phe Asn Ser Leu
        445                 450                 455

act gac ctt cct gga tgt ggc agc ttt agc agc ctt tct gta ttg atc     1488
Thr Asp Leu Pro Gly Cys Gly Ser Phe Ser Ser Leu Ser Val Leu Ile
    460                 465                 470

att gat cac aat tca gtt tcc cac cca tca gct gat ttc ttc cag agc     1536
Ile Asp His Asn Ser Val Ser His Pro Ser Ala Asp Phe Phe Gln Ser
475                 480                 485                 490

tgc cag aag atg agg tca ata aaa gca ggg gac aat cca ttc caa tgt     1584
Cys Gln Lys Met Arg Ser Ile Lys Ala Gly Asp Asn Pro Phe Gln Cys
                495                 500                 505

acc tgt gag ctc gga gaa ttt gtc aaa aat ata gac caa gta tca agt     1632
Thr Cys Glu Leu Gly Glu Phe Val Lys Asn Ile Asp Gln Val Ser Ser
            510                 515                 520

gaa gtg tta gag ggc tgg cct gat tct tat aag tgt gac tac ccg gaa     1680
Glu Val Leu Glu Gly Trp Pro Asp Ser Tyr Lys Cys Asp Tyr Pro Glu
        525                 530                 535

agt tat aga gga acc cta cta aag gac ttt cac atg tct gaa tta tcc     1728
Ser Tyr Arg Gly Thr Leu Leu Lys Asp Phe His Met Ser Glu Leu Ser
    540                 545                 550

tgc aac ata act ctg ctg atc gtc acc atc gtt gcc acc atg ctg gtg    1776
Cys Asn Ile Thr Leu Leu Ile Val Thr Ile Val Ala Thr Met Leu Val
555                 560                 565                 570

ttg gct gtg act gtg acc tcc ctc tgc atc tac ttg gat ctg ccc tgg    1824
Leu Ala Val Thr Val Thr Ser Leu Cys Ile Tyr Leu Asp Leu Pro Trp
                575                 580                 585

tat ctc agg atg gtg tgc cag tgg acc cag acc cgg cgc agg gcc agg    1872
Tyr Leu Arg Met Val Cys Gln Trp Thr Gln Thr Arg Arg Arg Ala Arg
            590                 595                 600

aac ata ccc tta gaa gaa ctc caa aga aat ctc cag ttt cat gca ttt    1920
Asn Ile Pro Leu Glu Glu Leu Gln Arg Asn Leu Gln Phe His Ala Phe
        605                 610                 615

att tca tat agt ggg cac gat tct ttc tgg gtg aag aat gaa tta ttg    1968
Ile Ser Tyr Ser Gly His Asp Ser Phe Trp Val Lys Asn Glu Leu Leu
    620                 625                 630

cca aac cta gag aaa gaa ggt atg cag att tgc ctt cat gag aga aac    2016
Pro Asn Leu Glu Lys Glu Gly Met Gln Ile Cys Leu His Glu Arg Asn
635                 640                 645                 650

ttt gtt cct ggc aag agc att gtg gaa aat atc atc acc tgc att gag    2064
Phe Val Pro Gly Lys Ser Ile Val Glu Asn Ile Ile Thr Cys Ile Glu
                655                 660                 665

aag agt tac aag tcc atc ttt gtt ttg tct ccc aac ttt gtc cag agt    2112
Lys Ser Tyr Lys Ser Ile Phe Val Leu Ser Pro Asn Phe Val Gln Ser
            670                 675                 680

gaa tgg tgc cat tat gaa ctc tac ttt gcc cat cac aat ctc ttt cat    2160
Glu Trp Cys His Tyr Glu Leu Tyr Phe Ala His His Asn Leu Phe His
        685                 690                 695

gaa gga tct aat agc tta atc ctg atc ttg ctg gaa ccc att ccg cag    2208
Glu Gly Ser Asn Ser Leu Ile Leu Ile Leu Leu Glu Pro Ile Pro Gln
    700                 705                 710

tac tcc att cct agc agt tat cac aag ctc aaa agt ctc atg gcc agg    2256
Tyr Ser Ile Pro Ser Ser Tyr His Lys Leu Lys Ser Leu Met Ala Arg
715                 720                 725                 730

agg act tat ttg gaa tgg ccc aag gaa aag agc aaa cgt ggc ctt ttt    2304
Arg Thr Tyr Leu Glu Trp Pro Lys Glu Lys Ser Lys Arg Gly Leu Phe
                735                 740                 745

tgg gct aac tta agg gca gcc att aat att aag ctg aca gag caa gca    2352
Trp Ala Asn Leu Arg Ala Ala Ile Asn Ile Lys Leu Thr Glu Gln Ala
            750                 755                 760

aag aaa tagtctaga                                                  2367
Lys Lys

<210> 2
<211> 786

<212> PRT
<213> Unknown

<400> 2
Met Thr Ser Ile Phe His Phe Ala Ile Ile Phe Met Leu Ile Leu Gln
        -20                 -15                 -10

Ile Arg Ile Gln Leu Ser Glu Glu Ser Glu Phe Leu Val Asp Arg Ser
    -5              -1   1               5                  10

Lys Asn Gly Leu Ile His Val Pro Lys Asp Leu Ser Gln Lys Thr Thr
                15                  20                  25

Ile Leu Asn Ile Ser Gln Asn Tyr Ile Ser Glu Leu Trp Thr Ser Asp
            30                  35                  40

Ile Leu Ser Leu Ser Lys Leu Arg Ile Leu Ile Ile Ser His Asn Arg
        45                  50                  55

Ile Gln Tyr Leu Asp Ile Ser Val Phe Lys Phe Asn Gln Glu Leu Glu
    60                  65                  70

Tyr Leu Asp Leu Ser His Asn Lys Leu Val Lys Ile Ser Cys His Pro
75                  80                  85                  90

Thr Val Asn Leu Lys His Leu Asp Leu Ser Phe Asn Ala Phe Asp Ala
                95                  100                 105

Leu Pro Ile Cys Lys Glu Phe Gly Asn Met Ser Gln Leu Lys Phe Leu
            110                 115                 120

Gly Leu Ser Thr Thr His Leu Glu Lys Ser Ser Val Leu Pro Ile Ala
        125                 130                 135

His Leu Asn Ile Ser Lys Val Leu Leu Val Leu Gly Glu Thr Tyr Gly
    140                 145                 150

Glu Lys Glu Asp Pro Glu Gly Leu Gln Asp Phe Asn Thr Glu Ser Leu
155                 160                 165                 170

His Ile Val Phe Pro Thr Asn Lys Glu Phe His Phe Ile Leu Asp Val
                175                 180                 185

Ser Val Lys Thr Val Ala Asn Leu Glu Leu Ser Asn Ile Lys Cys Val
            190                 195                 200

Leu Glu Asp Asn Lys Cys Ser Tyr Phe Leu Ser Ile Leu Ala Lys Leu
        205                 210                 215

Gln Thr Asn Pro Lys Leu Ser Ser Leu Thr Leu Asn Asn Ile Glu Thr
    220                 225                 230

Thr Trp Asn Ser Phe Ile Arg Ile Leu Gln Leu Val Trp His Thr Thr
235                 240                 245                 250

Val Trp Tyr Phe Ser Ile Ser Asn Val Lys Leu Gln Gly Gln Leu Asp
                255                 260                 265

Phe Arg Asp Phe Asp Tyr Ser Gly Thr Ser Leu Lys Ala Leu Ser Ile
            270                 275                 280

His Gln Val Val Ser Asp Val Phe Gly Phe Pro Gln Ser Tyr Ile Tyr
        285                 290                 295

Glu Ile Phe Ser Asn Met Asn Ile Lys Asn Phe Thr Val Ser Gly Thr
    300                 305                 310

Arg Met Val His Met Leu Cys Pro Ser Lys Ile Ser Pro Phe Leu His
315                 320                 325                 330

Leu Asp Phe Ser Asn Asn Leu Leu Thr Asp Thr Val Phe Glu Asn Cys
                335                 340                 345

Gly His Leu Thr Glu Leu Glu Thr Leu Ile Leu Gln Met Asn Gln Leu
            350                 355                 360

Lys Glu Leu Ser Lys Ile Ala Glu Met Thr Thr Gln Met Lys Ser Leu
        365                 370                 375

Gln Gln Leu Asp Ile Ser Gln Asn Ser Val Ser Tyr Asp Glu Lys Lys
    380                 385                 390

Gly Asp Cys Ser Trp Thr Lys Ser Leu Leu Ser Leu Asn Met Ser Ser
395                 400                 405                 410

Asn Ile Leu Thr Asp Thr Ile Phe Arg Cys Leu Pro Pro Arg Ile Lys
                415                 420                 425

Val Leu Asp Leu His Ser Asn Lys Ile Lys Ser Ile Pro Lys Gln Val
            430                 435                 440

Val Lys Leu Glu Ala Leu Gln Glu Leu Asn Val Ala Phe Asn Ser Leu
        445                 450                 455

Thr Asp Leu Pro Gly Cys Gly Ser Phe Ser Ser Leu Ser Val Leu Ile
    460                 465                 470

Ile Asp His Asn Ser Val Ser His Pro Ser Ala Asp Phe Phe Gln Ser
475                 480                 485                 490

Cys Gln Lys Met Arg Ser Ile Lys Ala Gly Asp Asn Pro Phe Gln Cys
                495                 500                 505

Thr Cys Glu Leu Gly Glu Phe Val Lys Asn Ile Asp Gln Val Ser Ser
            510                 515                 520

Glu Val Leu Glu Gly Trp Pro Asp Ser Tyr Lys Cys Asp Tyr Pro Glu
        525                 530                 535

Ser Tyr Arg Gly Thr Leu Leu Lys Asp Phe His Met Ser Glu Leu Ser
    540                 545                 550

Cys Asn Ile Thr Leu Leu Ile Val Thr Ile Val Ala Thr Met Leu Val
555                 560                 565                 570

Leu Ala Val Thr Val Thr Ser Leu Cys Ile Tyr Leu Asp Leu Pro Trp

575                 580                 585

Tyr Leu Arg Met Val Cys Gln Trp Thr Gln Thr Arg Arg Arg Ala Arg
            590                 595                 600

Asn Ile Pro Leu Glu Glu Leu Gln Arg Asn Leu Gln Phe His Ala Phe
        605                 610                 615

Ile Ser Tyr Ser Gly His Asp Ser Phe Trp Val Lys Asn Glu Leu Leu
    620                 625                 630

Pro Asn Leu Glu Lys Glu Gly Met Gln Ile Cys Leu His Glu Arg Asn
635                 640                 645                 650

Phe Val Pro Gly Lys Ser Ile Val Glu Asn Ile Ile Thr Cys Ile Glu
                655                 660                 665

Lys Ser Tyr Lys Ser Ile Phe Val Leu Ser Pro Asn Phe Val Gln Ser
            670                 675                 680

Glu Trp Cys His Tyr Glu Leu Tyr Phe Ala His His Asn Leu Phe His
        685                 690                 695

Glu Gly Ser Asn Ser Leu Ile Leu Ile Leu Leu Glu Pro Ile Pro Gln
    700                 705                 710

Tyr Ser Ile Pro Ser Ser Tyr His Lys Leu Lys Ser Leu Met Ala Arg
715                 720                 725                 730

Arg Thr Tyr Leu Glu Trp Pro Lys Glu Lys Ser Lys Arg Gly Leu Phe
                735                 740                 745

Trp Ala Asn Leu Arg Ala Ala Ile Asn Ile Lys Leu Thr Glu Gln Ala
            750                 755                 760

Lys Lys

<210> 3
<211> 2355
<212> DNA
<213> Unknown

<220>
<223> Description of Unknown Organism:primate; surmised
      Homo sapiens

<220>
<221> CDS
<222> (1)..(2352)

<220>
<221> mat_peptide
<222> (67)..(2352)

<400> 3
atg cca cat act ttg tgg atg gtg tgg gtc ttg ggg gtc atc atc agc      48
ctc tcc aag gaa gaa tcc tcc aat cag gct tct ctg tct tgt gac cgc 96
aat ggt atc tgc aag ggc agc tca gga tct tta aac tcc att ccc tca 144
ggg ctc aca gaa gct gta aaa agc ctt gac ctg tcc aac aac agg atc 192
acc tac att agc aac agt gac cta cag agg tgt gtg aac ctc cag gct 240
ctg gtg ctg aca tcc aat gga att aac aca ata gag gaa gat tct ttt 288
tct tcc ctg ggc agt ctt gaa cat tta gac tta tcc tat aat tac tta 336
tct aat tta tcg tct tcc tgg ttc aag ccc ctt tct tct tta aca ttc 384
tta aac tta ctg gga aat cct tac aaa acc cta ggg gaa aca tct ctt 432
ttt tct cat ctc aca aaa ttg caa atc ctg aga gtg gga aat atg gac 480
acc ttc act aag att caa aga aaa gat ttt gct gga ctt acc ttc ctt 528
gag gaa ctt gag att gat gct tca gat cta cag agc tat gag cca aaa 576
agt ttg aag tca att cag aac gta agt cat ctg atc ctt cat atg aag 624
cag cat att tta ctg ctg gag att ttt gta gat gtt aca agt tcc gtg 672
gaa tgt ttg gaa ctg cga gat act gat ttg gac act ttc cat ttt tca 720
gaa cta tcc act ggt gaa aca aat tca ttg att aaa aag ttt aca ttt 768
aga aat gtg aaa atc acc gat gaa agt ttg ttt cag gtt atg aaa ctt 816
ttg aat cag att tct gga ttg tta gaa tta gag ttt gat gac tgt acc 864
ctt aat gga gtt ggt aat ttt aga gca tct gat aat gac aga gtt ata 912
gat cca ggt aaa gtg gaa acg tta aca atc cgg agg ctg cat att cca 960
agg ttt tac tta ttt tat gat ctg agc act tta tat tca ctt aca gaa 1008
aga gtt aaa aga atc aca gta gaa aac agt aaa gtt ttt ctg gtt cct 1056
tgt tta ctt tca caa cat tta aaa tca tta gaa tac ttg gat ctc agt 1104
gaa aat ttg atg gtt gaa gaa tac ttg aaa aat tca gcc tgt gag gat 1152
gcc tgg ccc tct cta caa act tta att tta agg caa aat cat ttg gca 1200
tca ttg gaa aaa acc gga gag act ttg ctc act ctg aaa aac ttg act 1248
aac att gat atc agt aag aat agt ttt cat tct atg cct gaa act tgt 1296
cag tgg cca gaa aag atg aaa tat ttg aac tta tcc agc aca cga ata 1344
cac agt gta aca ggc tgc att ccc aag aca ctg gaa att tta gat gtt 1392
agc aac aac aat ctc aat tta ttt tct ttg aat ttg ccg caa ctc aaa 1440
gaa ctt tat att tcc aga aat aag ttg atg act cta cca gat gcc tcc 1488
ctc tta ccc atg tta cta gta ttg aaa atc agt agg aat gca ata act 1536
acg ttt tct aag gag caa ctt gac tca ttt cac aca ctg aag act ttg 1584
gaa gct ggt ggc aat aac ttc att tgc tcc tgt gaa ttc ctc tcc ttc 1632
act cag gag cag caa gca ctg gcc aaa gtc ttg att gat tgg cca gca 1680
aat tac ctg tgt gac tct cca tcc cat gtg cgt ggc cag cag gtt cag 1728
gat gtc cgc ctc tcg gtg tcg gaa tgt cac agg aca gca ctg gtg tct 1776
ggc atg tgc tgt gct ctg ttc ctg ctg atc ctg ctc acg ggg gtc ctg 1824
tgc cac cgt ttc cat ggc ctg tgg tat atg aaa atg atg tgg gcc tgg 1872
ctc cag gcc aaa agg aag ccc agg aaa gct ccc agc agg aac atc tgc 1920
tat gat gca ttt gtt tct tac agt gag cgg gat gcc tac tgg gtg gag 1968
aac ctt atg gtc cag gag ctg gag aac ttc aat ccc ccc ttc aag ttg 2016
tgt ctt cat aag cgg gac ttc att cct ggc aag tgg atc att gac aat 2064
atc att gac tcc att gaa aag agc cac aaa act gtc ttt gtg ctt tct 2112
gaa aac ttt gtg aag agt gag tgg tgc aag tat gaa ctg gac ttc tcc 2160
cat ttc cgt ctt ttt gaa gag aac aat gat gct gcc att ctc att ctt 2208
ctg gag ccc att gag aaa aaa gcc att ccc cag cgc ttc tgc aag ctg 2256
cgg aag ata atg aac acc aag acc tac ctg gag tgg ccc atg gac gag 2304
gct cag cgg gaa gga ttt tgg gta aat ctg aga gct gcg ata aag tcc 2352
tag 2355

<210> 4
<211> 784
<212> PRT
<213> Unknown

<400> 4

<210> 5
<211> 2715
<212> DNA
<213> Unknown

<220>
<223> Description of Unknown Organism:primate; surmised
      Homo sapiens

<220>
<221> CDS
<222> (1)..(2712)

<220>
<221> mat_peptide
<222> (64)..(2712)

<400> 5
atg aga cag act ttg cct tgt atc tac ttt tgg ggg ggc ctt ttg ccc      48
Met Arg Gln Thr Leu Pro Cys Ile Tyr Phe Trp Gly Gly Leu Leu Pro
    -20                 -15                 -10

ttt ggg atg ctg tgt gca tcc tcc acc acc aag tgc act gtt agc cat      96
Phe Gly Met Leu Cys Ala Ser Ser Thr Thr Lys Cys Thr Val Ser His
 -5                  -1   1               5                  10

gaa gtt gct gac tgc agc cac ctg aag ttg act cag gta ccc gat gat     144
Glu Val Ala Asp Cys Ser His Leu Lys Leu Thr Gln Val Pro Asp Asp
            15                  20                  25

cta ccc aca aac ata aca gtg ttg aac ctt acc cat aat caa ctc aga     192
Leu Pro Thr Asn Ile Thr Val Leu Asn Leu Thr His Asn Gln Leu Arg
        30                  35                  40

aga tta cca gcc gcc aac ttc aca agg tat agc cag cta act agc ttg     240
Arg Leu Pro Ala Ala Asn Phe Thr Arg Tyr Ser Gln Leu Thr Ser Leu
    45                  50                  55

gat gta gga ttt aac acc atc tca aaa ctg gag cca gaa ttg tgc cag     288
Asp Val Gly Phe Asn Thr Ile Ser Lys Leu Glu Pro Glu Leu Cys Gln
 60                  65                  70                  75

aaa ctt ccc atg tta aaa gtt ttg aac ctc cag cac aat gag cta tct     336
Lys Leu Pro Met Leu Lys Val Leu Asn Leu Gln His Asn Glu Leu Ser
                80                  85                  90

caa ctt tct gat aaa acc ttt gcc ttc tgc acg aat ttg act gaa ctc     384
Gln Leu Ser Asp Lys Thr Phe Ala Phe Cys Thr Asn Leu Thr Glu Leu
            95                 100                 105

cat ctc atg tcc aac tca atc cag aaa att aaa aat aat ccc ttt gtc     432
His Leu Met Ser Asn Ser Ile Gln Lys Ile Lys Asn Asn Pro Phe Val
        110                 115                 120

aag cag aag aat tta atc aca tta gat ctg tct cat aat ggc ttg tca 480
Lys Gln Lys Asn Leu Ile Thr Leu Asp Leu Ser His Asn Gly Leu Ser
125 130 135

tct aca aaa tta gga act cag gtt cag ctg gaa aat ctc caa gag ctt 528
Ser Thr Lys Leu Gly Thr Gln Val Gln Leu Glu Asn Leu Gln Glu Leu
140 145 150 155

cta tta tca aac aat aaa att caa gcg cta aaa agt gaa gaa ctg gat 576
Leu Leu Ser Asn Asn Lys Ile Gln Ala Leu Lys Ser Glu Glu Leu Asp
160 165 170

atc ttt gcc aat tca tct tta aaa aaa tta gag ttg tca tcg aat caa 624
Ile Phe Ala Asn Ser Ser Leu Lys Lys Leu Glu Leu Ser Ser Asn Gln
175 180 185

att aaa gag ttt tct cca ggg tgt ttt cac gca att gga aga tta ttt 672
Ile Lys Glu Phe Ser Pro Gly Cys Phe His Ala Ile Gly Arg Leu Phe
190 195 200

ggc ctc ttt ctg aac aat gtc cag ctg ggt ccc agc ctt aca gag aag 720
Gly Leu Phe Leu Asn Asn Val Gln Leu Gly Pro Ser Leu Thr Glu Lys
205 210 215

cta tgt ttg gaa tta gca aac aca agc att cgg aat ctg tct ctg agt 768
Leu Cys Leu Glu Leu Ala Asn Thr Ser Ile Arg Asn Leu Ser Leu Ser
220 225 230 235

aac agc cag ctg tcc acc acc agc aat aca act ttc ttg gga cta aag 816
Asn Ser Gln Leu Ser Thr Thr Ser Asn Thr Thr Phe Leu Gly Leu Lys
240 245 250

tgg aca aat ctc act atg ctc gat ctt tcc tac aac aac tta aat gtg 864
Trp Thr Asn Leu Thr Met Leu Asp Leu Ser Tyr Asn Asn Leu Asn Val
255 260 265

gtt ggt aac gat tcc ttt gct tgg ctt cca caa cta gaa tat ttc ttc 912
Val Gly Asn Asp Ser Phe Ala Trp Leu Pro Gln Leu Glu Tyr Phe Phe
270 275 280

cta gag tat aat aat ata cag cat ttg ttt tct cac tct ttg cac ggg 960
Leu Glu Tyr Asn Asn Ile Gln His Leu Phe Ser His Ser Leu His Gly
285 290 295

ctt ttc aat gtg agg tac ctg aat ttg aaa cgg tct ttt act aaa caa 1008
Leu Phe Asn Val Arg Tyr Leu Asn Leu Lys Arg Ser Phe Thr Lys Gln
300 305 310 315

agt att tcc ctt gcc tca ctc ccc aag att gat gat ttt tct ttt cag 1056
Ser Ile Ser Leu Ala Ser Leu Pro Lys Ile Asp Asp Phe Ser Phe Gln
320 325 330

tgg cta aaa tgt ttg gag cac ctt aac atg gaa gat aat gat att cca 1104
Trp Leu Lys Cys Leu Glu His Leu Asn Met Glu Asp Asn Asp Ile Pro
335 340 345

ggc ata aaa agc aat atg ttc aca gga ttg ata aac ctg aaa tac tta 1152
Gly Ile Lys Ser Asn Met Phe Thr Gly Leu Ile Asn Leu Lys Tyr Leu

350 355 360

agt cta tcc aac tcc ttt aca agt ttg cga act ttg aca aat gaa aca 1200
Ser Leu Ser Asn Ser Phe Thr Ser Leu Arg Thr Leu Thr Asn Glu Thr
365 370 375

ttt gta tca ctt gct cat tct ccc tta cac ata ctc aac cta acc aag 1248
Phe Val Ser Leu Ala His Ser Pro Leu His Ile Leu Asn Leu Thr Lys
380 385 390 395

aat aaa atc tca aaa ata gag agt gat gct ttc tct tgg ttg ggc cac 1296
Asn Lys Ile Ser Lys Ile Glu Ser Asp Ala Phe Ser Trp Leu Gly His
400 405 410

cta gaa gta ctt gac ctg ggc ctt aat gaa att ggg caa gaa ctc aca 1344
Leu Glu Val Leu Asp Leu Gly Leu Asn Glu Ile Gly Gln Glu Leu Thr
415 420 425

ggc cag gaa tgg aga ggt cta gaa aat att ttc gaa atc tat ctt tcc 1392
Gly Gln Glu Trp Arg Gly Leu Glu Asn Ile Phe Glu Ile Tyr Leu Ser
430 435 440

tac aac aag tac ctg cag ctg act agg aac tcc ttt gcc ttg gtc cca 1440
Tyr Asn Lys Tyr Leu Gln Leu Thr Arg Asn Ser Phe Ala Leu Val Pro
445 450 455

agc ctt caa cga ctg atg ctc cga agg gtg gcc ctt aaa aat gtg gat 1488
Ser Leu Gln Arg Leu Met Leu Arg Arg Val Ala Leu Lys Asn Val Asp
460 465 470 475

agc tct cct tca cca ttc cag cct ctt cgt aac ttg acc att ctg gat 1536
Ser Ser Pro Ser Pro Phe Gln Pro Leu Arg Asn Leu Thr Ile Leu Asp
480 485 490

cta agc aac aac aac ata gcc aac ata aat gat gac atg ttg gag ggt 1584
Leu Ser Asn Asn Asn Ile Ala Asn Ile Asn Asp Asp Met Leu Glu Gly
495 500 505

ctt gag aaa cta gaa att ctc gat ttg cag cat aac aac tta gca cgg 1632
Leu Glu Lys Leu Glu Ile Leu Asp Leu Gln His Asn Asn Leu Ala Arg
510 515 520

ctc tgg aaa cac gca aac cct ggt ggt ccc att tat ttc cta aag ggt 1680
Leu Trp Lys His Ala Asn Pro Gly Gly Pro Ile Tyr Phe Leu Lys Gly
525 530 535

ctg tct cac ctc cac atc ctt aac ttg gag tcc aac ggc ttt gac gag 1728
Leu Ser His Leu His Ile Leu Asn Leu Glu Ser Asn Gly Phe Asp Glu
540 545 550 555

atc cca gtt gag gtc ttc aag gat tta ttt gaa cta aag atc atc gat 1776
Ile Pro Val Glu Val Phe Lys Asp Leu Phe Glu Leu Lys Ile Ile Asp
560 565 570

tta gga ttg aat aat tta aac aca ctt cca gca tct gtc ttt aat aat 1824
Leu Gly Leu Asn Asn Leu Asn Thr Leu Pro Ala Ser Val Phe Asn Asn
575 580 585

cag gtg tct cta aag tca ttg aac ctt cag aag aat ctc ata aca tcc 1872
Gln Val Ser Leu Lys Ser Leu Asn Leu Gln Lys Asn Leu Ile Thr Ser
590 595 600

gtt gag aag aag gtt ttc ggg cca gct ttc agg aac ctg act gag tta 1920
Val Glu Lys Lys Val Phe Gly Pro Ala Phe Arg Asn Leu Thr Glu Leu
605 610 615

gat atg cgc ttt aat ccc ttt gat tgc acg tgt gaa agt att gcc tgg 1968
Asp Met Arg Phe Asn Pro Phe Asp Cys Thr Cys Glu Ser Ile Ala Trp
620 625 630 635

ttt gtt aat tgg att aac gag acc cat acc aac atc cct gag ctg tca 2016
Phe Val Asn Trp Ile Asn Glu Thr His Thr Asn Ile Pro Glu Leu Ser
640 645 650

agc cac tac ctt tgc aac act cca cct cac tat cat ggg ttc cca gtg 2064
Ser His Tyr Leu Cys Asn Thr Pro Pro His Tyr His Gly Phe Pro Val
655 660 665

aga ctt ttt gat aca tca tct tgc aaa gac agt gcc ccc ttt gaa ctc 2112
Arg Leu Phe Asp Thr Ser Ser Cys Lys Asp Ser Ala Pro Phe Glu Leu
670 675 680

ttt ttc atg atc aat acc agt atc ctg ttg att ttt atc ttt att gta 2160
Phe Phe Met Ile Asn Thr Ser Ile Leu Leu Ile Phe Ile Phe Ile Val
685 690 695

ctt ctc atc cac ttt gag ggc tgg agg ata tct ttt tat tgg aat gtt 2208
Leu Leu Ile His Phe Glu Gly Trp Arg Ile Ser Phe Tyr Trp Asn Val
700 705 710 715

tca gta cat cga gtt ctt ggt ttc aaa gaa ata gac aga cag aca gaa 2256
Ser Val His Arg Val Leu Gly Phe Lys Glu Ile Asp Arg Gln Thr Glu
720 725 730

cag ttt gaa tat gca gca tat ata att cat gcc tat aaa gat aag gat 2304
Gln Phe Glu Tyr Ala Ala Tyr Ile Ile His Ala Tyr Lys Asp Lys Asp
735 740 745

tgg gtc tgg gaa cat ttc tct tca atg gaa aag gaa gac caa tct ctc 2352
Trp Val Trp Glu His Phe Ser Ser Met Glu Lys Glu Asp Gln Ser Leu
750 755 760

aaa ttt tgt ctg gaa gaa agg gac ttt gag gcg ggt gtt ttt gaa cta 2400
Lys Phe Cys Leu Glu Glu Arg Asp Phe Glu Ala Gly Val Phe Glu Leu
765 770 775

gaa gca att gtt aac agc atc aaa aga agc aga aaa att att ttt gtt 2448
Glu Ala Ile Val Asn Ser Ile Lys Arg Ser Arg Lys Ile Ile Phe Val
780 785 790 795

ata aca cac cat cta tta aaa gac cca tta tgc aaa aga ttc aag gta 2496
Ile Thr His His Leu Leu Lys Asp Pro Leu Cys Lys Arg Phe Lys Val
800 805 810

cat cat gca gtt caa caa gct att gaa caa aat ctg gat tcc att ata 2544
His His Ala Val Gln Gln Ala Ile Glu Gln Asn Leu Asp Ser Ile Ile

815 820 825

ttg gtt ttc ctt gag gag att cca gat tat aaa ctg aac cat gca ctc 2592
Leu Val Phe Leu Glu Glu Ile Pro Asp Tyr Lys Leu Asn His Ala Leu
830 835 840

tgt ttg cga aga gga atg ttt aaa tct cac tgc atc ttg aac tgg cca 2640
Cys Leu Arg Arg Gly Met Phe Lys Ser His Cys Ile Leu Asn Trp Pro
845 850 855

gtt cag aaa gaa cgg ata ggt gcc ttt cgt cat aaa ttg caa gta gca 2688
Val Gln Lys Glu Arg Ile Gly Ala Phe Arg His Lys Leu Gln Val Ala
860 865 870 875

ctt gga tcc aaa aac tct gta cat taa 2715
Leu Gly Ser Lys Asn Ser Val His
880

<210> 6
<211> 904
<212> PRT
<213> Unknown

<400> 6
Met Arg Gln Thr Leu Pro Cys Ile Tyr Phe Trp Gly Gly Leu Leu Pro
-20 -15 -10

Phe Gly Met Leu Cys Ala Ser Ser Thr Thr Lys Cys Thr Val Ser His
-5 -1 1 5 10

Glu Val Ala Asp Cys Ser His Leu Lys Leu Thr Gln Val Pro Asp Asp
15 20 25

Leu Pro Thr Asn Ile Thr Val Leu Asn Leu Thr His Asn Gln Leu Arg
30 35 40

Arg Leu Pro Ala Ala Asn Phe Thr Arg Tyr Ser Gln Leu Thr Ser Leu
45 50 55

Asp Val Gly Phe Asn Thr Ile Ser Lys Leu Glu Pro Glu Leu Cys Gln
60 65 70 75

Lys Leu Pro Met Leu Lys Val Leu Asn Leu Gln His Asn Glu Leu Ser
80 85 90

Gln Leu Ser Asp Lys Thr Phe Ala Phe Cys Thr Asn Leu Thr Glu Leu
95 100 105

His Leu Met Ser Asn Ser Ile Gln Lys Ile Lys Asn Asn Pro Phe Val
110 115 120

Lys Gln Lys Asn Leu Ile Thr Leu Asp Leu Ser His Asn Gly Leu Ser
125 130 135

Ser Thr Lys Leu Gly Thr Gln Val Gln Leu Glu Asn Leu Gln Glu Leu
140 145 150 155

Leu Leu Ser Asn Asn Lys Ile Gln Ala Leu Lys Ser Glu Glu Leu Asp
160 165 170

Ile Phe Ala Asn Ser Ser Leu Lys Lys Leu Glu Leu Ser Ser Asn Gln
175 180 185

Ile Lys Glu Phe Ser Pro Gly Cys Phe His Ala Ile Gly Arg Leu Phe
190 195 200

Gly Leu Phe Leu Asn Asn Val Gln Leu Gly Pro Ser Leu Thr Glu Lys
205 210 215

Leu Cys Leu Glu Leu Ala Asn Thr Ser Ile Arg Asn Leu Ser Leu Ser
220 225 230 235

Asn Ser Gln Leu Ser Thr Thr Ser Asn Thr Thr Phe Leu Gly Leu Lys
240 245 250

Trp Thr Asn Leu Thr Met Leu Asp Leu Ser Tyr Asn Asn Leu Asn Val
255 260 265

Val Gly Asn Asp Ser Phe Ala Trp Leu Pro Gln Leu Glu Tyr Phe Phe
270 275 280

Leu Glu Tyr Asn Asn Ile Gln His Leu Phe Ser His Ser Leu His Gly
285 290 295

Leu Phe Asn Val Arg Tyr Leu Asn Leu Lys Arg Ser Phe Thr Lys Gln
300 305 310 315

Ser Ile Ser Leu Ala Ser Leu Pro Lys Ile Asp Asp Phe Ser Phe Gln
320 325 330

Trp Leu Lys Cys Leu Glu His Leu Asn Met Glu Asp Asn Asp Ile Pro
335 340 345

Gly Ile Lys Ser Asn Met Phe Thr Gly Leu Ile Asn Leu Lys Tyr Leu
350 355 360

Ser Leu Ser Asn Ser Phe Thr Ser Leu Arg Thr Leu Thr Asn Glu Thr
365 370 375

Phe Val Ser Leu Ala His Ser Pro Leu His Ile Leu Asn Leu Thr Lys
380 385 390 395

Asn Lys Ile Ser Lys Ile Glu Ser Asp Ala Phe Ser Trp Leu Gly His
400 405 410

Leu Glu Val Leu Asp Leu Gly Leu Asn Glu Ile Gly Gln Glu Leu Thr
415 420 425

Gly Gln Glu Trp Arg Gly Leu Glu Asn Ile Phe Glu Ile Tyr Leu Ser
430 435 440

Tyr Asn Lys Tyr Leu Gln Leu Thr Arg Asn Ser Phe Ala Leu Val Pro
445 450 455

Ser Leu Gln Arg Leu Met Leu Arg Arg Val Ala Leu Lys Asn Val Asp

460                 465                 470                 475

Ser Ser Pro Ser Pro Phe Gln Pro Leu Arg Asn Leu Thr Ile Leu Asp
                480                 485                 490

Leu Ser Asn Asn Asn Ile Ala Asn Ile Asn Asp Asp Met Leu Glu Gly
            495                 500                 505

Leu Glu Lys Leu Glu Ile Leu Asp Leu Gln His Asn Asn Leu Ala Arg
        510                 515                 520

Leu Trp Lys His Ala Asn Pro Gly Gly Pro Ile Tyr Phe Leu Lys Gly
    525                 530                 535

Leu Ser His Leu His Ile Leu Asn Leu Glu Ser Asn Gly Phe Asp Glu
540                 545                 550                 555

Ile Pro Val Glu Val Phe Lys Asp Leu Phe Glu Leu Lys Ile Ile Asp
                560                 565                 570

Leu Gly Leu Asn Asn Leu Asn Thr Leu Pro Ala Ser Val Phe Asn Asn
            575                 580                 585

Gln Val Ser Leu Lys Ser Leu Asn Leu Gln Lys Asn Leu Ile Thr Ser
        590                 595                 600

Val Glu Lys Lys Val Phe Gly Pro Ala Phe Arg Asn Leu Thr Glu Leu
    605                 610                 615

Asp Met Arg Phe Asn Pro Phe Asp Cys Thr Cys Glu Ser Ile Ala Trp
620                 625                 630                 635

Phe Val Asn Trp Ile Asn Glu Thr His Thr Asn Ile Pro Glu Leu Ser
                640                 645                 650

Ser His Tyr Leu Cys Asn Thr Pro Pro His Tyr His Gly Phe Pro Val
            655                 660                 665

Arg Leu Phe Asp Thr Ser Ser Cys Lys Asp Ser Ala Pro Phe Glu Leu
        670                 675                 680

Phe Phe Met Ile Asn Thr Ser Ile Leu Leu Ile Phe Ile Phe Ile Val
    685                 690                 695

Leu Leu Ile His Phe Glu Gly Trp Arg Ile Ser Phe Tyr Trp Asn Val
700                 705                 710                 715

Ser Val His Arg Val Leu Gly Phe Lys Glu Ile Asp Arg Gln Thr Glu
                720                 725                 730

Gln Phe Glu Tyr Ala Ala Tyr Ile Ile His Ala Tyr Lys Asp Lys Asp
            735                 740                 745

Trp Val Trp Glu His Phe Ser Ser Met Glu Lys Glu Asp Gln Ser Leu
        750                 755                 760

Lys Phe Cys Leu Glu Glu Arg Asp Phe Glu Ala Gly Val Phe Glu Leu
    765                 770                 775

Glu Ala Ile Val Asn Ser Ile Lys Arg Ser Arg Lys Ile Ile Phe Val
780                 785                 790                 795

Ile Thr His His Leu Leu Lys Asp Pro Leu Cys Lys Arg Phe Lys Val
                800                 805                 810

His His Ala Val Gln Gln Ala Ile Glu Gln Asn Leu Asp Ser Ile Ile
            815                 820                 825

Leu Val Phe Leu Glu Glu Ile Pro Asp Tyr Lys Leu Asn His Ala Leu
        830                 835                 840

Cys Leu Arg Arg Gly Met Phe Lys Ser His Cys Ile Leu Asn Trp Pro
    845                 850                 855

Val Gln Lys Glu Arg Ile Gly Ala Phe Arg His Lys Leu Gln Val Ala
860                 865                 870                 875

Leu Gly Ser Lys Asn Ser Val His
                880

<210> 7
<211> 2400
<212> DNA
<213> Unknown

<220>
<223> Description of Unknown Organism:primate; surmised
      Homo sapiens

<220>
<221> CDS
<222> (1)..(2397)

<400> 7
atg gag ctg aat ttc tac aaa atc ccc gac aac ctc ccc ttc tca acc      48
Met Glu Leu Asn Phe Tyr Lys Ile Pro Asp Asn Leu Pro Phe Ser Thr
  1               5                  10                  15

aag aac ctg gac ctg agc ttt aat ccc ctg agg cat tta ggc agc tat      96
Lys Asn Leu Asp Leu Ser Phe Asn Pro Leu Arg His Leu Gly Ser Tyr
             20                  25                  30

agc ttc ttc agt ttc cca gaa ctg cag gtg ctg gat tta tcc agg tgt     144
Ser Phe Phe Ser Phe Pro Glu Leu Gln Val Leu Asp Leu Ser Arg Cys
         35                  40                  45

gaa atc cag aca att gaa gat ggg gca tat cag agc cta agc cac ctc     192
Glu Ile Gln Thr Ile Glu Asp Gly Ala Tyr Gln Ser Leu Ser His Leu
     50                  55                  60

tct acc tta ata ttg aca gga aac ccc atc cag agt tta gcc ctg gga     240
Ser Thr Leu Ile Leu Thr Gly Asn Pro Ile Gln Ser Leu Ala Leu Gly
 65                  70                  75                  80

gcc ttt tct gga cta tca agt tta cag aag ctg gtg gct gtg gag aca     288

Ala Phe Ser Gly Leu Ser Ser Leu Gln Lys Leu Val Ala Val Glu Thr
                85                  90                  95

aat cta gca tct cta gag aac ttc ccc att gga cat ctc aaa act ttg 336
Asn Leu Ala Ser Leu Glu Asn Phe Pro Ile Gly His Leu Lys Thr Leu
            100                 105                 110

aaa gaa ctt aat gtg gct cac aat ctt atc caa tct ttc aaa tta cct 384
Lys Glu Leu Asn Val Ala His Asn Leu Ile Gln Ser Phe Lys Leu Pro
        115                 120                 125

gag tat ttt tct aat ctg acc aat cta gag cac ttg gac ctt tcc agc 432
Glu Tyr Phe Ser Asn Leu Thr Asn Leu Glu His Leu Asp Leu Ser Ser
    130                 135                 140

aac aag att caa agt att tat tgc aca gac ttg cgg gtt cta cat caa 480
Asn Lys Ile Gln Ser Ile Tyr Cys Thr Asp Leu Arg Val Leu His Gln
145                 150                 155                 160

atg ccc cta ctc aat ctc tct tta gac ctg tcc ctg aac cct atg aac 528
Met Pro Leu Leu Asn Leu Ser Leu Asp Leu Ser Leu Asn Pro Met Asn
                165                 170                 175

ttt atc caa cca ggt gca ttt aaa gaa att agg ctt cat aag ctg act 576
Phe Ile Gln Pro Gly Ala Phe Lys Glu Ile Arg Leu His Lys Leu Thr
            180                 185                 190

tta aga aat aat ttt gat agt tta aat gta atg aaa act tgt att caa 624
Leu Arg Asn Asn Phe Asp Ser Leu Asn Val Met Lys Thr Cys Ile Gln
        195                 200                 205

ggt ctg gct ggt tta gaa gtc cat cgt ttg gtt ctg gga gaa ttt aga 672
Gly Leu Ala Gly Leu Glu Val His Arg Leu Val Leu Gly Glu Phe Arg
    210                 215                 220

aat gaa gga aac ttg gaa aag ttt gac aaa tct gct cta gag ggc ctg 720
Asn Glu Gly Asn Leu Glu Lys Phe Asp Lys Ser Ala Leu Glu Gly Leu
225                 230                 235                 240

tgc aat ttg acc att gaa gaa ttc cga tta gca tac tta gac tac tac 768
Cys Asn Leu Thr Ile Glu Glu Phe Arg Leu Ala Tyr Leu Asp Tyr Tyr
                245                 250                 255

ctc gat gat att att gac tta ttt aat tgt ttg aca aat gtt tct tca 816
Leu Asp Asp Ile Ile Asp Leu Phe Asn Cys Leu Thr Asn Val Ser Ser
            260                 265                 270

ttt tcc ctg gtg agt gtg act att gaa agg gta aaa gac ttt tct tat 864
Phe Ser Leu Val Ser Val Thr Ile Glu Arg Val Lys Asp Phe Ser Tyr
        275                 280                 285

aat ttc gga tgg caa cat tta gaa tta gtt aac tgt aaa ttt gga cag 912
Asn Phe Gly Trp Gln His Leu Glu Leu Val Asn Cys Lys Phe Gly Gln
    290                 295                 300

ttt ccc aca ttg aaa ctc aaa tct ctc aaa agg ctt act ttc act tcc 960
Phe Pro Thr Leu Lys Leu Lys Ser Leu Lys Arg Leu Thr Phe Thr Ser
305                 310                 315                 320

aac aaa ggt ggg aat gct ttt tca gaa gtt gat cta cca agc ctt gag 1008
Asn Lys Gly Gly Asn Ala Phe Ser Glu Val Asp Leu Pro Ser Leu Glu
                325                 330                 335

ttt cta gat ctc agt aga aat ggc ttg agt ttc aaa ggt tgc tgt tct 1056
Phe Leu Asp Leu Ser Arg Asn Gly Leu Ser Phe Lys Gly Cys Cys Ser
            340                 345                 350

caa agt gat ttt ggg aca acc agc cta aag tat tta gat ctg agc ttc 1104
Gln Ser Asp Phe Gly Thr Thr Ser Leu Lys Tyr Leu Asp Leu Ser Phe
        355                 360                 365

aat ggt gtt att acc atg agt tca aac ttc ttg ggc tta gaa caa cta 1152
Asn Gly Val Ile Thr Met Ser Ser Asn Phe Leu Gly Leu Glu Gln Leu
    370                 375                 380

gaa cat ctg gat ttc cag cat tcc aat ttg aaa caa atg agt gag ttt 1200
Glu His Leu Asp Phe Gln His Ser Asn Leu Lys Gln Met Ser Glu Phe
385                 390                 395                 400

tca gta ttc cta tca ctc aga aac ctc att tac ctt gac att tct cat 1248
Ser Val Phe Leu Ser Leu Arg Asn Leu Ile Tyr Leu Asp Ile Ser His
                405                 410                 415

act cac acc aga gtt gct ttc aat ggc atc ttc aat ggc ttg tcc agt 1296
Thr His Thr Arg Val Ala Phe Asn Gly Ile Phe Asn Gly Leu Ser Ser
            420                 425                 430

ctc gaa gtc ttg aaa atg gct ggc aat tct ttc cag gaa aac ttc ctt 1344
Leu Glu Val Leu Lys Met Ala Gly Asn Ser Phe Gln Glu Asn Phe Leu
        435                 440                 445

cca gat atc ttc aca gag ctg aga aac ttg acc ttc ctg gac ctc tct 1392
Pro Asp Ile Phe Thr Glu Leu Arg Asn Leu Thr Phe Leu Asp Leu Ser
    450                 455                 460

cag tgt caa ctg gag cag ttg tct cca aca gca ttt aac tca ctc tcc 1440
Gln Cys Gln Leu Glu Gln Leu Ser Pro Thr Ala Phe Asn Ser Leu Ser
465                 470                 475                 480

agt ctt cag gta cta aat atg agc cac aac aac ttc ttt tca ttg gat 1488
Ser Leu Gln Val Leu Asn Met Ser His Asn Asn Phe Phe Ser Leu Asp
                485                 490                 495

acg ttt cct tat aag tgt ctg aac tcc ctc cag gtt ctt gat tac agt 1536
Thr Phe Pro Tyr Lys Cys Leu Asn Ser Leu Gln Val Leu Asp Tyr Ser
            500                 505                 510

ctc aat cac ata atg act tcc aaa aaa cag gaa cta cag cat ttt cca 1584
Leu Asn His Ile Met Thr Ser Lys Lys Gln Glu Leu Gln His Phe Pro
        515                 520                 525

agt agt cta gct ttc tta aat ctt act cag aat gac ttt gct tgt act 1632
Ser Ser Leu Ala Phe Leu Asn Leu Thr Gln Asn Asp Phe Ala Cys Thr
    530                 535                 540

tgt gaa cac cag agt ttc ctg caa tgg atc aag gac cag agg cag ctc 1680

Cys Glu His Gln Ser Phe Leu Gln Trp Ile Lys Asp Gln Arg Gln Leu
545                 550                 555                 560

ttg gtg gaa gtt gaa cga atg gaa tgt gca aca cct tca gat aag cag    1728
Leu Val Glu Val Glu Arg Met Glu Cys Ala Thr Pro Ser Asp Lys Gln
                565                 570                 575

ggc atg cct gtg ctg agt ttg aat atc acc tgt cag atg aat aag acc    1776
Gly Met Pro Val Leu Ser Leu Asn Ile Thr Cys Gln Met Asn Lys Thr
            580                 585                 590

atc att ggt gtg tcg gtc ctc agt gtg ctt gta gta tct gtt gta gca    1824
Ile Ile Gly Val Ser Val Leu Ser Val Leu Val Val Ser Val Val Ala
        595                 600                 605

gtt ctg gtc tat aag ttc tat ttt cac ctg atg ctt ctt gct ggc tgc    1872
Val Leu Val Tyr Lys Phe Tyr Phe His Leu Met Leu Leu Ala Gly Cys
    610                 615                 620

ata aag tat ggt aga ggt gaa aac atc tat gat gcc ttt gtt atc tac    1920
Ile Lys Tyr Gly Arg Gly Glu Asn Ile Tyr Asp Ala Phe Val Ile Tyr
625                 630                 635                 640

tca agc cag gat gag gac tgg gta agg aat gag cta gta aag aat tta    1968
Ser Ser Gln Asp Glu Asp Trp Val Arg Asn Glu Leu Val Lys Asn Leu
                645                 650                 655

gaa gaa ggg gtg cct cca ttt cag ctc tgc ctt cac tac aga gac ttt    2016
Glu Glu Gly Val Pro Pro Phe Gln Leu Cys Leu His Tyr Arg Asp Phe
            660                 665                 670

att ccc ggt gtg gcc att gct gcc aac atc atc cat gaa ggt ttc cat    2064
Ile Pro Gly Val Ala Ile Ala Ala Asn Ile Ile His Glu Gly Phe His
        675                 680                 685

aaa agc cga aag gtg att gtt gtg gtg tcc cag cac ttc atc cag agc    2112
Lys Ser Arg Lys Val Ile Val Val Val Ser Gln His Phe Ile Gln Ser
    690                 695                 700

cgc tgg tgt atc ttt gaa tat gag att gct cag acc tgg cag ttt ctg    2160
Arg Trp Cys Ile Phe Glu Tyr Glu Ile Ala Gln Thr Trp Gln Phe Leu
705                 710                 715                 720

agc agt cgt gct ggt atc atc ttc att gtc ctg cag aag gtg gag aag    2208
Ser Ser Arg Ala Gly Ile Ile Phe Ile Val Leu Gln Lys Val Glu Lys
                725                 730                 735

acc ctg ctc agg cag cag gtg gag ctg tac cgc ctt ctc agc agg aac    2256
Thr Leu Leu Arg Gln Gln Val Glu Leu Tyr Arg Leu Leu Ser Arg Asn
            740                 745                 750

act tac ctg gag tgg gag gac agt gtc ctg ggg cgg cac atc ttc tgg    2304
Thr Tyr Leu Glu Trp Glu Asp Ser Val Leu Gly Arg His Ile Phe Trp
        755                 760                 765

aga cga ctc aga aaa gcc ctg ctg gat ggt aaa tca tgg aat cca gaa    2352
Arg Arg Leu Arg Lys Ala Leu Leu Asp Gly Lys Ser Trp Asn Pro Glu
    770                 775                 780

gga aca gtg ggt aca gga tgc aat tgg cag gaa gca aca tct atc tga    2400
Gly Thr Val Gly Thr Gly Cys Asn Trp Gln Glu Ala Thr Ser Ile
785                 790                 795

<210> 8
<211> 799
<212> PRT
<213> Unknown

<400> 8
Met Glu Leu Asn Phe Tyr Lys Ile Pro Asp Asn Leu Pro Phe Ser Thr
1               5                   10                  15

Lys Asn Leu Asp Leu Ser Phe Asn Pro Leu Arg His Leu Gly Ser Tyr
            20                  25                  30

Ser Phe Phe Ser Phe Pro Glu Leu Gln Val Leu Asp Leu Ser Arg Cys
        35                  40                  45

Glu Ile Gln Thr Ile Glu Asp Gly Ala Tyr Gln Ser Leu Ser His Leu
    50                  55                  60

Ser Thr Leu Ile Leu Thr Gly Asn Pro Ile Gln Ser Leu Ala Leu Gly
65                  70                  75                  80

Ala Phe Ser Gly Leu Ser Ser Leu Gln Lys Leu Val Ala Val Glu Thr
                85                  90                  95

Asn Leu Ala Ser Leu Glu Asn Phe Pro Ile Gly His Leu Lys Thr Leu
            100                 105                 110

Lys Glu Leu Asn Val Ala His Asn Leu Ile Gln Ser Phe Lys Leu Pro
        115                 120                 125

Glu Tyr Phe Ser Asn Leu Thr Asn Leu Glu His Leu Asp Leu Ser Ser
    130                 135                 140

Asn Lys Ile Gln Ser Ile Tyr Cys Thr Asp Leu Arg Val Leu His Gln
145                 150                 155                 160

Met Pro Leu Leu Asn Leu Ser Leu Asp Leu Ser Leu Asn Pro Met Asn
                165                 170                 175

Phe Ile Gln Pro Gly Ala Phe Lys Glu Ile Arg Leu His Lys Leu Thr
            180                 185                 190

Leu Arg Asn Asn Phe Asp Ser Leu Asn Val Met Lys Thr Cys Ile Gln
        195                 200                 205

Gly Leu Ala Gly Leu Glu Val His Arg Leu Val Leu Gly Glu Phe Arg
    210                 215                 220

Asn Glu Gly Asn Leu Glu Lys Phe Asp Lys Ser Ala Leu Glu Gly Leu
225                 230                 235                 240

Cys Asn Leu Thr Ile Glu Glu Phe Arg Leu Ala Tyr Leu Asp Tyr Tyr

245                 250                 255

Leu Asp Asp Ile Ile Asp Leu Phe Asn Cys Leu Thr Asn Val Ser Ser
            260                 265                 270

Phe Ser Leu Val Ser Val Thr Ile Glu Arg Val Lys Asp Phe Ser Tyr
        275                 280                 285

Asn Phe Gly Trp Gln His Leu Glu Leu Val Asn Cys Lys Phe Gly Gln
    290                 295                 300

Phe Pro Thr Leu Lys Leu Lys Ser Leu Lys Arg Leu Thr Phe Thr Ser
305                 310                 315                 320

Asn Lys Gly Gly Asn Ala Phe Ser Glu Val Asp Leu Pro Ser Leu Glu
                325                 330                 335

Phe Leu Asp Leu Ser Arg Asn Gly Leu Ser Phe Lys Gly Cys Cys Ser
            340                 345                 350

Gln Ser Asp Phe Gly Thr Thr Ser Leu Lys Tyr Leu Asp Leu Ser Phe
        355                 360                 365

Asn Gly Val Ile Thr Met Ser Ser Asn Phe Leu Gly Leu Glu Gln Leu
    370                 375                 380

Glu His Leu Asp Phe Gln His Ser Asn Leu Lys Gln Met Ser Glu Phe
385                 390                 395                 400

Ser Val Phe Leu Ser Leu Arg Asn Leu Ile Tyr Leu Asp Ile Ser His
                405                 410                 415

Thr His Thr Arg Val Ala Phe Asn Gly Ile Phe Asn Gly Leu Ser Ser
            420                 425                 430

Leu Glu Val Leu Lys Met Ala Gly Asn Ser Phe Gln Glu Asn Phe Leu
        435                 440                 445

Pro Asp Ile Phe Thr Glu Leu Arg Asn Leu Thr Phe Leu Asp Leu Ser
    450                 455                 460

Gln Cys Gln Leu Glu Gln Leu Ser Pro Thr Ala Phe Asn Ser Leu Ser
465                 470                 475                 480

Ser Leu Gln Val Leu Asn Met Ser His Asn Asn Phe Phe Ser Leu Asp
                485                 490                 495

Thr Phe Pro Tyr Lys Cys Leu Asn Ser Leu Gln Val Leu Asp Tyr Ser
            500                 505                 510

Leu Asn His Ile Met Thr Ser Lys Lys Gln Glu Leu Gln His Phe Pro
        515                 520                 525

Ser Ser Leu Ala Phe Leu Asn Leu Thr Gln Asn Asp Phe Ala Cys Thr
    530                 535                 540

Cys Glu His Gln Ser Phe Leu Gln Trp Ile Lys Asp Gln Arg Gln Leu
545                 550                 555                 560

Leu Val Glu Val Glu Arg Met Glu Cys Ala Thr Pro Ser Asp Lys Gln
                565                 570                 575

Gly Met Pro Val Leu Ser Leu Asn Ile Thr Cys Gln Met Asn Lys Thr
            580                 585                 590

Ile Ile Gly Val Ser Val Leu Ser Val Leu Val Val Ser Val Val Ala
        595                 600                 605

Val Leu Val Tyr Lys Phe Tyr Phe His Leu Met Leu Leu Ala Gly Cys
    610                 615                 620

Ile Lys Tyr Gly Arg Gly Glu Asn Ile Tyr Asp Ala Phe Val Ile Tyr
625                 630                 635                 640

Ser Ser Gln Asp Glu Asp Trp Val Arg Asn Glu Leu Val Lys Asn Leu
                645                 650                 655

Glu Glu Gly Val Pro Pro Phe Gln Leu Cys Leu His Tyr Arg Asp Phe
            660                 665                 670

Ile Pro Gly Val Ala Ile Ala Ala Asn Ile Ile His Glu Gly Phe His
        675                 680                 685

Lys Ser Arg Lys Val Ile Val Val Val Ser Gln His Phe Ile Gln Ser
    690                 695                 700

Arg Trp Cys Ile Phe Glu Tyr Glu Ile Ala Gln Thr Trp Gln Phe Leu
705                 710                 715                 720

Ser Ser Arg Ala Gly Ile Ile Phe Ile Val Leu Gln Lys Val Glu Lys
                725                 730                 735

Thr Leu Leu Arg Gln Gln Val Glu Leu Tyr Arg Leu Leu Ser Arg Asn
            740                 745                 750

Thr Tyr Leu Glu Trp Glu Asp Ser Val Leu Gly Arg His Ile Phe Trp
        755                 760                 765

Arg Arg Leu Arg Lys Ala Leu Leu Asp Gly Lys Ser Trp Asn Pro Glu
    770                 775                 780

Gly Thr Val Gly Thr Gly Cys Asn Trp Gln Glu Ala Thr Ser Ile
785                 790                 795

<210> 9
<211> 1275
<212> DNA
<213> Unknown

<220>
<223> Description of Unknown Organism:primate; surmised
      Homo sapiens

<220>
<221> CDS

<222> (1)..(1095)

<400> 9
tgt tgg gat gtt ttt gag gga ctt tct cat ctt caa gtt ctg tat ttg 48
Cys Trp Asp Val Phe Glu Gly Leu Ser His Leu Gln Val Leu Tyr Leu
1               5                   10                  15

aat cat aac tat ctt aat tcc ctt cca cca gga gta ttt agc cat ctg 96
Asn His Asn Tyr Leu Asn Ser Leu Pro Pro Gly Val Phe Ser His Leu
            20                  25                  30

act gca tta agg gga cta agc ctc aac tcc aac agg ctg aca gtt ctt 144
Thr Ala Leu Arg Gly Leu Ser Leu Asn Ser Asn Arg Leu Thr Val Leu
        35                  40                  45

tct cac aat gat tta cct gct aat tta gag atc ctg gac ata tcc agg 192
Ser His Asn Asp Leu Pro Ala Asn Leu Glu Ile Leu Asp Ile Ser Arg
    50                  55                  60

aac cag ctc cta gct cct aat cct gat gta ttt gta tca ctt agt gtc 240
Asn Gln Leu Leu Ala Pro Asn Pro Asp Val Phe Val Ser Leu Ser Val
65                  70                  75                  80

ttg gat ata act cat aac aag ttc att tgt gaa tgt gaa ctt agc act 288
Leu Asp Ile Thr His Asn Lys Phe Ile Cys Glu Cys Glu Leu Ser Thr
                85                  90                  95

ttt atc aat tgg ctt aat cac acc aat gtc act ata gct ggg cct cct 336
Phe Ile Asn Trp Leu Asn His Thr Asn Val Thr Ile Ala Gly Pro Pro
            100                 105                 110

gca gac ata tat tgt gtg tac cct gac tcg ttc tct ggg gtt tcc ctc 384
Ala Asp Ile Tyr Cys Val Tyr Pro Asp Ser Phe Ser Gly Val Ser Leu
        115                 120                 125

ttc tct ctt tcc acg gaa ggt tgt gat gaa gag gaa gtc tta aag tcc 432
Phe Ser Leu Ser Thr Glu Gly Cys Asp Glu Glu Glu Val Leu Lys Ser
    130                 135                 140

cta aag ttc tcc ctt ttc att gta tgc act gtc act ctg act ctg ttc 480
Leu Lys Phe Ser Leu Phe Ile Val Cys Thr Val Thr Leu Thr Leu Phe
145                 150                 155                 160

ctc atg acc atc ctc aca gtc aca aag ttc cgg ggc ttc tgt ttt atc 528
Leu Met Thr Ile Leu Thr Val Thr Lys Phe Arg Gly Phe Cys Phe Ile
                165                 170                 175

tgt tat aag aca gcc cag aga ctg gtg ttc aag gac cat ccc cag ggc 576
Cys Tyr Lys Thr Ala Gln Arg Leu Val Phe Lys Asp His Pro Gln Gly
            180                 185                 190

aca gaa cct gat atg tac aaa tat gat gcc tat ttg tgc ttc agc agc 624
Thr Glu Pro Asp Met Tyr Lys Tyr Asp Ala Tyr Leu Cys Phe Ser Ser
        195                 200                 205

aaa gac ttc aca tgg gtg cag aat gct ttg ctc aaa cac ctg gac act 672
Lys Asp Phe Thr Trp Val Gln Asn Ala Leu Leu Lys His Leu Asp Thr
    210                 215                 220

caa tac agt gac caa aac aga ttc aac ctg tgc ttt gaa gaa aga gac 720
Gln Tyr Ser Asp Gln Asn Arg Phe Asn Leu Cys Phe Glu Glu Arg Asp
225                 230                 235                 240

ttt gtc cca gga gaa aac cgc att gcc aat atc cag gat gcc atc tgg 768
Phe Val Pro Gly Glu Asn Arg Ile Ala Asn Ile Gln Asp Ala Ile Trp
                245                 250                 255

aac agt aga aag atc gtt tgt ctt gtg agc aga cac ttc ctt aga gat 816
Asn Ser Arg Lys Ile Val Cys Leu Val Ser Arg His Phe Leu Arg Asp
            260                 265                 270

ggc tgg tgc ctt gaa gcc ttc agt tat gcc cag ggc agg tgc tta tct 864
Gly Trp Cys Leu Glu Ala Phe Ser Tyr Ala Gln Gly Arg Cys Leu Ser
        275                 280                 285

gac ctt aac agt gct ctc atc atg gtg gtg gtt ggg tcc ttg tcc cag 912
Asp Leu Asn Ser Ala Leu Ile Met Val Val Val Gly Ser Leu Ser Gln
    290                 295                 300

tac cag ttg atg aaa cat caa tcc atc aga ggc ttt gta cag aaa cag 960
Tyr Gln Leu Met Lys His Gln Ser Ile Arg Gly Phe Val Gln Lys Gln
305                 310                 315                 320

cag tat ttg agg tgg cct gag gat ctc cag gat gtt ggc tgg ttt ctt 1008
Gln Tyr Leu Arg Trp Pro Glu Asp Leu Gln Asp Val Gly Trp Phe Leu
                325                 330                 335

cat aaa ctc tct caa cag ata cta aag aaa gaa aag gaa aag aag aaa 1056
His Lys Leu Ser Gln Gln Ile Leu Lys Lys Glu Lys Glu Lys Lys Lys
            340                 345                 350

gac aat aac att ccg ttg caa act gta gca acc atc tcc taatcaaagg 1105
Asp Asn Asn Ile Pro Leu Gln Thr Val Ala Thr Ile Ser
        355                 360                 365

agcaatttcc aacttatctc aagccacaaa taactcttca ctttgtattt gcaccaagtt 1165

atcattttgg ggtcctctct ggaggttttt tttttctttt tgctactatg aaaacaacat 1225

aaatctctca attttcgtat caaaaaaaaa aaaaaaaaaa tggcggccgc 1275

<210> 10
<211> 365
<212> PRT
<213> Unknown

<400> 10
Cys Trp Asp Val Phe Glu Gly Leu Ser His Leu Gln Val Leu Tyr Leu
1               5                   10                  15

Asn His Asn Tyr Leu Asn Ser Leu Pro Pro Gly Val Phe Ser His Leu
            20                  25                  30

Thr Ala Leu Arg Gly Leu Ser Leu Asn Ser Asn Arg Leu Thr Val Leu
        35                  40                  45

Ser His Asn Asp Leu Pro Ala Asn Leu Glu Ile Leu Asp Ile Ser Arg
    50                  55                  60

Asn Gln Leu Leu Ala Pro Asn Pro Asp Val Phe Val Ser Leu Ser Val
65                  70                  75                  80

Leu Asp Ile Thr His Asn Lys Phe Ile Cys Glu Cys Glu Leu Ser Thr
                85                  90                  95

Phe Ile Asn Trp Leu Asn His Thr Asn Val Thr Ile Ala Gly Pro Pro
            100                 105                 110

Ala Asp Ile Tyr Cys Val Tyr Pro Asp Ser Phe Ser Gly Val Ser Leu
        115                 120                 125

Phe Ser Leu Ser Thr Glu Gly Cys Asp Glu Glu Glu Val Leu Lys Ser
    130                 135                 140

Leu Lys Phe Ser Leu Phe Ile Val Cys Thr Val Thr Leu Thr Leu Phe
145                 150                 155                 160

Leu Met Thr Ile Leu Thr Val Thr Lys Phe Arg Gly Phe Cys Phe Ile
                165                 170                 175

Cys Tyr Lys Thr Ala Gln Arg Leu Val Phe Lys Asp His Pro Gln Gly
            180                 185                 190

Thr Glu Pro Asp Met Tyr Lys Tyr Asp Ala Tyr Leu Cys Phe Ser Ser
        195                 200                 205

Lys Asp Phe Thr Trp Val Gln Asn Ala Leu Leu Lys His Leu Asp Thr
    210                 215                 220

Gln Tyr Ser Asp Gln Asn Arg Phe Asn Leu Cys Phe Glu Glu Arg Asp
225                 230                 235                 240

Phe Val Pro Gly Glu Asn Arg Ile Ala Asn Ile Gln Asp Ala Ile Trp
                245                 250                 255

Asn Ser Arg Lys Ile Val Cys Leu Val Ser Arg His Phe Leu Arg Asp
            260                 265                 270

Gly Trp Cys Leu Glu Ala Phe Ser Tyr Ala Gln Gly Arg Cys Leu Ser
        275                 280                 285

Asp Leu Asn Ser Ala Leu Ile Met Val Val Val Gly Ser Leu Ser Gln
    290                 295                 300

Tyr Gln Leu Met Lys His Gln Ser Ile Arg Gly Phe Val Gln Lys Gln
305                 310                 315                 320

Gln Tyr Leu Arg Trp Pro Glu Asp Leu Gln Asp Val Gly Trp Phe Leu
                325                 330                 335

His Lys Leu Ser Gln Gln Ile Leu Lys Lys Glu Lys Glu Lys Lys Lys
            340                 345                 350

Asp Asn Asn Ile Pro Leu Gln Thr Val Ala Thr Ile Ser
        355                 360                 365

<210> 11
<211> 3138
<212> DNA
<213> Unknown

<220>
<223> Description of Unknown Organism:primate; surmised
Homo sapiens

<220>
<221> CDS
<222> (1)..(3135)

<220>
<221> mat_peptide
<222> (67)..(3135)

<400> 11
atg tgg aca ctg aag aga cta att ctt atc ctt ttt aac ata atc cta 48
att tcc aaa ctc ctt ggg gct aga tgg ttt cct aaa act ctg ccc tgt 96
gat gtc act ctg gat gtt cca aag aac cat gtg atc gtg gac tgc aca 144
gac aag cat ttg aca gaa att cct gga ggt att ccc acg aac acc acg 192
aac ctc acc ctc acc att aac cac ata cca gac atc tcc cca gcg tcc 240
ttt cac aga ctg gac cat ctg gta gag atc gat ttc aga tgc aac tgt 288
gta cct att cca ctg ggg tca aaa aac aac atg tgc atc aag agg ctg 336
cag att aaa ccc aga agc ttt agt gga ctc act tat tta aaa tcc ctt 384
tac ctg gat gga aac cag cta cta gag ata ccg cag ggc ctc ccg cct 432
agc tta cag ctt ctc agc ctt gag gcc aac aac atc ttt tcc atc aga 480
aaa gag aat cta aca gaa ctg gcc aac ata gaa ata ctc tac ctg ggc 528
caa aac tgt tat tat cga aat cct tgt tat gtt tca tat tca ata gag 576
aaa gat gcc ttc cta aac ttg aca aag tta aaa gtg ctc tcc ctg aaa 624
gat aac aat gtc aca gcc gtc cct act gtt ttg cca tct act tta aca 672
gaa cta tat ctc tac aac aac atg att gca aaa atc caa gaa gat gat 720
ttt aat aac ctc aac caa tta caa att ctt gac cta agt gga aat tgc 768
cct cgt tgt tat aat gcc cca ttt cct tgt gcg ccg tgt aaa aat aat 816
tct ccc cta cag atc cct gta aat gct ttt gat gcg ctg aca gaa tta 864
aaa gtt tta cgt cta cac agt aac tct ctt cag cat gtg ccc cca aga 912
tgg ttt aag aac atc aac aaa ctc cag gaa ctg gat ctg tcc caa aac 960
ttc ttg gcc aaa gaa att ggg gat gct aaa ttt ctg cat ttt ctc ccc 1008
agc ctc atc caa ttg gat ctg tct ttc aat ttt gaa ctt cag gtc tat 1056
cgt gca tct atg aat cta tca caa gca ttt tct tca ctg aaa agc ctg 1104
aaa att ctg cgg atc aga gga tat gtc ttt aaa gag ttg aaa agc ttt 1152
aac ctc tcg cca tta cat aat ctt caa aat ctt gaa gtt ctt gat ctt    1200
ggc act aac ttt ata aaa att gct aac ctc agc atg ttt aaa caa ttt    1248
aaa aga ctg aaa gtc ata gat ctt tca gtg aat aaa ata tca cct tca    1296
gga gat tca agt gaa gtt ggc ttc tgc tca aat gcc aga act tct gta    1344
gaa agt tat gaa ccc cag gtc ctg gaa caa tta cat tat ttc aga tat    1392
gat aag tat gca agg agt tgc aga ttc aaa aac aaa gag gct tct ttc    1440
atg tct gtt aat gaa agc tgc tac aag tat ggg cag acc ttg gat cta    1488
agt aaa aat agt ata ttt ttt gtc aag tcc tct gat ttt cag cat ctt    1536
tct ttc ctc aaa tgc ctg aat ctg tca gga aat ctc att agc caa act    1584
ctt aat ggc agt gaa ttc caa cct tta gca gag ctg aga tat ttg gac    1632
ttc tcc aac aac cgg ctt gat tta ctc cat tca aca gca ttt gaa gag    1680
ctt cac aaa ctg gaa gtt ctg gat ata agc agt aat agc cat tat ttt    1728
caa tca gaa gga att act cat atg cta aac ttt acc aag aac cta aag    1776
gtt ctg cag aaa ctg atg atg aac gac aat gac atc tct tcc tcc acc    1824
agc agg acc atg gag agt gag tct ctt aga act ctg gaa ttc aga gga 1872
aat cac tta gat gtt tta tgg aga gaa ggt gat aac aga tac tta caa 1920
tta ttc aag aat ctg cta aaa tta gag gaa tta gac atc tct aaa aat 1968
tcc cta agt ttc ttg cct tct gga gtt ttt gat ggt atg cct cca aat 2016
cta aag aat ctc tct ttg gcc aaa aat ggg ctc aaa tct ttc agt tgg 2064
aag aaa ctc cag tgt cta aag aac ctg gaa act ttg gac ctc agc cac 2112
aac caa ctg acc act gtc cct gag aga tta tcc aac tgt tcc aga agc 2160
ctc aag aat ctg att ctt aag aat aat caa atc agg agt ctg acg aag 2208
tat ttt cta caa gat gcc ttc cag ttg cga tat ctg gat ctc agc tca 2256
aat aaa atc cag atg atc caa aag acc agc ttc cca gaa aat gtc ctc 2304
aac aat ctg aag atg ttg ctt ttg cat cat aat cgg ttt ctg tgc acc 2352
tgt gat gct gtg tgg ttt gtc tgg tgg gtt aac cat acg gag gtg act 2400
att cct tac ctg gcc aca gat gtg act tgt gtg ggg cca gga gca cac 2448
aag ggc caa agt gtg atc tcc ctg gat ctg tac acc tgt gag tta gat 2496
ctg act aac ctg att ctg ttc tca ctt tcc ata tct gta tct ctc ttt 2544
ctc atg gtg atg atg aca gca agt cac ctc tat ttc tgg gat gtg tgg     2592
tat att tac cat ttc tgt aag gcc aag ata aag ggg tat cag cgt cta     2640
ata tca cca gac tgt tgc tat gat gct ttt att gtg tat gac act aaa     2688
gac cca gct gtg acc gag tgg gtt ttg gct gag ctg gtg gcc aaa ctg     2736
gaa gac cca aga gag aaa cat ttt aat tta tgt ctc gag gaa agg gac     2784
tgg tta cca ggg cag cca gtt ctg gaa aac ctt tcc cag agc ata cag     2832
ctt agc aaa aag aca gtg ttt gtg atg aca gac aag tat gca aag act     2880
gaa aat ttt aag ata gca ttt tac ttg tcc cat cag agg ctc atg gat     2928
gaa aaa gtt gat gtg att atc ttg ata ttt ctt gag aag ccc ttt cag     2976
aag tcc aag ttc ctc cag ctc cgg aaa agg ctc tgt ggg agt tct gtc     3024
ctt gag tgg cca aca aac ccg caa gct cac cca tac ttc tgg cag tgt     3072
cta aag aac gcc ctg gcc aca gac aat cat gtg gcc tat agt cag gtg     3120
ttc aag gaa acg gtc tag                                             3138


<210> 12
<211> 1045
<212> PRT

<213> Unknown

<400> 12

<210> 13
<211> 180
<212> DNA
<213> Unknown

<220>
<223> Description of Unknown Organism:rodent; surmised
      Mus musculus

<220>
<221> CDS
<222> (1)..(177)

<400> 13
ctt gga aaa cct ctt cag aag tct aag ttt ctt cag ctc agg aag aga      48
Leu Gly Lys Pro Leu Gln Lys Ser Lys Phe Leu Gln Leu Arg Lys Arg
  1               5                  10                  15

ctc tgc agg agc tct gtc ctt gag tgg cct gca aat cca cag gct cac      96
Leu Cys Arg Ser Ser Val Leu Glu Trp Pro Ala Asn Pro Gln Ala His
             20                  25                  30

cca tac ttc tgg cag tgc ctg aaa aat gcc ctg acc aca gac aat cat      144
Pro Tyr Phe Trp Gln Cys Leu Lys Asn Ala Leu Thr Thr Asp Asn His
         35                  40                  45

gtg gct tat agt caa atg ttc aag gaa aca gtc tag                      180
Val Ala Tyr Ser Gln Met Phe Lys Glu Thr Val
     50                  55

<210> 14
<211> 59
<212> PRT
<213> Unknown

<400> 14
Leu Gly Lys Pro Leu Gln Lys Ser Lys Phe Leu Gln Leu Arg Lys Arg
1               5                   10                  15

Leu Cys Arg Ser Ser Val Leu Glu Trp Pro Ala Asn Pro Gln Ala His
            20                  25                  30

Pro Tyr Phe Trp Gln Cys Leu Lys Asn Ala Leu Thr Thr Asp Asn His
        35                  40                  45

Val Ala Tyr Ser Gln Met Phe Lys Glu Thr Val
    50                  55


<210> 15
<211> 990
<212> DNA
<213> Unknown

<220>
<223> Description of Unknown Organism:primate; surmised
      Homo sapiens

<220>
<221> CDS
<222> (2)..(988)

<400> 15
g aat tcc aga ctt ata aac ttg aaa aat ctc tat ttg gcc tgg aac tgc 49
  Asn Ser Arg Leu Ile Asn Leu Lys Asn Leu Tyr Leu Ala Trp Asn Cys
    1               5                   10                  15

tat ttt aac aaa gtt tgc gag aaa act aac ata gaa gat gga gta ttt   97
Tyr Phe Asn Lys Val Cys Glu Lys Thr Asn Ile Glu Asp Gly Val Phe
                20                  25                  30

gaa acg ctg aca aat ttg gag ttg cta tca cta tct ttc aat tct ctt   145
Glu Thr Leu Thr Asn Leu Glu Leu Leu Ser Leu Ser Phe Asn Ser Leu
            35                  40                  45

tca cat gtg cca ccc aaa ctg cca agc tcc cta cgc aaa ctt ttt ctg   193
Ser His Val Pro Pro Lys Leu Pro Ser Ser Leu Arg Lys Leu Phe Leu
    50                  55                  60

agc aac acc cag atc aaa tac att agt gaa gaa gat ttc aag gga ttg   241
Ser Asn Thr Gln Ile Lys Tyr Ile Ser Glu Glu Asp Phe Lys Gly Leu
65                  70                  75                  80

ata aat tta aca tta cta gat tta agc ggg aac tgt ccg agg tgc ttc   289
Ile Asn Leu Thr Leu Leu Asp Leu Ser Gly Asn Cys Pro Arg Cys Phe
                85                  90                  95

aat gcc cca ttt cca tgc gtg cct tgt gat ggt ggt gct tca att aat 337
Asn Ala Pro Phe Pro Cys Val Pro Cys Asp Gly Gly Ala Ser Ile Asn
            100                 105                 110

ata gat cgt ttt gct ttt caa aac ttg acc caa ctt cga tac cta aac 385
Ile Asp Arg Phe Ala Phe Gln Asn Leu Thr Gln Leu Arg Tyr Leu Asn
        115                 120                 125

ctc tct agc act tcc ctc agg aag att aat gct gcc tgg ttt aaa aat 433
Leu Ser Ser Thr Ser Leu Arg Lys Ile Asn Ala Ala Trp Phe Lys Asn
    130                 135                 140

atg cct cat ctg aag gtg ctg gat ctt gaa ttc aac tat tta gtg gga 481
Met Pro His Leu Lys Val Leu Asp Leu Glu Phe Asn Tyr Leu Val Gly
145                 150                 155                 160

gaa ata gcc tct ggg gca ttt tta acg atg ctg ccc cgc tta gaa ata 529
Glu Ile Ala Ser Gly Ala Phe Leu Thr Met Leu Pro Arg Leu Glu Ile
                165                 170                 175

ctt gac ttg tct ttt aac tat ata aag ggg agt tat cca cag cat att 577
Leu Asp Leu Ser Phe Asn Tyr Ile Lys Gly Ser Tyr Pro Gln His Ile
            180                 185                 190

aat att tcc aga aac ttc tct aaa ctt ttg tct cta cgg gca ttg cat 625
Asn Ile Ser Arg Asn Phe Ser Lys Leu Leu Ser Leu Arg Ala Leu His
        195                 200                 205

tta aga ggt tat gtg ttc cag gaa ctc aga gaa gat gat ttc cag ccc 673
Leu Arg Gly Tyr Val Phe Gln Glu Leu Arg Glu Asp Asp Phe Gln Pro
    210                 215                 220

ctg atg cag ctt cca aac tta tcg act atc aac ttg ggt att aat ttt 721
Leu Met Gln Leu Pro Asn Leu Ser Thr Ile Asn Leu Gly Ile Asn Phe
225                 230                 235                 240

att aag caa atc gat ttc aaa ctt ttc caa aat ttc tcc aat ctg gaa 769
Ile Lys Gln Ile Asp Phe Lys Leu Phe Gln Asn Phe Ser Asn Leu Glu
                245                 250                 255

att att tac ttg tca gaa aac aga ata tca ccg ttg gta aaa gat acc 817
Ile Ile Tyr Leu Ser Glu Asn Arg Ile Ser Pro Leu Val Lys Asp Thr
            260                 265                 270

cgg cag agt tat gca aat agt tcc tct ttt caa cgt cat atc cgg aaa 865
Arg Gln Ser Tyr Ala Asn Ser Ser Ser Phe Gln Arg His Ile Arg Lys
        275                 280                 285

cga cgc tca aca gat ttt gag ttt gac cca cat tcg aac ttt tat cat 913
Arg Arg Ser Thr Asp Phe Glu Phe Asp Pro His Ser Asn Phe Tyr His
    290                 295                 300

ttc acc cgt cct tta ata aag cca caa tgt gct gct tat gga aaa gcc 961
Phe Thr Arg Pro Leu Ile Lys Pro Gln Cys Ala Ala Tyr Gly Lys Ala
305                 310                 315                 320

tta gat tta agc ctc aac agt att ttc tt 990
Leu Asp Leu Ser Leu Asn Ser Ile Phe

                325

<210> 16
<211> 329
<212> PRT
<213> Unknown

<400> 16
Asn Ser Arg Leu Ile Asn Leu Lys Asn Leu Tyr Leu Ala Trp Asn Cys
1               5                   10                  15

Tyr Phe Asn Lys Val Cys Glu Lys Thr Asn Ile Glu Asp Gly Val Phe
            20                  25                  30

Glu Thr Leu Thr Asn Leu Glu Leu Leu Ser Leu Ser Phe Asn Ser Leu
        35                  40                  45

Ser His Val Pro Pro Lys Leu Pro Ser Ser Leu Arg Lys Leu Phe Leu
    50                  55                  60

Ser Asn Thr Gln Ile Lys Tyr Ile Ser Glu Glu Asp Phe Lys Gly Leu
65                  70                  75                  80

Ile Asn Leu Thr Leu Leu Asp Leu Ser Gly Asn Cys Pro Arg Cys Phe
                85                  90                  95

Asn Ala Pro Phe Pro Cys Val Pro Cys Asp Gly Gly Ala Ser Ile Asn
            100                 105                 110

Ile Asp Arg Phe Ala Phe Gln Asn Leu Thr Gln Leu Arg Tyr Leu Asn
        115                 120                 125

Leu Ser Ser Thr Ser Leu Arg Lys Ile Asn Ala Ala Trp Phe Lys Asn
    130                 135                 140

Met Pro His Leu Lys Val Leu Asp Leu Glu Phe Asn Tyr Leu Val Gly
145                 150                 155                 160

Glu Ile Ala Ser Gly Ala Phe Leu Thr Met Leu Pro Arg Leu Glu Ile
                165                 170                 175

Leu Asp Leu Ser Phe Asn Tyr Ile Lys Gly Ser Tyr Pro Gln His Ile
            180                 185                 190

Asn Ile Ser Arg Asn Phe Ser Lys Leu Leu Ser Leu Arg Ala Leu His
        195                 200                 205

Leu Arg Gly Tyr Val Phe Gln Glu Leu Arg Glu Asp Asp Phe Gln Pro
    210                 215                 220

Leu Met Gln Leu Pro Asn Leu Ser Thr Ile Asn Leu Gly Ile Asn Phe
225                 230                 235                 240

Ile Lys Gln Ile Asp Phe Lys Leu Phe Gln Asn Phe Ser Asn Leu Glu
                245                 250                 255

Ile Ile Tyr Leu Ser Glu Asn Arg Ile Ser Pro Leu Val Lys Asp Thr

260                 265                 270

Arg Gln Ser Tyr Ala Asn Ser Ser Ser Phe Gln Arg His Ile Arg Lys
        275                 280                 285

Arg Arg Ser Thr Asp Phe Glu Phe Asp Pro His Ser Asn Phe Tyr His
    290                 295                 300

Phe Thr Arg Pro Leu Ile Lys Pro Gln Cys Ala Ala Tyr Gly Lys Ala
305                 310                 315                 320

Leu Asp Leu Ser Leu Asn Ser Ile Phe
                325

<210> 17
<211> 1557
<212> DNA
<213> Unknown

<220>
<223> Description of Unknown Organism:primate; surmised
      Homo sapiens

<220>
<221> CDS
<222> (1)..(513)

<220>
<221> misc_feature
<222> (93)..(149)
<223> Xaa translation depends on genetic code

<400> 17
cag tct ctt tcc aca tcc caa act ttc tat gat gct tac att tct tat      48
Gln Ser Leu Ser Thr Ser Gln Thr Phe Tyr Asp Ala Tyr Ile Ser Tyr
  1               5                  10                  15

gac acc aaa gat gcc tct gtt act gac tgg gtg ata aat gag ctg cgc      96
Asp Thr Lys Asp Ala Ser Val Thr Asp Trp Val Ile Asn Glu Leu Arg
             20                  25                  30

tac cac ctt gaa gag agc cga gac aaa aac gtt ctc ctt tgt cta gag     144
Tyr His Leu Glu Glu Ser Arg Asp Lys Asn Val Leu Leu Cys Leu Glu
         35                  40                  45

gag agg gat tgg gac ccg gga ttg gcc atc atc gac aac ctc atg cag     192
Glu Arg Asp Trp Asp Pro Gly Leu Ala Ile Ile Asp Asn Leu Met Gln
     50                  55                  60

agc atc aac caa agc aag aaa aca gta ttt gtt tta acc aaa aaa tat     240
Ser Ile Asn Gln Ser Lys Lys Thr Val Phe Val Leu Thr Lys Lys Tyr
 65                  70                  75                  80

gca aaa agc tgg aac ttt aaa aca gct ttt tac ttg gsc ttg cag agg     288
Ala Lys Ser Trp Asn Phe Lys Thr Ala Phe Tyr Leu Xaa Leu Gln Arg
                 85                  90                  95

cta atg ggt gag aac atg gat gtg att ata ttt atc ctg ctg gag cca 336
Leu Met Gly Glu Asn Met Asp Val Ile Ile Phe Ile Leu Leu Glu Pro
            100                 105                 110

gtg tta cag cat tct ccg tat ttg agg cta cgg cag cgg atc tgt aag 384
Val Leu Gln His Ser Pro Tyr Leu Arg Leu Arg Gln Arg Ile Cys Lys
        115                 120                 125

agc tcc atc ctc cag tgg cct gac aac ccg aag gca gaa agg ttg ttt 432
Ser Ser Ile Leu Gln Trp Pro Asp Asn Pro Lys Ala Glu Arg Leu Phe
    130                 135                 140

tgg caa act ctg wga aat gtg gtc ttg act gaa aat gat tca cgg tat 480
Trp Gln Thr Leu Xaa Asn Val Val Leu Thr Glu Asn Asp Ser Arg Tyr
145                 150                 155                 160

aac aat atg tat gtc gat tcc att aag caa tac taactgacgt taagtcatga 533
Asn Asn Met Tyr Val Asp Ser Ile Lys Gln Tyr
                165                 170

tttcgcgcca taataaagat gcaaaggaat gacatttcng tattagttat ctattgctan 593

ggtaacnaaa ttantcccaa aaancttang tnggtttnaa aacaacnaca ttntgctggn 653

cccacagttt ttgagggtca ggagtccagg cccagcataa ctgggtcttc tgcttcaggg 713

tgtctncaga ggctgcaatg taggtgttca ccagagacat aggcatcact ggggtcacac 773

tncatgtggt tgttttctgg attcaattcc tcctgggcta ttggccaaag gctatactca 833

tgtaagccat gcgagcctat cccacaangg cagcttgctt catcagagct agcaaaaaag 893

agaggttgct agcaagatga agtcacaatc ttttgtaatc gaatcaaaaa agtgatatct 953

catcactttg gccatattct atttgttaga agtaaaccac aggtcccacc agctccatgg 1013

gagtgaccac ctcagtccag ggaaaacagc tgaagaccaa gatggtgagc tctgattgct 1073

tcagttggtc atcaactatt ttcccttgac tgctgtcctg ggatggccgg ctatcttgat 1133

ggatagattg tgaatatcag gaggccaggg atcactgtgg accatcttag cagttgacct 1193

aacacatctt cttttcaata tctaagaact tttgccactg tgactaatgg tcctaatatt 1253

aagctgttgt ttatatttat catatatcta tggctacatg gttatattat gctgtggttg 1313

cgttcggttt tatttacagt tgcttttaca aatatttgct gtaacatttg acttctaagg 1373

tttagatgcc atttaagaac tgagatggat agcttttaaa gcatctttta cttcttacca 1433

tttttaaaa gtatgcagct aaattcgaag cttttggtct atattgttaa ttgccattgc 1493

tgtaaatctt aaaatgaatg aataaaaatg tttcatttta aaaaaaaaaa aaaaaaaaaa 1553

aaaa 1557

<210> 18

<211> 171
<212> PRT
<213> Unknown

<400> 18
Gln Ser Leu Ser Thr Ser Gln Thr Phe Tyr Asp Ala Tyr Ile Ser Tyr
1               5                   10                  15

Asp Thr Lys Asp Ala Ser Val Thr Asp Trp Val Ile Asn Glu Leu Arg
            20                  25                  30

Tyr His Leu Glu Glu Ser Arg Asp Lys Asn Val Leu Leu Cys Leu Glu
        35                  40                  45

Glu Arg Asp Trp Asp Pro Gly Leu Ala Ile Ile Asp Asn Leu Met Gln
    50                  55                  60

Ser Ile Asn Gln Ser Lys Lys Thr Val Phe Val Leu Thr Lys Lys Tyr
65                  70                  75                  80

Ala Lys Ser Trp Asn Phe Lys Thr Ala Phe Tyr Leu Xaa Leu Gln Arg
                85                  90                  95

Leu Met Gly Glu Asn Met Asp Val Ile Ile Phe Ile Leu Leu Glu Pro
            100                 105                 110

Val Leu Gln His Ser Pro Tyr Leu Arg Leu Arg Gln Arg Ile Cys Lys
        115                 120                 125

Ser Ser Ile Leu Gln Trp Pro Asp Asn Pro Lys Ala Glu Arg Leu Phe
    130                 135                 140

Trp Gln Thr Leu Xaa Asn Val Val Leu Thr Glu Asn Asp Ser Arg Tyr
145                 150                 155                 160

Asn Asn Met Tyr Val Asp Ser Ile Lys Gln Tyr
                165                 170


<210> 19
<211> 629
<212> DNA
<213> Unknown

<220>
<223> Description of Unknown Organism:primate; surmised
      Homo sapiens

<220>
<221> CDS
<222> (1)..(486)

<220>
<221> misc_feature
<222> (48)..(75)
<223> Xaa translation depends on genetic code

<400> 19

aat gaa ttg atc ccc aat cta gag aag gaa gat ggt tct atc ttg att 48
Asn Glu Leu Ile Pro Asn Leu Glu Lys Glu Asp Gly Ser Ile Leu Ile
1               5                   10                  15

tgc ctt tat gaa agc tac ttt gac cct ggc aaa agc att agt gaa aat 96
Cys Leu Tyr Glu Ser Tyr Phe Asp Pro Gly Lys Ser Ile Ser Glu Asn
            20                  25                  30

att gta agc ttc att gag aaa agc tat aag tcc atc ttt gtt ttg tcy 144
Ile Val Ser Phe Ile Glu Lys Ser Tyr Lys Ser Ile Phe Val Leu Xaa
        35                  40                  45

ccc aac ttt gtc cag aat gag tgg tgc cat tat gaa ttc tac ttt gcc 192
Pro Asn Phe Val Gln Asn Glu Trp Cys His Tyr Glu Phe Tyr Phe Ala
    50                  55                  60

cac cac aat ctc ttc cat gaa aat tct gat cay ata att ctt atc tta 240
His His Asn Leu Phe His Glu Asn Ser Asp Xaa Ile Ile Leu Ile Leu
65                  70                  75                  80

ctg gaa ccc att cca ttc tat tgc att ccc acc agg tat cat aaa ctg 288
Leu Glu Pro Ile Pro Phe Tyr Cys Ile Pro Thr Arg Tyr His Lys Leu
                85                  90                  95

gaa gct ctc ctg gaa aaa aaa gca tac ttg gaa tgg ccc aag gat agg 336
Glu Ala Leu Leu Glu Lys Lys Ala Tyr Leu Glu Trp Pro Lys Asp Arg
            100                 105                 110

cgt aaa tgt ggg ctt ttc tgg gca aac ctt cga gct gct gtt aat gtt 384
Arg Lys Cys Gly Leu Phe Trp Ala Asn Leu Arg Ala Ala Val Asn Val
        115                 120                 125

aat gta tta gcc acc aga gaa atg tat gaa ctg cag aca ttc aca gag 432
Asn Val Leu Ala Thr Arg Glu Met Tyr Glu Leu Gln Thr Phe Thr Glu
    130                 135                 140

tta aat gaa gag tct cga ggt tct aca atc tct ctg atg aga aca gac 480
Leu Asn Glu Glu Ser Arg Gly Ser Thr Ile Ser Leu Met Arg Thr Asp
145                 150                 155                 160

tgt cta taaaatccca cagtccttgg gaagttgggg accacataca ctgttgggat 536
Cys Leu

gtacattgat acaaccttta tgatggcaat ttgacaatat ttattaaaat aaaaaatggt 596

tattcccttc aaaaaaaaaa aaaaaaaaaa aaa 629

<210> 20
<211> 162
<212> PRT
<213> Unknown

<400> 20
Asn Glu Leu Ile Pro Asn Leu Glu Lys Glu Asp Gly Ser Ile Leu Ile
1               5                   10                  15

Cys Leu Tyr Glu Ser Tyr Phe Asp Pro Gly Lys Ser Ile Ser Glu Asn

20                  25                  30

Ile Val Ser Phe Ile Glu Lys Ser Tyr Lys Ser Ile Phe Val Leu Xaa
        35                  40                  45

Pro Asn Phe Val Gln Asn Glu Trp Cys His Tyr Glu Phe Tyr Phe Ala
    50                  55                  60

His His Asn Leu Phe His Glu Asn Ser Asp Xaa Ile Ile Leu Ile Leu
65                  70                  75                  80

Leu Glu Pro Ile Pro Phe Tyr Cys Ile Pro Thr Arg Tyr His Lys Leu
                85                  90                  95

Glu Ala Leu Leu Glu Lys Lys Ala Tyr Leu Glu Trp Pro Lys Asp Arg
            100                 105                 110

Arg Lys Cys Gly Leu Phe Trp Ala Asn Leu Arg Ala Ala Val Asn Val
        115                 120                 125

Asn Val Leu Ala Thr Arg Glu Met Tyr Glu Leu Gln Thr Phe Thr Glu
    130                 135                 140

Leu Asn Glu Glu Ser Arg Gly Ser Thr Ile Ser Leu Met Arg Thr Asp
145                 150                 155                 160

Cys Leu


<210> 21
<211> 427
<212> DNA
<213> Unknown

<220>
<223> Description of Unknown Organism:primate; surmised
      Homo sapiens

<220>
<221> CDS
<222> (1)..(426)

<400> 21
aag aac tcc aaa gaa aac ctc cag ttt cat gct ttt att tca tat agt      48
Lys Asn Ser Lys Glu Asn Leu Gln Phe His Ala Phe Ile Ser Tyr Ser
  1               5                  10                  15

gaa cat gat tct gcc tgg gtg aaa agt gaa ttg gta cct tac cta gaa      96
Glu His Asp Ser Ala Trp Val Lys Ser Glu Leu Val Pro Tyr Leu Glu
             20                  25                  30

aaa gaa gat ata cag att tgt ctt cat gag aga aac ttt gtc cct ggc      144
Lys Glu Asp Ile Gln Ile Cys Leu His Glu Arg Asn Phe Val Pro Gly
         35                  40                  45

aag agc att gtg gaa aat atc atc aac tgc att gag aag agt tac aag      192
Lys Ser Ile Val Glu Asn Ile Ile Asn Cys Ile Glu Lys Ser Tyr Lys

    50                  55                  60

tcc atc ttt gtt ttg tct ccc aac ttt gtc cag agt gag tgg tgc cat 240
Ser Ile Phe Val Leu Ser Pro Asn Phe Val Gln Ser Glu Trp Cys His
65                  70                  75                  80

tac gaa ctc tat ttt gcc cat cac aat ctc ttt cat gaa gga tct aat 288
Tyr Glu Leu Tyr Phe Ala His His Asn Leu Phe His Glu Gly Ser Asn
                85                  90                  95

aac tta atc ctc atc tta ctg gaa ccc att cca cag aac agc att ccc 336
Asn Leu Ile Leu Ile Leu Leu Glu Pro Ile Pro Gln Asn Ser Ile Pro
            100                 105                 110

aac aag tac cac aag ctg aag gct ctc atg acg cag cgg act tat ttg 384
Asn Lys Tyr His Lys Leu Lys Ala Leu Met Thr Gln Arg Thr Tyr Leu
        115                 120                 125

cag tgg ccc aag gag aaa agc aaa cgt ggg ctc ttt tgg gct a 427
Gln Trp Pro Lys Glu Lys Ser Lys Arg Gly Leu Phe Trp Ala
    130                 135                 140

<210> 22
<211> 142
<212> PRT
<213> Unknown

<400> 22
Lys Asn Ser Lys Glu Asn Leu Gln Phe His Ala Phe Ile Ser Tyr Ser
1               5                   10                  15

Glu His Asp Ser Ala Trp Val Lys Ser Glu Leu Val Pro Tyr Leu Glu
            20                  25                  30

Lys Glu Asp Ile Gln Ile Cys Leu His Glu Arg Asn Phe Val Pro Gly
        35                  40                  45

Lys Ser Ile Val Glu Asn Ile Ile Asn Cys Ile Glu Lys Ser Tyr Lys
    50                  55                  60

Ser Ile Phe Val Leu Ser Pro Asn Phe Val Gln Ser Glu Trp Cys His
65                  70                  75                  80

Tyr Glu Leu Tyr Phe Ala His His Asn Leu Phe His Glu Gly Ser Asn
                85                  90                  95

Asn Leu Ile Leu Ile Leu Leu Glu Pro Ile Pro Gln Asn Ser Ile Pro
            100                 105                 110

Asn Lys Tyr His Lys Leu Lys Ala Leu Met Thr Gln Arg Thr Tyr Leu
        115                 120                 125

Gln Trp Pro Lys Glu Lys Ser Lys Arg Gly Leu Phe Trp Ala
    130                 135                 140

<210> 23

<211> 662
<212> DNA
<213> Unknown

<220>
<223> Description of Unknown Organism:primate; surmised
      Homo sapiens

<220>
<221> CDS
<222> (1)..(627)

<220>
<221> misc_feature
<222> (18)..(136)
<223> Xaa translation depends on genetic code

<400> 23
gct tcc acc tgt gcc tgg cct ggc ttc cct ggc ggg ggc ggc aaa gtg     48
Ala Ser Thr Cys Ala Trp Pro Gly Phe Pro Gly Gly Gly Gly Lys Val
  1               5                  10                  15

ggc gar atg agg atg ccc tgc cct acg atg cct tcg tgg tct tcg aca     96
Gly Xaa Met Arg Met Pro Cys Pro Thr Met Pro Ser Trp Ser Ser Thr
             20                  25                  30

aaa cgc rga gcg cag tgg cag act ggg tgt aca acg agc ttc ggg ggc    144
Lys Arg Xaa Ala Gln Trp Gln Thr Gly Cys Thr Thr Ser Phe Gly Gly
         35                  40                  45

agc tgg agg agt gcc gtg ggc gct ggg cac tcc gcc tgt gcc tgg agg    192
Ser Trp Arg Ser Ala Val Gly Ala Gly His Ser Ala Cys Ala Trp Arg
     50                  55                  60

aac gcg act ggc tgc ctg gca aaa ccc tct ttg aga acc tgt ggg cct    240
Asn Ala Thr Gly Cys Leu Ala Lys Pro Ser Leu Arg Thr Cys Gly Pro
 65                  70                  75                  80

cgg tct atg gca gcc gca aga cgc tgt ttg tgc tgg ccc aca cgg acc    288
Arg Ser Met Ala Ala Ala Arg Arg Cys Leu Cys Trp Pro Thr Arg Thr
                 85                  90                  95

ggg tca gtg gtc tct tgc gcg cca ktt ntc ctg ctg gcc cag cag cgc    336
Gly Ser Val Val Ser Cys Ala Pro Xaa Xaa Leu Leu Ala Gln Gln Arg
            100                 105                 110

ctg ctg gar gac cgc aag gac gtc gtg gtg ctg gtg atc cta ang cct    384
Leu Leu Xaa Asp Arg Lys Asp Val Val Val Leu Val Ile Leu Xaa Pro
        115                 120                 125

gac ggc caa gcc tcc cga cta cnn gat gcg ctg acc agc gcc tct gcc    432
Asp Gly Gln Ala Ser Arg Leu Xaa Asp Ala Leu Thr Ser Ala Ser Ala
    130                 135                 140

gcc aga gtg tcc tcc tct ggc ccc acc agc cca gtg gtc gcg cag ctt    480
Ala Arg Val Ser Ser Ser Gly Pro Thr Ser Pro Val Val Ala Gln Leu
145                 150                 155                 160

ctg agg cca gca tgc atg gcc ctg acc agg gac aac cac cac ttc tat     528
Leu Arg Pro Ala Cys Met Ala Leu Thr Arg Asp Asn His His Phe Tyr
                165                 170                 175

aac cgg aac ttc tgc cag gga acc cac ggc cga ata gcc gtg agc cgg     576
Asn Arg Asn Phe Cys Gln Gly Thr His Gly Arg Ile Ala Val Ser Arg
            180                 185                 190

aat cct gca cgg tgc cac ctc cac aca cac cta aca tat gcc tgc ctg     624
Asn Pro Ala Arg Cys His Leu His Thr His Leu Thr Tyr Ala Cys Leu
        195                 200                 205

atc tgaccaacac atgctcgcca ccctcaccac acacc                          662
Ile

<210> 24
<211> 209
<212> PRT
<213> Unknown

<400> 24
Ala Ser Thr Cys Ala Trp Pro Gly Phe Pro Gly Gly Gly Gly Lys Val
1               5                   10                  15

Gly Xaa Met Arg Met Pro Cys Pro Thr Met Pro Ser Trp Ser Ser Thr
            20                  25                  30

Lys Arg Xaa Ala Gln Trp Gln Thr Gly Cys Thr Thr Ser Phe Gly Gly
        35                  40                  45

Ser Trp Arg Ser Ala Val Gly Ala Gly His Ser Ala Cys Ala Trp Arg
    50                  55                  60

Asn Ala Thr Gly Cys Leu Ala Lys Pro Ser Leu Arg Thr Cys Gly Pro
65                  70                  75                  80

Arg Ser Met Ala Ala Ala Arg Arg Cys Leu Cys Trp Pro Thr Arg Thr
                85                  90                  95

Gly Ser Val Val Ser Cys Ala Pro Xaa Xaa Leu Leu Ala Gln Gln Arg
            100                 105                 110

Leu Leu Xaa Asp Arg Lys Asp Val Val Val Leu Val Ile Leu Xaa Pro
        115                 120                 125

Asp Gly Gln Ala Ser Arg Leu Xaa Asp Ala Leu Thr Ser Ala Ser Ala
    130                 135                 140

Ala Arg Val Ser Ser Ser Gly Pro Thr Ser Pro Val Val Ala Gln Leu
145                 150                 155                 160

Leu Arg Pro Ala Cys Met Ala Leu Thr Arg Asp Asn His His Phe Tyr
                165                 170                 175

Asn Arg Asn Phe Cys Gln Gly Thr His Gly Arg Ile Ala Val Ser Arg
            180                 185                 190

Asn Pro Ala Arg Cys His Leu His Thr His Leu Thr Tyr Ala Cys Leu
        195                 200                 205

Ile

<210> 25
<211> 4865
<212> DNA
<213> Unknown

<220>
<223> Description of Unknown Organism:primate; surmised
Homo sapiens

<220>
<221> CDS
<222> (107)..(2617)

<220>
<221> mat_peptide
<222> (173)..(2617)

<220>
<221> misc_feature
<222> (189)
<223> Xaa translation depends on genetic code

<400> 25
aaaatactcc cttgcctcaa aaactgctcg gtcaaacggt gatagcaaac cacgcattca 60

cagggccact gctgctcaca naascagtga ggatgatgcc aggatg atg tct gcc 115
Met Ser Ala
-20

tcg cgc ctg gct ggg act ctg atc cca gcc atg gcc ttc ctc tcc tgc 163
Ser Arg Leu Ala Gly Thr Leu Ile Pro Ala Met Ala Phe Leu Ser Cys
-15 -10 -5

gtg aga cca gaa agc tgg gag ccc tgc gtg gag gtt cct aat att act 211
Val Arg Pro Glu Ser Trp Glu Pro Cys Val Glu Val Pro Asn Ile Thr
-1 1 5 10

tat caa tgc atg gag ctg aat ttc tac aaa atc ccc gac aac ctc ccc 259
Tyr Gln Cys Met Glu Leu Asn Phe Tyr Lys Ile Pro Asp Asn Leu Pro
15 20 25

ttc tca acc aag aac ctg gac ctg agc ttt aat ccc ctg agg cat tta 307
Phe Ser Thr Lys Asn Leu Asp Leu Ser Phe Asn Pro Leu Arg His Leu
30 35 40 45

ggc agc tat agc ttc ttc agt ttc cca gaa ctg cag gtg ctg gat tta 355
Gly Ser Tyr Ser Phe Phe Ser Phe Pro Glu Leu Gln Val Leu Asp Leu
50 55 60

tcc agg tgt gaa atc cag aca att gaa gat ggg gca tat cag agc cta 403
Ser Arg Cys Glu Ile Gln Thr Ile Glu Asp Gly Ala Tyr Gln Ser Leu

65                  70                  75

agc cac ctc tct acc tta ata ttg aca gga aac ccc atc cag agt tta    451
Ser His Leu Ser Thr Leu Ile Leu Thr Gly Asn Pro Ile Gln Ser Leu
        80                  85                  90

gcc ctg gga gcc ttt tct gga cta tca agt tta cag aag ctg gtg gct    499
Ala Leu Gly Ala Phe Ser Gly Leu Ser Ser Leu Gln Lys Leu Val Ala
    95                  100                 105

gtg gag aca aat cta gca tct cta gag aac ttc ccc att gga cat ctc    547
Val Glu Thr Asn Leu Ala Ser Leu Glu Asn Phe Pro Ile Gly His Leu
110                 115                 120                 125

aaa act ttg aaa gaa ctt aat gtg gct cac aat ctt atc caa tct ttc    595
Lys Thr Leu Lys Glu Leu Asn Val Ala His Asn Leu Ile Gln Ser Phe
                130                 135                 140

aaa tta cct gag tat ttt tct aat ctg acc aat cta gag cac ttg gac    643
Lys Leu Pro Glu Tyr Phe Ser Asn Leu Thr Asn Leu Glu His Leu Asp
            145                 150                 155

ctt tcc agc aac aag att caa agt att tat tgc aca gac ttg cgg gtt    691
Leu Ser Ser Asn Lys Ile Gln Ser Ile Tyr Cys Thr Asp Leu Arg Val
        160                 165                 170

cta cat caa atg ccc cta ctc aat ctc tct tta gac ctg tcc ctg aay    739
Leu His Gln Met Pro Leu Leu Asn Leu Ser Leu Asp Leu Ser Leu Xaa
    175                 180                 185

cct atg aac ttt atc caa cca ggt gca ttt aaa gaa att agg ctt cat    787
Pro Met Asn Phe Ile Gln Pro Gly Ala Phe Lys Glu Ile Arg Leu His
190                 195                 200                 205

aag ctg act tta aga aat aat ttt gat agt tta aat gta atg aaa act    835
Lys Leu Thr Leu Arg Asn Asn Phe Asp Ser Leu Asn Val Met Lys Thr
                210                 215                 220

tgt att caa ggt ctg gct ggt tta gaa gtc cat cgt ttg gtt ctg gga    883
Cys Ile Gln Gly Leu Ala Gly Leu Glu Val His Arg Leu Val Leu Gly
            225                 230                 235

gaa ttt aga aat gaa gga aac ttg gaa aag ttt gac aaa tct gct cta    931
Glu Phe Arg Asn Glu Gly Asn Leu Glu Lys Phe Asp Lys Ser Ala Leu
        240                 245                 250

gag ggc ctg tgc aat ttg acc att gaa gaa ttc cga tta gca tac tta    979
Glu Gly Leu Cys Asn Leu Thr Ile Glu Glu Phe Arg Leu Ala Tyr Leu
    255                 260                 265

gac tac tac ctc gat gat att att gac tta ttt aat tgt ttg aca aat    1027
Asp Tyr Tyr Leu Asp Asp Ile Ile Asp Leu Phe Asn Cys Leu Thr Asn
270                 275                 280                 285

gtt tct tca ttt tcc ctg gtg agt gtg act att gaa agg gta aaa gac    1075
Val Ser Ser Phe Ser Leu Val Ser Val Thr Ile Glu Arg Val Lys Asp
                290                 295                 300

ttt tct tat aat ttc gga tgg caa cat tta gaa tta gtt aac tgt aaa 1123
Phe Ser Tyr Asn Phe Gly Trp Gln His Leu Glu Leu Val Asn Cys Lys
305 310 315

ttt gga cag ttt ccc aca ttg aaa ctc aaa tct ctc aaa agg ctt act 1171
Phe Gly Gln Phe Pro Thr Leu Lys Leu Lys Ser Leu Lys Arg Leu Thr
320 325 330

ttc act tcc aac aaa ggt ggg aat gct ttt tca gaa gtt gat cta cca 1219
Phe Thr Ser Asn Lys Gly Gly Asn Ala Phe Ser Glu Val Asp Leu Pro
335 340 345

agc ctt gag ttt cta gat ctc agt aga aat ggc ttg agt ttc aaa ggt 1267
Ser Leu Glu Phe Leu Asp Leu Ser Arg Asn Gly Leu Ser Phe Lys Gly
350 355 360 365

tgc tgt tct caa agt gat ttt ggg aca acc agc cta aag tat tta gat 1315
Cys Cys Ser Gln Ser Asp Phe Gly Thr Thr Ser Leu Lys Tyr Leu Asp
370 375 380

ctg agc ttc aat ggt gtt att acc atg agt tca aac ttc ttg ggc tta 1363
Leu Ser Phe Asn Gly Val Ile Thr Met Ser Ser Asn Phe Leu Gly Leu
385 390 395

gaa caa cta gaa cat ctg gat ttc cag cat tcc aat ttg aaa caa atg 1411
Glu Gln Leu Glu His Leu Asp Phe Gln His Ser Asn Leu Lys Gln Met
400 405 410

agt gag ttt tca gta ttc cta tca ctc aga aac ctc att tac ctt gac 1459
Ser Glu Phe Ser Val Phe Leu Ser Leu Arg Asn Leu Ile Tyr Leu Asp
415 420 425

att tct cat act cac acc aga gtt gct ttc aat ggc atc ttc aat ggc 1507
Ile Ser His Thr His Thr Arg Val Ala Phe Asn Gly Ile Phe Asn Gly
430 435 440 445

ttg tcc agt ctc gaa gtc ttg aaa atg gct ggc aat tct ttc cag gaa 1555
Leu Ser Ser Leu Glu Val Leu Lys Met Ala Gly Asn Ser Phe Gln Glu
450 455 460

aac ttc ctt cca gat atc ttc aca gag ctg aga aac ttg acc ttc ctg 1603
Asn Phe Leu Pro Asp Ile Phe Thr Glu Leu Arg Asn Leu Thr Phe Leu
465 470 475

gac ctc tct cag tgt caa ctg gag cag ttg tct cca aca gca ttt aac 1651
Asp Leu Ser Gln Cys Gln Leu Glu Gln Leu Ser Pro Thr Ala Phe Asn
480 485 490

tca ctc tcc agt ctt cag gta cta aat atg agc cac aac aac ttc ttt 1699
Ser Leu Ser Ser Leu Gln Val Leu Asn Met Ser His Asn Asn Phe Phe
495 500 505

tca ttg gat acg ttt cct tat aag tgt ctg aac tcc ctc cag gtt ctt 1747
Ser Leu Asp Thr Phe Pro Tyr Lys Cys Leu Asn Ser Leu Gln Val Leu
510 515 520 525

gat tac agt ctc aat cac ata atg act tcc aaa aaa cag gaa cta cag 1795
Asp Tyr Ser Leu Asn His Ile Met Thr Ser Lys Lys Gln Glu Leu Gln

530 535 540

cat ttt cca agt agt cta gct ttc tta aat ctt act cag aat gac ttt 1843
His Phe Pro Ser Ser Leu Ala Phe Leu Asn Leu Thr Gln Asn Asp Phe
545 550 555

gct tgt act tgt gaa cac cag agt ttc ctg caa tgg atc aag gac cag 1891
Ala Cys Thr Cys Glu His Gln Ser Phe Leu Gln Trp Ile Lys Asp Gln
560 565 570

agg cag ctc ttg gtg gaa gtt gaa cga atg gaa tgt gca aca cct tca 1939
Arg Gln Leu Leu Val Glu Val Glu Arg Met Glu Cys Ala Thr Pro Ser
575 580 585

gat aag cag ggc atg cct gtg ctg agt ttg aat atc acc tgt cag atg 1987
Asp Lys Gln Gly Met Pro Val Leu Ser Leu Asn Ile Thr Cys Gln Met
590 595 600 605

aat aag acc atc att ggt gtg tcg gtc ctc agt gtg ctt gta gta tct 2035
Asn Lys Thr Ile Ile Gly Val Ser Val Leu Ser Val Leu Val Val Ser
610 615 620

gtt gta gca gtt ctg gtc tat aag ttc tat ttt cac ctg atg ctt ctt 2083
Val Val Ala Val Leu Val Tyr Lys Phe Tyr Phe His Leu Met Leu Leu
625 630 635

gct ggc tgc ata aag tat ggt aga ggt gaa aac atc tat gat gcc ttt 2131
Ala Gly Cys Ile Lys Tyr Gly Arg Gly Glu Asn Ile Tyr Asp Ala Phe
640 645 650

gtt atc tac tca agc cag gat gag gac tgg gta agg aat gag cta gta 2179
Val Ile Tyr Ser Ser Gln Asp Glu Asp Trp Val Arg Asn Glu Leu Val
655 660 665

aag aat tta gaa gaa ggg gtg cct cca ttt cag ctc tgc ctt cac tac 2227
Lys Asn Leu Glu Glu Gly Val Pro Pro Phe Gln Leu Cys Leu His Tyr
670 675 680 685

aga gac ttt att ccc ggt gtg gcc att gct gcc aac atc atc cat gaa 2275
Arg Asp Phe Ile Pro Gly Val Ala Ile Ala Ala Asn Ile Ile His Glu
690 695 700

ggt ttc cat aaa agc cga aag gtg att gtt gtg gtg tcc cag cac ttc 2323
Gly Phe His Lys Ser Arg Lys Val Ile Val Val Val Ser Gln His Phe
705 710 715

atc cag agc cgc tgg tgt atc ttt gaa tat gag att gct cag acc tgg 2371
Ile Gln Ser Arg Trp Cys Ile Phe Glu Tyr Glu Ile Ala Gln Thr Trp
720 725 730

cag ttt ctg agc agt cgt gct ggt atc atc ttc att gtc ctg cag aag 2419
Gln Phe Leu Ser Ser Arg Ala Gly Ile Ile Phe Ile Val Leu Gln Lys
735 740 745

gtg gag aag acc ctg ctc agg cag cag gtg gag ctg tac cgc ctt ctc 2467
Val Glu Lys Thr Leu Leu Arg Gln Gln Val Glu Leu Tyr Arg Leu Leu
750 755 760 765

agc agg aac act tac ctg gag tgg gag gac agt gtc ctg ggg cgg cac    2515
Ser Arg Asn Thr Tyr Leu Glu Trp Glu Asp Ser Val Leu Gly Arg His
                770                 775                 780

atc ttc tgg aga cga ctc aga aaa gcc ctg ctg gat ggt aaa tca tgg    2563
Ile Phe Trp Arg Arg Leu Arg Lys Ala Leu Leu Asp Gly Lys Ser Trp
            785                 790                 795

aat cca gaa gga aca gtg ggt aca gga tgc aat tgg cag gaa gca aca    2611
Asn Pro Glu Gly Thr Val Gly Thr Gly Cys Asn Trp Gln Glu Ala Thr
        800                 805                 810

tct atc tgaagaggaa aaataaaaac ctcctgaggc atttcttgcc cagctgggtc    2667
Ser Ile
    815

caacacttgt tcagttaata agtattaaat gctgccacat gtcaggcctt atgctaaggg 2727

tgagtaattc catggtgcac tagatatgca gggctgctaa tctcaaggag cttccagtgc 2787

agagggaata aatgctagac taaaatacag agtcttccag gtgggcattt caaccaactc 2847

agtcaaggaa cccatgacaa agaaagtcat ttcaactctt acctcatcaa gttgaataaa 2907

gacagagaaa acagaaagag acattgttct tttcctgagt cttttgaatg gaaattgtat 2967

tatgttatag ccatcataaa accattttgg tagttttgac tgaactgggt gttcactttt 3027

tcctttttga ttgaatacaa tttaaattct acttgatgac tgcagtcgtc aaggggctcc 3087

tgatgcaaga tgccccttcc atttaagtc tgtctcctta cagakgttaa agtctantgg 3147

ctaattccta aggaaacctg attaacacat gctcacaacc atcctggtca ttctcganca 3207

tgttctattt tttaactaat cacccctgat atatttttat ttttatatat ccagttttca 3267

tttttttacg tcttgcctat aagctaatat cataaataag gttgtttaag acgtgcttca 3327

aatatccata ttaaccacta tttttcaagg aagtatggaa aagtacactc tgtcactttg 3387

tcactcgatg tcattccaaa gttattgcct actaagtaat gactgtcatg aaagcagcat 3447

tgaaataatt tgtttaaagg gggcactctt ttaaacggga agaaaatttc cgcttcctgg 3507

tcttatcatg gacaatttgg gctakaggca kgaaggaagt gggatkacct caggangtca 3567

ccttttcttg attccagaaa catatgggct gataaacccg gggtgacctc atgaaatgag 3627

ttgcagcaga wgtttatttt tttcagaaca agtgatgttt gatggacctm tgaatctmtt 3687

tagggagaca cagatggctg ggatccctcc cctgtaccct tctcactgmc aggagaacta 3747

cgtgtgaagg tattcaaggc agggagtata cattgctgtt tcctgttggg caatgctcct 3807

tgaccacatt ttgggaagag tggatgttat cattgagaaa acaatgtgtc tggaattaat 3867

ggggttctta taaagaaggt tcccagaaaa gaatgttcat tccagcttct tcaggaaaca 3927

ggaacattca aggaaaagga caatcaggat gtcatcaggg aaatgaaaat aaaaaccaca 3987

atgagatatc accttatacc aggtagatgg ctactataaa aaaatgaagt gtcatcaagg 4047

atatagagaa attggaaccc ttcttcactg ctggagggaa tggaaaatgg tgtagccgtt 4107

atgaaaaaca gtacggaggt ttctcaaaaa ttaaaaatag aactgctata tgatccagca 4167

atctcacttc tgtatatata cccaaaataa ttgaaatcag aatttcaaga aaatatttac 4227

actcccatgt tcattgtggc actcttcaca atcactgttt ccaaagttat ggaaacaacc 4287

caaatttcca ttggaaaata aatggacaaa ggaaatgtgc atataacgta caatggggat 4347

attattcagc ctaaaaaaag gggggatcct gttatttatg acaacatgaa taaacccgga 4407

ggccattatg ctatgtaaaa tgagcaagta acagaaagac aaatactgcc tgatttcatt 4467

tatatgaggt tctaaaatag tcaaactcat agaagcagag aatagaacag tggttcctag 4527

ggaaaaggag gaagggagaa atgaggaaat agggagttgt ctaattggta taaaattata 4587

gtatgcaaga tgaattagct ctaaagatca gctgtatagc agagttcgta taatgaacaa 4647

tactgtatta tgcacttaac attttgttaa gagggtacct ctcatgttaa gtgttcttac 4707

catatacata tacacaagga agcttttgga ggtgatggat atatttatta ccttgattgt 4767

ggtgatggtt tgacaggtat gtgactatgt ctaaactcat caaattgtat acattaaata 4827

tatgcagttt tataatatca aaaaaaaaaa aaaaaaaa 4865

<210> 26
<211> 837
<212> PRT
<213> Unknown

<400> 26
Met Ser Ala Ser Arg Leu Ala Gly Thr Leu Ile Pro Ala Met Ala Phe
-20 -15 -10

Leu Ser Cys Val Arg Pro Glu Ser Trp Glu Pro Cys Val Glu Val Pro
-5 -1 1 5 10

Asn Ile Thr Tyr Gln Cys Met Glu Leu Asn Phe Tyr Lys Ile Pro Asp
15 20 25

Asn Leu Pro Phe Ser Thr Lys Asn Leu Asp Leu Ser Phe Asn Pro Leu
30 35 40

Arg His Leu Gly Ser Tyr Ser Phe Phe Ser Phe Pro Glu Leu Gln Val
45 50 55

Leu Asp Leu Ser Arg Cys Glu Ile Gln Thr Ile Glu Asp Gly Ala Tyr
60 65 70

Gln Ser Leu Ser His Leu Ser Thr Leu Ile Leu Thr Gly Asn Pro Ile

75                    80                    85                    90

Gln Ser Leu Ala Leu Gly Ala Phe Ser Gly Leu Ser Ser Leu Gln Lys
                95                  100                 105

Leu Val Ala Val Glu Thr Asn Leu Ala Ser Leu Glu Asn Phe Pro Ile
            110                 115                 120

Gly His Leu Lys Thr Leu Lys Glu Leu Asn Val Ala His Asn Leu Ile
        125                 130                 135

Gln Ser Phe Lys Leu Pro Glu Tyr Phe Ser Asn Leu Thr Asn Leu Glu
    140                 145                 150

His Leu Asp Leu Ser Ser Asn Lys Ile Gln Ser Ile Tyr Cys Thr Asp
155                 160                 165                 170

Leu Arg Val Leu His Gln Met Pro Leu Leu Asn Leu Ser Leu Asp Leu
                175                 180                 185

Ser Leu Xaa Pro Met Asn Phe Ile Gln Pro Gly Ala Phe Lys Glu Ile
            190                 195                 200

Arg Leu His Lys Leu Thr Leu Arg Asn Asn Phe Asp Ser Leu Asn Val
        205                 210                 215

Met Lys Thr Cys Ile Gln Gly Leu Ala Gly Leu Glu Val His Arg Leu
    220                 225                 230

Val Leu Gly Glu Phe Arg Asn Glu Gly Asn Leu Glu Lys Phe Asp Lys
235                 240                 245                 250

Ser Ala Leu Glu Gly Leu Cys Asn Leu Thr Ile Glu Glu Phe Arg Leu
                255                 260                 265

Ala Tyr Leu Asp Tyr Tyr Leu Asp Asp Ile Ile Asp Leu Phe Asn Cys
            270                 275                 280

Leu Thr Asn Val Ser Ser Phe Ser Leu Val Ser Val Thr Ile Glu Arg
        285                 290                 295

Val Lys Asp Phe Ser Tyr Asn Phe Gly Trp Gln His Leu Glu Leu Val
    300                 305                 310

Asn Cys Lys Phe Gly Gln Phe Pro Thr Leu Lys Leu Lys Ser Leu Lys
315                 320                 325                 330

Arg Leu Thr Phe Thr Ser Asn Lys Gly Gly Asn Ala Phe Ser Glu Val
                335                 340                 345

Asp Leu Pro Ser Leu Glu Phe Leu Asp Leu Ser Arg Asn Gly Leu Ser
            350                 355                 360

Phe Lys Gly Cys Cys Ser Gln Ser Asp Phe Gly Thr Thr Ser Leu Lys
        365                 370                 375

Tyr Leu Asp Leu Ser Phe Asn Gly Val Ile Thr Met Ser Ser Asn Phe
    380                 385                 390

Leu Gly Leu Glu Gln Leu Glu His Leu Asp Phe Gln His Ser Asn Leu
395                 400                 405                 410

Lys Gln Met Ser Glu Phe Ser Val Phe Leu Ser Leu Arg Asn Leu Ile
                415                 420                 425

Tyr Leu Asp Ile Ser His Thr His Thr Arg Val Ala Phe Asn Gly Ile
            430                 435                 440

Phe Asn Gly Leu Ser Ser Leu Glu Val Leu Lys Met Ala Gly Asn Ser
        445                 450                 455

Phe Gln Glu Asn Phe Leu Pro Asp Ile Phe Thr Glu Leu Arg Asn Leu
    460                 465                 470

Thr Phe Leu Asp Leu Ser Gln Cys Gln Leu Glu Gln Leu Ser Pro Thr
475                 480                 485                 490

Ala Phe Asn Ser Leu Ser Ser Leu Gln Val Leu Asn Met Ser His Asn
                495                 500                 505

Asn Phe Phe Ser Leu Asp Thr Phe Pro Tyr Lys Cys Leu Asn Ser Leu
            510                 515                 520

Gln Val Leu Asp Tyr Ser Leu Asn His Ile Met Thr Ser Lys Lys Gln
        525                 530                 535

Glu Leu Gln His Phe Pro Ser Ser Leu Ala Phe Leu Asn Leu Thr Gln
    540                 545                 550

Asn Asp Phe Ala Cys Thr Cys Glu His Gln Ser Phe Leu Gln Trp Ile
555                 560                 565                 570

Lys Asp Gln Arg Gln Leu Leu Val Glu Val Glu Arg Met Glu Cys Ala
                575                 580                 585

Thr Pro Ser Asp Lys Gln Gly Met Pro Val Leu Ser Leu Asn Ile Thr
            590                 595                 600

Cys Gln Met Asn Lys Thr Ile Ile Gly Val Ser Val Leu Ser Val Leu
        605                 610                 615

Val Val Ser Val Val Ala Val Leu Val Tyr Lys Phe Tyr Phe His Leu
    620                 625                 630

Met Leu Leu Ala Gly Cys Ile Lys Tyr Gly Arg Gly Glu Asn Ile Tyr
635                 640                 645                 650

Asp Ala Phe Val Ile Tyr Ser Ser Gln Asp Glu Asp Trp Val Arg Asn
                655                 660                 665

Glu Leu Val Lys Asn Leu Glu Glu Gly Val Pro Pro Phe Gln Leu Cys
            670                 675                 680

Leu His Tyr Arg Asp Phe Ile Pro Gly Val Ala Ile Ala Ala Asn Ile
        685                 690                 695

Ile His Glu Gly Phe His Lys Ser Arg Lys Val Ile Val Val Val Ser
    700                 705                 710

Gln His Phe Ile Gln Ser Arg Trp Cys Ile Phe Glu Tyr Glu Ile Ala
715                 720                 725                 730

Gln Thr Trp Gln Phe Leu Ser Ser Arg Ala Gly Ile Ile Phe Ile Val
                735                 740                 745

Leu Gln Lys Val Glu Lys Thr Leu Leu Arg Gln Gln Val Glu Leu Tyr
            750                 755                 760

Arg Leu Leu Ser Arg Asn Thr Tyr Leu Glu Trp Glu Asp Ser Val Leu
        765                 770                 775

Gly Arg His Ile Phe Trp Arg Arg Leu Arg Lys Ala Leu Leu Asp Gly
    780                 785                 790

Lys Ser Trp Asn Pro Glu Gly Thr Val Gly Thr Gly Cys Asn Trp Gln
795                 800                 805                 810

Glu Ala Thr Ser Ile
                815

<210> 27
<211> 300
<212> DNA
<213> Unknown

<220>
<223> Description of Unknown Organism:rodent; surmised
      Mus musculus

<220>
<221> CDS
<222> (1)..(300)

<220>
<221> misc_feature
<222> (62)..(100)
<223> Xaa translation depends on genetic code

<400> 27
tcc tat tct atg gaa aaa gat gct ttc cta ttt atg aga aat ttg aag      48
Ser Tyr Ser Met Glu Lys Asp Ala Phe Leu Phe Met Arg Asn Leu Lys
  1               5                  10                  15

gtt ctc tca cta aaa gat aac aat gtc aca gct gtc ccc acc act ttg      96
Val Leu Ser Leu Lys Asp Asn Asn Val Thr Ala Val Pro Thr Thr Leu
             20                  25                  30

cca cct aat tta cta gag ctc tat ctt tat aac aat atc att aag aaa     144
Pro Pro Asn Leu Leu Glu Leu Tyr Leu Tyr Asn Asn Ile Ile Lys Lys
         35                  40                  45

atc caa gaa aat gat ttc aat aac ctc aat gag ttg caa gtn ctt gac     192
Ile Gln Glu Asn Asp Phe Asn Asn Leu Asn Glu Leu Gln Xaa Leu Asp

50                          55                          60

cta ngt gga aat tgc cct cga tgt nat aat gtc cca tat ccg tgt aca     240
Leu Xaa Gly Asn Cys Pro Arg Cys Xaa Asn Val Pro Tyr Pro Cys Thr
 65                      70                      75                  80

ccg tgt gaa aat aat tcc ccc tta cag atc cat gan aat gct ttc aat     288
Pro Cys Glu Asn Asn Ser Pro Leu Gln Ile His Xaa Asn Ala Phe Asn
                     85                      90                      95

tca tcg aca gan                                                     300
Ser Ser Thr Xaa
            100


<210> 28
<211> 100
<212> PRT
<213> Unknown

<400> 28
Ser Tyr Ser Met Glu Lys Asp Ala Phe Leu Phe Met Arg Asn Leu Lys
  1               5                  10                  15

Val Leu Ser Leu Lys Asp Asn Asn Val Thr Ala Val Pro Thr Thr Leu
             20                  25                  30

Pro Pro Asn Leu Leu Glu Leu Tyr Leu Tyr Asn Asn Ile Ile Lys Lys
         35                  40                  45

Ile Gln Glu Asn Asp Phe Asn Asn Leu Asn Glu Leu Gln Xaa Leu Asp
     50                  55                  60

Leu Xaa Gly Asn Cys Pro Arg Cys Xaa Asn Val Pro Tyr Pro Cys Thr
 65                  70                  75                  80

Pro Cys Glu Asn Asn Ser Pro Leu Gln Ile His Xaa Asn Ala Phe Asn
                 85                  90                  95

Ser Ser Thr Xaa
            100


<210> 29
<211> 1756
<212> DNA
<213> Unknown

<220>
<223> Description of Unknown Organism:rodent; surmised
      Mus musculus

<220>
<221> CDS
<222> (1)..(1182)

<400> 29
tct cca gaa att ccc tgg aat tcc ttg cct cct gag gtt ttt gag ggt     48

Ser Pro Glu Ile Pro Trp Asn Ser Leu Pro Pro Glu Val Phe Glu Gly
1               5                   10                  15

atg ccg cca aat cta aag aat ctc tcc ttg gcc aaa aat ggg ctc aaa     96
Met Pro Pro Asn Leu Lys Asn Leu Ser Leu Ala Lys Asn Gly Leu Lys
            20                  25                  30

tct ttc ttt tgg gac aga ctc cag tta ctg aag cat ttg gaa att ttg     144
Ser Phe Phe Trp Asp Arg Leu Gln Leu Leu Lys His Leu Glu Ile Leu
        35                  40                  45

gac ctc agc cat aac cag ctg aca aaa gta cct gag aga ttg gcc aac     192
Asp Leu Ser His Asn Gln Leu Thr Lys Val Pro Glu Arg Leu Ala Asn
    50                  55                  60

tgt tcc aaa agt ctc aca aca ctg att ctt aag cat aat caa atc agg     240
Cys Ser Lys Ser Leu Thr Thr Leu Ile Leu Lys His Asn Gln Ile Arg
65                  70                  75                  80

caa ttg aca aaa tat ttt cta gaa gat gct ttg caa ttg cgc tat cta     288
Gln Leu Thr Lys Tyr Phe Leu Glu Asp Ala Leu Gln Leu Arg Tyr Leu
                85                  90                  95

gac atc agt tca aat aaa atc cag gtc att cag aag act agc ttc cca     336
Asp Ile Ser Ser Asn Lys Ile Gln Val Ile Gln Lys Thr Ser Phe Pro
            100                 105                 110

gaa aat gtc ctc aac aat ctg gag atg ttg gtt tta cat cac aat cgc     384
Glu Asn Val Leu Asn Asn Leu Glu Met Leu Val Leu His His Asn Arg
        115                 120                 125

ttt ctt tgc aac tgt gat gct gtg tgg ttt gtc tgg tgg gtt aac cat     432
Phe Leu Cys Asn Cys Asp Ala Val Trp Phe Val Trp Trp Val Asn His
    130                 135                 140

aca gat gtt act att cca tac ctg gcc act gat gtg act tgt gta ggt     480
Thr Asp Val Thr Ile Pro Tyr Leu Ala Thr Asp Val Thr Cys Val Gly
145                 150                 155                 160

cca gga gca cac aaa ggt caa agt gtc ata tcc ctt gat ctg tat acg     528
Pro Gly Ala His Lys Gly Gln Ser Val Ile Ser Leu Asp Leu Tyr Thr
                165                 170                 175

tgt gag tta gat ctc aca aac ctg att ctg ttc tca gtt tcc ata tca     576
Cys Glu Leu Asp Leu Thr Asn Leu Ile Leu Phe Ser Val Ser Ile Ser
            180                 185                 190

tca gtc ctc ttt ctt atg gta gtt atg aca aca agt cac ctc ttt ttc     624
Ser Val Leu Phe Leu Met Val Val Met Thr Thr Ser His Leu Phe Phe
        195                 200                 205

tgg gat atg tgg tac att tat tat ttt tgg aaa gca aag ata aag ggg     672
Trp Asp Met Trp Tyr Ile Tyr Tyr Phe Trp Lys Ala Lys Ile Lys Gly
    210                 215                 220

tat cca gca tct gca atc cca tgg agt cct tgt tat gat gct ttt att     720
Tyr Pro Ala Ser Ala Ile Pro Trp Ser Pro Cys Tyr Asp Ala Phe Ile
225                 230                 235                 240

gtg tat gac act aaa aac tca gct gtg aca gaa tgg gtt ttg cag gag 768
Val Tyr Asp Thr Lys Asn Ser Ala Val Thr Glu Trp Val Leu Gln Glu
                245                 250                 255

ctg gtg gca aaa ttg gaa gat cca aga gaa aaa cac ttc aat ttg tgt 816
Leu Val Ala Lys Leu Glu Asp Pro Arg Glu Lys His Phe Asn Leu Cys
            260                 265                 270

cta gaa gaa aga gac tgg cta cca gga cag cca gtt cta gaa aac ctt 864
Leu Glu Glu Arg Asp Trp Leu Pro Gly Gln Pro Val Leu Glu Asn Leu
        275                 280                 285

tcc cag agc ata cag ctc agc aaa aag aca gtg ttt gtg atg aca cag 912
Ser Gln Ser Ile Gln Leu Ser Lys Lys Thr Val Phe Val Met Thr Gln
    290                 295                 300

aaa tat gct aag act gag agt ttt aag atg gca ttt tat ttg tct cat 960
Lys Tyr Ala Lys Thr Glu Ser Phe Lys Met Ala Phe Tyr Leu Ser His
305                 310                 315                 320

cag agg ctc ctg gat gaa aaa gtg gat gtg att atc ttg ata ttc ttg 1008
Gln Arg Leu Leu Asp Glu Lys Val Asp Val Ile Ile Leu Ile Phe Leu
                325                 330                 335

gaa aga cct ctt cag aag tct aag ttt ctt cag ctc agg aag aga ctc 1056
Glu Arg Pro Leu Gln Lys Ser Lys Phe Leu Gln Leu Arg Lys Arg Leu
            340                 345                 350

tgc agg agc tct gtc ctt gag tgg cct gca aat cca cag gct cac cca 1104
Cys Arg Ser Ser Val Leu Glu Trp Pro Ala Asn Pro Gln Ala His Pro
        355                 360                 365

tac ttc tgg cag tgc ctg aaa aat gcc ctg acc aca gac aat cat gtg 1152
Tyr Phe Trp Gln Cys Leu Lys Asn Ala Leu Thr Thr Asp Asn His Val
    370                 375                 380

gct tat agt caa atg ttc aag gaa aca gtc tagctctctg aagaatgtca 1202
Ala Tyr Ser Gln Met Phe Lys Glu Thr Val
385                 390

ccacctagga catgccttgg tacctgaagt tttcataaag gtttccataa atgaaggtct 1262

gaatttttcc taacagttgt catggctcag attggtggga aatcatcaat atatggctaa 1322

gaaattaaga aggggagact gatagaagat aatttctttc ttcatgtgcc atgctcagtt 1382

aaatatttcc cctagctcaa atctgaaaaa ctgtgcctag gagacaacac aaggctttga 1442

tttatctgca tacaattgat aagagccaca catctgccct gaagaagtac tagtagtttt 1502

agtagtaggg taaaaattac acaagctttc tctctctctg atactgaact gtaccagagt 1562

tcaatgaaat aaaagcccag agaacttctc agtaaatggt ttcattatca tgtagtatcc 1622

accatgcaat atgccacaaa rccgctactg gtacaggaca gntggtagct gcttcaakgc 1682

ctcttatcat tttcttgggg cccatggagg ggttctytgg gaaadaggga agkttttttt 1742

tggccatcca tgaa                                                    1756

<210> 30
<211> 394
<212> PRT
<213> Unknown

<400> 30
Ser Pro Glu Ile Pro Trp Asn Ser Leu Pro Pro Glu Val Phe Glu Gly
1               5                   10                  15

Met Pro Pro Asn Leu Lys Asn Leu Ser Leu Ala Lys Asn Gly Leu Lys
            20                  25                  30

Ser Phe Phe Trp Asp Arg Leu Gln Leu Leu Lys His Leu Glu Ile Leu
        35                  40                  45

Asp Leu Ser His Asn Gln Leu Thr Lys Val Pro Glu Arg Leu Ala Asn
    50                  55                  60

Cys Ser Lys Ser Leu Thr Thr Leu Ile Leu Lys His Asn Gln Ile Arg
65                  70                  75                  80

Gln Leu Thr Lys Tyr Phe Leu Glu Asp Ala Leu Gln Leu Arg Tyr Leu
                85                  90                  95

Asp Ile Ser Ser Asn Lys Ile Gln Val Ile Gln Lys Thr Ser Phe Pro
            100                 105                 110

Glu Asn Val Leu Asn Asn Leu Glu Met Leu Val Leu His His Asn Arg
        115                 120                 125

Phe Leu Cys Asn Cys Asp Ala Val Trp Phe Val Trp Trp Val Asn His
    130                 135                 140

Thr Asp Val Thr Ile Pro Tyr Leu Ala Thr Asp Val Thr Cys Val Gly
145                 150                 155                 160

Pro Gly Ala His Lys Gly Gln Ser Val Ile Ser Leu Asp Leu Tyr Thr
                165                 170                 175

Cys Glu Leu Asp Leu Thr Asn Leu Ile Leu Phe Ser Val Ser Ile Ser
            180                 185                 190

Ser Val Leu Phe Leu Met Val Val Met Thr Thr Ser His Leu Phe Phe
        195                 200                 205

Trp Asp Met Trp Tyr Ile Tyr Tyr Phe Trp Lys Ala Lys Ile Lys Gly
    210                 215                 220

Tyr Pro Ala Ser Ala Ile Pro Trp Ser Pro Cys Tyr Asp Ala Phe Ile
225                 230                 235                 240

Val Tyr Asp Thr Lys Asn Ser Ala Val Thr Glu Trp Val Leu Gln Glu
                245                 250                 255

Leu Val Ala Lys Leu Glu Asp Pro Arg Glu Lys His Phe Asn Leu Cys
            260                 265                 270

Leu Glu Glu Arg Asp Trp Leu Pro Gly Gln Pro Val Leu Glu Asn Leu
        275                 280                 285

Ser Gln Ser Ile Gln Leu Ser Lys Lys Thr Val Phe Val Met Thr Gln
    290                 295                 300

Lys Tyr Ala Lys Thr Glu Ser Phe Lys Met Ala Phe Tyr Leu Ser His
305                 310                 315                 320

Gln Arg Leu Leu Asp Glu Lys Val Asp Val Ile Ile Leu Ile Phe Leu
                325                 330                 335

Glu Arg Pro Leu Gln Lys Ser Lys Phe Leu Gln Leu Arg Lys Arg Leu
            340                 345                 350

Cys Arg Ser Ser Val Leu Glu Trp Pro Ala Asn Pro Gln Ala His Pro
        355                 360                 365

Tyr Phe Trp Gln Cys Leu Lys Asn Ala Leu Thr Thr Asp Asn His Val
    370                 375                 380

Ala Tyr Ser Gln Met Phe Lys Glu Thr Val
385                 390

<210> 31
<211> 999
<212> DNA
<213> Unknown

<220>
<223> Description of Unknown Organism:primate; surmised
Homo sapiens

<220>
<221> CDS
<222> (2)..(847)

<220>
<221> misc_feature
<222> (1)..(282)
<223> Xaa translation depends on genetic code

<400> 31
c tcn gat gcc aag att cgg cac nag gca tat tca gag gtc atg atg gtt 49
Xaa Asp Ala Lys Ile Arg His Xaa Ala Tyr Ser Glu Val Met Met Val
1               5                   10                  15

gga tgg tca gat tca tac acc tgt gaa tac cct tta aac cta agg gga 97
Gly Trp Ser Asp Ser Tyr Thr Cys Glu Tyr Pro Leu Asn Leu Arg Gly
            20                  25                  30

act agg tta aaa gac gtt cat ctc cac gaa tta tct tgc aac aca gct 145
Thr Arg Leu Lys Asp Val His Leu His Glu Leu Ser Cys Asn Thr Ala
        35                  40                  45

ctg ttg att gtc acc att gtg gtt att atg cta gtt ctg ggg ttg gct 193
Leu Leu Ile Val Thr Ile Val Val Ile Met Leu Val Leu Gly Leu Ala
    50                  55                  60

gtg gcc ttc tgc tgt ctc cac ttt gat ctg ccc tgg tat ctc agg atg 241
Val Ala Phe Cys Cys Leu His Phe Asp Leu Pro Trp Tyr Leu Arg Met
65                  70                  75                  80

cta ggt caa tgc aca caa aca tgg cac agg gtt agg aaa aca acc caa 289
Leu Gly Gln Cys Thr Gln Thr Trp His Arg Val Arg Lys Thr Thr Gln
                85                  90                  95

gaa caa ctc aag aga aat gtc cga ttc cac gca ttt att tca tac agt 337
Glu Gln Leu Lys Arg Asn Val Arg Phe His Ala Phe Ile Ser Tyr Ser
            100                 105                 110

gaa cat gat tct ctg tgg gtg aag aat gaa ttg atc ccc aat cta gag 385
Glu His Asp Ser Leu Trp Val Lys Asn Glu Leu Ile Pro Asn Leu Glu
        115                 120                 125

aag gaa gat ggt tct atc ttg att tgc ctt tat gaa agc tac ttt gac 433
Lys Glu Asp Gly Ser Ile Leu Ile Cys Leu Tyr Glu Ser Tyr Phe Asp
    130                 135                 140

cct ggc aaa agc att agt gaa aat att gta agc ttc att gag aaa agc 481
Pro Gly Lys Ser Ile Ser Glu Asn Ile Val Ser Phe Ile Glu Lys Ser
145                 150                 155                 160

tat aag tcc atc ttt gtt ttg tct ccc aac ttt gtc cag aat gag tgg 529
Tyr Lys Ser Ile Phe Val Leu Ser Pro Asn Phe Val Gln Asn Glu Trp
                165                 170                 175

tgc cat tat gaa ttc tac ttt gcc cac cac aat ctc ttc cat gaa aat 577
Cys His Tyr Glu Phe Tyr Phe Ala His His Asn Leu Phe His Glu Asn
            180                 185                 190

tct gat cac ata att ctt atc tta ctg gaa ccc att cca ttc tat tgc 625
Ser Asp His Ile Ile Leu Ile Leu Leu Glu Pro Ile Pro Phe Tyr Cys
        195                 200                 205

att ccc acc agg tat cat aaa ctg raa gct ctc ctg gaa aaa aaa gca 673
Ile Pro Thr Arg Tyr His Lys Leu Xaa Ala Leu Leu Glu Lys Lys Ala
    210                 215                 220

tac ttg gaa tgg ccc aag gat agg cgt aaa tgt ggg ctt tty tgg gca 721
Tyr Leu Glu Trp Pro Lys Asp Arg Arg Lys Cys Gly Leu Xaa Trp Ala
225                 230                 235                 240

aac ctt cga gct gct gtt aat gtt aat gta tta gcc acc aga gaa atg 769
Asn Leu Arg Ala Ala Val Asn Val Asn Val Leu Ala Thr Arg Glu Met
                245                 250                 255

tat gaa ctg cag aca ttc aca gag tta aat gaa gag tct cga ggt tct 817
Tyr Glu Leu Gln Thr Phe Thr Glu Leu Asn Glu Glu Ser Arg Gly Ser
            260                 265                 270

aca atc tyt ctg atg aga aca gac tgt yta taaaatccca cagtccttgg 867

Thr Ile Xaa Leu Met Arg Thr Asp Cys Xaa
        275                 280

gaagttgggg accacataca ctgttgggat gtacattgat acaaccttta tgatggcaat 927

ttgacaatat ttattaaaat aaaaaatggt tattcccttc aaaaaaaaaa aaaaaaaaaa 987

aaaaaaaaaa aa                                                     999

<210> 32
<211> 282
<212> PRT
<213> Unknown

<400> 32
Xaa Asp Ala Lys Ile Arg His Xaa Ala Tyr Ser Glu Val Met Met Val
1               5                   10                  15

Gly Trp Ser Asp Ser Tyr Thr Cys Glu Tyr Pro Leu Asn Leu Arg Gly
            20                  25                  30

Thr Arg Leu Lys Asp Val His Leu His Glu Leu Ser Cys Asn Thr Ala
        35                  40                  45

Leu Leu Ile Val Thr Ile Val Val Ile Met Leu Val Leu Gly Leu Ala
    50                  55                  60

Val Ala Phe Cys Cys Leu His Phe Asp Leu Pro Trp Tyr Leu Arg Met
65                  70                  75                  80

Leu Gly Gln Cys Thr Gln Thr Trp His Arg Val Arg Lys Thr Thr Gln
                85                  90                  95

Glu Gln Leu Lys Arg Asn Val Arg Phe His Ala Phe Ile Ser Tyr Ser
            100                 105                 110

Glu His Asp Ser Leu Trp Val Lys Asn Glu Leu Ile Pro Asn Leu Glu
        115                 120                 125

Lys Glu Asp Gly Ser Ile Leu Ile Cys Leu Tyr Glu Ser Tyr Phe Asp
    130                 135                 140

Pro Gly Lys Ser Ile Ser Glu Asn Ile Val Ser Phe Ile Glu Lys Ser
145                 150                 155                 160

Tyr Lys Ser Ile Phe Val Leu Ser Pro Asn Phe Val Gln Asn Glu Trp
                165                 170                 175

Cys His Tyr Glu Phe Tyr Phe Ala His His Asn Leu Phe His Glu Asn
            180                 185                 190

Ser Asp His Ile Ile Leu Ile Leu Leu Glu Pro Ile Pro Phe Tyr Cys
        195                 200                 205

Ile Pro Thr Arg Tyr His Lys Leu Xaa Ala Leu Leu Glu Lys Lys Ala
    210                 215                 220

Tyr Leu Glu Trp Pro Lys Asp Arg Arg Lys Cys Gly Leu Xaa Trp Ala
225                 230                 235                 240

Asn Leu Arg Ala Ala Val Asn Val Asn Val Leu Ala Thr Arg Glu Met
                245                 250                 255

Tyr Glu Leu Gln Thr Phe Thr Glu Leu Asn Glu Glu Ser Arg Gly Ser
            260                 265                 270

Thr Ile Xaa Leu Met Arg Thr Asp Cys Xaa
        275                 280

<210> 33
<211> 1173
<212> DNA
<213> Unknown

<220>
<223> Description of Unknown Organism:primate; surmised
      Homo sapiens

<220>
<221> CDS
<222> (1)..(1008)

<220>
<221> misc_feature
<222> (285)
<223> Xaa translation depends on genetic code

<400> 33
ctg cct gct ggc acc cgg ctc cgg agg ctg gat gtc agc tgc aac agc      48
Leu Pro Ala Gly Thr Arg Leu Arg Arg Leu Asp Val Ser Cys Asn Ser
  1               5                  10                  15

atc agc ttc gtg gcc ccc ggc ttc ttt tcc aag gcc aag gag ctg cga      96
Ile Ser Phe Val Ala Pro Gly Phe Phe Ser Lys Ala Lys Glu Leu Arg
             20                  25                  30

gag ctc aac ctt agc gcc aac gcc ctc aag aca gtg gac cac tcc tgg     144
Glu Leu Asn Leu Ser Ala Asn Ala Leu Lys Thr Val Asp His Ser Trp
         35                  40                  45

ttt ggg ccc ctg gcg agt gcc ctg caa ata cta gat gta agc gcc aac     192
Phe Gly Pro Leu Ala Ser Ala Leu Gln Ile Leu Asp Val Ser Ala Asn
     50                  55                  60

cct ctg cac tgc gcc tgt ggg gcg gcc ttt atg gac ttc ctg ctg gag     240
Pro Leu His Cys Ala Cys Gly Ala Ala Phe Met Asp Phe Leu Leu Glu
 65                  70                  75                  80

gtg cag gct gcc gtg ccc ggt ctg ccc agc cgg gtg aag tgt ggc agt     288
Val Gln Ala Ala Val Pro Gly Leu Pro Ser Arg Val Lys Cys Gly Ser
                 85                  90                  95

ccg ggc cag ctc cag ggc ctc agc atc ttt gca cag gac ctg cgc ctc     336
Pro Gly Gln Leu Gln Gly Leu Ser Ile Phe Ala Gln Asp Leu Arg Leu

            100                 105                 110

tgc ctg gat gag gcc ctc tcc tgg gac tgt ttc gcc ctc tcg ctg ctg 384
Cys Leu Asp Glu Ala Leu Ser Trp Asp Cys Phe Ala Leu Ser Leu Leu
        115                 120                 125

gct gtg gct ctg ggc ctg ggt gtg ccc atg ctg cat cac ctc tgt ggc 432
Ala Val Ala Leu Gly Leu Gly Val Pro Met Leu His His Leu Cys Gly
    130                 135                 140

tgg gac ctc tgg tac tgc ttc cac ctg tgc ctg gcc tgg ctt ccc tgg 480
Trp Asp Leu Trp Tyr Cys Phe His Leu Cys Leu Ala Trp Leu Pro Trp
145                 150                 155                 160

cgg ggg cgg caa agt ggg cga gat gag gat gcc ctg ccc tac gat gcc 528
Arg Gly Arg Gln Ser Gly Arg Asp Glu Asp Ala Leu Pro Tyr Asp Ala
                165                 170                 175

ttc gtg gtc ttc gac aaa acg cag agc gca gtg gca gac tgg gtg tac 576
Phe Val Val Phe Asp Lys Thr Gln Ser Ala Val Ala Asp Trp Val Tyr
            180                 185                 190

aac gag ctt cgg ggg cag ctg gag gag tgc cgt ggg cgc tgg gca ctc 624
Asn Glu Leu Arg Gly Gln Leu Glu Glu Cys Arg Gly Arg Trp Ala Leu
        195                 200                 205

cgc ctg tgc ctg gag gaa cgc gac tgg ctg cct ggc aaa acc ctc ttt 672
Arg Leu Cys Leu Glu Glu Arg Asp Trp Leu Pro Gly Lys Thr Leu Phe
    210                 215                 220

gag aac ctg tgg gcc tcg gtc tat ggc agc cgc aag acg ctg ttt gtg 720
Glu Asn Leu Trp Ala Ser Val Tyr Gly Ser Arg Lys Thr Leu Phe Val
225                 230                 235                 240

ctg gcc cac acg gac cgg gtc agt ggt ctc ttg cgc gcc agc ttc ctg 768
Leu Ala His Thr Asp Arg Val Ser Gly Leu Leu Arg Ala Ser Phe Leu
                245                 250                 255

ctg gcc cag cag cgc ctg ctg gag gac cgc aag gac gtc gtg gtg ctg 816
Leu Ala Gln Gln Arg Leu Leu Glu Asp Arg Lys Asp Val Val Val Leu
            260                 265                 270

gtg atc ctg agc cct gac ggc cgc cgc tcc cgc tac gkg cgg ctg cgc 864
Val Ile Leu Ser Pro Asp Gly Arg Arg Ser Arg Tyr Xaa Arg Leu Arg
        275                 280                 285

cag cgc ctc tgc cgc cag agt gtc ctc ctc tgg ccc cac cag ccc agt 912
Gln Arg Leu Cys Arg Gln Ser Val Leu Leu Trp Pro His Gln Pro Ser
    290                 295                 300

ggt cag cgc agc ttc tgg gcc cag ctg ggc atg gcc ctg acc agg gac 960
Gly Gln Arg Ser Phe Trp Ala Gln Leu Gly Met Ala Leu Thr Arg Asp
305                 310                 315                 320

aac cac cac ttc tat aac cgg aac ttc tgc cag gga ccc acg gcc gaa 1008
Asn His His Phe Tyr Asn Arg Asn Phe Cys Gln Gly Pro Thr Ala Glu
                325                 330                 335

tagccgtgag ccggaatcct gcacggtgcc acctccacac tcacctcacc tctgcctgcc 1068

tggtctgacc ctcccctgct cgcctccctc accccacacc tgacacagag caggcactca 1128

ataaatgcta ccgaaggcta aaaaaaaaaa aaaaaaaaaa aanna 1173

<210> 34
<211> 336
<212> PRT
<213> Unknown

<400> 34
Leu Pro Ala Gly Thr Arg Leu Arg Arg Leu Asp Val Ser Cys Asn Ser
1               5                   10                  15

Ile Ser Phe Val Ala Pro Gly Phe Phe Ser Lys Ala Lys Glu Leu Arg
            20                  25                  30

Glu Leu Asn Leu Ser Ala Asn Ala Leu Lys Thr Val Asp His Ser Trp
        35                  40                  45

Phe Gly Pro Leu Ala Ser Ala Leu Gln Ile Leu Asp Val Ser Ala Asn
    50                  55                  60

Pro Leu His Cys Ala Cys Gly Ala Ala Phe Met Asp Phe Leu Leu Glu
65                  70                  75                  80

Val Gln Ala Ala Val Pro Gly Leu Pro Ser Arg Val Lys Cys Gly Ser
                85                  90                  95

Pro Gly Gln Leu Gln Gly Leu Ser Ile Phe Ala Gln Asp Leu Arg Leu
            100                 105                 110

Cys Leu Asp Glu Ala Leu Ser Trp Asp Cys Phe Ala Leu Ser Leu Leu
        115                 120                 125

Ala Val Ala Leu Gly Leu Gly Val Pro Met Leu His His Leu Cys Gly
    130                 135                 140

Trp Asp Leu Trp Tyr Cys Phe His Leu Cys Leu Ala Trp Leu Pro Trp
145                 150                 155                 160

Arg Gly Arg Gln Ser Gly Arg Asp Glu Asp Ala Leu Pro Tyr Asp Ala
                165                 170                 175

Phe Val Val Phe Asp Lys Thr Gln Ser Ala Val Ala Asp Trp Val Tyr
            180                 185                 190

Asn Glu Leu Arg Gly Gln Leu Glu Glu Cys Arg Gly Arg Trp Ala Leu
        195                 200                 205

Arg Leu Cys Leu Glu Glu Arg Asp Trp Leu Pro Gly Lys Thr Leu Phe
    210                 215                 220

Glu Asn Leu Trp Ala Ser Val Tyr Gly Ser Arg Lys Thr Leu Phe Val
225                 230                 235                 240

Leu Ala His Thr Asp Arg Val Ser Gly Leu Leu Arg Ala Ser Phe Leu
                245                 250                 255

Leu Ala Gln Gln Arg Leu Leu Glu Asp Arg Lys Asp Val Val Val Leu
            260                 265                 270

Val Ile Leu Ser Pro Asp Gly Arg Arg Ser Arg Tyr Xaa Arg Leu Arg
        275                 280                 285

Gln Arg Leu Cys Arg Gln Ser Val Leu Leu Trp Pro His Gln Pro Ser
    290                 295                 300

Gly Gln Arg Ser Phe Trp Ala Gln Leu Gly Met Ala Leu Thr Arg Asp
305                 310                 315                 320

Asn His His Phe Tyr Asn Arg Asn Phe Cys Gln Gly Pro Thr Ala Glu
                325                 330                 335

<210> 35
<211> 497
<212> DNA
<213> Unknown

<220>
<223> Description of Unknown Organism:rodent; surmised
Mus musculus

<400> 35
tggcccacac ggaccgcgtc agtggcctcc tgcgcaccag cttcctgctg gctcagcagc 60

gcctgttgga agaccgcaag gacgtggtgg tgttggtgat cctgcgtccg gatgccccac 120

cgtcccgcta tgtgcgactg cgccagcgtc tctgccgcca gagtgtgctc ttctggcccc 180

agcgacccaa cgggcagggg ggcttctggg cccagctgag tacagccctg actagggaca 240

accgccactt ctataaccag aacttctgcc ggggacctac agcagaatag ctcagagcaa 300

cagctggaaa cagctgcatc ttcatgtctg gttcccgagt tgctctgcct gccttgctct 360

gtcttactac accgctattt ggcaagtgcg caatatatgc taccaagcca ccaggcccac 420

ggagcaaagg ttggctgtaa agggtagttt tcttcccatg catctttcag gagagtgaag 480

atagacacca aacccac 497

<210> 36
<211> 3099
<212> DNA
<213> Unknown

<220>
<223> Description of Unknown Organism:primate; surmised
Homo sapiens

<220>

<221> CDS
<222> (1)..(3096)

<220>
<221> mat_peptide
<222> (52)..(3096)

<220>
<221> misc_feature
<222> (725)
<223> Xaa translation depends on genetic code

<400> 36
atg ctg acc tgc att ttc ctg cta ata tct ggt tcc tgt gag tta tgc     48
Met Leu Thr Cys Ile Phe Leu Leu Ile Ser Gly Ser Cys Glu Leu Cys
        -15                 -10                 -5

gcc gaa gaa aat ttt tct aga agc tat cct tgt gat gag aaa aag caa     96
Ala Glu Glu Asn Phe Ser Arg Ser Tyr Pro Cys Asp Glu Lys Lys Gln
 -1   1               5                  10                  15

aat gac tca gtt att gca gag tgc agc aat cgt cga cta cag gaa gtt     144
Asn Asp Ser Val Ile Ala Glu Cys Ser Asn Arg Arg Leu Gln Glu Val
                 20                  25                  30

ccc caa acg gtg ggc aaa tat gtg aca gaa cta gac ctg tct gat aat     192
Pro Gln Thr Val Gly Lys Tyr Val Thr Glu Leu Asp Leu Ser Asp Asn
             35                  40                  45

ttc atc aca cac ata acg aat gaa tca ttt caa ggg ctg caa aat ctc     240
Phe Ile Thr His Ile Thr Asn Glu Ser Phe Gln Gly Leu Gln Asn Leu
         50                  55                  60

act aaa ata aat cta aac cac aac ccc aat gta cag cac cag aac gga     288
Thr Lys Ile Asn Leu Asn His Asn Pro Asn Val Gln His Gln Asn Gly
     65                  70                  75

aat ccc ggt ata caa tca aat ggc ttg aat atc aca gac ggg gca ttc     336
Asn Pro Gly Ile Gln Ser Asn Gly Leu Asn Ile Thr Asp Gly Ala Phe
 80                  85                  90                  95

ctc aac cta aaa aac cta agg gag tta ctg ctt gaa gac aac cag tta     384
Leu Asn Leu Lys Asn Leu Arg Glu Leu Leu Leu Glu Asp Asn Gln Leu
                100                 105                 110

ccc caa ata ccc tct ggt ttg cca gag tct ttg aca gaa ctt agt cta     432
Pro Gln Ile Pro Ser Gly Leu Pro Glu Ser Leu Thr Glu Leu Ser Leu
            115                 120                 125

att caa aac aat ata tac aac ata act aaa gag ggc att tca aga ctt     480
Ile Gln Asn Asn Ile Tyr Asn Ile Thr Lys Glu Gly Ile Ser Arg Leu
        130                 135                 140

ata aac ttg aaa aat ctc tat ttg gcc tgg aac tgc tat ttt aac aaa     528
Ile Asn Leu Lys Asn Leu Tyr Leu Ala Trp Asn Cys Tyr Phe Asn Lys
    145                 150                 155

gtt tgc gag aaa act aac ata gaa gat gga gta ttt gaa acg ctg aca     576

Val Cys Glu Lys Thr Asn Ile Glu Asp Gly Val Phe Glu Thr Leu Thr
160                 165                 170                 175

aat ttg gag ttg cta tca cta tct ttc aat tct ctt tca cat gtg cca     624
Asn Leu Glu Leu Leu Ser Leu Ser Phe Asn Ser Leu Ser His Val Pro
                180                 185                 190

ccc aaa ctg cca agc tcc cta cgc aaa ctt ttt ctg agc aac acc cag     672
Pro Lys Leu Pro Ser Ser Leu Arg Lys Leu Phe Leu Ser Asn Thr Gln
            195                 200                 205

atc aaa tac att agt gaa gaa gat ttc aag gga ttg ata aat tta aca     720
Ile Lys Tyr Ile Ser Glu Glu Asp Phe Lys Gly Leu Ile Asn Leu Thr
        210                 215                 220

tta cta gat tta agc ggg aac tgt ccg agg tgc ttc aat gcc cca ttt     768
Leu Leu Asp Leu Ser Gly Asn Cys Pro Arg Cys Phe Asn Ala Pro Phe
    225                 230                 235

cca tgc gtg cct tgt gat ggt ggt gct tca att aat ata gat cgt ttt     816
Pro Cys Val Pro Cys Asp Gly Gly Ala Ser Ile Asn Ile Asp Arg Phe
240                 245                 250                 255

gct ttt caa aac ttg acc caa ctt cga tac cta aac ctc tct agc act     864
Ala Phe Gln Asn Leu Thr Gln Leu Arg Tyr Leu Asn Leu Ser Ser Thr
                260                 265                 270

tcc ctc agg aag att aat gct gcc tgg ttt aaa aat atg cct cat ctg     912
Ser Leu Arg Lys Ile Asn Ala Ala Trp Phe Lys Asn Met Pro His Leu
            275                 280                 285

aag gtg ctg gat ctt gaa ttc aac tat tta gtg gga gaa ata gcc tct     960
Lys Val Leu Asp Leu Glu Phe Asn Tyr Leu Val Gly Glu Ile Ala Ser
        290                 295                 300

ggg gca ttt tta acg atg ctg ccc cgc tta gaa ata ctt gac ttg tct     1008
Gly Ala Phe Leu Thr Met Leu Pro Arg Leu Glu Ile Leu Asp Leu Ser
    305                 310                 315

ttt aac tat ata aag ggg agt tat cca cag cat att aat att tcc aga     1056
Phe Asn Tyr Ile Lys Gly Ser Tyr Pro Gln His Ile Asn Ile Ser Arg
320                 325                 330                 335

aac ttc tct aaa ctt ttg tct cta cgg gca ttg cat tta aga ggt tat     1104
Asn Phe Ser Lys Leu Leu Ser Leu Arg Ala Leu His Leu Arg Gly Tyr
                340                 345                 350

gtg ttc cag gaa ctc aga gaa gat gat ttc cag ccc ctg atg cag ctt     1152
Val Phe Gln Glu Leu Arg Glu Asp Asp Phe Gln Pro Leu Met Gln Leu
            355                 360                 365

cca aac tta tcg act atc aac ttg ggt att aat ttt att aag caa atc     1200
Pro Asn Leu Ser Thr Ile Asn Leu Gly Ile Asn Phe Ile Lys Gln Ile
        370                 375                 380

gat ttc aaa ctt ttc caa aat ttc tcc aat ctg gaa att att tac ttg     1248
Asp Phe Lys Leu Phe Gln Asn Phe Ser Asn Leu Glu Ile Ile Tyr Leu
    385                 390                 395

tca gaa aac aga ata tca ccg ttg gta aaa gat acc cgg cag agt tat 1296
Ser Glu Asn Arg Ile Ser Pro Leu Val Lys Asp Thr Arg Gln Ser Tyr
400 405 410 415

gca aat agt tcc tct ttt caa cgt cat atc cgg aaa cga cgc tca aca 1344
Ala Asn Ser Ser Ser Phe Gln Arg His Ile Arg Lys Arg Arg Ser Thr
420 425 430

gat ttt gag ttt gac cca cat tcg aac ttt tat cat ttc acc cgt cct 1392
Asp Phe Glu Phe Asp Pro His Ser Asn Phe Tyr His Phe Thr Arg Pro
435 440 445

tta ata aag cca caa tgt gct gct tat gga aaa gcc tta gat tta agc 1440
Leu Ile Lys Pro Gln Cys Ala Ala Tyr Gly Lys Ala Leu Asp Leu Ser
450 455 460

ctc aac agt att ttc ttc att ggg cca aac caa ttt gaa aat ctt cct 1488
Leu Asn Ser Ile Phe Phe Ile Gly Pro Asn Gln Phe Glu Asn Leu Pro
465 470 475

gac att gcc tgt tta aat ctg tct gca aat agc aat gct caa gtg tta 1536
Asp Ile Ala Cys Leu Asn Leu Ser Ala Asn Ser Asn Ala Gln Val Leu
480 485 490 495

agt gga act gaa ttt tca gcc att cct cat gtc aaa tat ttg gat ttg 1584
Ser Gly Thr Glu Phe Ser Ala Ile Prc His Val Lys Tyr Leu Asp Leu
500 505 510

aca aac aat aga cta gac ttt gat aat gct agt gct ctt act gaa ttg 1632
Thr Asn Asn Arg Leu Asp Phe Asp Asn Ala Ser Ala Leu Thr Glu Leu
515 520 525

tcc gac ttg gaa gtt cta gat ctc agc tat aat tca cac tat ttc aga 1680
Ser Asp Leu Glu Val Leu Asp Leu Ser Tyr Asn Ser His Tyr Phe Arg
530 535 540

ata gca ggc gta aca cat cat cta gaa ttt att caa aat ttc aca aat 1728
Ile Ala Gly Val Thr His His Leu Glu Phe Ile Gln Asn Phe Thr Asn
545 550 555

cta aaa gtt tta aac ttg agc cac aac aac att tat act tta aca gat 1776
Leu Lys Val Leu Asn Leu Ser His Asn Asn Ile Tyr Thr Leu Thr Asp
560 565 570 575

aag tat aac ctg gaa agc aag tcc ctg gta gaa tta gtt ttc agt ggc 1824
Lys Tyr Asn Leu Glu Ser Lys Ser Leu Val Glu Leu Val Phe Ser Gly
580 585 590

aat cgc ctt gac att ttg tgg aat gat gat gac aac agg tat atc tcc 1872
Asn Arg Leu Asp Ile Leu Trp Asn Asp Asp Asp Asn Arg Tyr Ile Ser
595 600 605

att ttc aaa ggt ctc aag aat ctg aca cgt ctg gat tta tcc ctt aat 1920
Ile Phe Lys Gly Leu Lys Asn Leu Thr Arg Leu Asp Leu Ser Leu Asn
610 615 620

agg ctc aag cac atc cca aat gaa gca ttc ctt aat ttg cca gcg agt 1968

Arg Leu Lys His Ile Pro Asn Glu Ala Phe Leu Asn Leu Pro Ala Ser
    625                 630                 635

ctc act gaa cta cat ata aat gat aat atg tta aag ttt ttt aac tgg    2016
Leu Thr Glu Leu His Ile Asn Asp Asn Met Leu Lys Phe Phe Asn Trp
640                 645                 650                 655

aca tta ctc cag cag ttt cct cgt ctc gag ttg ctt gac tta cgt gga    2064
Thr Leu Leu Gln Gln Phe Pro Arg Leu Glu Leu Leu Asp Leu Arg Gly
                660                 665                 670

aac aaa cta ctc ttt tta act gat agc cta tct gac ttt aca tct tcc    2112
Asn Lys Leu Leu Phe Leu Thr Asp Ser Leu Ser Asp Phe Thr Ser Ser
            675                 680                 685

ctt cgg aca ctg ctg ctg agt cat aac agg att tcc cac cta ccc tct    2160
Leu Arg Thr Leu Leu Leu Ser His Asn Arg Ile Ser His Leu Pro Ser
        690                 695                 700

ggc ttt ctt tct gaa gtc agt agt ctg aag cac ctc gat tta agt tcc    2208
Gly Phe Leu Ser Glu Val Ser Ser Leu Lys His Leu Asp Leu Ser Ser
    705                 710                 715

aat ctg cta aaa aca atm aac aaa tcc gca ctt gaa act aag acc acc    2256
Asn Leu Leu Lys Thr Xaa Asn Lys Ser Ala Leu Glu Thr Lys Thr Thr
720                 725                 730                 735

acc aaa tta tct atg ttg gaa cta cac gga aac ccc ttt gaa tgc acc    2304
Thr Lys Leu Ser Met Leu Glu Leu His Gly Asn Pro Phe Glu Cys Thr
                740                 745                 750

tgt gac att gga gat ttc cga aga tgg atg gat gaa cat ctg aat gtc    2352
Cys Asp Ile Gly Asp Phe Arg Arg Trp Met Asp Glu His Leu Asn Val
            755                 760                 765

aaa att ccc aga ctg gta gat gtc att tgt gcc agt cct ggg gat caa    2400
Lys Ile Pro Arg Leu Val Asp Val Ile Cys Ala Ser Pro Gly Asp Gln
        770                 775                 780

aga ggg aag agt att gtg agt ctg gag cta aca act tgt gtt tca gat    2448
Arg Gly Lys Ser Ile Val Ser Leu Glu Leu Thr Thr Cys Val Ser Asp
    785                 790                 795

gtc act gca gtg ata tta ttt ttc ttc acg ttc ttt atc acc acc atg    2496
Val Thr Ala Val Ile Leu Phe Phe Phe Thr Phe Phe Ile Thr Thr Met
800                 805                 810                 815

gtt atg ttg gct gcc ctg gct cac cat ttg ttt tac tgg gat gtt tgg    2544
Val Met Leu Ala Ala Leu Ala His His Leu Phe Tyr Trp Asp Val Trp
                820                 825                 830

ttt ata tat aat gtg tgt tta gct aag tta aaa ggc tac agg tct ctt    2592
Phe Ile Tyr Asn Val Cys Leu Ala Lys Leu Lys Gly Tyr Arg Ser Leu
            835                 840                 845

tcc aca tcc caa act ttc tat gat gct tac att tct tat gac acc aaa    2640
Ser Thr Ser Gln Thr Phe Tyr Asp Ala Tyr Ile Ser Tyr Asp Thr Lys
        850                 855                 860

gat gcc tct gtt act gac tgg gtg ata aat gag ctg cgc tac cac ctt 2688
Asp Ala Ser Val Thr Asp Trp Val Ile Asn Glu Leu Arg Tyr His Leu
865 870 875

gaa gag agc cga gac aaa aac gtt ctc ctt tgt cta gag gag agg gat 2736
Glu Glu Ser Arg Asp Lys Asn Val Leu Leu Cys Leu Glu Glu Arg Asp
880 885 890 895

tgg gac ccg gga ttg gcc atc atc gac aac ctc atg cag agc atc aac 2784
Trp Asp Pro Gly Leu Ala Ile Ile Asp Asn Leu Met Gln Ser Ile Asn
900 905 910

caa agc aag aaa aca gta ttt gtt tta acc aaa aaa tat gca aaa agc 2832
Gln Ser Lys Lys Thr Val Phe Val Leu Thr Lys Lys Tyr Ala Lys Ser
915 920 925

tgg aac ttt aaa aca gct ttt tac ttg gcc ttg cag agg cta atg ggt 2880
Trp Asn Phe Lys Thr Ala Phe Tyr Leu Ala Leu Gln Arg Leu Met Gly
930 935 940

gag aac atg gat gtg att ata ttt atc ctg ctg gag cca gtg tta cag 2928
Glu Asn Met Asp Val Ile Ile Phe Ile Leu Leu Glu Pro Val Leu Gln
945 950 955

cat tct ccg tat ttg agg cta cgg cag cgg atc tgt aag agc tcc atc 2976
His Ser Pro Tyr Leu Arg Leu Arg Gln Arg Ile Cys Lys Ser Ser Ile
960 965 970 975

ctc cag tgg cct gac aac ccg aag gca gaa ggc ttg ttt tgg caa act 3024
Leu Gln Trp Pro Asp Asn Pro Lys Ala Glu Gly Leu Phe Trp Gln Thr
980 985 990

ctg aga aat gtg gtc ttg act gaa aat gat tca cgg tat aac aat atg 3072
Leu Arg Asn Val Val Leu Thr Glu Asn Asp Ser Arg Tyr Asn Asn Met
995 1000 1005

tat gtc gat tcc att aag caa tac taa 3099
Tyr Val Asp Ser Ile Lys Gln Tyr
1010 1015

<210> 37
<211> 1032
<212> PRT
<213> Unknown

<400> 37
Met Leu Thr Cys Ile Phe Leu Leu Ile Ser Gly Ser Cys Glu Leu Cys
-15 -10 -5

Ala Glu Glu Asn Phe Ser Arg Ser Tyr Pro Cys Asp Glu Lys Lys Gln
-1 1 5 10 15

Asn Asp Ser Val Ile Ala Glu Cys Ser Asn Arg Arg Leu Gln Glu Val
20 25 30

Pro Gln Thr Val Gly Lys Tyr Val Thr Glu Leu Asp Leu Ser Asp Asn

35                  40                  45

Phe Ile Thr His Ile Thr Asn Glu Ser Phe Gln Gly Leu Gln Asn Leu
        50                  55                  60

Thr Lys Ile Asn Leu Asn His Asn Pro Asn Val Gln His Gln Asn Gly
    65                  70                  75

Asn Pro Gly Ile Gln Ser Asn Gly Leu Asn Ile Thr Asp Gly Ala Phe
80                  85                  90                  95

Leu Asn Leu Lys Asn Leu Arg Glu Leu Leu Leu Glu Asp Asn Gln Leu
                100                 105                 110

Pro Gln Ile Pro Ser Gly Leu Pro Glu Ser Leu Thr Glu Leu Ser Leu
            115                 120                 125

Ile Gln Asn Asn Ile Tyr Asn Ile Thr Lys Glu Gly Ile Ser Arg Leu
        130                 135                 140

Ile Asn Leu Lys Asn Leu Tyr Leu Ala Trp Asn Cys Tyr Phe Asn Lys
    145                 150                 155

Val Cys Glu Lys Thr Asn Ile Glu Asp Gly Val Phe Glu Thr Leu Thr
160                 165                 170                 175

Asn Leu Glu Leu Leu Ser Leu Ser Phe Asn Ser Leu Ser His Val Pro
                180                 185                 190

Pro Lys Leu Pro Ser Ser Leu Arg Lys Leu Phe Leu Ser Asn Thr Gln
            195                 200                 205

Ile Lys Tyr Ile Ser Glu Glu Asp Phe Lys Gly Leu Ile Asn Leu Thr
        210                 215                 220

Leu Leu Asp Leu Ser Gly Asn Cys Pro Arg Cys Phe Asn Ala Pro Phe
    225                 230                 235

Pro Cys Val Pro Cys Asp Gly Gly Ala Ser Ile Asn Ile Asp Arg Phe
240                 245                 250                 255

Ala Phe Gln Asn Leu Thr Gln Leu Arg Tyr Leu Asn Leu Ser Ser Thr
                260                 265                 270

Ser Leu Arg Lys Ile Asn Ala Ala Trp Phe Lys Asn Met Pro His Leu
            275                 280                 285

Lys Val Leu Asp Leu Glu Phe Asn Tyr Leu Val Gly Glu Ile Ala Ser
        290                 295                 300

Gly Ala Phe Leu Thr Met Leu Pro Arg Leu Glu Ile Leu Asp Leu Ser
    305                 310                 315

Phe Asn Tyr Ile Lys Gly Ser Tyr Pro Gln His Ile Asn Ile Ser Arg
320                 325                 330                 335

Asn Phe Ser Lys Leu Leu Ser Leu Arg Ala Leu His Leu Arg Gly Tyr
                340                 345                 350

Val Phe Gln Glu Leu Arg Glu Asp Asp Phe Gln Pro Leu Met Gln Leu
355 360 365

Pro Asn Leu Ser Thr Ile Asn Leu Gly Ile Asn Phe Ile Lys Gln Ile
370 375 380

Asp Phe Lys Leu Phe Gln Asn Phe Ser Asn Leu Glu Ile Ile Tyr Leu
385 390 395

Ser Glu Asn Arg Ile Ser Pro Leu Val Lys Asp Thr Arg Gln Ser Tyr
400 405 410 415

Ala Asn Ser Ser Ser Phe Gln Arg His Ile Arg Lys Arg Arg Ser Thr
420 425 430

Asp Phe Glu Phe Asp Pro His Ser Asn Phe Tyr His Phe Thr Arg Pro
435 440 445

Leu Ile Lys Pro Gln Cys Ala Ala Tyr Gly Lys Ala Leu Asp Leu Ser
450 455 460

Leu Asn Ser Ile Phe Phe Ile Gly Pro Asn Gln Phe Glu Asn Leu Pro
465 470 475

Asp Ile Ala Cys Leu Asn Leu Ser Ala Asn Ser Asn Ala Gln Val Leu
480 485 490 495

Ser Gly Thr Glu Phe Ser Ala Ile Pro His Val Lys Tyr Leu Asp Leu
500 505 510

Thr Asn Asn Arg Leu Asp Phe Asp Asn Ala Ser Ala Leu Thr Glu Leu
515 520 525

Ser Asp Leu Glu Val Leu Asp Leu Ser Tyr Asn Ser His Tyr Phe Arg
530 535 540

Ile Ala Gly Val Thr His His Leu Glu Phe Ile Gln Asn Phe Thr Asn
545 550 555

Leu Lys Val Leu Asn Leu Ser His Asn Asn Ile Tyr Thr Leu Thr Asp
560 565 570 575

Lys Tyr Asn Leu Glu Ser Lys Ser Leu Val Glu Leu Val Phe Ser Gly
580 585 590

Asn Arg Leu Asp Ile Leu Trp Asn Asp Asp Asp Asn Arg Tyr Ile Ser
595 600 605

Ile Phe Lys Gly Leu Lys Asn Leu Thr Arg Leu Asp Leu Ser Leu Asn
610 615 620

Arg Leu Lys His Ile Pro Asn Glu Ala Phe Leu Asn Leu Pro Ala Ser
625 630 635

Leu Thr Glu Leu His Ile Asn Asp Asn Met Leu Lys Phe Phe Asn Trp
640 645 650 655

Thr Leu Leu Gln Gln Phe Pro Arg Leu Glu Leu Leu Asp Leu Arg Gly
                660                 665                 670

Asn Lys Leu Leu Phe Leu Thr Asp Ser Leu Ser Asp Phe Thr Ser Ser
            675                 680                 685

Leu Arg Thr Leu Leu Leu Ser His Asn Arg Ile Ser His Leu Pro Ser
        690                 695                 700

Gly Phe Leu Ser Glu Val Ser Ser Leu Lys His Leu Asp Leu Ser Ser
    705                 710                 715

Asn Leu Leu Lys Thr Xaa Asn Lys Ser Ala Leu Glu Thr Lys Thr Thr
720                 725                 730                 735

Thr Lys Leu Ser Met Leu Glu Leu His Gly Asn Pro Phe Glu Cys Thr
                740                 745                 750

Cys Asp Ile Gly Asp Phe Arg Arg Trp Met Asp Glu His Leu Asn Val
            755                 760                 765

Lys Ile Pro Arg Leu Val Asp Val Ile Cys Ala Ser Pro Gly Asp Gln
        770                 775                 780

Arg Gly Lys Ser Ile Val Ser Leu Glu Leu Thr Thr Cys Val Ser Asp
    785                 790                 795

Val Thr Ala Val Ile Leu Phe Phe Phe Thr Phe Phe Ile Thr Thr Met
800                 805                 810                 815

Val Met Leu Ala Ala Leu Ala His His Leu Phe Tyr Trp Asp Val Trp
                820                 825                 830

Phe Ile Tyr Asn Val Cys Leu Ala Lys Leu Lys Gly Tyr Arg Ser Leu
            835                 840                 845

Ser Thr Ser Gln Thr Phe Tyr Asp Ala Tyr Ile Ser Tyr Asp Thr Lys
        850                 855                 860

Asp Ala Ser Val Thr Asp Trp Val Ile Asn Glu Leu Arg Tyr His Leu
    865                 870                 875

Glu Glu Ser Arg Asp Lys Asn Val Leu Leu Cys Leu Glu Glu Arg Asp
880                 885                 890                 895

Trp Asp Pro Gly Leu Ala Ile Ile Asp Asn Leu Met Gln Ser Ile Asn
                900                 905                 910

Gln Ser Lys Lys Thr Val Phe Val Leu Thr Lys Lys Tyr Ala Lys Ser
            915                 920                 925

Trp Asn Phe Lys Thr Ala Phe Tyr Leu Ala Leu Gln Arg Leu Met Gly
        930                 935                 940

Glu Asn Met Asp Val Ile Ile Phe Ile Leu Leu Glu Pro Val Leu Gln
    945                 950                 955

His Ser Pro Tyr Leu Arg Leu Arg Gln Arg Ile Cys Lys Ser Ser Ile

960                 965                 970                 975

Leu Gln Trp Pro Asp Asn Pro Lys Ala Glu Gly Leu Phe Trp Gln Thr
            980                 985                 990

Leu Arg Asn Val Val Leu Thr Glu Asn Asp Ser Arg Tyr Asn Asn Met
        995                 1000                1005

Tyr Val Asp Ser Ile Lys Gln Tyr
    1010                1015

<210> 38
<211> 3046
<212> DNA
<213> Unknown

<220>
<223> Description of Unknown Organism:primate; surmised
      Homo sapiens

<220>
<221> CDS
<222> (111)..(2543)

<220>
<221> mat_peptide
<222> (168)..(2543)

<400> 38
gaatcatcca cgcacctgca gctctgctga gagagtgcaa gccgtggggg ttttgagctc 60

atcttcatca ttcatatgag gaaataagtg gtaaaatcct tggaaataca atg aga    116
                                                       Met Arg

ctc atc aga aac att tac ata ttt tgt agt att gtt atg aca gca gag    164
Leu Ile Arg Asn Ile Tyr Ile Phe Cys Ser Ile Val Met Thr Ala Glu
    -15                 -10                 -5

ggt gat gct cca gag ctg cca gaa gaa agg gaa ctg atg acc aac tgc    212
Gly Asp Ala Pro Glu Leu Pro Glu Glu Arg Glu Leu Met Thr Asn Cys
-1   1               5                  10                  15

tcc aac atg tct cta aga aag gtt ccc gca gac ttg acc cca gcc aca    260
Ser Asn Met Ser Leu Arg Lys Val Pro Ala Asp Leu Thr Pro Ala Thr
                20                  25                  30

acg aca ctg gat tta tcc tat aac ctc ctt ttt caa ctc cag agt tca    308
Thr Thr Leu Asp Leu Ser Tyr Asn Leu Leu Phe Gln Leu Gln Ser Ser
            35                  40                  45

gat ttt cat tct gtc tcc aaa ctg aga gtt ttg att cta tgc cat aac    356
Asp Phe His Ser Val Ser Lys Leu Arg Val Leu Ile Leu Cys His Asn
        50                  55                  60

aga att caa cag ctg gat ctc aaa acc ttt gaa ttc aac aag gag tta    404
Arg Ile Gln Gln Leu Asp Leu Lys Thr Phe Glu Phe Asn Lys Glu Leu
    65                  70                  75

aga tat tta gat ttg tct aat aac aga ctg aag agt gta act tgg tat 452
Arg Tyr Leu Asp Leu Ser Asn Asn Arg Leu Lys Ser Val Thr Trp Tyr
80 85 90 95

tta ctg gca ggt ctc agg tat tta gat ctt tct ttt aat gac ttt gac 500
Leu Leu Ala Gly Leu Arg Tyr Leu Asp Leu Ser Phe Asn Asp Phe Asp
100 105 110

acc atg cct atc tgt gag gaa gct ggc aac atg tca cac ctg gaa atc 548
Thr Met Pro Ile Cys Glu Glu Ala Gly Asn Met Ser His Leu Glu Ile
115 120 125

cta ggt ttg agt ggg gca aaa ata caa aaa tca gat ttc cag aaa att 596
Leu Gly Leu Ser Gly Ala Lys Ile Gln Lys Ser Asp Phe Gln Lys Ile
130 135 140

gct cat ctg cat cta aat act gtc ttc tta gga ttc aga act ctt cct 644
Ala His Leu His Leu Asn Thr Val Phe Leu Gly Phe Arg Thr Leu Pro
145 150 155

cat tat gaa gaa ggt agc ctg ccc atc tta aac aca aca aaa ctg cac 692
His Tyr Glu Glu Gly Ser Leu Pro Ile Leu Asn Thr Thr Lys Leu His
160 165 170 175

att gtt tta cca atg gac aca aat ttc tgg gtt ctt ttg cgt gat gga 740
Ile Val Leu Pro Met Asp Thr Asn Phe Trp Val Leu Leu Arg Asp Gly
180 185 190

atc aag act tca aaa ata tta gaa atg aca aat ata gat ggc aaa agc 788
Ile Lys Thr Ser Lys Ile Leu Glu Met Thr Asn Ile Asp Gly Lys Ser
195 200 205

caa ttt gta agt tat gaa atg caa cga aat ctt agt tta gaa aat gct 836
Gln Phe Val Ser Tyr Glu Met Gln Arg Asn Leu Ser Leu Glu Asn Ala
210 215 220

aag aca tcg gtt cta ttg ctt aat aaa gtt gat tta ctc tgg gac gac 884
Lys Thr Ser Val Leu Leu Leu Asn Lys Val Asp Leu Leu Trp Asp Asp
225 230 235

ctt ttc ctt atc tta caa ttt gtt tgg cat aca tca gtg gaa cac ttt 932
Leu Phe Leu Ile Leu Gln Phe Val Trp His Thr Ser Val Glu His Phe
240 245 250 255

cag atc cga aat gtg act ttt ggt ggt aag gct tat ctt gac cac aat 980
Gln Ile Arg Asn Val Thr Phe Gly Gly Lys Ala Tyr Leu Asp His Asn
260 265 270

tca ttt gac tac tca aat act gta atg aga act ata aaa ttg gag cat 1028
Ser Phe Asp Tyr Ser Asn Thr Val Met Arg Thr Ile Lys Leu Glu His
275 280 285

gta cat ttc aga gtg ttt tac att caa cag gat aaa atc tat ttg ctt 1076
Val His Phe Arg Val Phe Tyr Ile Gln Gln Asp Lys Ile Tyr Leu Leu
290 295 300

ttg acc aaa atg gac ata gaa aac ctg aca ata tca aat gca caa atg 1124

Leu Thr Lys Met Asp Ile Glu Asn Leu Thr Ile Ser Asn Ala Gln Met
305 310 315

cca cac atg ctt ttc ccg aat tat cct acg aaa ttc caa tat tta aat 1172
Pro His Met Leu Phe Pro Asn Tyr Pro Thr Lys Phe Gln Tyr Leu Asn
320 325 330 335

ttt gcc aat aat atc tta aca gac gag ttg ttt aaa aga act atc caa 1220
Phe Ala Asn Asn Ile Leu Thr Asp Glu Leu Phe Lys Arg Thr Ile Gln
340 345 350

ctg cct cac ttg aaa act ctc att ttg aat ggc aat aaa ctg gag aca 1268
Leu Pro His Leu Lys Thr Leu Ile Leu Asn Gly Asn Lys Leu Glu Thr
355 360 365

ctt tct tta gta agt tgc ttt gct aac aac aca ccc ttg gaa cac ttg 1316
Leu Ser Leu Val Ser Cys Phe Ala Asn Asn Thr Pro Leu Glu His Leu
370 375 380

gat ctg agt caa aat cta tta caa cat aaa aat gat gaa aat tgc tca 1364
Asp Leu Ser Gln Asn Leu Leu Gln His Lys Asn Asp Glu Asn Cys Ser
385 390 395

tgg cca gaa act gtg gtc aat atg aat ctg tca tac aat aaa ttg tct 1412
Trp Pro Glu Thr Val Val Asn Met Asn Leu Ser Tyr Asn Lys Leu Ser
400 405 410 415

gat tct gtc ttc agg tgc ttg ccc aaa agt att caa ata ctt gac cta 1460
Asp Ser Val Phe Arg Cys Leu Pro Lys Ser Ile Gln Ile Leu Asp Leu
420 425 430

aat aat aac caa atc caa act gta cct aaa gag act att cat ctg atg 1508
Asn Asn Asn Gln Ile Gln Thr Val Pro Lys Glu Thr Ile His Leu Met
435 440 445

gcc tta cga gaa cta aat att gca ttt aat ttt cta act gat ctc cct 1556
Ala Leu Arg Glu Leu Asn Ile Ala Phe Asn Phe Leu Thr Asp Leu Pro
450 455 460

gga tgc agt cat ttc agt aga ctt tca gtt ctg aac att gaa atg aac 1604
Gly Cys Ser His Phe Ser Arg Leu Ser Val Leu Asn Ile Glu Met Asn
465 470 475

ttc att ctc agc cca tct ctg gat ttt gtt cag agc tgc cag gaa gtt 1652
Phe Ile Leu Ser Pro Ser Leu Asp Phe Val Gln Ser Cys Gln Glu Val
480 485 490 495

aaa act cta aat gcg gga aga aat cca ttc cgg tgt acc tgt gaa tta 1700
Lys Thr Leu Asn Ala Gly Arg Asn Pro Phe Arg Cys Thr Cys Glu Leu
500 505 510

aaa aat ttc att cag ctt gaa aca tat tca gag gtc atg atg gtt gga 1748
Lys Asn Phe Ile Gln Leu Glu Thr Tyr Ser Glu Val Met Met Val Gly
515 520 525

tgg tca gat tca tac acc tgt gaa tac cct tta aac cta agg gga act 1796
Trp Ser Asp Ser Tyr Thr Cys Glu Tyr Pro Leu Asn Leu Arg Gly Thr
530 535 540

agg tta aaa gac gtt cat ctc cac gaa tta tct tgc aac aca gct ctg 1844
Arg Leu Lys Asp Val His Leu His Glu Leu Ser Cys Asn Thr Ala Leu
545 550 555

ttg att gtc acc att gtg gtt att atg cta gtt ctg ggg ttg gct gtg 1892
Leu Ile Val Thr Ile Val Val Ile Met Leu Val Leu Gly Leu Ala Val
560 565 570 575

gcc ttc tgc tgt ctc cac ttt gat ctg ccc tgg tat ctc agg atg cta 1940
Ala Phe Cys Cys Leu His Phe Asp Leu Pro Trp Tyr Leu Arg Met Leu
580 585 590

ggt caa tgc aca caa aca tgg cac agg gtt agg aaa aca acc caa gaa 1988
Gly Gln Cys Thr Gln Thr Trp His Arg Val Arg Lys Thr Thr Gln Glu
595 600 605

caa ctc aag aga aat gtc cga ttc cac gca ttt att tca tac agt gaa 2036
Gln Leu Lys Arg Asn Val Arg Phe His Ala Phe Ile Ser Tyr Ser Glu
610 615 620

cat gat tct ctg tgg gtg aag aat gaa ttg atc ccc aat cta gag aag 2084
His Asp Ser Leu Trp Val Lys Asn Glu Leu Ile Pro Asn Leu Glu Lys
625 630 635

gaa gat ggt tct atc ttg att tgc ctt tat gaa agc tac ttt gac cct 2132
Glu Asp Gly Ser Ile Leu Ile Cys Leu Tyr Glu Ser Tyr Phe Asp Pro
640 645 650 655

ggc aaa agc att agt gaa aat att gta agc ttc att gag aaa agc tat 2180
Gly Lys Ser Ile Ser Glu Asn Ile Val Ser Phe Ile Glu Lys Ser Tyr
660 665 670

aag tcc atc ttt gtt ttg tct ccc aac ttt gtc cag aat gag tgg tgc 2228
Lys Ser Ile Phe Val Leu Ser Pro Asn Phe Val Gln Asn Glu Trp Cys
675 680 685

cat tat gaa ttc tac ttt gcc cac cac aat ctc ttc cat gaa aat tct 2276
His Tyr Glu Phe Tyr Phe Ala His His Asn Leu Phe His Glu Asn Ser
690 695 700

gat cat ata att ctt atc tta ctg gaa ccc att cca ttc tat tgc att 2324
Asp His Ile Ile Leu Ile Leu Leu Glu Pro Ile Pro Phe Tyr Cys Ile
705 710 715

ccc acc agg tat cat aaa ctg aaa gct ctc ctg gaa aaa aaa gca tac 2372
Pro Thr Arg Tyr His Lys Leu Lys Ala Leu Leu Glu Lys Lys Ala Tyr
720 725 730 735

ttg gaa tgg ccc aag gat agg cgt aaa tgt ggg ctt ttc tgg gca aac 2420
Leu Glu Trp Pro Lys Asp Arg Arg Lys Cys Gly Leu Phe Trp Ala Asn
740 745 750

ctt cga gct gct att aat gtt aat gta tta gcc acc aga gaa atg tat 2468
Leu Arg Ala Ala Ile Asn Val Asn Val Leu Ala Thr Arg Glu Met Tyr
755 760 765

gaa ctg cag aca ttc aca gag tta aat gaa gag tct cga ggt tct aca 2516

Glu Leu Gln Thr Phe Thr Glu Leu Asn Glu Glu Ser Arg Gly Ser Thr
770 775 780

atc tct ctg atg aga aca gat tgt cta taaaatccca cagtccttgg 2563
Ile Ser Leu Met Arg Thr Asp Cys Leu
785 790

gaagttgggg accacataca ctgttgggat gtacattgat acaaccttta tgatggcaat 2623

ttgacaatat ttattaaaat aaaaaatggt tattcccttc atatcagttt ctagaaggat 2683

ttctaagaat gtatcctata gaaacacctt cacaagttta taagggctta tggaaaaagg 2743

tgttcatccc aggattgttt ataatcatga aaaatgtggc caggtgcagt ggctcactct 2803

tgtaatccca gcactatggg aggccaaggt gggtgaccca cgaggtcaag agatggagac 2863

catcctggcc aacatggtga aaccctgtct ctactaaaaa tacaaaaatt agctgggcgt 2923

gatggtgcac gcctgtagtc ccagctactt gggaggctga ggcaggagaa tcgcttgaac 2983

ccgggaggtg gcagttgcag tgagctgaga tcgagccact gcactccagc ctggtgacag 3043

agc 3046

<210> 39
<211> 811
<212> PRT
<213> Unknown

<400> 39
Met Arg Leu Ile Arg Asn Ile Tyr Ile Phe Cys Ser Ile Val Met Thr
-15 -10 -5

Ala Glu Gly Asp Ala Pro Glu Leu Pro Glu Glu Arg Glu Leu Met Thr
-1 1 5 10

Asn Cys Ser Asn Met Ser Leu Arg Lys Val Pro Ala Asp Leu Thr Pro
15 20 25

Ala Thr Thr Thr Leu Asp Leu Ser Tyr Asn Leu Leu Phe Gln Leu Gln
30 35 40 45

Ser Ser Asp Phe His Ser Val Ser Lys Leu Arg Val Leu Ile Leu Cys
50 55 60

His Asn Arg Ile Gln Gln Leu Asp Leu Lys Thr Phe Glu Phe Asn Lys
65 70 75

Glu Leu Arg Tyr Leu Asp Leu Ser Asn Asn Arg Leu Lys Ser Val Thr
80 85 90

Trp Tyr Leu Leu Ala Gly Leu Arg Tyr Leu Asp Leu Ser Phe Asn Asp
95 100 105

Phe Asp Thr Met Pro Ile Cys Glu Glu Ala Gly Asn Met Ser His Leu
110 115 120 125

Glu Ile Leu Gly Leu Ser Gly Ala Lys Ile Gln Lys Ser Asp Phe Gln
                130                 135                 140

Lys Ile Ala His Leu His Leu Asn Thr Val Phe Leu Gly Phe Arg Thr
            145                 150                 155

Leu Pro His Tyr Glu Glu Gly Ser Leu Pro Ile Leu Asn Thr Thr Lys
        160                 165                 170

Leu His Ile Val Leu Pro Met Asp Thr Asn Phe Trp Val Leu Leu Arg
    175                 180                 185

Asp Gly Ile Lys Thr Ser Lys Ile Leu Glu Met Thr Asn Ile Asp Gly
190                 195                 200                 205

Lys Ser Gln Phe Val Ser Tyr Glu Met Gln Arg Asn Leu Ser Leu Glu
                210                 215                 220

Asn Ala Lys Thr Ser Val Leu Leu Leu Asn Lys Val Asp Leu Leu Trp
            225                 230                 235

Asp Asp Leu Phe Leu Ile Leu Gln Phe Val Trp His Thr Ser Val Glu
        240                 245                 250

His Phe Gln Ile Arg Asn Val Thr Phe Gly Gly Lys Ala Tyr Leu Asp
    255                 260                 265

His Asn Ser Phe Asp Tyr Ser Asn Thr Val Met Arg Thr Ile Lys Leu
270                 275                 280                 285

Glu His Val His Phe Arg Val Phe Tyr Ile Gln Gln Asp Lys Ile Tyr
                290                 295                 300

Leu Leu Leu Thr Lys Met Asp Ile Glu Asn Leu Thr Ile Ser Asn Ala
            305                 310                 315

Gln Met Pro His Met Leu Phe Pro Asn Tyr Pro Thr Lys Phe Gln Tyr
        320                 325                 330

Leu Asn Phe Ala Asn Asn Ile Leu Thr Asp Glu Leu Phe Lys Arg Thr
    335                 340                 345

Ile Gln Leu Pro His Leu Lys Thr Leu Ile Leu Asn Gly Asn Lys Leu
350                 355                 360                 365

Glu Thr Leu Ser Leu Val Ser Cys Phe Ala Asn Asn Thr Pro Leu Glu
                370                 375                 380

His Leu Asp Leu Ser Gln Asn Leu Leu Gln His Lys Asn Asp Glu Asn
            385                 390                 395

Cys Ser Trp Pro Glu Thr Val Val Asn Met Asn Leu Ser Tyr Asn Lys
        400                 405                 410

Leu Ser Asp Ser Val Phe Arg Cys Leu Pro Lys Ser Ile Gln Ile Leu
    415                 420                 425

Asp Leu Asn Asn Asn Gln Ile Gln Thr Val Pro Lys Glu Thr Ile His
430                 435                 440                 445

Leu Met Ala Leu Arg Glu Leu Asn Ile Ala Phe Asn Phe Leu Thr Asp
                450                 455                 460

Leu Pro Gly Cys Ser His Phe Ser Arg Leu Ser Val Leu Asn Ile Glu
            465                 470                 475

Met Asn Phe Ile Leu Ser Pro Ser Leu Asp Phe Val Gln Ser Cys Gln
        480                 485                 490

Glu Val Lys Thr Leu Asn Ala Gly Arg Asn Pro Phe Arg Cys Thr Cys
    495                 500                 505

Glu Leu Lys Asn Phe Ile Gln Leu Glu Thr Tyr Ser Glu Val Met Met
510                 515                 520                 525

Val Gly Trp Ser Asp Ser Tyr Thr Cys Glu Tyr Pro Leu Asn Leu Arg
                530                 535                 540

Gly Thr Arg Leu Lys Asp Val His Leu His Glu Leu Ser Cys Asn Thr
            545                 550                 555

Ala Leu Leu Ile Val Thr Ile Val Val Ile Met Leu Val Leu Gly Leu
        560                 565                 570

Ala Val Ala Phe Cys Cys Leu His Phe Asp Leu Pro Trp Tyr Leu Arg
    575                 580                 585

Met Leu Gly Gln Cys Thr Gln Thr Trp His Arg Val Arg Lys Thr Thr
590                 595                 600                 605

Gln Glu Gln Leu Lys Arg Asn Val Arg Phe His Ala Phe Ile Ser Tyr
                610                 615                 620

Ser Glu His Asp Ser Leu Trp Val Lys Asn Glu Leu Ile Pro Asn Leu
            625                 630                 635

Glu Lys Glu Asp Gly Ser Ile Leu Ile Cys Leu Tyr Glu Ser Tyr Phe
        640                 645                 650

Asp Pro Gly Lys Ser Ile Ser Glu Asn Ile Val Ser Phe Ile Glu Lys
    655                 660                 665

Ser Tyr Lys Ser Ile Phe Val Leu Ser Pro Asn Phe Val Gln Asn Glu
670                 675                 680                 685

Trp Cys His Tyr Glu Phe Tyr Phe Ala His His Asn Leu Phe His Glu
                690                 695                 700

Asn Ser Asp His Ile Ile Leu Ile Leu Leu Glu Pro Ile Pro Phe Tyr
            705                 710                 715

Cys Ile Pro Thr Arg Tyr His Lys Leu Lys Ala Leu Leu Glu Lys Lys
        720                 725                 730

Ala Tyr Leu Glu Trp Pro Lys Asp Arg Arg Lys Cys Gly Leu Phe Trp

735                 740                 745

Ala Asn Leu Arg Ala Ala Ile Asn Val Asn Val Leu Ala Thr Arg Glu
750                 755                 760                 765

Met Tyr Glu Leu Gln Thr Phe Thr Glu Leu Asn Glu Glu Ser Arg Gly
                770                 775                 780

Ser Thr Ile Ser Leu Met Arg Thr Asp Cys Leu
            785                 790


<210> 40
<211> 2760
<212> DNA
<213> Unknown

<220>
<223> Description of Unknown Organism:primate; surmised
      Homo sapiens

<220>
<221> CDS
<222> (68)..(2455)

<220>
<221> mat_peptide
<222> (161)..(2455)

<220>
<221> misc_feature
<222> (2529)
<223> n may be a, c, g, or t

<400> 40
aagaatttgg actcatatca agatgctctg aagaagaaca accctttagg atagccactg 60

caacatc atg acc aaa gac aaa gaa cct att gtt aaa agc ttc cat ttt   109
        Met Thr Lys Asp Lys Glu Pro Ile Val Lys Ser Phe His Phe
            -30                 -25                 -20

gtt tgc ctt atg atc ata ata gtt gga acc aga atc cag ttc tcc gac   157
Val Cys Leu Met Ile Ile Ile Val Gly Thr Arg Ile Gln Phe Ser Asp
        -15                 -10                 -5

gga aat gaa ttt gca gta gac aag tca aaa aga ggt ctt att cat gtt   205
Gly Asn Glu Phe Ala Val Asp Lys Ser Lys Arg Gly Leu Ile His Val
 -1   1               5                  10                  15

cca aaa gac cta ccg ctg aaa acc aaa gtc tta gat atg tct cag aac   253
Pro Lys Asp Leu Pro Leu Lys Thr Lys Val Leu Asp Met Ser Gln Asn
                20                  25                  30

tac atc gct gag ctt cag gtc tct gac atg agc ttt cta tca gag ttg   301
Tyr Ile Ala Glu Leu Gln Val Ser Asp Met Ser Phe Leu Ser Glu Leu
            35                  40                  45

aca gtt ttg aga ctt tcc cat aac aga atc cag cta ctt gat tta agt   349

Thr Val Leu Arg Leu Ser His Asn Arg Ile Gln Leu Leu Asp Leu Ser
50 55 60

gtt ttc aag ttc aac cag gat tta gaa tat ttg gat tta tct cat aat 397
Val Phe Lys Phe Asn Gln Asp Leu Glu Tyr Leu Asp Leu Ser His Asn
65 70 75

cag ttg caa aag ata tcc tgc cat cct att gtg agt ttc agg cat tta 445
Gln Leu Gln Lys Ile Ser Cys His Pro Ile Val Ser Phe Arg His Leu
80 85 90 95

gat ctc tca ttc aat gat ttc aag gcc ctg ccc atc tgt aag gaa ttt 493
Asp Leu Ser Phe Asn Asp Phe Lys Ala Leu Pro Ile Cys Lys Glu Phe
100 105 110

ggc aac tta tca caa ctg aat ttc ttg gga ttg agt gct atg aag ctg 541
Gly Asn Leu Ser Gln Leu Asn Phe Leu Gly Leu Ser Ala Met Lys Leu
115 120 125

caa aaa tta gat ttg ctg cca att gct cac ttg cat cta agt tat atc 589
Gln Lys Leu Asp Leu Leu Pro Ile Ala His Leu His Leu Ser Tyr Ile
130 135 140

ctt ctg gat tta aga aat tat tat ata aaa gaa aat gag aca gaa agt 637
Leu Leu Asp Leu Arg Asn Tyr Tyr Ile Lys Glu Asn Glu Thr Glu Ser
145 150 155

cta caa att ctg aat gca aaa acc ctt cac ctt gtt ttt cac cca act 685
Leu Gln Ile Leu Asn Ala Lys Thr Leu His Leu Val Phe His Pro Thr
160 165 170 175

agt tta ttc gct atc caa gtg aac ata tca gtt aat act tta ggg tgc 733
Ser Leu Phe Ala Ile Gln Val Asn Ile Ser Val Asn Thr Leu Gly Cys
180 185 190

tta caa ctg act aat att aaa ttg aat gat gac aac tgt caa gtt ttc 781
Leu Gln Leu Thr Asn Ile Lys Leu Asn Asp Asp Asn Cys Gln Val Phe
195 200 205

att aaa ttt tta tca gaa ctc acc aga ggt cca acc tta ctg aat ttt 829
Ile Lys Phe Leu Ser Glu Leu Thr Arg Gly Pro Thr Leu Leu Asn Phe
210 215 220

acc ctc aac cac ata gaa acg act tgg aaa tgc ctg gtc aga gtc ttt 877
Thr Leu Asn His Ile Glu Thr Thr Trp Lys Cys Leu Val Arg Val Phe
225 230 235

caa ttt ctt tgg ccc aaa cct gtg gaa tat ctc aat att tac aat tta 925
Gln Phe Leu Trp Pro Lys Pro Val Glu Tyr Leu Asn Ile Tyr Asn Leu
240 245 250 255

aca ata att gaa agc att cgt gaa gaa gat ttt act tat tct aaa acg 973
Thr Ile Ile Glu Ser Ile Arg Glu Glu Asp Phe Thr Tyr Ser Lys Thr
260 265 270

aca ttg aaa gca ttg aca ata gaa cat atc acg aac caa gtt ttt ctg 1021
Thr Leu Lys Ala Leu Thr Ile Glu His Ile Thr Asn Gln Val Phe Leu
275 280 285

ttt tca cag aca gct ttg tac acc gtg ttt tct gag atg aac att atg 1069
Phe Ser Gln Thr Ala Leu Tyr Thr Val Phe Ser Glu Met Asn Ile Met
290 295 300

atg tta acc att tca gat aca cct ttt ata cac atg ctg tgt cct cat 1117
Met Leu Thr Ile Ser Asp Thr Pro Phe Ile His Met Leu Cys Pro His
305 310 315

gca cca agc aca ttc aag ttt ttg aac ttt acc cag aac gtt ttc aca 1165
Ala Pro Ser Thr Phe Lys Phe Leu Asn Phe Thr Gln Asn Val Phe Thr
320 325 330 335

gat agt att ttt gaa aaa tgt tcc acg tta gtt aaa ttg gag aca ctt 1213
Asp Ser Ile Phe Glu Lys Cys Ser Thr Leu Val Lys Leu Glu Thr Leu
340 345 350

atc tta caa aag aat gga tta aaa gac ctt ttc aaa gta ggt ctc atg 1261
Ile Leu Gln Lys Asn Gly Leu Lys Asp Leu Phe Lys Val Gly Leu Met
355 360 365

acg aag gat atg cct tct ttg gaa ata ctg gat gtt agc tgg aat tct 1309
Thr Lys Asp Met Pro Ser Leu Glu Ile Leu Asp Val Ser Trp Asn Ser
370 375 380

ttg gaa tct ggt aga cat aaa gaa aac tgc act tgg gtt gag agt ata 1357
Leu Glu Ser Gly Arg His Lys Glu Asn Cys Thr Trp Val Glu Ser Ile
385 390 395

gtg gtg tta aat ttg tct tca aat atg ctt act gac tct gtt ttc aga 1405
Val Val Leu Asn Leu Ser Ser Asn Met Leu Thr Asp Ser Val Phe Arg
400 405 410 415

tgt tta cct ccc agg atc aag gta ctt gat ctt cac agc aat aaa ata 1453
Cys Leu Pro Pro Arg Ile Lys Val Leu Asp Leu His Ser Asn Lys Ile
420 425 430

aag agc gtt cct aaa caa gtc gta aaa ctg gaa gct ttg caa gaa ctc 1501
Lys Ser Val Pro Lys Gln Val Val Lys Leu Glu Ala Leu Gln Glu Leu
435 440 445

aat gtt gct ttc aat tct tta act gac ctt cct gga tgt ggc agc ttt 1549
Asn Val Ala Phe Asn Ser Leu Thr Asp Leu Pro Gly Cys Gly Ser Phe
450 455 460

agc agc ctt tct gta ttg atc att gat cac aat tca gtt tcc cac cca 1597
Ser Ser Leu Ser Val Leu Ile Ile Asp His Asn Ser Val Ser His Pro
465 470 475

tcg gct gat ttc ttc cag agc tgc cag aag atg agg tca ata aaa gca 1645
Ser Ala Asp Phe Phe Gln Ser Cys Gln Lys Met Arg Ser Ile Lys Ala
480 485 490 495

ggg gac aat cca ttc caa tgt acc tgt gag cta aga gaa ttt gtc aaa 1693
Gly Asp Asn Pro Phe Gln Cys Thr Cys Glu Leu Arg Glu Phe Val Lys
500 505 510

aat ata gac caa gta tca agt gaa gtg tta gag ggc tgg cct gat tct 1741

DX0724XK

Asn Ile Asp Gln Val Ser Ser Glu Val Leu Glu Gly Trp Pro Asp Ser
            515                 520                 525

tat aag tgt gac tac cca gaa agt tat aga gga agc cca cta aag gac    1789
Tyr Lys Cys Asp Tyr Pro Glu Ser Tyr Arg Gly Ser Pro Leu Lys Asp
        530                 535                 540

ttt cac atg tct gaa tta tcc tgc aac ata act ctg ctg atc gtc acc    1837
Phe His Met Ser Glu Leu Ser Cys Asn Ile Thr Leu Leu Ile Val Thr
    545                 550                 555

atc ggt gcc acc atg ctg gtg ttg gct gtg act gtg acc tcc ctc tgc    1885
Ile Gly Ala Thr Met Leu Val Leu Ala Val Thr Val Thr Ser Leu Cys
560                 565                 570                 575

atc tac ttg gat ctg ccc tgg tat ctc agg atg gtg tgc cag tgg acc    1933
Ile Tyr Leu Asp Leu Pro Trp Tyr Leu Arg Met Val Cys Gln Trp Thr
                580                 585                 590

cag act cgg cgc agg gcc agg aac ata ccc tta gaa gaa ctc caa aga    1981
Gln Thr Arg Arg Arg Ala Arg Asn Ile Pro Leu Glu Glu Leu Gln Arg
            595                 600                 605

aac ctc cag ttt cat gct ttt att tca tat agt gaa cat gat tct gcc    2029
Asn Leu Gln Phe His Ala Phe Ile Ser Tyr Ser Glu His Asp Ser Ala
        610                 615                 620

tgg gtg aaa agt gaa ttg gta cct tac cta gaa aaa gaa gat ata cag    2077
Trp Val Lys Ser Glu Leu Val Pro Tyr Leu Glu Lys Glu Asp Ile Gln
    625                 630                 635

att tgt ctt cat gag agg aac ttt gtc cct ggc aag agc att gtg gaa    2125
Ile Cys Leu His Glu Arg Asn Phe Val Pro Gly Lys Ser Ile Val Glu
640                 645                 650                 655

aat atc atc aac tgc att gag aag agt tac aag tcc atc ttt gtt ttg    2173
Asn Ile Ile Asn Cys Ile Glu Lys Ser Tyr Lys Ser Ile Phe Val Leu
                660                 665                 670

tct ccc aac ttt gtc cag agt gag tgg tgc cat tac gaa ctc tat ttt    2221
Ser Pro Asn Phe Val Gln Ser Glu Trp Cys His Tyr Glu Leu Tyr Phe
            675                 680                 685

gcc cat cac aat ctc ttt cat gaa gga tct aat aac tta atc ctc atc    2269
Ala His His Asn Leu Phe His Glu Gly Ser Asn Asn Leu Ile Leu Ile
        690                 695                 700

tta ctg gaa ccc att cca cag aac agc att ccc aac aag tac cac aag    2317
Leu Leu Glu Pro Ile Pro Gln Asn Ser Ile Pro Asn Lys Tyr His Lys
    705                 710                 715

ctg aag gct ctc atg acg cag cgg act tat ttg cag tgg ccc aag gag    2365
Leu Lys Ala Leu Met Thr Gln Arg Thr Tyr Leu Gln Trp Pro Lys Glu
720                 725                 730                 735

aaa agc aaa cgt ggg ctc ttt tgg gct aac att aga gcc gct ttt aat    2413
Lys Ser Lys Arg Gly Leu Phe Trp Ala Asn Ile Arg Ala Ala Phe Asn
                740                 745                 750

DX0724XK

atg aaa tta aca cta gtc act gaa aac aat gat gtg aaa tct          2455
Met Lys Leu Thr Leu Val Thr Glu Asn Asn Asp Val Lys Ser
            755                 760                 765

taaaaaaatt taggaaattc aacttaagaa accattattt acttggatga tggtgaatag 2515

tacagtcgta agtnactgtc tggaggtgcc tccattatcc tcatgccttc aggaaagact 2575

taacaaaaac aatgtttcat ctggggaact gagctaggcg gtgaggttag cctgccagtt 2635

agagacagcc cagtctcttc tggtttaatc attatgtttc aaattgaaac agtctctttt 2695

gagtaaatgc tcagtttttc agctcctctc cactctgctt tcccaaatgg attctgttgg 2755

tgaag                                                             2760

<210> 41
<211> 796
<212> PRT
<213> Unknown

<400> 41
Met Thr Lys Asp Lys Glu Pro Ile Val Lys Ser Phe His Phe Val Cys
    -30                 -25                 -20

Leu Met Ile Ile Ile Val Gly Thr Arg Ile Gln Phe Ser Asp Gly Asn
-15                 -10                 -5                  -1   1

Glu Phe Ala Val Asp Lys Ser Lys Arg Gly Leu Ile His Val Pro Lys
            5                   10                  15

Asp Leu Pro Leu Lys Thr Lys Val Leu Asp Met Ser Gln Asn Tyr Ile
        20                  25                  30

Ala Glu Leu Gln Val Ser Asp Met Ser Phe Leu Ser Glu Leu Thr Val
    35                  40                  45

Leu Arg Leu Ser His Asn Arg Ile Gln Leu Leu Asp Leu Ser Val Phe
50                  55                  60                  65

Lys Phe Asn Gln Asp Leu Glu Tyr Leu Asp Leu Ser His Asn Gln Leu
                70                  75                  80

Gln Lys Ile Ser Cys His Pro Ile Val Ser Phe Arg His Leu Asp Leu
            85                  90                  95

Ser Phe Asn Asp Phe Lys Ala Leu Pro Ile Cys Lys Glu Phe Gly Asn
        100                 105                 110

Leu Ser Gln Leu Asn Phe Leu Gly Leu Ser Ala Met Lys Leu Gln Lys
    115                 120                 125

Leu Asp Leu Leu Pro Ile Ala His Leu His Leu Ser Tyr Ile Leu Leu

150                 155                 160

Ile Leu Asn Ala Lys Thr Leu His Leu Val Phe His Pro Thr Ser Leu
            165                 170                 175

Phe Ala Ile Gln Val Asn Ile Ser Val Asn Thr Leu Gly Cys Leu Gln
        180                 185                 190

Leu Thr Asn Ile Lys Leu Asn Asp Asp Asn Cys Gln Val Phe Ile Lys
    195                 200                 205

Phe Leu Ser Glu Leu Thr Arg Gly Pro Thr Leu Leu Asn Phe Thr Leu
210                 215                 220                 225

Asn His Ile Glu Thr Thr Trp Lys Cys Leu Val Arg Val Phe Gln Phe
                230                 235                 240

Leu Trp Pro Lys Pro Val Glu Tyr Leu Asn Ile Tyr Asn Leu Thr Ile
            245                 250                 255

Ile Glu Ser Ile Arg Glu Glu Asp Phe Thr Tyr Ser Lys Thr Thr Leu
        260                 265                 270

Lys Ala Leu Thr Ile Glu His Ile Thr Asn Gln Val Phe Leu Phe Ser
    275                 280                 285

Gln Thr Ala Leu Tyr Thr Val Phe Ser Glu Met Asn Ile Met Met Leu
290                 295                 300                 305

Thr Ile Ser Asp Thr Pro Phe Ile His Met Leu Cys Pro His Ala Pro
                310                 315                 320

Ser Thr Phe Lys Phe Leu Asn Phe Thr Gln Asn Val Phe Thr Asp Ser
            325                 330                 335

Ile Phe Glu Lys Cys Ser Thr Leu Val Lys Leu Glu Thr Leu Ile Leu
        340                 345                 350

Gln Lys Asn Gly Leu Lys Asp Leu Phe Lys Val Gly Leu Met Thr Lys
    355                 360                 365

Asp Met Pro Ser Leu Glu Ile Leu Asp Val Ser Trp Asn Ser Leu Glu
370                 375                 380                 385

Ser Gly Arg His Lys Glu Asn Cys Thr Trp Val Glu Ser Ile Val Val
                390                 395                 400

Leu Asn Leu Ser Ser Asn Met Leu Thr Asp Ser Val Phe Arg Cys Leu
            405                 410                 415

Pro Pro Arg Ile Lys Val Leu Asp Leu His Ser Asn Lys Ile Lys Ser
        420                 425                 430

Val Pro Lys Gln Val Val Lys Leu Glu Ala Leu Gln Glu Leu Asn Val
    435                 440                 445

Ala Phe Asn Ser Leu Thr Asp Leu Pro Gly Cys Gly Ser Phe Ser Ser
450                 455                 460                 465

Leu Ser Val Leu Ile Ile Asp His Asn Ser Val Ser His Pro Ser Ala
470 475 480

Asp Phe Phe Gln Ser Cys Gln Lys Met Arg Ser Ile Lys Ala Gly Asp
485 490 495

Asn Pro Phe Gln Cys Thr Cys Glu Leu Arg Glu Phe Val Lys Asn Ile
500 505 510

Asp Gln Val Ser Ser Glu Val Leu Glu Gly Trp Pro Asp Ser Tyr Lys
515 520 525

Cys Asp Tyr Pro Glu Ser Tyr Arg Gly Ser Pro Leu Lys Asp Phe His
530 535 540 545


550 555 560


565 570 575


580 585 590


595 600 605


610 615 620 625


630 635 640


645 650 655


660 665 670


675 680 685


690 695 700 705


710 715 720


725 730 735


740 745 750


755 760 765

<210> 42
<211> 3168
<212> DNA
<213> Unknown

<220>
<223> Description of Unknown Organism:primate; surmised
      Homo sapiens

<220>
<221> CDS
<222> (1)..(3165)

<220>
<221> mat_peptide
<222> (144)..(3165)

<400> 42
atg ccc atg aag tgg agt ggg tgg agg tgg agc tgg ggg ccg gcc act     48
Met Pro Met Lys Trp Ser Gly Trp Arg Trp Ser Trp Gly Pro Ala Thr
            -45                 -40                 -35

cac aca gcc ctc cca ccc cca cag ggt ttc tgc cgc agc gcc ctg cac     96
His Thr Ala Leu Pro Pro Pro Gln Gly Phe Cys Arg Ser Ala Leu His
        -30                 -25                 -20

ccg ctg tct ctc ctg gtg cag gcc atc atg ctg gcc atg acc ctg gcc     144
Pro Leu Ser Leu Leu Val Gln Ala Ile Met Leu Ala Met Thr Leu Ala
    -15                 -10                 -5                  -1

ctg ggt acc ttg cct gcc ttc cta ccc tgt gag ctc cag ccc cac ggc     192
Leu Gly Thr Leu Pro Ala Phe Leu Pro Cys Glu Leu Gln Pro His Gly
1               5                   10                  15

ctg gtg aac tgc aac tgg ctg ttc ctg aag tct gtg ccc cac ttc tcc     240
Leu Val Asn Cys Asn Trp Leu Phe Leu Lys Ser Val Pro His Phe Ser
            20                  25                  30

atg gca gca ccc cgt ggc aat gtc acc agc ctt tcc ttg tcc tcc aac     288
Met Ala Ala Pro Arg Gly Asn Val Thr Ser Leu Ser Leu Ser Ser Asn
        35                  40                  45

cgc atc cac cac ctc cat gat tct gac ttt gcc cac ctg ccc agc ctg     336
Arg Ile His His Leu His Asp Ser Asp Phe Ala His Leu Pro Ser Leu
    50                  55                  60

cgg cat ctc aac ctc aag tgg aac tgc ccg ccg gtt ggc ctc agc ccc     384
Arg His Leu Asn Leu Lys Trp Asn Cys Pro Pro Val Gly Leu Ser Pro
65                  70                  75                  80

atg cac ttc ccc tgc cac atg acc atc gag ccc agc acc ttc ttg gct     432
Met His Phe Pro Cys His Met Thr Ile Glu Pro Ser Thr Phe Leu Ala
                85                  90                  95

gtg ccc acc ctg gaa gag cta aac ctg agc tac aac aac atc atg act     480
Val Pro Thr Leu Glu Glu Leu Asn Leu Ser Tyr Asn Asn Ile Met Thr
            100                 105                 110

gtg cct gcg ctg ccc aaa tcc ctc ata tcc ctg tcc ctc agc cat acc 528
Val Pro Ala Leu Pro Lys Ser Leu Ile Ser Leu Ser Leu Ser His Thr
        115                 120                 125

aac atc ctg atg cta gac tct gcc agc ctc gcc ggc ctg cat gcc ctg 576
Asn Ile Leu Met Leu Asp Ser Ala Ser Leu Ala Gly Leu His Ala Leu
    130                 135                 140

cgc ttc cta ttc atg gac ggc aac tgt tat tac aag aac ccc tgc agg 624
Arg Phe Leu Phe Met Asp Gly Asn Cys Tyr Tyr Lys Asn Pro Cys Arg
145                 150                 155                 160

cag gca ctg gag gtg gcc ccg ggt gcc ctc ctt ggc ctg ggc aac ctc 672
Gln Ala Leu Glu Val Ala Pro Gly Ala Leu Leu Gly Leu Gly Asn Leu
                165                 170                 175

acc cac ctg tca ctc aag tac aac aac ctc act gtg gtg ccc cgc aac 720
Thr His Leu Ser Leu Lys Tyr Asn Asn Leu Thr Val Val Pro Arg Asn
            180                 185                 190

ctg cct tcc agc ctg gag tat ctg ctg ttg tcc tac aac cgc atc gtc 768
Leu Pro Ser Ser Leu Glu Tyr Leu Leu Leu Ser Tyr Asn Arg Ile Val
        195                 200                 205

aaa ctg gcg cct gag gac ctg gcc aat ctg acc gcc ctg cgt gtg ctc 816
Lys Leu Ala Pro Glu Asp Leu Ala Asn Leu Thr Ala Leu Arg Val Leu
    210                 215                 220

gat gtg ggc gga aat tgc cgc cgc tgc gac cac gct ccc aac ccc tgc 864
Asp Val Gly Gly Asn Cys Arg Arg Cys Asp His Ala Pro Asn Pro Cys
225                 230                 235                 240

atg gag tgc cct cgt cac ttc ccc cag cta cat ccc gat acc ttc agc 912
Met Glu Cys Pro Arg His Phe Pro Gln Leu His Pro Asp Thr Phe Ser
                245                 250                 255

cac ctg agc cgt ctt gaa ggc ctg gtg ttg aag gac agt tct ctc tcc 960
His Leu Ser Arg Leu Glu Gly Leu Val Leu Lys Asp Ser Ser Leu Ser
            260                 265                 270

tgg ctg aat gcc agt tgg ttc cgt ggg ctg gga aac ctc cga gtg ctg 1008
Trp Leu Asn Ala Ser Trp Phe Arg Gly Leu Gly Asn Leu Arg Val Leu
        275                 280                 285

gac ctg agt gag aac ttc ctc tac aaa tgc atc act aaa acc aag gcc 1056
Asp Leu Ser Glu Asn Phe Leu Tyr Lys Cys Ile Thr Lys Thr Lys Ala
    290                 295                 300

ttc cag ggc cta aca cag ctg cgc aag ctt aac ctg tcc ttc aat tac 1104
Phe Gln Gly Leu Thr Gln Leu Arg Lys Leu Asn Leu Ser Phe Asn Tyr
305                 310                 315                 320

caa aag agg gtg tcc ttt gcc cac ctg tct ctg gcc cct tcc ttc ggg 1152
Gln Lys Arg Val Ser Phe Ala His Leu Ser Leu Ala Pro Ser Phe Gly
                325                 330                 335

agc ctg gtc gcc ctg aag gag ctg gac atg cac ggc atc ttc ttc cgc 1200

Ser Leu Val Ala Leu Lys Glu Leu Asp Met His Gly Ile Phe Phe Arg
            340                 345                 350

tca ctc gat gag acc acg ctc cgg cca ctg gcc cgc ctg ccc atg ctc 1248
Ser Leu Asp Glu Thr Thr Leu Arg Pro Leu Ala Arg Leu Pro Met Leu
        355                 360                 365

cag act ctg cgt ctg cag atg aac ttc atc aac cag gcc cag ctc ggc 1296
Gln Thr Leu Arg Leu Gln Met Asn Phe Ile Asn Gln Ala Gln Leu Gly
    370                 375                 380

atc ttc agg gcc ttc cct ggc ctg cgc tac gtg gac ctg tcg gac aac 1344
Ile Phe Arg Ala Phe Pro Gly Leu Arg Tyr Val Asp Leu Ser Asp Asn
385                 390                 395                 400

cgc atc agc gga gct tcg gag ctg aca gcc acc atg ggg gag gca gat 1392
Arg Ile Ser Gly Ala Ser Glu Leu Thr Ala Thr Met Gly Glu Ala Asp
                405                 410                 415

gga ggg gag aag gtc tgg ctg cag cct ggg gac ctt gct ccg gcc cca 1440
Gly Gly Glu Lys Val Trp Leu Gln Pro Gly Asp Leu Ala Pro Ala Pro
            420                 425                 430

gtg gac act ccc agc tct gaa gac ttc agg ccc aac tgc agc acc ctc 1488
Val Asp Thr Pro Ser Ser Glu Asp Phe Arg Pro Asn Cys Ser Thr Leu
        435                 440                 445

aac ttc acc ttg gat ctg tca cgg aac aac ctg gtg acc gtg cag ccg 1536
Asn Phe Thr Leu Asp Leu Ser Arg Asn Asn Leu Val Thr Val Gln Pro
    450                 455                 460

gag atg ttt gcc cag ctc tcg cac ctg cag tgc ctg cgc ctg agc cac 1584
Glu Met Phe Ala Gln Leu Ser His Leu Gln Cys Leu Arg Leu Ser His
465                 470                 475                 480

aac tgc atc tcg cag gca gtc aat ggc tcc cag ttc ctg ccg ctg acc 1632
Asn Cys Ile Ser Gln Ala Val Asn Gly Ser Gln Phe Leu Pro Leu Thr
                485                 490                 495

ggt ctg cag gtg cta gac ctg tcc cac aat aag ctg gac ctc tac cac 1680
Gly Leu Gln Val Leu Asp Leu Ser His Asn Lys Leu Asp Leu Tyr His
            500                 505                 510

gag cac tca ttc acg gag cta cca cga ctg gag gcc ctg gac ctc agc 1728
Glu His Ser Phe Thr Glu Leu Pro Arg Leu Glu Ala Leu Asp Leu Ser
        515                 520                 525

tac aac agc cag ccc ttt ggc atg cag ggc gtg ggc cac aac ttc agc 1776
Tyr Asn Ser Gln Pro Phe Gly Met Gln Gly Val Gly His Asn Phe Ser
    530                 535                 540

ttc gtg gct cac ctg cgc acc ctg cgc cac ctc agc ctg gcc cac aac 1824
Phe Val Ala His Leu Arg Thr Leu Arg His Leu Ser Leu Ala His Asn
545                 550                 555                 560

aac atc cac agc caa gtg tcc cag cag ctc tgc agt acg tcg ctg cgg 1872
Asn Ile His Ser Gln Val Ser Gln Gln Leu Cys Ser Thr Ser Leu Arg
                565                 570                 575

gcc ctg gac ttc agc ggc aat gca ctg ggc cat atg tgg gcc gag gga 1920
Ala Leu Asp Phe Ser Gly Asn Ala Leu Gly His Met Trp Ala Glu Gly
            580                 585                 590

gac ctc tat ctg cac ttc ttc caa ggc ctg agc ggt ttg atc tgg ctg 1968
Asp Leu Tyr Leu His Phe Phe Gln Gly Leu Ser Gly Leu Ile Trp Leu
        595                 600                 605

gac ttg tcc cag aac cgc ctg cac acc ctc ctg ccc caa acc ctg cgc 2016
Asp Leu Ser Gln Asn Arg Leu His Thr Leu Leu Pro Gln Thr Leu Arg
    610                 615                 620

aac ctc ccc aag agc cta cag gtg ctg cgt ctc cgt gac aat tac ctg 2064
Asn Leu Pro Lys Ser Leu Gln Val Leu Arg Leu Arg Asp Asn Tyr Leu
625                 630                 635                 640

gcc ttc ttt aag tgg tgg agc ctc cac ttc ctg ccc aaa ctg gaa gtc 2112
Ala Phe Phe Lys Trp Trp Ser Leu His Phe Leu Pro Lys Leu Glu Val
                645                 650                 655

ctc gac ctg gca gga aac cag ctg aag gcc ctg acc aat ggc agc ctg 2160
Leu Asp Leu Ala Gly Asn Gln Leu Lys Ala Leu Thr Asn Gly Ser Leu
            660                 665                 670

cct gct ggc acc cgg ctc cgg agg ctg gat gtc agc tgc aac agc atc 2208
Pro Ala Gly Thr Arg Leu Arg Arg Leu Asp Val Ser Cys Asn Ser Ile
        675                 680                 685

agc ttc gtg gcc ccc ggc ttc ttt tcc aag gcc aag gag ctg cga gag 2256
Ser Phe Val Ala Pro Gly Phe Phe Ser Lys Ala Lys Glu Leu Arg Glu
    690                 695                 700

ctc aac ctt agc gcc aac gcc ctc aag aca gtg gac cac tcc tgg ttt 2304
Leu Asn Leu Ser Ala Asn Ala Leu Lys Thr Val Asp His Ser Trp Phe
705                 710                 715                 720

ggg ccc ctg gcg agt gcc ctg caa ata cta gat gta agc gcc aac cct 2352
Gly Pro Leu Ala Ser Ala Leu Gln Ile Leu Asp Val Ser Ala Asn Pro
                725                 730                 735

ctg cac tgc gcc tgt ggg gcg gcc ttt atg gac ttc ctg ctg gag gtg 2400
Leu His Cys Ala Cys Gly Ala Ala Phe Met Asp Phe Leu Leu Glu Val
            740                 745                 750

cag gct gcc gtg ccc ggt ctg ccc agc cgg gtg aag tgt ggc agt ccg 2448
Gln Ala Ala Val Pro Gly Leu Pro Ser Arg Val Lys Cys Gly Ser Pro
        755                 760                 765

ggc cag ctc cag ggc ctc agc atc ttt gca cag gac ctg cgc ctc tgc 2496
Gly Gln Leu Gln Gly Leu Ser Ile Phe Ala Gln Asp Leu Arg Leu Cys
    770                 775                 780

ctg gat gag gcc ctc tcc tgg gac tgt ttc gcc ctc tcg ctg ctg gct 2544
Leu Asp Glu Ala Leu Ser Trp Asp Cys Phe Ala Leu Ser Leu Leu Ala
785                 790                 795                 800

gtg gct ctg ggc ctg ggt gtg ccc atg ctg cat cac ctc tgt ggc tgg 2592

Val Ala Leu Gly Leu Gly Val Pro Met Leu His His Leu Cys Gly Trp
                805                 810                 815

gac ctc tgg tac tgc ttc cac ctg tgc ctg gcc tgg ctt ccc tgg cgg     2640
Asp Leu Trp Tyr Cys Phe His Leu Cys Leu Ala Trp Leu Pro Trp Arg
            820                 825                 830

ggg cgg caa agt ggg cga gat gag gat gcc ctg ccc tac gat gcc ttc     2688
Gly Arg Gln Ser Gly Arg Asp Glu Asp Ala Leu Pro Tyr Asp Ala Phe
        835                 840                 845

gtg gtc ttc gac aaa acg cag agc gca gtg gca gac tgg gtg tac aac     2736
Val Val Phe Asp Lys Thr Gln Ser Ala Val Ala Asp Trp Val Tyr Asn
    850                 855                 860

gag ctt cgg ggg cag ctg gag gag tgc cgt ggg cgc tgg gca ctc cgc     2784
Glu Leu Arg Gly Gln Leu Glu Glu Cys Arg Gly Arg Trp Ala Leu Arg
865                 870                 875                 880

ctg tgc ctg gag gaa cgc gac tgg ctg cct ggc aaa acc ctc ttt gag     2832
Leu Cys Leu Glu Glu Arg Asp Trp Leu Pro Gly Lys Thr Leu Phe Glu
                885                 890                 895

aac ctg tgg gcc tcg gtc tat ggc agc cgc aag acg ctg ttt gtg ctg     2880
Asn Leu Trp Ala Ser Val Tyr Gly Ser Arg Lys Thr Leu Phe Val Leu
            900                 905                 910

gcc cac acg gac cgg gtc agt ggt ctc ttg cgc gcc agc ttc ctg ctg     2928
Ala His Thr Asp Arg Val Ser Gly Leu Leu Arg Ala Ser Phe Leu Leu
        915                 920                 925

gcc cag cag cgc ctg ctg gag gac cgc aag gac gtc gtg gtg ctg gtg     2976
Ala Gln Gln Arg Leu Leu Glu Asp Arg Lys Asp Val Val Val Leu Val
    930                 935                 940

atc ctg agc cct gac ggc cgc cgc tcc cgc tat gtg cgg ctg cgc cag     3024
Ile Leu Ser Pro Asp Gly Arg Arg Ser Arg Tyr Val Arg Leu Arg Gln
945                 950                 955                 960

cgc ctc tgc cgc cag agt gtc ctc ctc tgg ccc cac cag ccc agt ggt     3072
Arg Leu Cys Arg Gln Ser Val Leu Leu Trp Pro His Gln Pro Ser Gly
                965                 970                 975

cag cgc agc ttc tgg gcc cag ctg ggc atg gcc ctg acc agg gac aac     3120
Gln Arg Ser Phe Trp Ala Gln Leu Gly Met Ala Leu Thr Arg Asp Asn
            980                 985                 990

cac cac ttc tat aac cgg aac ttc tgc cag gga ccc acg gcc gaa tag     3168
His His Phe Tyr Asn Arg Asn Phe Cys Gln Gly Pro Thr Ala Glu
        995                 1000                1005

<210> 43
<211> 1055
<212> PRT
<213> Unknown

<400> 43

Met Pro Met Lys Trp Ser Gly Trp Arg Trp Ser Trp Gly Pro Ala Thr
-45 -40 -35

His Thr Ala Leu Pro Pro Pro Gln Gly Phe Cys Arg Ser Ala Leu His
-30 -25 -20

Pro Leu Ser Leu Leu Val Gln Ala Ile Met Leu Ala Met Thr Leu Ala
-15 -10 -5 -1

Leu Gly Thr Leu Pro Ala Phe Leu Pro Cys Glu Leu Gln Pro His Gly
1               5                   10                  15

Leu Val Asn Cys Asn Trp Leu Phe Leu Lys Ser Val Pro His Phe Ser
            20                  25                  30

Met Ala Ala Pro Arg Gly Asn Val Thr Ser Leu Ser Leu Ser Ser Asn
        35                  40                  45

Arg Ile His His Leu His Asp Ser Asp Phe Ala His Leu Pro Ser Leu
    50                  55                  60

Arg His Leu Asn Leu Lys Trp Asn Cys Pro Pro Val Gly Leu Ser Pro
65                  70                  75                  80

Met His Phe Pro Cys His Met Thr Ile Glu Pro Ser Thr Phe Leu Ala
                85                  90                  95

Val Pro Thr Leu Glu Glu Leu Asn Leu Ser Tyr Asn Asn Ile Met Thr
            100                 105                 110

Val Pro Ala Leu Pro Lys Ser Leu Ile Ser Leu Ser Leu Ser His Thr
        115                 120                 125

Asn Ile Leu Met Leu Asp Ser Ala Ser Leu Ala Gly Leu His Ala Leu
    130                 135                 140

Arg Phe Leu Phe Met Asp Gly Asn Cys Tyr Tyr Lys Asn Pro Cys Arg
145                 150                 155                 160

Gln Ala Leu Glu Val Ala Pro Gly Ala Leu Leu Gly Leu Gly Asn Leu
                165                 170                 175

Thr His Leu Ser Leu Lys Tyr Asn Asn Leu Thr Val Val Pro Arg Asn
            180                 185                 190

Leu Pro Ser Ser Leu Glu Tyr Leu Leu Leu Ser Tyr Asn Arg Ile Val
        195                 200                 205

Lys Leu Ala Pro Glu Asp Leu Ala Asn Leu Thr Ala Leu Arg Val Leu
    210                 215                 220

Asp Val Gly Gly Asn Cys Arg Arg Cys Asp His Ala Pro Asn Pro Cys
225                 230                 235                 240

Met Glu Cys Pro Arg His Phe Pro Gln Leu His Pro Asp Thr Phe Ser
                245                 250                 255

His Leu Ser Arg Leu Glu Gly Leu Val Leu Lys Asp Ser Ser Leu Ser

            260                 265                 270

Trp Leu Asn Ala Ser Trp Phe Arg Gly Leu Gly Asn Leu Arg Val Leu
        275                 280                 285

Asp Leu Ser Glu Asn Phe Leu Tyr Lys Cys Ile Thr Lys Thr Lys Ala
    290                 295                 300

Phe Gln Gly Leu Thr Gln Leu Arg Lys Leu Asn Leu Ser Phe Asn Tyr
305                 310                 315                 320

Gln Lys Arg Val Ser Phe Ala His Leu Ser Leu Ala Pro Ser Phe Gly
                325                 330                 335

Ser Leu Val Ala Leu Lys Glu Leu Asp Met His Gly Ile Phe Phe Arg
            340                 345                 350

Ser Leu Asp Glu Thr Thr Leu Arg Pro Leu Ala Arg Leu Pro Met Leu
        355                 360                 365

Gln Thr Leu Arg Leu Gln Met Asn Phe Ile Asn Gln Ala Gln Leu Gly
    370                 375                 380

Ile Phe Arg Ala Phe Pro Gly Leu Arg Tyr Val Asp Leu Ser Asp Asn
385                 390                 395                 400

Arg Ile Ser Gly Ala Ser Glu Leu Thr Ala Thr Met Gly Glu Ala Asp
                405                 410                 415

Gly Gly Glu Lys Val Trp Leu Gln Pro Gly Asp Leu Ala Pro Ala Pro
            420                 425                 430

Val Asp Thr Pro Ser Ser Glu Asp Phe Arg Pro Asn Cys Ser Thr Leu
        435                 440                 445

Asn Phe Thr Leu Asp Leu Ser Arg Asn Asn Leu Val Thr Val Gln Pro
    450                 455                 460

Glu Met Phe Ala Gln Leu Ser His Leu Gln Cys Leu Arg Leu Ser His
465                 470                 475                 480

Asn Cys Ile Ser Gln Ala Val Asn Gly Ser Gln Phe Leu Pro Leu Thr
                485                 490                 495

Gly Leu Gln Val Leu Asp Leu Ser His Asn Lys Leu Asp Leu Tyr His
            500                 505                 510

Glu His Ser Phe Thr Glu Leu Pro Arg Leu Glu Ala Leu Asp Leu Ser
        515                 520                 525

Tyr Asn Ser Gln Pro Phe Gly Met Gln Gly Val Gly His Asn Phe Ser
    530                 535                 540

Phe Val Ala His Leu Arg Thr Leu Arg His Leu Ser Leu Ala His Asn
545                 550                 555                 560

Asn Ile His Ser Gln Val Ser Gln Gln Leu Cys Ser Thr Ser Leu Arg
                565                 570                 575

Ala Leu Asp Phe Ser Gly Asn Ala Leu Gly His Met Trp Ala Glu Gly
            580                 585                 590

Asp Leu Tyr Leu His Phe Phe Gln Gly Leu Ser Gly Leu Ile Trp Leu
        595                 600                 605

Asp Leu Ser Gln Asn Arg Leu His Thr Leu Leu Pro Gln Thr Leu Arg
    610                 615                 620

Asn Leu Pro Lys Ser Leu Gln Val Leu Arg Leu Arg Asp Asn Tyr Leu
625                 630                 635                 640

Ala Phe Phe Lys Trp Trp Ser Leu His Phe Leu Pro Lys Leu Glu Val
                645                 650                 655

Leu Asp Leu Ala Gly Asn Gln Leu Lys Ala Leu Thr Asn Gly Ser Leu
            660                 665                 670

Pro Ala Gly Thr Arg Leu Arg Arg Leu Asp Val Ser Cys Asn Ser Ile
        675                 680                 685

Ser Phe Val Ala Pro Gly Phe Phe Ser Lys Ala Lys Glu Leu Arg Glu
    690                 695                 700

Leu Asn Leu Ser Ala Asn Ala Leu Lys Thr Val Asp His Ser Trp Phe
705                 710                 715                 720

Gly Pro Leu Ala Ser Ala Leu Gln Ile Leu Asp Val Ser Ala Asn Pro
                725                 730                 735

Leu His Cys Ala Cys Gly Ala Ala Phe Met Asp Phe Leu Leu Glu Val
            740                 745                 750

Gln Ala Ala Val Pro Gly Leu Pro Ser Arg Val Lys Cys Gly Ser Pro
        755                 760                 765

Gly Gln Leu Gln Gly Leu Ser Ile Phe Ala Gln Asp Leu Arg Leu Cys
    770                 775                 780

Leu Asp Glu Ala Leu Ser Trp Asp Cys Phe Ala Leu Ser Leu Leu Ala
785                 790                 795                 800

Val Ala Leu Gly Leu Gly Val Pro Met Leu His His Leu Cys Gly Trp
                805                 810                 815

Asp Leu Trp Tyr Cys Phe His Leu Cys Leu Ala Trp Leu Pro Trp Arg
            820                 825                 830

Gly Arg Gln Ser Gly Arg Asp Glu Asp Ala Leu Pro Tyr Asp Ala Phe
        835                 840                 845

Val Val Phe Asp Lys Thr Gln Ser Ala Val Ala Asp Trp Val Tyr Asn
    850                 855                 860

Glu Leu Arg Gly Gln Leu Glu Glu Cys Arg Gly Arg Trp Ala Leu Arg
865                 870                 875                 880

Leu Cys Leu Glu Glu Arg Asp Trp Leu Pro Gly Lys Thr Leu Phe Glu
                885                 890                 895

Asn Leu Trp Ala Ser Val Tyr Gly Ser Arg Lys Thr Leu Phe Val Leu
            900                 905                 910

Ala His Thr Asp Arg Val Ser Gly Leu Leu Arg Ala Ser Phe Leu Leu
        915                 920                 925

Ala Gln Gln Arg Leu Leu Glu Asp Arg Lys Asp Val Val Val Leu Val
    930                 935                 940

Ile Leu Ser Pro Asp Gly Arg Arg Ser Arg Tyr Val Arg Leu Arg Gln
945                 950                 955                 960

Arg Leu Cys Arg Gln Ser Val Leu Leu Trp Pro His Gln Pro Ser Gly
                965                 970                 975

Gln Arg Ser Phe Trp Ala Gln Leu Gly Met Ala Leu Thr Arg Asp Asn
            980                 985                 990

His His Phe Tyr Asn Arg Asn Phe Cys Gln Gly Pro Thr Ala Glu
        995                 1000                1005

<210> 44
<211> 2289
<212> DNA
<213> Unknown

<220>
<223> Description of Unknown Organism:rodent; surmised
      Mus musculus

<220>
<221> CDS
<222> (1)..(2079)

<400> 44
aac ctg tcc ttc aat tac cgc aag aag gta tcc ttt gcc cgc ctc cac      48
Asn Leu Ser Phe Asn Tyr Arg Lys Lys Val Ser Phe Ala Arg Leu His
  1               5                  10                  15

ctg gca agt tcc ttt aag aac ctg gtg tca ctg cag gag ctg aac atg      96
Leu Ala Ser Ser Phe Lys Asn Leu Val Ser Leu Gln Glu Leu Asn Met
             20                  25                  30

aac ggc atc ttc ttc cgc ttg ctc aac aag tac acg ctc aga tgg ctg     144
Asn Gly Ile Phe Phe Arg Leu Leu Asn Lys Tyr Thr Leu Arg Trp Leu
         35                  40                  45

gcc gat ctg ccc aaa ctc cac act ctg cat ctt caa atg aac ttc atc     192
Ala Asp Leu Pro Lys Leu His Thr Leu His Leu Gln Met Asn Phe Ile
     50                  55                  60

aac cag gca cag ctc agc atc ttt ggt acc ttc cga gcc ctt cgc ttt     240
Asn Gln Ala Gln Leu Ser Ile Phe Gly Thr Phe Arg Ala Leu Arg Phe
 65                  70                  75                  80

gtg gac ttg tca gac aat cgc atc agt ggg cct tca acg ctg tca gaa 288
Val Asp Leu Ser Asp Asn Arg Ile Ser Gly Pro Ser Thr Leu Ser Glu
                85                  90                  95

gcc acc cct gaa gag gca gat gat gca gag cag gag gag ctg ttg tct 336
Ala Thr Pro Glu Glu Ala Asp Asp Ala Glu Gln Glu Glu Leu Leu Ser
            100                 105                 110

gcg gat cct cac cca gct ccg ctg agc acc cct gct tct aag aac ttc 384
Ala Asp Pro His Pro Ala Pro Leu Ser Thr Pro Ala Ser Lys Asn Phe
        115                 120                 125

atg gac agg tgt aag aac ttc aag ttc aac atg gac ctg tct cgg aac 432
Met Asp Arg Cys Lys Asn Phe Lys Phe Asn Met Asp Leu Ser Arg Asn
    130                 135                 140

aac ctg gtg act atc aca gca gag atg ttt gta aat ctc tca cgc ctc 480
Asn Leu Val Thr Ile Thr Ala Glu Met Phe Val Asn Leu Ser Arg Leu
145                 150                 155                 160

cag tgt ctt agc ctg agc cac aac tca att gca cag gct gtc aat ggc 528
Gln Cys Leu Ser Leu Ser His Asn Ser Ile Ala Gln Ala Val Asn Gly
                165                 170                 175

tct cag ttc ctg ccg ctg acc ggt ctg cag gtg cta gac ctg tcc cac 576
Ser Gln Phe Leu Pro Leu Thr Gly Leu Gln Val Leu Asp Leu Ser His
            180                 185                 190

aat aag ctg gac ctc tac cac gag cac tca ttc acg gag cta cca cga 624
Asn Lys Leu Asp Leu Tyr His Glu His Ser Phe Thr Glu Leu Pro Arg
        195                 200                 205

ctg gag gcc ctg gac ctc agc tac aac agc cag ccc ttt agc atg aag 672
Leu Glu Ala Leu Asp Leu Ser Tyr Asn Ser Gln Pro Phe Ser Met Lys
    210                 215                 220

ggt ata ggc cac aat ttc agt ttt gtg acc cat ctg tcc atg cta cag 720
Gly Ile Gly His Asn Phe Ser Phe Val Thr His Leu Ser Met Leu Gln
225                 230                 235                 240

agc ctt agc ctg gca cac aat gac att cat acc cgt gtg tcc tca cat 768
Ser Leu Ser Leu Ala His Asn Asp Ile His Thr Arg Val Ser Ser His
                245                 250                 255

ctc aac agc aac tca gtg agg ttt ctt gac ttc agc ggc aac ggt atg 816
Leu Asn Ser Asn Ser Val Arg Phe Leu Asp Phe Ser Gly Asn Gly Met
            260                 265                 270

ggc cgc atg tgg gat gag ggg ggc ctt tat ctc cat ttc ttc caa ggc 864
Gly Arg Met Trp Asp Glu Gly Gly Leu Tyr Leu His Phe Phe Gln Gly
        275                 280                 285

ctg agt ggc gtg ctg aag ctg gac ctg tct caa aat aac ctg cat atc 912
Leu Ser Gly Val Leu Lys Leu Asp Leu Ser Gln Asn Asn Leu His Ile
    290                 295                 300

ctc cgg ccc cag aac ctt gac aac ctc ccc aag agc ctg aag ctg ctg 960

Leu Arg Pro Gln Asn Leu Asp Asn Leu Pro Lys Ser Leu Lys Leu Leu
305                 310                 315                 320

agc ctc cga gac aac tac cta tct ttc ttt aac tgg acc agt ctg tcc    1008
Ser Leu Arg Asp Asn Tyr Leu Ser Phe Phe Asn Trp Thr Ser Leu Ser
                325                 330                 335

ttc cta ccc aac ctg gaa gtc cta gac ctg gca ggc aac cag cta aag    1056
Phe Leu Pro Asn Leu Glu Val Leu Asp Leu Ala Gly Asn Gln Leu Lys
            340                 345                 350

gcc ctg acc aat ggc acc ctg cct aat ggc acc ctc ctc cag aaa ctc    1104
Ala Leu Thr Asn Gly Thr Leu Pro Asn Gly Thr Leu Leu Gln Lys Leu
        355                 360                 365

gat gtc agt agc aac agt atc gtc tct gtg gcc ccc ggc ttc ttt tcc    1152
Asp Val Ser Ser Asn Ser Ile Val Ser Val Ala Pro Gly Phe Phe Ser
    370                 375                 380

aag gcc aag gag ctg cga gag ctc aac ctt agc gcc aac gcc ctc aag    1200
Lys Ala Lys Glu Leu Arg Glu Leu Asn Leu Ser Ala Asn Ala Leu Lys
385                 390                 395                 400

aca gtg gac cac tcc tgg ttt ggg ccc att gtg atg aac ctg aca gtt    1248
Thr Val Asp His Ser Trp Phe Gly Pro Ile Val Met Asn Leu Thr Val
                405                 410                 415

cta gac gtg aga agc aac cct ctg cac tgt gcc tgt ggg gca gcc ttc    1296
Leu Asp Val Arg Ser Asn Pro Leu His Cys Ala Cys Gly Ala Ala Phe
            420                 425                 430

gta gac tta ctg ttg gag gtg cag acc aag gtg cct ggc ctg gct aat    1344
Val Asp Leu Leu Leu Glu Val Gln Thr Lys Val Pro Gly Leu Ala Asn
        435                 440                 445

ggt gtg aag tgt ggc agc ccc ggc cag ctg cag ggc cgt agc atc ttc    1392
Gly Val Lys Cys Gly Ser Pro Gly Gln Leu Gln Gly Arg Ser Ile Phe
    450                 455                 460

gcg cag gac ctg cgg ctg tgc ctg gat gag gtc ctc tct tgg gac tgc    1440
Ala Gln Asp Leu Arg Leu Cys Leu Asp Glu Val Leu Ser Trp Asp Cys
465                 470                 475                 480

ttt ggc ctt tca ctc ttg gct gtg gcc gtg ggc atg gtg gtg cct ata    1488
Phe Gly Leu Ser Leu Leu Ala Val Ala Val Gly Met Val Val Pro Ile
                485                 490                 495

ctg cac cat ctc tgc ggc tgg gac gtc tgg tac tgt ttt cat ctg tgc    1536
Leu His His Leu Cys Gly Trp Asp Val Trp Tyr Cys Phe His Leu Cys
            500                 505                 510

ctg gca tgg cta cct ttg cta gcc cgc agc cga cgc agc gcc caa act    1584
Leu Ala Trp Leu Pro Leu Leu Ala Arg Ser Arg Arg Ser Ala Gln Thr
        515                 520                 525

ctc cct tat gat gcc ttc gtg gtg ttc gat aag gca cag agc gca gtt    1632
Leu Pro Tyr Asp Ala Phe Val Val Phe Asp Lys Ala Gln Ser Ala Val
    530                 535                 540

gcc gac tgg gtg tat aac gag ctg cgg gtg cgg ctg gag gag cgg cgc 1680
Ala Asp Trp Val Tyr Asn Glu Leu Arg Val Arg Leu Glu Glu Arg Arg
545                 550                 555                 560

ggc cgc tgg gca ctc cgc ctg tgc ctg gag gac cga gat tgg ctg cct 1728
Gly Arg Trp Ala Leu Arg Leu Cys Leu Glu Asp Arg Asp Trp Leu Pro
                565                 570                 575

ggc cag acg ctc ttc gag aac ctc tgg gct tcc atc tat ggg agc cgc 1776
Gly Gln Thr Leu Phe Glu Asn Leu Trp Ala Ser Ile Tyr Gly Ser Arg
            580                 585                 590

aag act cta ttt gtg ctg gcc cac acg gac cgc gtc agt ggc ctc ctg 1824
Lys Thr Leu Phe Val Leu Ala His Thr Asp Arg Val Ser Gly Leu Leu
        595                 600                 605

cgc acc agc ttc ctg ctg gct cag cag cgc ctg ttg gaa gac cgc aag 1872
Arg Thr Ser Phe Leu Leu Ala Gln Gln Arg Leu Leu Glu Asp Arg Lys
    610                 615                 620

gac gtg gtg gtg ttg gtg atc ctg cgt ccg gat gcc cac cgc tcc cgc 1920
Asp Val Val Val Leu Val Ile Leu Arg Pro Asp Ala His Arg Ser Arg
625                 630                 635                 640

tat gtg cga ctg cgc cag cgt ctc tgc cgc cag agt gtg ctc ttc tgg 1968
Tyr Val Arg Leu Arg Gln Arg Leu Cys Arg Gln Ser Val Leu Phe Trp
                645                 650                 655

ccc cag cag ccc aac ggg cag ggg ggc ttc tgg gcc cag ctg agt aca 2016
Pro Gln Gln Pro Asn Gly Gln Gly Gly Phe Trp Ala Gln Leu Ser Thr
            660                 665                 670

gcc ctg act agg gac aac cgc cac ttc tat aac cag aac ttc tgc cgg 2064
Ala Leu Thr Arg Asp Asn Arg His Phe Tyr Asn Gln Asn Phe Cys Arg
        675                 680                 685

gga cct aca gca gaa tagctcagag caacagctgg aaacagctgc atcttcatgt 2119
Gly Pro Thr Ala Glu
    690

ctggttcccg agttgctctg cctgccttgc tctgtcttac tacaccgcta tttggcaagt 2179

gcgcaatata tgctaccaag ccaccaggcc cacggagcaa aggttggctg taaagggtag 2239

ttttcttccc atgcatcttt caggagagtg aagatagaca ccaaacccac 2289

<210> 45
<211> 693
<212> PRT
<213> Unknown

<400> 45
Asn Leu Ser Phe Asn Tyr Arg Lys Lys Val Ser Phe Ala Arg Leu His
1               5                   10                  15

Leu Ala Ser Ser Phe Lys Asn Leu Val Ser Leu Gln Glu Leu Asn Met

            20                  25                  30

Asn Gly Ile Phe Phe Arg Leu Leu Asn Lys Tyr Thr Leu Arg Trp Leu
        35                  40                  45

Ala Asp Leu Pro Lys Leu His Thr Leu His Leu Gln Met Asn Phe Ile
    50                  55                  60

Asn Gln Ala Gln Leu Ser Ile Phe Gly Thr Phe Arg Ala Leu Arg Phe
65                  70                  75                  80

Val Asp Leu Ser Asp Asn Arg Ile Ser Gly Pro Ser Thr Leu Ser Glu
                85                  90                  95

Ala Thr Pro Glu Glu Ala Asp Asp Ala Glu Gln Glu Glu Leu Leu Ser
            100                 105                 110

Ala Asp Pro His Pro Ala Pro Leu Ser Thr Pro Ala Ser Lys Asn Phe
        115                 120                 125

Met Asp Arg Cys Lys Asn Phe Lys Phe Asn Met Asp Leu Ser Arg Asn
    130                 135                 140

Asn Leu Val Thr Ile Thr Ala Glu Met Phe Val Asn Leu Ser Arg Leu
145 150 [?] 160

Gln Cys Leu Ser Leu Ser His Asn Ser Ile Ala Gln Ala Val Asn Gly
165 170 175

Ser Gln Phe Leu Pro Leu Thr Gly Leu Gln Val Leu Asp Leu Ser His
180 185 190

Asn Lys Leu Asp Leu Tyr His Glu His Ser Phe Thr Glu Leu Pro Arg
195 200 205

Leu Glu Ala Leu Asp Leu Ser Tyr Asn Ser Gln Pro Phe Ser Met Lys
210 215 220

Gly Ile Gly His Asn Phe Ser Phe Val Thr His Leu Ser Met Leu Gln
225 230 235 240

Ser Leu Ser Leu Ala His Asn Asp Ile His Thr Arg Val Ser Ser His
245 250 255

Leu Asn Ser Asn Ser Val Arg Phe Leu Asp Phe Ser Gly Asn Gly Met
260 265 270

Gly Arg Met Trp Asp Glu Gly Gly Leu Tyr Leu His Phe Phe Gln Gly
275 280 285

Leu Ser Gly Val Leu Lys Leu Asp Leu Ser Gln Asn Asn Leu His Ile
290 295 300

Leu Arg Pro Gln Asn Leu Asp Asn Leu Pro Lys Ser Leu Lys Leu Leu
305 310 315 320

Ser Leu Arg Asp Asn Tyr Leu Ser Phe Phe Asn Trp Thr Ser Leu Ser
325 330 335

Phe Leu Pro Asn Leu Glu Val Leu Asp Leu Ala Gly Asn Gln Leu Lys
            340                 345                 350

Ala Leu Thr Asn Gly Thr Leu Pro Asn Gly Thr Leu Leu Gln Lys Leu
        355                 360                 365

Asp Val Ser Ser Asn Ser Ile Val Ser Val Ala Pro Gly Phe Phe Ser
    370                 375                 380

Lys Ala Lys Glu Leu Arg Glu Leu Asn Leu Ser Ala Asn Ala Leu Lys
385                 390                 395                 400

Thr Val Asp His Ser Trp Phe Gly Pro Ile Val Met Asn Leu Thr Val
                405                 410                 415

Leu Asp Val Arg Ser Asn Pro Leu His Cys Ala Cys Gly Ala Ala Phe
            420                 425                 430

Val Asp Leu Leu Leu Glu Val Gln Thr Lys Val Pro Gly Leu Ala Asn
        435                 440                 445

Gly Val Lys Cys Gly Ser Pro Gly Gln Leu Gln Gly Arg Ser Ile Phe
    450                 455                 460

Ala Gln Asp Leu Arg Leu Cys Leu Asp Glu Val Leu Ser Trp Asp Cys
465                 470                 475                 480

Phe Gly Leu Ser Leu Leu Ala Val Ala Val Gly Met Val Val Pro Ile
                485                 490                 495

Leu His His Leu Cys Gly Trp Asp Val Trp Tyr Cys Phe His Leu Cys
            500                 505                 510

Leu Ala Trp Leu Pro Leu Leu Ala Arg Ser Arg Arg Ser Ala Gln Thr
        515                 520                 525

Leu Pro Tyr Asp Ala Phe Val Val Phe Asp Lys Ala Gln Ser Ala Val
    530                 535                 540

Ala Asp Trp Val Tyr Asn Glu Leu Arg Val Arg Leu Glu Glu Arg Arg
545                 550                 555                 560

Gly Arg Trp Ala Leu Arg Leu Cys Leu Glu Asp Arg Asp Trp Leu Pro
                565                 570                 575

Gly Gln Thr Leu Phe Glu Asn Leu Trp Ala Ser Ile Tyr Gly Ser Arg
            580                 585                 590

Lys Thr Leu Phe Val Leu Ala His Thr Asp Arg Val Ser Gly Leu Leu
        595                 600                 605

Arg Thr Ser Phe Leu Leu Ala Gln Gln Arg Leu Leu Glu Asp Arg Lys
    610                 615                 620

Asp Val Val Val Leu Val Ile Leu Arg Pro Asp Ala His Arg Ser Arg
625                 630                 635                 640

Tyr Val Arg Leu Arg Gln Arg Leu Cys Arg Gln Ser Val Leu Phe Trp
                645                 650                 655

Pro Gln Gln Pro Asn Gly Gln Gly Gly Phe Trp Ala Gln Leu Ser Thr
            660                 665                 670

Ala Leu Thr Arg Asp Asn Arg His Phe Tyr Asn Gln Asn Phe Cys Arg
        675                 680                 685

Gly Pro Thr Ala Glu
    690